(19) **Europäisches Patentamt / European Patent Office / Office européen des brevets**

(11) **EP 4 545 564 A1**

(12) **EUROPEAN PATENT APPLICATION**
published in accordance with Art. 153(4) EPC

(43) Date of publication:
**30.04.2025 Bulletin 2025/18**

(21) Application number: **23826530.0**

(22) Date of filing: **21.06.2023**

(51) International Patent Classification (IPC):
*C07K 16/46* (2006.01)    *C07K 16/30* (2006.01)
*C07K 16/28* (2006.01)    *C07K 19/00* (2006.01)
*A61K 39/395* (2006.01)    *A61P 35/00* (2006.01)
*C12N 15/13* (2006.01)    *C12N 5/10* (2006.01)

(52) Cooperative Patent Classification (CPC):
**A61K 39/395; A61P 35/00; C07K 16/00;
C07K 16/28; C07K 16/30; C07K 16/46;
C07K 19/00; C12N 5/10**

(86) International application number:
**PCT/CN2023/101778**

(87) International publication number:
**WO 2023/246885 (28.12.2023 Gazette 2023/52)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.06.2022 CN 202210725979**

(71) Applicants:
- **Jiangsu Hengrui Pharmaceuticals Co., Ltd.
  Lianyungang, Jiangsu 222047 (CN)**
- **Shanghai Hengrui Pharmaceutical Co., Ltd.
  Shanghai 200245 (CN)**

(72) Inventors:
- **YE, Xin
  Shanghai 200245 (CN)**
- **YAO, Qingqing
  Shanghai 200245 (CN)**
- **JIN, Xinsheng
  Shanghai 200245 (CN)**
- **YING, Hua
  Shanghai 200245 (CN)**
- **TAO, Weikang
  Shanghai 200245 (CN)**

(74) Representative: **Dragotti & Associati S.R.L.
Via Nino Bixio, 7
20129 Milano (IT)**

Remarks:
The complete document including Reference Table(s) and the Sequence Listing(s) can be downloaded from the EPO website

(54) **ANTIGEN-BINDING MOLECULE SPECIFICALLY BINDING TO DLL3 AND CD3, AND PHARMACEUTICAL USE THEREOF**

(57) The present disclosure relates to an antigen-binding molecule specifically binding to DLL3 and CD3, and pharmaceutical use thereof. The antigen-binding molecule can be used for treating cancers.

**EP 4 545 564 A1**

**Description**

**TECHNICAL FIELD**

The present disclosure pertains to the technical field of biology. More specifically, the present disclosure relates to a DLL3/CD3 antigen-binding molecule and use thereof.

**BACKGROUND**

The statements herein merely provide background information related to the present disclosure and may not necessarily constitute the prior art.

Small cell lung cancer (SCLC) is a relatively malignant type of lung cancer, accounting for 10%-15% of all lung cancer cases. Tumors of small cell lung cancer grow fast and easily metastasize, with a five-year survival rate below 7%. The drugs for treating small cell lung cancer are quite limited and are primarily chemotherapy drugs, such as platinum/etoposide combined chemotherapy. Patients with small cell lung cancer initially respond well to chemotherapy but are very likely to develop resistance and relapse. In recent years, immunotherapies such as PD-L1 antibodies and PD1 antibodies have shown some efficacy in SCLC patients, with an effective rate of about 15%. There are still no specific targeted therapy drugs developed for this disease.

DLL3 is a ligand that inhibits Notch. Under normal conditions, DLL3 is located on the Golgi apparatus, but in cancer cells (such as cells of small cell lung cancer), DLL3 can be expressed on the cell surface and bind to Notch in a cis manner, impeding cell-to-cell binding and the endocytosis of Notch in target cells, thereby inhibiting the Notch signaling pathway and promoting tumor cell growth. DLL3 is primarily expressed in nerve or neuroendocrine tumors, including small cell lung cancer, large cell neuroendocrine cancer, gastrointestinal neuroendocrine tumors, small cell bladder cancer, glioblastoma multiforme, metastatic castration prostate cancer, melanoma, and the like, especially SCLC. More than 80% of SCLC cases have positive expression of DLL3. However, DLL3 is not expressed in normal lung cancer tissue and paracancerous tissue. This differential expression makes DLL3 a very potential therapeutic target for SCLC treatment.

CD3 is a homodimeric or heterodimeric antigen expressed on T cells. Functional CD3 is formed by dimer binding of two of four different chains: $\varepsilon$, $\zeta$, $\delta$, and $\gamma$. CD3 dimer arrangements include $\gamma/\varepsilon$, $\delta/\varepsilon$, and $\zeta/\zeta$. CD3 binds to the T-cell receptor complex (TCR) and is essential for T-cell activation. Therefore, it has been proposed to use anti-CD3 antibodies that activate T cells for cancer treatment. However, administration of anti-CD3 antibodies may trigger T cell activation and release of related cytokines. Excessive cytokine release leads to severe cytokine release syndrome (CRS), which is a significant challenge in the clinical use of anti-CD3 antibodies.

**SUMMARY**

The present disclosure provides an antigen-binding molecule binding specifically to DLL3 and CD3.

In one aspect, the present disclosure provides an antigen-binding molecule comprising at least one antigen-binding moiety binding specifically to DLL3 and at least one antigen-binding moiety binding specifically to CD3; the antigen-binding moiety binding specifically to DLL3 comprises a heavy chain variable region DLL3-VH and a light chain variable region DLL3-VL, and the antigen-binding moiety binding specifically to CD3 comprises a heavy chain variable region CD3-VH and a light chain variable region CD3-VL.

In some embodiments, provided is the antigen-binding molecule according to the foregoing, wherein:

(i) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 12, 56, or 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 13, 63, 64, 65, 66, or 67, respectively; or
(ii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 14, 79, 80, 81, or 82, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 15, respectively; or
(iii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 16, 91, 92, 93, or 94, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 17, respectively; or
(iv) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 18, 107, 108, 109, 110, 112, 113, 114, or 115, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 19, respectively.

In some embodiments, provided is the antigen-binding molecule according to the foregoing, wherein:

(i) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 12, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 13, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 54, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 64, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 54, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 63, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 56, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 65, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 56, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 66, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 56, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 67, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 65, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 66, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 67, respectively; or

(ii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 14, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 15, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 81, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 77, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 79, 80, or 82, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 77, respectively; or

(iii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 16, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 17, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 91, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 87, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 92, 93, or 94, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 87, respectively; or

(iv) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 18, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 19, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 109, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 100, respectively; or
a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 107, 108, 110, 112, 113, 114, or 115, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 100, respectively.

In some embodiments, the DLL3-HCDR1, DLL3-HCDR2, DLL3-HCDR3, DLL3-LCDR1, DLL3-LCDR2, and DLL3-LCDR3 are defined according to the Kabat, IMGT, Chothia, AbM, or Contact numbering scheme.

In some embodiments, provided is the antigen-binding molecule according to any one of the foregoing, wherein:

i) the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, 71, 72, or 73, and a DLL3-LCDR3 comprising the amino acid sequence of SEQ ID NO: 25 or 74; or
ii) the DLL3-VH comprises: a DLL3-HCDR1 comprising the amino acid sequence of SEQ ID NO: 26 or 84, a DLL3-HCDR2 comprising the amino acid sequence of SEQ ID NO: 27 or 85, and a DLL3-HCDR3 comprising the amino acid sequence of SEQ ID NO: 28 or 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or
iii) the DLL3-VH comprises: a DLL3-HCDR1 comprising the amino acid sequence of SEQ ID NO: 20, a DLL3-HCDR2 comprising the amino acid sequence of SEQ ID NO: 32, 96, 97, 98, or 99, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or
iv) the DLL3-VH comprises: a DLL3-HCDR1 comprising the amino acid sequence of SEQ ID NO: 20, a DLL3-HCDR2 comprising the amino acid sequence of SEQ ID NO: 37, 117, 118, 119, or 120, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 69 or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25;

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25.

In some embodiments, provided is the antigen-binding molecule according to any one of the foregoing, wherein:

the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 84, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 84, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

the DLL3-VH comprises: a DLL3-HCDR1 comprising the amino acid sequence of SEQ ID NO: 20, a DLL3-HCDR2 comprising the amino acid sequence of SEQ ID NO: 97, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

the DLL3-VH comprises: a DLL3-HCDR1 comprising the amino acid sequence of SEQ ID NO: 20, a DLL3-HCDR2 comprising the amino acid sequence of SEQ ID NO: 98, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

the DLL3-VH comprises: a DLL3-HCDR1 comprising the amino acid sequence of SEQ ID NO: 20, a DLL3-HCDR2 comprising the amino acid sequence of SEQ ID NO: 99, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40; or

the DLL3-VH comprises: a DLL3-HCDR1 comprising the amino acid sequence of SEQ ID NO: 20, a DLL3-HCDR2 comprising the amino acid sequence of SEQ ID NO: 117, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40; or

the DLL3-VH comprises: a DLL3-HCDR1 comprising the amino acid sequence of SEQ ID NO: 20, a DLL3-HCDR2 comprising the amino acid sequence of SEQ ID NO: 118, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40; or

the DLL3-VH comprises: a DLL3-HCDR1 comprising the amino acid sequence of SEQ ID NO: 20, a DLL3-HCDR2 comprising the amino acid sequence of SEQ ID NO: 120, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40.

In some embodiments, provided is the antigen-binding molecule according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25.

In some embodiments, provided is the antigen-binding molecule according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25.

In some embodiments, provided is the antigen-binding molecule according to any one of the foregoing, wherein: the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31.

In some embodiments, provided is the antigen-binding molecule according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36.

In some embodiments, provided is the antigen-binding molecule according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40.

In some embodiments, in the antigen-binding molecule according to any one of the foregoing, the DLL3-HCDR1, DLL3-HCDR2, DLL3-HCDR3, DLL3-LCDR1, DLL3-LCDR2, and DLL3-LCDR3 are defined according to the Kabat numbering scheme.

In some embodiments, the antigen-binding molecule according to any one of the foregoing binds to human DLL3 at 25 °C with a KD of less than $4 \times 10^{-9}$ M, $3 \times 10^{-9}$ M, $2 \times 10^{-9}$ M, $1 \times 10^{-9}$ M, $9 \times 10^{-10}$ M, $8 \times 10^{-10}$ M, $7 \times 10^{-10}$ M, $6 \times 10^{-10}$ M, $5 \times 10^{-10}$ M, $4 \times 10^{-10}$ M, $3 \times 10^{-10}$ M, $2 \times 10^{-10}$ M, $1 \times 10^{-10}$ M, or $9 \times 10^{-11}$ M, as measured by surface plasmon resonance.

In some embodiments, provided is the antigen-binding molecule according to any one of the foregoing, wherein the DLL3-VH and DLL3-VL are humanized and comprise FR regions of a human antibody.

In some embodiments, provided is the antigen-binding molecule according to any one of the foregoing, wherein:

(i) the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, 71, 72, or 73, and a DLL3-LCDR3 comprising the amino acid sequence of SEQ ID NO: 25 or 74, and FR regions of the DLL3-VL comprise one or more amino acid substitutions selected from the group consisting of 1N, 36L, 42K, 43S, 44F, 46G, 69A, 71Y, 79Q, and 85D; or

(ii) the DLL3-VH comprises: a DLL3-HCDR1 comprising the amino acid sequence of SEQ ID NO: 26 or 84, a DLL3-HCDR2 comprising the amino acid sequence of SEQ ID NO: 27 or 85, and a DLL3-HCDR3 comprising the amino acid sequence of SEQ ID NO: 28 or 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or

(iii) the DLL3-VH comprises: a DLL3-HCDR1 comprising the amino acid sequence of SEQ ID NO: 20, a DLL3-HCDR2 comprising the amino acid sequence of SEQ ID NO: 32, 96, 97, 98, or 99, and a DLL3-HCDR3 comprising the amino acid sequence of SEQ ID NO: 33, and FR regions of the DLL3-VH comprise one or more amino acid substitutions selected from the group consisting of 1E, 24T, 44S, 71V, and 73K; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36, and FR regions of the DLL3-VL comprise one or more amino acid substitutions selected from the group consisting of 1N, 9K, 43S, 68A, and 85D; or

(iv) the DLL3-VH comprises: a DLL3-HCDR1 comprising the amino acid sequence of SEQ ID NO: 20, a DLL3-HCDR2 comprising the amino acid sequence of SEQ ID NO: 37, 117, 118, 119, or 120, and a DLL3-HCDR3 comprising the amino acid sequence of SEQ ID NO: 38, and FR regions of the DLL3-VH comprise one or more amino acid substitutions selected from the group consisting of 1E, 3R, 5Q, 7F, 9P, 10V, 12V, 40S, 41H, 44S, 48I, 67A, 69L, 71V, 73K, 75S, and 83T; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40, and FR regions of the DLL3-VL comprise one or more amino acid substitutions selected from the group consisting of 9K, 43S, 60D, 68A, 87H, and 100S.

In some embodiments, the antigen-binding molecule according to any one of the foregoing binds to human DLL3 at 25 °C with a KD of less than $4 \times 10^{-9}$ M, $3 \times 10^{-9}$ M, $2 \times 10^{-9}$ M, $1 \times 10^{-9}$ M, $9 \times 10^{-10}$ M, $8 \times 10^{-10}$ M, $7 \times 10^{-10}$ M, $6 \times 10^{-10}$ M, $5 \times 10^{-10}$ M, $4 \times 10^{-10}$ M, $3 \times 10^{-10}$ M, $2 \times 10^{-10}$ M, $1 \times 10^{-10}$ M, or $9 \times 10^{-11}$ M, as measured by surface plasmon resonance.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D.

In some embodiments, provided is the antigen-binding molecule according to any one of the foregoing, wherein:

the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or

the DLL3-VH comprises: a DLL3-HCDR1 comprising the amino acid sequence of SEQ ID NO: 84, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or

the DLL3-VH comprises: a DLL3-HCDR1 comprising the amino acid sequence of SEQ ID NO: 84, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y.

In some embodiments, provided is the antigen-binding molecule according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33, and FR regions of the DLL3-VH comprise one or more amino acid substitutions selected from the group consisting of 1E, 24T, 44S, 71V, and 73K; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36, and FR regions of the DLL3-VL comprise one or more amino acid substitutions selected from the group consisting of 1N, 9K, 43S, 68A, and 85D.

In some embodiments, provided is the antigen-binding molecule according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38, and FR regions of the DLL3-VH comprise one or more amino acid substitutions selected from the group consisting of 1E, 3R, 5Q, 7F, 9P, 10V, 12V, 40S, 41H, 44S, 48I, 67A, 69L, 71V, 73K, 75S, and 83T; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40, and FR regions of the DLL3-VL comprise one or more amino acid substitutions selected from the group consisting of 9K, 43S, 60D, 68A, 87H, and 100S.

In some embodiments, provided is the antigen-binding molecule according to any one of the foregoing, wherein: the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein: the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein: the DLL3-VH comprises the amino acid sequence of SEQ ID NO: 50 or a variant thereof. In some embodiments, the variant comprises one or more amino acid substitutions selected from the group consisting of Q1E, Q43K, M48I, V67A, I69L, R71V, T73K, and S76N in FR regions of SEQ ID NO: 50.

In some embodiments, provided is the antigen-binding molecule according to any one of the foregoing, wherein: the DLL3-VH comprises the amino acid sequence of SEQ ID NO: 51 or a variant thereof. In some embodiments, the variant comprises one or more amino acid substitutions selected from the group consisting of Q1E, 5Q, R38K, V68A, F69L, L71V, T73K, V75S, and S76N in FR regions of SEQ ID NO: 51.

In some embodiments, provided is the antigen-binding molecule according to any one of the foregoing, wherein: the DLL3-VH comprises the amino acid sequence of SEQ ID NO: 52 or a variant thereof. In some embodiments, the variant comprises one or more amino acid substitutions selected from the group consisting of F27Y, S30T, I69L, R71V, D73K, and T93A in FR regions of SEQ ID NO: 52.

In some embodiments, provided is the antigen-binding molecule according to any one of the foregoing, wherein: the DLL3-VL comprises the amino acid sequence of SEQ ID NO: 62 or a variant thereof. In some embodiments, the variant comprises one or more amino acid substitutions selected from the group consisting of F36L, A43S, P44F, S46G, T69A, F71Y, and T85D in FR regions of SEQ ID NO: 62.

In some embodiments, provided is the antigen-binding molecule according to any one of the foregoing, wherein: the DLL3-VL comprises the amino acid sequence of SEQ ID NO: 68 or a variant thereof. In some embodiments, the variant comprises one or more amino acid substitutions selected from the group consisting of Y36L, Q42K, L46G, T69A, F71Y, E79Q, and V85D in FR regions of SEQ ID NO: 68.

In some embodiments, provided is the antigen-binding molecule according to any one of the foregoing, wherein: the DLL3-VH comprises the amino acid sequence of SEQ ID NO: 78 or a variant thereof. In some embodiments, the variant comprises one or more amino acid substitutions in FR regions of SEQ ID NO: 78.

In some embodiments, provided is the antigen-binding molecule according to any one of the foregoing, wherein: the DLL3-VL comprises the amino acid sequence of SEQ ID NO: 75 or a variant thereof. In some embodiments, the variant comprises one or more amino acid substitutions selected from the group consisting of K42G, P44V, and F71Y in FR regions of SEQ ID NO: 75.

In some embodiments, provided is the antigen-binding molecule according to any one of the foregoing, wherein: the DLL3-VH comprises the amino acid sequence of SEQ ID NO: 95 or a variant thereof. In some embodiments, the variant comprises one or more amino acid substitutions selected from the group consisting of Q1E, A24T, R44S, R71V, and T73K in FR regions of SEQ ID NO: 95.

In some embodiments, provided is the antigen-binding molecule according to any one of the foregoing, wherein: the DLL3-VL comprises the amino acid sequence of SEQ ID NO: 88 or a variant thereof. In some embodiments, the variant comprises one or more amino acid substitutions selected from the group consisting of D1N, D9K, P43S, G68A, and V85D in FR regions of SEQ ID NO: 88.

In some embodiments, provided is the antigen-binding molecule according to any one of the foregoing, wherein: the DLL3-VH comprises the amino acid sequence of SEQ ID NO: 116 or a variant thereof. In some embodiments, the variant comprises one or more amino acid substitutions selected from the group consisting of Q1E, Q3R, V5Q, S7F, A9P, E10V, K12V, A40S, P41H, R44S, M48I, V67A, I69L, R71V, T73K, A75S, and R83T in FR regions of SEQ ID NO: 116.

In some embodiments, provided is the antigen-binding molecule according to any one of the foregoing, wherein: the DLL3-VL comprises the amino acid sequence of SEQ ID NO: 100 or a variant thereof. In some embodiments, the variant comprises one or more amino acid substitutions selected from the group consisting of D9K, P43S, G68A, and Y87H in FR regions of SEQ ID NO: 100.

In some embodiments, provided is the antigen-binding molecule according to any one of the foregoing, wherein: the DLL3-VL comprises the amino acid sequence of SEQ ID NO: 103 or a variant thereof. In some embodiments, the variant comprises one or more amino acid substitutions selected from the group consisting of A43S, S60D, and Q100S in FR regions of SEQ ID NO: 103.

In some embodiments, provided is the antigen-binding molecule according to any one of the foregoing, wherein: the DLL3-VH comprises the amino acid sequence of SEQ ID NO: 56 or a variant thereof. In some embodiments, the variant comprises one or more amino acid substitutions in FR regions of SEQ ID NO: 56.

In some embodiments, provided is the antigen-binding molecule according to any one of the foregoing, wherein: the DLL3-VH comprises the amino acid sequence of SEQ ID NO: 57 or a variant thereof. In some embodiments, the variant comprises one or more amino acid substitutions in FR regions of SEQ ID NO: 57.

In some embodiments, provided is the antigen-binding molecule according to any one of the foregoing, wherein: the DLL3-VL comprises the amino acid sequence of SEQ ID NO: 63 or a variant thereof. In some embodiments, the variant comprises one or more amino acid substitutions in FR regions of SEQ ID NO: 63; in some embodiments, the amino acid substitutions are one or more amino acid substitutions selected from the group consisting of A43S and P44F.

In some embodiments, provided is the antigen-binding molecule according to any one of the foregoing, wherein: the DLL3-VL comprises the amino acid sequence of SEQ ID NO: 64 or a variant thereof. In some embodiments, the variant comprises one or more amino acid substitutions in FR regions of SEQ ID NO: 64; in some embodiments, the amino acid substitutions are one or more amino acid substitutions selected from the group consisting of A43S and P44F.

In some embodiments, provided is the antigen-binding molecule according to any one of the foregoing, wherein: the DLL3-VL comprises the amino acid sequence of SEQ ID NO: 65 or a variant thereof. In some embodiments, the variant comprises one or more amino acid substitutions in FR regions of SEQ ID NO: 65; in some embodiments, the amino acid substitutions are one or more amino acid substitutions selected from the group consisting of A43S and P44F.

In some embodiments, provided is the antigen-binding molecule according to any one of the foregoing, wherein: the DLL3-VL comprises the amino acid sequence of SEQ ID NO: 66 or a variant thereof. In some embodiments, the variant comprises one or more amino acid substitutions in FR regions of SEQ ID NO: 66; in some embodiments, the amino acid substitutions are one or more amino acid substitutions selected from the group consisting of A43S and P44F.

In some embodiments, provided is the antigen-binding molecule according to any one of the foregoing, wherein: the DLL3-VL comprises the amino acid sequence of SEQ ID NO: 67 or a variant thereof. In some embodiments, the variant comprises one or more amino acid substitutions in FR regions of SEQ ID NO: 67.

In some embodiments, provided is the antigen-binding molecule according to any one of the foregoing, wherein:

the DLL3-VH comprises the amino acid sequence of SEQ ID NO: 79, 80, 81, or 82, or a variant of SEQ ID NO: 79, 80, 81, or 82. In some embodiments, the variant comprises one or more amino acid substitutions in FR regions of SEQ ID NO: 79, 80, 81, or 82; and

the DLL3-VL comprises the amino acid sequence of SEQ ID NO: 75 or a variant thereof. In some embodiments, the variant comprises one or more amino acid substitutions selected from the group consisting of K42G, P44V, and F71Y in FR regions of SEQ ID NO: 75.

In some embodiments, provided is the antigen-binding molecule according to any one of the foregoing, wherein:

the DLL3-VH comprises the amino acid sequence of SEQ ID NO: 91, 92, 93, or 94, or a variant of SEQ ID NO: 91, 92, 93, or 94. In some embodiments, the variant comprises one or more amino acid substitutions in FR regions of SEQ ID NO: 91, 92, 93, or 94; and

the DLL3-VL comprises the amino acid sequence of SEQ ID NO: 88 or a variant thereof. In some embodiments, the variant comprises one or more amino acid substitutions selected from the group consisting of D1N, D9K, P43S, G68A, and V85D in FR regions of SEQ ID NO: 88.

In some embodiments, provided is the antigen-binding molecule according to any one of the foregoing, wherein: the DLL3-VH comprises the amino acid sequence of SEQ ID NO: 107, 108, 109, or 110, or a variant of SEQ ID NO: 107, 108, 109, or 110. In some embodiments, the variant comprises one or more amino acid substitutions in FR regions of SEQ ID NO: 107, 108, 109, or 110; in some embodiments, the amino acid substitutions are one or more amino acid substitutions selected from the group consisting of V5Q, S7F, A9P, E10V, K12V, A40S, P41H, M48I, V67A, A75S, and R83T.

In some embodiments, provided is the antigen-binding molecule according to any one of the foregoing, wherein: the DLL3-VH comprises the amino acid sequence of SEQ ID NO: 112, or a variant of SEQ ID NO: 112. In some embodiments, the variant comprises one or more amino acid substitutions in FR regions of SEQ ID NO: 112; in some embodiments, the amino acid substitutions are one or more amino acid substitutions selected from the group consisting of V5Q, A9P, K12V, A40S, P41H, 4M8I, V67A, and R83T.

In some embodiments, provided is the antigen-binding molecule according to any one of the foregoing, wherein: the DLL3-VH comprises the amino acid sequence of SEQ ID NO: 113, or a variant of SEQ ID NO: 113. In some embodiments, the variant comprises one or more amino acid substitutions in FR regions of SEQ ID NO: 113; in some embodiments, the amino acid substitutions are one or more amino acid substitutions selected from the group consisting of V5Q, S7F, A9P, E10V, K12V, A40S, P41H, A75S, and R83T.

In some embodiments, provided is the antigen-binding molecule according to any one of the foregoing, wherein: the DLL3-VH comprises the amino acid sequence of SEQ ID NO: 114, or a variant of SEQ ID NO: 114. In some embodiments, the variant comprises one or more amino acid substitutions in FR regions of SEQ ID NO: 114; in some embodiments, the amino acid substitutions are one or more amino acid substitutions selected from the group consisting of V5Q, S7F, A9P, E10V, K12V, M48I, V67A, A75S, and R83T.

In some embodiments, provided is the antigen-binding molecule according to any one of the foregoing, wherein: the DLL3-VH comprises the amino acid sequence of SEQ ID NO: 115, or a variant of SEQ ID NO: 115. In some embodiments, the variant comprises one or more amino acid substitutions in FR regions of SEQ ID NO: 115; in some embodiments, the amino acid substitutions are one or more amino acid substitutions selected from the group consisting of V5Q, A9P, K12V, A40S, P41H, M48I, and V67A.

In some embodiments, provided is the antigen-binding molecule according to any one of the foregoing, wherein: the DLL3-VL comprises the amino acid sequence of SEQ ID NO: 100 or a variant thereof. In some embodiments, the variant comprises one or more amino acid substitutions selected from the group consisting of D9K, P43S, G68A, and Y87H in FR regions of SEQ ID NO: 100.

In some embodiments, provided is the antigen-binding molecule according to any one of the foregoing, wherein: the DLL3-VL comprises the amino acid sequence of SEQ ID NO: 103 or a variant thereof. In some embodiments, the variant comprises one or more amino acid substitutions selected from the group consisting of A43S, S60D, and Q100S in FR regions of SEQ ID NO: 103.

In some embodiments, provided is the antigen-binding molecule according to any one of the foregoing, wherein:

i) the DLL3-VH comprises the amino acid sequence of SEQ ID NO: 12, 43, 44, 45, 46, 47, 48, 49, 50, 51, 52, 53, 54, 55, 56, or 57, and the DLL3-VL comprises the amino acid sequence of SEQ ID NO: 13, 58, 59, 60, 61, 62, 63, 64, 65, 66, 67, or 68; or
ii) the DLL3-VH comprises the amino acid sequence of SEQ ID NO: 14, 78, 79, 80, 81, or 82, and the DLL3-VL comprises the amino acid sequence of SEQ ID NO: 15, 75, 76, or 77; or
iii) the DLL3-VH comprises the amino acid sequence of SEQ ID NO: 16, 89, 90, 91, 92, 93, 94, or 95, and the DLL3-VL comprises the amino acid sequence of SEQ ID NO: 17, 86, 87, or 88; or

iv) the DLL3-VH comprises the amino acid sequence of SEQ ID NO: 18, 105, 106, 107, 108, 109, 110, 111, 112, 113, 114, 115, or 116, and the DLL3-VL comprises the amino acid sequence of SEQ ID NO: 19, 100, 101, 102, 103, or 104.

In some embodiments, provided is the antigen-binding molecule according to any one of the foregoing, wherein:

the DLL3-VH comprises the amino acid sequence of SEQ ID NO: 43, 44, or 45, and the DLL3-VL comprises the amino acid sequence of SEQ ID NO: 58, 59, or 60; or
the DLL3-VH comprises the amino acid sequence of SEQ ID NO: 47, 48, 49, 53, 54, or 55, and the DLL3-VL comprises the amino acid sequence of SEQ ID NO: 59; or
the DLL3-VH comprises the amino acid sequence of SEQ ID NO: 43, 44, or 45, and the DLL3-VL comprises the amino acid sequence of SEQ ID NO: 60; or
the DLL3-VH comprises the amino acid sequence of SEQ ID NO: 46, 48, 53, 54, or 55, and the DLL3-VL comprises the amino acid sequence of SEQ ID NO: 61; or
the DLL3-VH comprises the amino acid sequence of SEQ ID NO: 54, and the DLL3-VL comprises the amino acid sequence of SEQ ID NO: 63 or 64; or
the DLL3-VH comprises the amino acid sequence of SEQ ID NO: 56 or 57, and the DLL3-VL comprises the amino acid sequence of SEQ ID NO: 65, 66, or 67.

In some embodiments, provided is the antigen-binding molecule according to any one of the foregoing, wherein:

the DLL3-VH comprises the amino acid sequence of SEQ ID NO: 78 or 79, and the DLL3-VL comprises the amino acid sequence of SEQ ID NO: 75, 76, or 77; or
the DLL3-VH comprises the amino acid sequence of SEQ ID NO: 78, 79, 80, 81, or 82, and the DLL3-VL comprises the amino acid sequence of SEQ ID NO: 77.

In some embodiments, provided is the antigen-binding molecule according to any one of the foregoing, wherein: the DLL3-VH comprises the amino acid sequence of SEQ ID NO: 89, 90, 91, 92, 93, or 94, and the DLL3-VL comprises the amino acid sequence of SEQ ID NO: 86 or 87; or the DLL3-VH comprises the amino acid sequence of SEQ ID NO: 95, and the DLL3-VL comprises the amino acid sequence of SEQ ID NO: 87.

In some embodiments, provided is the antigen-binding molecule according to any one of the foregoing, wherein:

the DLL3-VH comprises the amino acid sequence of SEQ ID NO: 105, 106, 107, 108, 109, 110, or 111, and the DLL3-VL comprises the amino acid sequence of SEQ ID NO: 100, 101, or 102; or
the DLL3-VH comprises the amino acid sequence of SEQ ID NO: 112, 113, 114, or 115, and the DLL3-VL comprises the amino acid sequence of SEQ ID NO: 100, 103, or 104; or
the DLL3-VH comprises the amino acid sequence of SEQ ID NO: 109, and the DLL3-VL comprises the amino acid sequence of SEQ ID NO: 103.

In some embodiments, provided is the antigen-binding molecule according to any one of the foregoing, wherein:

i) the DLL3-VH comprises the amino acid sequence of SEQ ID NO: 54, and the DLL3-VL comprises the amino acid sequence of SEQ ID NO: 63; or

the DLL3-VH comprises the amino acid sequence of SEQ ID NO: 54, and the DLL3-VL comprises the amino acid sequence of SEQ ID NO: 61; or
the DLL3-VH comprises the amino acid sequence of SEQ ID NO: 54, and the DLL3-VL comprises the amino acid sequence of SEQ ID NO: 64; or
the DLL3-VH comprises the amino acid sequence of SEQ ID NO: 56, and the DLL3-VL comprises the amino acid sequence of SEQ ID NO: 65; or
the DLL3-VH comprises the amino acid sequence of SEQ ID NO: 56, and the DLL3-VL comprises the amino acid sequence of SEQ ID NO: 66; or
the DLL3-VH comprises the amino acid sequence of SEQ ID NO: 56, and the DLL3-VL comprises the amino acid sequence of SEQ ID NO: 67; or
the DLL3-VH comprises the amino acid sequence of SEQ ID NO: 57, and the DLL3-VL comprises the amino acid sequence of SEQ ID NO: 65; or
the DLL3-VH comprises the amino acid sequence of SEQ ID NO: 57, and the DLL3-VL comprises the amino acid sequence of SEQ ID NO: 66; or

the DLL3-VH comprises the amino acid sequence of SEQ ID NO: 57, and the DLL3-VL comprises the amino acid sequence of SEQ ID NO: 67; or

ii) the DLL3-VH comprises the amino acid sequence of SEQ ID NO: 81, and the DLL3-VL comprises the amino acid sequence of SEQ ID NO: 77; or
iii) the DLL3-VH comprises the amino acid sequence of SEQ ID NO: 91, and the DLL3-VL comprises the amino acid sequence of SEQ ID NO: 87; or
iv) the DLL3-VH comprises the amino acid sequence of SEQ ID NO: 109, and the DLL3-VL comprises the amino acid sequence of SEQ ID NO: 100.

In some embodiments, provided is the antigen-binding molecule according to any one of the foregoing, wherein:

i) the DLL3-VH comprises the amino acid sequence of SEQ ID NO: 12, and the DLL3-VL comprises the amino acid sequence of SEQ ID NO: 13; or
ii) the DLL3-VH comprises the amino acid sequence of SEQ ID NO: 14, and the DLL3-VL comprises the amino acid sequence of SEQ ID NO: 15; or
iii) the DLL3-VH comprises the amino acid sequence of SEQ ID NO: 16, and the DLL3-VL comprises the amino acid sequence of SEQ ID NO: 17; or
iv) the DLL3-VH comprises the amino acid sequence of SEQ ID NO: 18, and the DLL3-VL comprises the amino acid sequence of SEQ ID NO: 19.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 63.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 61.

In some embodiments, provided is the antigen-binding molecule according to any one of the foregoing, wherein: the DLL3-VH comprises the amino acid sequence of SEQ ID NO: 81, and the DLL3-VL comprises the amino acid sequence of SEQ ID NO: 77.

In some embodiments, provided is the antigen-binding molecule according to any one of the foregoing, wherein: the DLL3-VH comprises the amino acid sequence of SEQ ID NO: 91, and the DLL3-VL comprises the amino acid sequence of SEQ ID NO: 87.

In some embodiments, provided is the antigen-binding molecule according to any one of the foregoing, wherein: the DLL3-VH comprises the amino acid sequence of SEQ ID NO: 109, and the DLL3-VL comprises the amino acid sequence of SEQ ID NO: 100.

In some embodiments, provided is the antigen-binding molecule according to any one of the foregoing, wherein: the CD3-VH comprises the amino acid sequence of SEQ ID NO: 136, and the CD3-VL comprises the amino acid sequence of SEQ ID NO: 137.

In some embodiments, provided is the antigen-binding molecule according to any one of the foregoing, wherein: the CD3-VH comprises the amino acid sequence of SEQ ID NO: 138, and the CD3-VL comprises the amino acid sequence of SEQ ID NO: 137.

In some embodiments, provided is the antigen-binding molecule according to any one of the foregoing, wherein the antigen-binding moiety binding specifically to DLL3 or the antigen-binding moiety binding specifically to CD3 comprises a Titin chain and an Obscurin chain capable of forming a dimer.

In some embodiments, provided is the antigen-binding molecule according to any one of the foregoing, wherein the antigen-binding moiety binding specifically to DLL3 comprises a Titin chain and an Obscurin chain capable of forming a dimer.

In some embodiments, provided is the antigen-binding molecule according to any one of the foregoing, wherein the antigen-binding moiety binding specifically to CD3 comprises a Titin chain and an Obscurin chain capable of forming a dimer.

In some embodiments, provided is the antigen-binding molecule according to any one of the foregoing, wherein the Titin chain comprises an amino acid sequence selected from the group consisting of SEQ ID NO: 192 to SEQ ID NO: 210, and the Obscurin chain comprises an amino acid sequence selected from the group consisting of SEQ ID NO: 211 to SEQ ID NO: 251.

In some embodiments, provided is the antigen-binding molecule according to any one of the foregoing, wherein the Titin chain comprises the amino acid sequence of SEQ ID NO: 208.

In some embodiments, provided is the antigen-binding molecule according to any one of the foregoing, wherein the Obscurin chain comprises the amino acid sequence of SEQ ID NO: 246.

In some embodiments, provided is the antigen-binding molecule according to any one of the foregoing, wherein the Titin chain comprises the amino acid sequence of SEQ ID NO: 208, and the Obscurin chain comprises the amino acid sequence of SEQ ID NO: 246.

In some embodiments, provided is the antigen-binding molecule according to any one of the foregoing, wherein the antigen-binding molecule comprises an Fc region.

In some embodiments, provided is the antigen-binding molecule according to any one of the foregoing, wherein the antigen-binding molecule comprises a human IgG Fc region.

In some embodiments, provided is the antigen-binding molecule according to any one of the foregoing, wherein the antigen-binding molecule comprises a human IgG1 Fc region.

In some embodiments, provided is the antigen-binding molecule according to any one of the foregoing, wherein the antigen-binding molecule comprises an Fc region comprising one or more amino acid substitutions capable of reducing binding of the Fc region to an Fcγ receptor.

In some embodiments, provided is the antigen-binding molecule according to any one of the foregoing, wherein the antigen-binding molecule comprises an Fc region, and the Fc region is a human IgG1 Fc region and has amino acids A at positions 234 and 235, with numbering in accordance with the EU index.

In some embodiments, provided is the antigen-binding molecule according to any one of the foregoing, wherein the antigen-binding molecule comprises an Fc region comprising a first subunit Fc1 and a second subunit Fc2 capable of associating with each other, and the Fc1 and Fc2 each independently have one or more amino acid substitutions that reduce homodimerization of the Fc region.

In some embodiments, provided is the antigen-binding molecule according to any one of the foregoing, wherein the antigen-binding molecule comprises an Fc region comprising a first subunit Fc1 and a second subunit Fc2 capable of associating with each other; the Fc1 has a knob structure according to the knob-into-hole technique, and the Fc2 has a hole structure according to the knob-into-hole technique.

In some embodiments, provided is the antigen-binding molecule according to any one of the foregoing, wherein the antigen-binding molecule comprises an Fc region comprising a first subunit Fc1 and a second subunit Fc2 capable of associating with each other; the Fc1 has a knob structure according to the knob-into-hole technique, and the Fc2 has a hole structure according to the knob-into-hole technique; the Fc1 has an amino acid W at position 366, and the Fc2 has an amino acid S at position 366, an amino acid A at position 368, and an amino acid V at position 407, with numbering in accordance with the EU index.

In some embodiments, provided is the antigen-binding molecule according to any one of the foregoing, wherein the antigen-binding molecule comprises an Fc region comprising a first subunit Fc1 and a second subunit Fc2 capable of associating with each other; the Fc1 has a knob structure according to the knob-into-hole technique, and the Fc2 has a hole structure according to the knob-into-hole technique, wherein the Fc1 has an amino acid C at position 354 and an amino acid W at position 366, and the Fc2 has an amino acid C at position 349, an amino acid S at position 366, an amino acid A at position 368, and an amino acid V at position 407, with numbering in accordance with the EU index.

In some embodiments, provided is the antigen-binding molecule according to any one of the foregoing, wherein the antigen-binding molecule comprises an Fc region comprising a first subunit Fc1 and a second subunit Fc2 capable of associating with each other, wherein the Fc1 comprises the amino acid sequence of SEQ ID NO: 143, and the Fc2 comprises the amino acid sequence of SEQ ID NO: 144.

In some embodiments, provided is the antigen-binding molecule according to any one of the foregoing, wherein the antigen-binding molecule comprises an Fc region comprising a first subunit Fc1 and a second subunit Fc2 capable of associating with each other, wherein the Fc1 has an amino acid C at position 349, an amino acid W at position 366, and an amino acid A at position 237, and the Fc2 has an amino acid C at position 354, an amino acid S at position 366, an amino acid A at position 368, an amino acid V at position 407, and an amino acid A at position 237, with numbering in accordance with the EU index.

In some embodiments, provided is the antigen-binding molecule according to any one of the foregoing, wherein the antigen-binding molecule comprises an Fc region comprising a first subunit Fc1 and a second subunit Fc2 capable of associating with each other, wherein the Fc1 comprises the amino acid sequence of SEQ ID NO: 145, and the Fc2 comprises the amino acid sequence of SEQ ID NO: 146.

It should be understood that the ordinal numbers "first", "second", "formula a", "formula b", "Fc1", "linker 3", etc. in the present disclosure are used only to distinguish between different technical features, elements, components, or steps and are not intended to limit the level, order, or number.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3, wherein:
the antigen-binding moiety binding specifically to DLL3 is a Fab, and the antigen-binding moiety binding specifically to CD3 is a replaced Fab comprising a Titin chain and an Obscurin chain capable of forming a dimer.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3, wherein:
the antigen-binding moiety binding specifically to CD3 is a Fab, and the antigen-binding moiety binding specifically to DLL3 is a replaced Fab comprising a Titin chain and an Obscurin chain capable of forming a dimer.

In some embodiments, provided is the antigen-binding molecule according to any one of the foregoing, wherein the antigen-binding molecule comprises one first chain having a structure represented by formula (a), one second chain having a structure represented by formula (b), one third chain having a structure represented by formula (c), and one fourth chain having a structure represented by formula (d):

(a) [DLL3-VH]-[linker 1]-[Titin chain]/[Obscurin chain]-[Fc2]/[Fc1],
(b) [DLL3-VL]-[linker 2]-[Obscurin chain]/[Titin chain],
(c) [CD3-VH]-[CH1]-[Fc1]/[Fc2], and
(d) [CD3-VL]-[CL],

wherein the linker 1 and the linker 2 are identical or different peptide linkers; the structures represented by formulas (a), (b), (c), and (d) are arranged from N-terminus to C-terminus. "/" means "or"; that is, the Fc1 and Fc2 can be interchanged, and the Titin chain and Obscurin chain can be interchanged.

In some embodiments, provided is the antigen-binding molecule according to any one of the foregoing, wherein the antigen-binding molecule comprises one first chain having a structure represented by formula (a), one second chain having a structure represented by formula (b), one third chain having a structure represented by formula (c), and one fourth chain having a structure represented by formula (d):

(a) [DLL3-VH]-[linker 1]-[Titin chain]-[Fc2],
(b) [DLL3-VL]-[linker 2]-[Obscurin chain],
(c) [CD3-VH]-[CH1]-[Fc1], and
(d) [CD3-VL]-[CL],

wherein the linker 1 and the linker 2 are identical or different peptide linkers; the structures represented by formulas (a), (b), (c), and (d) are arranged from N-terminus to C-terminus; the Fc1 and Fc2 can be interchanged, and the Titin chain and Obscurin chain can be interchanged.

In some embodiments, provided is the antigen-binding molecule according to any one of the foregoing, wherein the antigen-binding molecule comprises one first chain having a structure represented by formula (a), one second chain having a structure represented by formula (b), one third chain having a structure represented by formula (c), and one fourth chain having a structure represented by formula (d):

(a) [DLL3-VH]-[linker 1]-[Titin chain]-[Fc2],
(b) [DLL3-VL]-[linker 2]-[Obscurin chain],
(c) [CD3-VH]-[CH1]-[Fc1], and
(d) [CD3-VL]-[CL],

wherein the linker 1 and the linker 2 are identical or different peptide linkers; the structures represented by formulas (a), (b), (c), and (d) are arranged from N-terminus to C-terminus.

In some embodiments, provided is the antigen-binding molecule according to any one of the foregoing, wherein the antigen-binding molecule comprises one first chain having a structure represented by formula (a'), one second chain having a structure represented by formula (b'), one third chain having a structure represented by formula (c'), and one fourth chain having a structure represented by formula (d'):

(a') [CD3-VH]-[linker 1]-[Titin chain]/Obscurin chain]-[Fc2]/[Fc1],
(b') [CD3-VL]-[linker 2]-[Obscurin chain]/[Titin chain],
(c') [DLL3-VH]-[CH1]-[Fc1]/[Fc2], and
(d') [DLL3-VL]-[CL],

wherein the linker 1 and the linker 2 are absent; that is, both the linker 1 and the linker 2 are peptide bonds; the structures represented by formulas (a'), (b'), (c'), and (d') are arranged from N-terminus to C-terminus. "/" means "or"; that is, the Fc1 and Fc2 can be interchanged, and the Titin chain and Obscurin chain can be interchanged.

In some embodiments, provided is the antigen-binding molecule according to any one of the foregoing, wherein the antigen-binding molecule comprises one first chain having a structure represented by formula (a'), one second chain having a structure represented by formula (b'), one third chain having a structure represented by formula (c'), and one fourth chain having a structure represented by formula (d'):

(a') [CD3-VH]-[linker 1]-[Titin chain]-[Fc2],
(b') [CD3-VL]-[linker 2]-[Obscurin chain],
(c') [DLL3-VH]-[CH1]-[Fc1], and
(d') [DLL3-VL]-[CL],

wherein the linker 1 and the linker 2 are absent; that is, both the linker 1 and the linker 2 are peptide bonds; the structures represented by formulas (a'), (b'), (c'), and (d') are arranged from N-terminus to C-terminus; the Fc1 and Fc2 can be interchanged, and the Titin chain and Obscurin chain can be interchanged.

In some embodiments, provided is the antigen-binding molecule according to any one of the foregoing, wherein the antigen-binding molecule comprises one first chain having a structure represented by formula (a), one second chain having a structure represented by formula (b), one third chain having a structure represented by formula (c), and one fourth chain having a structure represented by formula (d):

(a') [CD3-VH]-[linker 1]-[Titin chain]-[Fc2],
(b') [CD3-VL]-[linker 2]-[Obscurin chain],
(c') [DLL3-VH]-[CH1]-[Fc1], and
(d') [DLL3-VL]-[CL],

wherein the linker 1 and the linker 2 are absent; that is, both the linker 1 and the linker 2 are peptide bonds; the structures represented by formulas (a), (b), (c), and (d) are arranged from N-terminus to C-terminus.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; and
the CD3-VH comprises the amino acid sequence of SEQ ID NO: 136, and the CD3-VL comprises the amino acid sequence of SEQ ID NO: 137.

In some embodiments, provided is the antigen-binding molecule according to any one of the foregoing, wherein:

the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40; and

the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and

the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 61; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 63; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 63; and the CD3-VH comprises the amino acid sequence of SEQ ID NO: 136, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 81, and the DLL3-VL comprises the amino acid sequence of SEQ ID NO: 77; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 91, and the DLL3-VL comprises the amino acid sequence of SEQ ID NO: 87; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 109, and the DLL3-VL comprises the amino acid sequence of SEQ ID NO: 100; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 91, and the DLL3-VL comprises the amino acid sequence of SEQ ID NO: 87; and the CD3-VH comprises the amino acid sequence of SEQ ID NO: 136, and the CD3-VL comprises the amino acid sequence of SEQ ID NO: 137.

In some embodiments, provided is the antigen-binding molecule according to any one of the foregoing, wherein the Titin chain and the Obscurin chain are any Titin chain and any Obscurin chain from Tables 3-1 and 3-2 in the present disclosure that can form a dimer. In some embodiments, provided is the antigen-binding molecule according to any one of the foregoing, wherein the Titin chain comprises the amino acid sequence of SEQ ID NO: 208. In some embodiments, provided is the antigen-binding molecule according to any one of the foregoing, wherein the Obscurin chain comprises the amino acid sequence of SEQ ID NO: 246.

In some embodiments, provided is the antigen-binding molecule according to any one of the foregoing, wherein both the linker 1 and the linker 2 are peptide linkers known in the art, as long as the antigen-binding molecule is capable of exhibiting the desired antigen binding activity. For example, the peptide linkers may be flexible peptides having 1-50 or 3-20 amino acid residues. In some embodiments, the peptide linkers are 3-15 amino acid residues in length. In some embodiments, the peptide linker 1 and linker 2 each independently have a structure of (GGGGS)n, wherein n is 1, 2, or 3. In some embodiments, the sequences of the linker 1 and linker 2 are both GGGGS (SEQ ID NO: 147).

In some embodiments, provided is the antigen-binding molecule according to any one of the foregoing, wherein the CH1 is a CH1 sequence of an IgG. In some embodiments, the CH1 is the CH1 of IgG1. In some embodiments, the CH1 comprises the amino acid sequence of SEQ ID NO: 141.

In some embodiments, provided is the antigen-binding molecule according to any one of the foregoing, wherein the CL is a light chain constant region of an antibody. In some embodiments, provided is the antigen-binding molecule according to any one of the foregoing, wherein the CL is a light chain constant region of a kappa chain or a lambda chain. In some embodiments, the CL comprises the amino acid sequence of SEQ ID NO: 142.

In some embodiments, provided is the antigen-binding molecule according to any one of the foregoing, wherein the antigen-binding molecule comprises:

one first chain comprising the amino acid sequence of SEQ ID NO: 171, one second chain comprising the amino acid sequence of SEQ ID NO: 172, one third chain comprising the amino acid sequence of SEQ ID NO: 154, and one fourth chain comprising the amino acid sequence of SEQ ID NO: 155; or

one first chain comprising the amino acid sequence of SEQ ID NO: 171, one second chain comprising the amino acid sequence of SEQ ID NO: 172, one third chain comprising the amino acid sequence of SEQ ID NO: 156, and one fourth chain comprising the amino acid sequence of SEQ ID NO: 155; or

one first chain comprising the amino acid sequence of SEQ ID NO: 152, one second chain comprising the amino acid sequence of SEQ ID NO: 153, one third chain comprising the amino acid sequence of SEQ ID NO: 154, and one fourth chain comprising the amino acid sequence of SEQ ID NO: 155; or

one first chain comprising the amino acid sequence of SEQ ID NO: 152, one second chain comprising the amino acid sequence of SEQ ID NO: 153, one third chain comprising the amino acid sequence of SEQ ID NO: 156, and one fourth chain comprising the amino acid sequence of SEQ ID NO: 155; or

one first chain comprising the amino acid sequence of SEQ ID NO: 164, one second chain comprising the amino acid sequence of SEQ ID NO: 165, one third chain comprising the amino acid sequence of SEQ ID NO: 154, and one fourth chain comprising the amino acid sequence of SEQ ID NO: 155; or

one first chain comprising the amino acid sequence of SEQ ID NO: 164, one second chain comprising the amino acid sequence of SEQ ID NO: 165, one third chain comprising the amino acid sequence of SEQ ID NO: 156, and one fourth chain comprising the amino acid sequence of SEQ ID NO: 155; or

one first chain comprising the amino acid sequence of SEQ ID NO: 178, one second chain comprising the amino acid sequence of SEQ ID NO: 179, one third chain comprising the amino acid sequence of SEQ ID NO: 154, and one fourth chain comprising the amino acid sequence of SEQ ID NO: 155; or

one first chain comprising the amino acid sequence of SEQ ID NO: 178, one second chain comprising the amino acid sequence of SEQ ID NO: 179, one third chain comprising the amino acid sequence of SEQ ID NO: 156, and one fourth chain comprising the amino acid sequence of SEQ ID NO: 155.

In some embodiments, provided is the antigen-binding molecule according to any one of the foregoing, wherein the antigen-binding molecule comprises:
one first chain comprising the amino acid sequence of SEQ ID NO: 171, one second chain comprising the amino acid sequence of SEQ ID NO: 172, one third chain comprising the amino acid sequence of SEQ ID NO: 154, and one fourth chain comprising the amino acid sequence of SEQ ID NO: 155.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3; the antigen-binding moiety binding specifically to DLL3 is a Fab, and the antigen-binding moiety binding specifically to CD3 is an scFv. In some embodiments, provided is the antigen-binding molecule according to any one of the foregoing, wherein the antigen-binding molecule comprises one first chain having a structure represented by formula (e), one second chain having a structure represented by formula (f), and one third chain having a structure represented by formula (g):

(e) [DLL3-VL]-[CL],
(f) [DLL3-VH]-[CH1]-[Fc2]/[Fc1], and
(g) [CD3-VH]-[linker 3]-[CD3-VL]-[linker 4]-[Fc1]/[Fc2],
wherein the linker 3 and the linker 4 are identical or different peptide linkers;

the structures represented by formulas (e), (f), and (g) are arranged from N-terminus to C-terminus. "/" means "or"; that is, the Fc1 and Fc2 can be interchanged.

In some embodiments, provided is the antigen-binding molecule according to any one of the foregoing, wherein the antigen-binding molecule comprises one first chain having a structure represented by formula (e), one second chain having a structure represented by formula (f), and one third chain having a structure represented by formula (g):

(e) [DLL3-VL]-[CL],
(f) [DLL3-VH]-[CH1]-[Fc2], and
(g) [CD3-VH]-[linker 3]-[CD3-VL]-[linker 4]-[Fc1],
wherein the linker 3 and the linker 4 are identical or different peptide linkers;
the structures represented by formulas (e), (f), and (g) are arranged from N-terminus to C-terminus; the Fc1 and Fc2 can be interchanged.

In some embodiments, provided is the antigen-binding molecule according to any one of the foregoing, wherein the antigen-binding molecule comprises one first chain having a structure represented by formula (e), one second chain having a structure represented by formula (f), and one third chain having a structure represented by formula (g):

(e) [DLL3-VL]-[CL],
(f) [DLL3-VH]-[CH1]-[Fc2], and
(g) [CD3-VH]-[linker 3]-[CD3-VL]-[linker 4]-[Fc1],
wherein the linker 3 and the linker 4 are identical or different peptide linkers;
the structures represented by formulas (e), (f), and (g) are arranged from N-terminus to C-terminus.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3; the antigen-binding moiety binding specifically to DLL3 is an scFv, and the antigen-binding moiety binding specifically to CD3 is a Fab.

In some embodiments, provided is the antigen-binding molecule according to any one of the foregoing, wherein the antigen-binding molecule comprises one first chain having a structure represented by formula (e'), one second chain having a structure represented by formula (f), and one third chain having a structure represented by formula (g'):

(e') [CD3-VL]-[CL],
(f) [CD3-VH]-[CH1]-[Fc2]/[Fc1], and
(g') [DLL3-VH]-[linker 3]-[DLL3-VL]-[linker 4]-[Fc1]/[Fc2],
wherein the linker 3 and the linker 4 are identical or different peptide linkers;
the structures represented by formulas (e'), (f), and (g') are arranged from N-terminus to C-terminus. "/" means "or".

In some embodiments, provided is the antigen-binding molecule according to any one of the foregoing, wherein the antigen-binding molecule comprises one first chain having a structure represented by formula (e'), one second chain having a structure represented by formula (f), and one third chain having a structure represented by formula (g'):

(e') [CD3-VL]-[CL],
(f) [CD3-VH]-[CH1]-[Fc2], and
(g') [DLL3-VH]-[linker 3]-[DLL3-VL]-[linker 4]-[Fc1],
wherein the linker 3 and the linker 4 are identical or different peptide linkers;
the structures represented by formulas (e'), (f), and (g') are arranged from N-terminus to C-terminus; the Fc1 and Fc2 can be interchanged.

In some embodiments, provided is the antigen-binding molecule according to any one of the foregoing, wherein the antigen-binding molecule comprises one first chain having a structure represented by formula (e'), one second chain having a structure represented by formula (f), and one third chain having a structure represented by formula (g'):

(e') [CD3-VL]-[CL],
(f) [CD3-VH]-[CH1]-[Fc2], and
(g') [DLL3-VH]-[linker 3]-[DLL3-VL]-[linker 4]-[Fc1],
wherein the linker 3 and the linker 4 are identical or different peptide linkers;
the structures represented by formulas (e'), (f), and (g') are arranged from N-terminus to C-terminus.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 61; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 63; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 91, and the DLL3-VL comprises the amino acid sequence of SEQ ID NO: 87; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 81, and the DLL3-VL comprises the amino acid sequence of SEQ ID NO: 77; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 109, and the DLL3-VL comprises the amino acid sequence of SEQ ID NO: 100; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 91, and the DLL3-VL comprises the amino acid sequence of SEQ ID NO: 87; and the CD3-VH comprises the amino acid sequence of SEQ ID NO: 138, and the CD3-VL comprises the amino acid sequence of SEQ ID NO: 137.

In some embodiments, provided is the antigen-binding molecule according to any one of the foregoing, wherein both the linker 3 and the linker 4 are peptide linkers known in the art, as long as the antigen-binding molecule is capable of exhibiting the desired antigen binding activity. For example, the peptide linkers may be flexible peptides having 1-50 or 3-20 amino acid residues. In some embodiments, the peptide linkers are 3-15 amino acid residues in length. In some embodiments, the peptide linker 3 and linker 4 each independently have a structure of (GGGGS)n, wherein n is 1-5. In some embodiments, n is 1, 2, 3, or 4. In some embodiments, the sequence of the linker 3 is GGGGSGGGGSGGGGS (SEQ ID NO: 148). In some embodiments, the sequence of the linker 4 is GGGGS (SEQ ID NO: 147).

In some embodiments, provided is the antigen-binding molecule according to any one of the foregoing, wherein the CH1 is a CH1 sequence of an IgG. In some embodiments, the CH1 is the CH1 of IgG1. In some embodiments, the CH1 comprises the amino acid sequence of SEQ ID NO: 141.

In some embodiments, provided is the antigen-binding molecule according to any one of the foregoing, wherein the CL is a light chain constant region of an antibody. In some embodiments, provided is the antigen-binding molecule according to any one of the foregoing, wherein the CL is a light chain constant region of a kappa chain or a lambda chain. In some embodiments, the CL comprises the amino acid sequence of SEQ ID NO: 142.

In some embodiments, provided is the antigen-binding molecule according to any one of the foregoing, wherein the antigen-binding molecule comprises:

one first chain comprising the amino acid sequence of SEQ ID NO: 173, one second chain comprising the amino acid sequence of SEQ ID NO: 174, and one third chain comprising the amino acid sequence of SEQ ID NO: 160; or
one first chain comprising the amino acid sequence of SEQ ID NO: 173, one second chain comprising the amino acid sequence of SEQ ID NO: 174, and one third chain comprising the amino acid sequence of SEQ ID NO: 159; or
one first chain comprising the amino acid sequence of SEQ ID NO: 157, one second chain comprising the amino acid sequence of SEQ ID NO: 158, and one third chain comprising the amino acid sequence of SEQ ID NO: 159; or
one first chain comprising the amino acid sequence of SEQ ID NO: 157, one second chain comprising the amino acid sequence of SEQ ID NO: 158, and one third chain comprising the amino acid sequence of SEQ ID NO: 160; or
one first chain comprising the amino acid sequence of SEQ ID NO: 166, one second chain comprising the amino acid sequence of SEQ ID NO: 167, and one third chain comprising the amino acid sequence of SEQ ID NO: 159; or

one first chain comprising the amino acid sequence of SEQ ID NO: 166, one second chain comprising the amino acid sequence of SEQ ID NO: 167, and one third chain comprising the amino acid sequence of SEQ ID NO: 160; or one first chain comprising the amino acid sequence of SEQ ID NO: 180, one second chain comprising the amino acid sequence of SEQ ID NO: 181, and one third chain comprising the amino acid sequence of SEQ ID NO: 159; or one first chain comprising the amino acid sequence of SEQ ID NO: 180, one second chain comprising the amino acid sequence of SEQ ID NO: 181, and one third chain comprising the amino acid sequence of SEQ ID NO: 160.

In some embodiments, provided is the antigen-binding molecule according to any one of the foregoing, wherein the antigen-binding molecule comprises:

one first chain comprising the amino acid sequence of SEQ ID NO: 173, one second chain comprising the amino acid sequence of SEQ ID NO: 174, and one third chain comprising the amino acid sequence of SEQ ID NO: 160.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3;

the antigen-binding molecule comprises one first chain having a structure represented by formula (h) and one second chain having a structure represented by formula (i):

(h) [DLL3-VL]-[linker 5]-[CD3-VH]-[linker 6]-[Fc1]/[Fc1], and
(i) [CD3-VL]-[linker 7]-[DLL3-VH]-[linker 8]-[Fc2]/[Fc2],
wherein the linker 5, linker 6, linker 7, and linker 8 are identical or different peptide linkers;
the structures represented by formulas (h) and (i) are arranged from N-terminus to C-terminus. "/" means "or"; that is, the Fc1 and Fc2 can be interchanged.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3;

the antigen-binding molecule comprises one first chain having a structure represented by formula (h) and one second chain having a structure represented by formula (i):

(h) [DLL3-VL]-[linker 5]-[CD3-VH]-[linker 6]-[Fc1], and
(i) [CD3-VL]-[linker 7]-[DLL3-VH]-[linker 8]-[Fc2],
wherein the linker 5, linker 6, linker 7, and linker 8 are identical or different peptide linkers;
the structures represented by formulas (h) and (i) are arranged from N-terminus to C-terminus; the Fc1 and Fc2 can be interchanged.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3;

the antigen-binding molecule comprises one first chain having a structure represented by formula (h) and one second chain having a structure represented by formula (i):

(h) [DLL3-VL]-[linker 5]-[CD3-VH]-[linker 6]-[Fc1], and
(i) [CD3-VL]-[linker 7]-[DLL3-VH]-[linker 8]-[Fc2],
wherein the linker 5, linker 6, linker 7, and linker 8 are identical or different peptide linkers;
the structures represented by formulas (h) and (i) are arranged from N-terminus to C-terminus.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3;

the antigen-binding molecule comprises one first chain having a structure represented by formula (h') and one second chain having a structure represented by formula (i'):

(h') [CD3-VL]-[linker 5]-[DLL3-VH]-[linker 6]-[Fc1]/[Fc2], and
(I') [DLL3-VL]-[linker 7]-[CD3-VH]-[linker 8]-[Fc2]/[Fc1],
wherein the linker 5, linker 6, linker 7, and linker 8 are identical or different peptide linkers;
the structures represented by formulas (h') and (i') are arranged from N-terminus to C-terminus. "/" means "or".

In some embodiments, provided is the antigen-binding molecule according to any one of the foregoing, wherein the antigen-binding molecule comprises one antigen-binding moiety binding specifically to DLL3 and one antigen-binding moiety binding specifically to CD3;
the antigen-binding molecule comprises one first chain having a structure represented by formula (h') and one second chain having a structure represented by formula (i'):

(h') [CD3-VL]-[linker 5]-[DLL3-VH]-[linker 6]-[Fc1], and
(I') [DLL3-VL]-[linker 7]-[CD3-VH]-[linker 8]-[Fc2],
wherein the linker 5, linker 6, linker 7, and linker 8 are identical or different peptide linkers;
the structures represented by formulas (h') and (i') are arranged from N-terminus to C-terminus; the Fc1 and Fc2 can be interchanged.

In some embodiments, provided is the antigen-binding molecule according to any one of the foregoing, wherein the antigen-binding molecule comprises one antigen-binding moiety binding specifically to DLL3 and one antigen-binding moiety binding specifically to CD3;
the antigen-binding molecule comprises one first chain having a structure represented by formula (h') and one second chain having a structure represented by formula (i'):

(h') [CD3-VL]-[linker 5]-[DLL3-VH]-[linker 6]-[Fc1], and
(I') [DLL3-VL]-[linker 7]-[CD3-VH]-[linker 8]-[Fc2],
wherein the linker 5, linker 6, linker 7, and linker 8 are identical or different peptide linkers;
the structures represented by formulas (h') and (i') are arranged from N-terminus to C-terminus.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; and

the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; and

the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; and

the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and

the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40; and

the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131 or 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; and
the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 61; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 63; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 64; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 56, and the DLL3-VL comprises the amino acid sequence of SEQ ID NO: 65; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 56, and the DLL3-VL comprises the amino acid sequence of SEQ ID NO: 66; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 56, and the DLL3-VL comprises the amino acid sequence of SEQ ID NO: 67; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 57, and the DLL3-VL comprises the amino acid sequence of SEQ ID NO: 65; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 57, and the DLL3-VL comprises the amino acid sequence of SEQ ID NO: 66; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 57, and the DLL3-VL comprises the amino acid sequence of SEQ ID NO: 67; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 91, and the DLL3-VL comprises the amino acid sequence of SEQ ID NO: 87; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 81, and the DLL3-VL comprises the amino acid sequence of SEQ ID NO: 77; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 109, and the DLL3-VL comprises the amino acid sequence of SEQ ID NO: 100; and the CD3-VH comprises the amino acid sequence of SEQ ID NO: 136 or 138, and the CD3-VL comprises the amino acid sequence of SEQ ID NO: 137.

In some embodiments, provided is the antigen-binding molecule according to any one of the foregoing, wherein: the DLL3-VH comprises the amino acid sequence of SEQ ID NO: 54, and the DLL3-VL comprises the amino acid sequence of SEQ ID NO: 63; and the CD3-VH comprises the amino acid sequence of SEQ ID NO: 136, and the CD3-VL comprises the amino acid sequence of SEQ ID NO: 137;

In some embodiments, provided is the antigen-binding molecule according to any one of the foregoing, wherein the linker 5, linker 6, linker 7, and linker 8 are all peptide linkers known in the art, as long as the antigen-binding molecule is capable of exhibiting the desired antigen binding activity. For example, the peptide linkers may be flexible peptides comprising 1-50 or 3-20 amino acid residues. In some embodiments, the peptide linkers are 1-15 amino acid residues in length. In some embodiments, the linker 5 has a structure of (GGGS)nGm, wherein n is 1-5, preferably 1, 2, or 3; m is 1-10, preferably 4, 5, 6, 7, or 8. In some embodiments, the sequence of the linker 5 is GGGSGGGG (SEQ ID NO: 149). In some embodiments, the linker 6 and linker 8 have a structure of Gm, wherein m is 1-5, preferably 1, 2, or 3. In some embodiments, the sequences of linker 6 and linker 8 are G (SEQ ID NO: 150). In some embodiments, the linker 7 has a structure of (GGGGS)nGm, wherein n is 1-5, preferably 1, 2, or 3; m is 1-10, preferably 4, 5, 6, 7, or 8. In some embodiments, the sequence of linker 7 is GGGGSGGGG (SEQ ID NO: 151).

In some embodiments, provided is the antigen-binding molecule according to any one of the foregoing, wherein the antigen-binding molecule comprises:

one first chain comprising the amino acid sequence of SEQ ID NO: 185 and one second chain comprising the amino acid sequence of SEQ ID NO: 162; or

one first chain comprising the amino acid sequence of SEQ ID NO: 161 and one second chain comprising the amino acid sequence of SEQ ID NO: 162; or

one first chain comprising the amino acid sequence of SEQ ID NO: 163 and one second chain comprising the amino acid sequence of SEQ ID NO: 162; or

one first chain comprising the amino acid sequence of SEQ ID NO: 259 and one second chain comprising the amino acid sequence of SEQ ID NO: 162; or

one first chain comprising the amino acid sequence of SEQ ID NO: 260 and one second chain comprising the amino acid sequence of SEQ ID NO: 162; or

one first chain comprising the amino acid sequence of SEQ ID NO: 168 and one second chain comprising the amino acid sequence of SEQ ID NO: 169; or

one first chain comprising the amino acid sequence of SEQ ID NO: 170 and one second chain comprising the amino acid sequence of SEQ ID NO: 169; or

one first chain comprising the amino acid sequence of SEQ ID NO: 175 and one second chain comprising the amino acid sequence of SEQ ID NO: 176; or

one first chain comprising the amino acid sequence of SEQ ID NO: 177 and one second chain comprising the amino acid sequence of SEQ ID NO: 176; or

one first chain comprising the amino acid sequence of SEQ ID NO: 182 and one second chain comprising the amino acid sequence of SEQ ID NO: 183; or

one first chain comprising the amino acid sequence of SEQ ID NO: 184 and one second chain comprising the amino acid sequence of SEQ ID NO: 183; or

one first chain comprising the amino acid sequence of SEQ ID NO: 186 and one second chain comprising the amino acid sequence of SEQ ID NO: 162; or

one first chain comprising the amino acid sequence of SEQ ID NO: 187 and one second chain comprising the amino acid sequence of SEQ ID NO: 188; or

one first chain comprising the amino acid sequence of SEQ ID NO: 187 and one second chain comprising the amino acid sequence of SEQ ID NO: 189; or

one first chain comprising the amino acid sequence of SEQ ID NO: 190 and one second chain comprising the amino acid sequence of SEQ ID NO: 188; or

one first chain comprising the amino acid sequence of SEQ ID NO: 190 and one second chain comprising the amino acid sequence of SEQ ID NO: 189; or

one first chain comprising the amino acid sequence of SEQ ID NO: 191 and one second chain comprising the amino acid sequence of SEQ ID NO: 188; or

one first chain comprising the amino acid sequence of SEQ ID NO: 191 and one second chain comprising the amino acid sequence of SEQ ID NO: 189.

In some embodiments, provided is the antigen-binding molecule according to any one of the foregoing, wherein the antigen-binding molecule comprises:

one first chain comprising the amino acid sequence of SEQ ID NO: 185 and one second chain comprising the amino acid sequence of SEQ ID NO: 162.

In another aspect, the present disclosure also provides an anti-DLL3 antibody capable of binding specifically to DLL3, the anti-DLL3 antibody comprising a heavy chain variable region DLL3-VH and a light chain variable region DLL3-VL, wherein:

(i) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 12, 56, or 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 13, 63, 64, 65, 66, or 67, respectively; or

(ii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 14, 79, 80, 81, or 82, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 15, respectively; or

(iii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 16, 91, 92, 93, or 94, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 17, respectively; or

(iv) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 18, 107, 108, 109, 110, 112, 113, 114, or 115, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 19, respectively.

In some embodiments, provided is the anti-DLL3 antibody according to any one of the foregoing, wherein:

(i) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 12, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 13, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 54, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 64, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 54, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 63, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 56, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 65, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 56, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 66, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 56, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 67, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 65, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 66, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 57, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 67, respectively; or

(ii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 14, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 15, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 81, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 77, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 79, 80, or 82, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 77, respectively; or

(iii) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 16, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 17, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 91, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 87, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 92, 93, or 94, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 87, respectively; or

(iv) a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 18, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 19, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 109, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 100, respectively; or

a DLL3-HCDR1, a DLL3-HCDR2, and a DLL3-HCDR3 in the DLL3-VH comprise the amino acid sequences of a DLL3-HCDR1, a DLL3-HCDR2, and a DLL3-HCDR3 in SEQ ID NO: 107, 108, 110, 112, 113, 114, or 115, respectively, and a DLL3-LCDR1, a DLL3-LCDR2, and a DLL3-LCDR3 in the DLL3-VL comprise the amino acid sequences of a DLL3-LCDR1, a DLL3-LCDR2, and a DLL3-LCDR3 in SEQ ID NO: 100, respectively.

In some embodiments, the DLL3-HCDR1, DLL3-HCDR2, DLL3-HCDR3, DLL3-LCDR1, DLL3-LCDR2, and DLL3-LCDR3 are defined according to the Kabat, IMGT, Chothia, AbM, or Contact numbering scheme.

In some embodiments, provided is the anti-DLL3 antibody according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 69 or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25;

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

ii) the DLL3-VH comprises: a DLL3-HCDR1 comprising the amino acid sequence of SEQ ID NO: 26 or 84, a DLL3-HCDR2 comprising the amino acid sequence of SEQ ID NO: 27 or 85, and a DLL3-HCDR3 comprising the amino acid sequence of SEQ ID NO: 28 or 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

iii) the DLL3-VH comprises: a DLL3-HCDR1 comprising the amino acid sequence of SEQ ID NO: 20, a DLL3-HCDR2 comprising the amino acid sequence of SEQ ID NO: 32, 96, 97, 98, or 99, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

iv) the DLL3-VH comprises: a DLL3-HCDR1 comprising the amino acid sequence of SEQ ID NO: 20, a DLL3-HCDR2 comprising the amino acid sequence of SEQ ID NO: 37, 117, 118, 119, or 120, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40.

In some embodiments, provided is the anti-DLL3 antibody according to any one of the foregoing, wherein:

i) the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74.

In some embodiments, provided is the anti-DLL3 antibody according to any one of the foregoing, wherein:

i) the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

ii) the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 84, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 84, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

iii) the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

iv) the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40.

In some embodiments, provided is the anti-DLL3 antibody according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25.

In some embodiments, provided is the anti-DLL3 antibody according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25.

In some embodiments, provided is the anti-DLL3 antibody according to any one of the foregoing, wherein: the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31.

In some embodiments, provided is the anti-DLL3 antibody according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36.

In some embodiments, provided is the anti-DLL3 antibody according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40.

In some embodiments, in the anti-DLL3 antibody according to any one of the foregoing, the DLL3-HCDR1, DLL3-HCDR2, DLL3-HCDR3, DLL3-LCDR1, DLL3-LCDR2, and DLL3-LCDR3 are defined according to the Kabat numbering scheme.

In some embodiments, the anti-DLL3 antibody according to any one of the foregoing binds to human DLL3 at 25 °C with a KD of less than $4 \times 10^{-9}$ M, $3 \times 10^{-9}$ M, $2 \times 10^{-9}$ M, $1 \times 10^{-9}$ M, $9 \times 10^{-10}$ M, $8 \times 10^{-10}$ M, $7 \times 10^{-10}$ M, $6 \times 10^{-10}$ M, $5 \times 10^{-10}$ M, $4 \times 10^{-10}$ M, $3 \times 10^{-10}$ M, $2 \times 10^{-10}$ M, $1 \times 10^{-10}$ M, or $9 \times 10^{-11}$ M, as measured by surface plasmon resonance.

In some embodiments, provided is the anti-DLL3 antibody according to the foregoing, wherein:

(i) the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, 71, 72, or 73, and a DLL3-LCDR3 comprising the amino acid sequence of SEQ ID NO: 25 or 74, and FR regions of the DLL3-VL comprise one or more amino acid substitutions selected from the group consisting of 1N, 36L, 42K, 43S, 44F, 46G, 69A, 71Y, 79Q, and 85D; or

(ii) the DLL3-VH comprises: a DLL3-HCDR1 comprising the amino acid sequence of SEQ ID NO: 26 or 84, a DLL3-HCDR2 comprising the amino acid sequence of SEQ ID NO: 27 or 85, and a DLL3-HCDR3 comprising the amino acid sequence of SEQ ID NO: 28 or 83; and

the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or

(iii) the DLL3-VH comprises: a DLL3-HCDR1 comprising the amino acid sequence of SEQ ID NO: 20, a DLL3-HCDR2 comprising the amino acid sequence of SEQ ID NO: 32, 96, 97, 98, or 99, and a DLL3-HCDR3 comprising the amino acid sequence of SEQ ID NO: 33, and FR regions of the DLL3-VH comprise one or more amino acid substitutions selected from the group consisting of 1E, 24T, 44S, 71V, and 73K; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36, and FR regions of the DLL3-VL comprise one or more amino acid substitutions selected from the group consisting of 1N, 9K, 43S, 68A, and 85D; or

(iv) the DLL3-VH comprises: a DLL3-HCDR1 comprising the amino acid sequence of SEQ ID NO: 20, a DLL3-HCDR2 comprising the amino acid sequence of SEQ ID NO: 37, 117, 118, 119, or 120, and a DLL3-HCDR3 comprising the amino acid sequence of SEQ ID NO: 38, and FR regions of the DLL3-VH comprise one or more amino acid substitutions selected from the group consisting of 1E, 3R, 5Q, 7F, 9P, 10V, 12V, 40S, 41H, 44S, 48I, 67A, 69L, 71V, 73K, 75S, and 83T; and

the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40, and FR regions of the DLL3-VL comprise one or more amino acid substitutions selected from the group consisting of 9K, 43S, 60D, 68A, 87H, and 100S.

In some embodiments, the antigen-binding molecule according to any one of the foregoing binds to human DLL3 at 25 °C with a KD of less than $4 \times 10^{-9}$ M, $3 \times 10^{-9}$ M, $2 \times 10^{-9}$ M, $1 \times 10^{-9}$ M, $9 \times 10^{-10}$ M, $8 \times 10^{-10}$ M, $7 \times 10^{-10}$ M, $6 \times 10^{-10}$ M, $5 \times 10^{-10}$ M, $4 \times 10^{-10}$ M, $3 \times 10^{-10}$ M, $2 \times 10^{-10}$ M, $1 \times 10^{-10}$ M, or $9 \times 10^{-11}$ M, as measured by surface plasmon resonance.

In some embodiments, provided is the anti-DLL3 antibody according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D.

In some embodiments, provided is the anti-DLL3 antibody according to any one of the foregoing, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22, and FR regions of the DLL3-VH comprise one or more amino acid substitutions selected from the group consisting of 1E, 5Q, 27Y, 30T, 38K, 43K, 48I, 67A, 68A, 69L, 71V, 73K, 75S, 76N, and 93A; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25, and FR regions of the DLL3-VL comprise one or more amino acid substitutions selected from the group consisting of 1N, 36L, 42K, 43S, 44F, 46G, 69A, 71Y, 79Q, and 85D.

In some embodiments, provided is the anti-DLL3 antibody according to any one of the foregoing, wherein:

the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or
the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or
the DLL3-VH comprises: a DLL3-HCDR1 comprising the amino acid sequence of SEQ ID NO: 84, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y; or
the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31, and FR regions of the DLL3-VL comprise one or more amino acid substitutions selected from the group consisting of 42G, 44V, and 71Y.

In some embodiments, provided is the anti-DLL3 antibody according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33, and FR regions of the DLL3-VH comprise one or more amino acid substitutions selected from the group consisting of 1E, 24T, 44S, 71V, and 73K; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36, and FR regions of the DLL3-VL comprise one or more amino acid substitutions selected from the group consisting of 1N, 9K, 43S, 68A, and 85D.
In some embodiments, provided is the anti-DLL3 antibody according to any one of the foregoing, wherein: the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38, and FR regions of the DLL3-VH comprise one or more amino acid substitutions selected from the group consisting of 1E, 3R, 5Q, 7F, 9P, 10V, 12V, 40S, 41H, 44S, 48I, 67A, 69L, 71V, 73K, 75S, and 83T; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40, and FR regions of the DLL3-VL comprise one or more amino acid substitutions selected from the group consisting of 9K, 43S, 60D, 68A, 87H, and 100S.
In some embodiments, provided is the anti-DLL3 antibody according to any one of the foregoing, wherein: the DLL3-VH comprises the amino acid sequence of SEQ ID NO: 50 or a variant thereof. In some embodiments, the variant comprises one or more amino acid substitutions selected from the group consisting of Q1E, Q43K, M48I, V67A, I69L, R71V, T73K, and S76N in FR regions of SEQ ID NO: 50.
In some embodiments, provided is the anti-DLL3 antibody according to any one of the foregoing, wherein: the DLL3-VH comprises the amino acid sequence of SEQ ID NO: 51 or a variant thereof. In some embodiments, the variant comprises one or more amino acid substitutions selected from the group consisting of Q1E, 5Q, R38K, V68A, F69L, L71V, T73K, V75S, and S76N in FR regions of SEQ ID NO: 51.

In some embodiments, provided is the anti-DLL3 antibody according to any one of the foregoing, wherein: the DLL3-VH comprises the amino acid sequence of SEQ ID NO: 52 or a variant thereof. In some embodiments, the variant comprises one or more amino acid substitutions selected from the group consisting of F27Y, S30T, I69L, R71V, D73K, and T93A in FR regions of SEQ ID NO: 52.

In some embodiments, provided is the anti-DLL3 antibody according to any one of the foregoing, wherein: the DLL3-VL comprises the amino acid sequence of SEQ ID NO: 62 or a variant thereof. In some embodiments, the variant comprises one or more amino acid substitutions selected from the group consisting of F36L, A43S, P44F, S46G, T69A, F71Y, and T85D in FR regions of SEQ ID NO: 62.

In some embodiments, provided is the anti-DLL3 antibody according to any one of the foregoing, wherein: the DLL3-VL comprises the amino acid sequence of SEQ ID NO: 68 or a variant thereof. In some embodiments, the variant comprises one or more amino acid substitutions selected from the group consisting of Y36L, Q42K, L46G, T69A, F71Y, E79Q, and V85D in FR regions of SEQ ID NO: 68.

In some embodiments, provided is the anti-DLL3 antibody according to any one of the foregoing, wherein: the DLL3-VH comprises the amino acid sequence of SEQ ID NO: 78 or a variant thereof. In some embodiments, the variant comprises one or more amino acid substitutions in FR regions of SEQ ID NO: 78.

In some embodiments, provided is the anti-DLL3 antibody according to any one of the foregoing, wherein: the DLL3-VL comprises the amino acid sequence of SEQ ID NO: 75 or a variant thereof. In some embodiments, the variant comprises one or more amino acid substitutions selected from the group consisting of K42G, P44V, and F71Y in FR regions of SEQ ID NO: 75.

In some embodiments, provided is the anti-DLL3 antibody according to any one of the foregoing, wherein: the DLL3-VH comprises the amino acid sequence of SEQ ID NO: 95 or a variant thereof. In some embodiments, the variant comprises one or more amino acid substitutions selected from the group consisting of Q1E, A24T, R44S, R71V, and T73K in FR regions of SEQ ID NO: 95.

In some embodiments, provided is the anti-DLL3 antibody according to any one of the foregoing, wherein: the DLL3-VL comprises the amino acid sequence of SEQ ID NO: 88 or a variant thereof. In some embodiments, the variant comprises one or more amino acid substitutions selected from the group consisting of D1N, D9K, P43S, G68A, and V85D in FR regions of SEQ ID NO: 88.

In some embodiments, provided is the anti-DLL3 antibody according to any one of the foregoing, wherein: the DLL3-VH comprises the amino acid sequence of SEQ ID NO: 116 or a variant thereof. In some embodiments, the variant comprises one or more amino acid substitutions selected from the group consisting of Q1E, Q3R, V5Q, S7F, A9P, E10V, K12V, A40S, P41H, R44S, M48I, V67A, I69L, R71V, T73K, A75S, and R83T in FR regions of SEQ ID NO: 116.

In some embodiments, provided is the anti-DLL3 antibody according to any one of the foregoing, wherein: the DLL3-VL comprises the amino acid sequence of SEQ ID NO: 100 or a variant thereof. In some embodiments, the variant comprises one or more amino acid substitutions selected from the group consisting of D9K, P43S, G68A, and Y87H in FR regions of SEQ ID NO: 100.

In some embodiments, provided is the anti-DLL3 antibody according to any one of the foregoing, wherein: the DLL3-VL comprises the amino acid sequence of SEQ ID NO: 103 or a variant thereof. In some embodiments, the variant comprises one or more amino acid substitutions selected from the group consisting of A43S, S60D, and Q100S in FR regions of SEQ ID NO: 103.

In some embodiments, provided is the anti-DLL3 antibody according to any one of the foregoing, wherein: the DLL3-VH comprises the amino acid sequence of SEQ ID NO: 56 or a variant thereof. In some embodiments, the variant comprises one or more amino acid substitutions in FR regions of SEQ ID NO: 56.

In some embodiments, provided is the anti-DLL3 antibody according to any one of the foregoing, wherein: the DLL3-VH comprises the amino acid sequence of SEQ ID NO: 57 or a variant thereof. In some embodiments, the variant comprises one or more amino acid substitutions in FR regions of SEQ ID NO: 57.

In some embodiments, provided is the anti-DLL3 antibody according to any one of the foregoing, wherein: the DLL3-VL comprises the amino acid sequence of SEQ ID NO: 63 or a variant thereof. In some embodiments, the variant comprises one or more amino acid substitutions in FR regions of SEQ ID NO: 63; in some embodiments, the amino acid substitutions are one or more amino acid substitutions selected from the group consisting of A43S and P44F.

In some embodiments, provided is the anti-DLL3 antibody according to any one of the foregoing, wherein: the DLL3-VL comprises the amino acid sequence of SEQ ID NO: 64 or a variant thereof. In some embodiments, the variant comprises one or more amino acid substitutions in FR regions of SEQ ID NO: 64; in some embodiments, the amino acid substitutions are one or more amino acid substitutions selected from the group consisting of A43S and P44F.

In some embodiments, provided is the anti-DLL3 antibody according to any one of the foregoing, wherein: the DLL3-VL comprises the amino acid sequence of SEQ ID NO: 65 or a variant thereof. In some embodiments, the variant comprises one or more amino acid substitutions in FR regions of SEQ ID NO: 65; in some embodiments, the amino acid substitutions are one or more amino acid substitutions selected from the group consisting of A43S and P44F.

In some embodiments, provided is the anti-DLL3 antibody according to any one of the foregoing, wherein: the DLL3-VL comprises the amino acid sequence of SEQ ID NO: 66 or a variant thereof. In some embodiments, the variant comprises one or more amino acid substitutions in FR regions of SEQ ID NO: 66; in some embodiments, the amino acid substitutions are one or more amino acid substitutions selected from the group consisting of A43S and P44F.

In some embodiments, provided is the anti-DLL3 antibody according to any one of the foregoing, wherein: the DLL3-VL comprises the amino acid sequence of SEQ ID NO: 67 or a variant thereof. In some embodiments, the variant comprises one or more amino acid substitutions in FR regions of SEQ ID NO: 67.

In some embodiments, provided is the anti-DLL3 antibody according to any one of the foregoing, wherein:

the DLL3-VH comprises the amino acid sequence of SEQ ID NO: 79, 80, 81, or 82, or a variant of SEQ ID NO: 79, 80, 81, or 82. In some embodiments, the variant comprises one or more amino acid substitutions in FR regions of SEQ ID NO: 79, 80, 81, or 82; and

the DLL3-VL comprises the amino acid sequence of SEQ ID NO: 75 or a variant thereof. In some embodiments, the variant comprises one or more amino acid substitutions selected from the group consisting of K42G, P44V, and F71Y in FR regions of SEQ ID NO: 75.

In some embodiments, provided is the anti-DLL3 antibody according to any one of the foregoing, wherein:

the DLL3-VH comprises the amino acid sequence of SEQ ID NO: 91, 92, 93, or 94, or a variant of SEQ ID NO: 91, 92, 93, or 94. In some embodiments, the variant comprises one or more amino acid substitutions in FR regions of SEQ ID NO: 91, 92, 93, or 94; and
the DLL3-VL comprises the amino acid sequence of SEQ ID NO: 88 or a variant thereof. In some embodiments, the variant comprises one or more amino acid substitutions selected from the group consisting of D1N, D9K, P43S, G68A, and V85D in FR regions of SEQ ID NO: 88.

In some embodiments, provided is the anti-DLL3 antibody according to any one of the foregoing, wherein: the DLL3-VH comprises the amino acid sequence of SEQ ID NO: 107, 108, 109, or 110, or a variant of SEQ ID NO: 107, 108, 109, or 110. In some embodiments, the variant comprises one or more amino acid substitutions in FR regions of SEQ ID NO: 107, 108, 109, or 110; in some embodiments, the amino acid substitutions are one or more amino acid substitutions selected from the group consisting of V5Q, S7F, A9P, E10V, K12V, A40S, P41H, M48I, V67A, A75S, and R83T.

In some embodiments, provided is the anti-DLL3 antibody according to any one of the foregoing, wherein: the DLL3-VH comprises the amino acid sequence of SEQ ID NO: 112, or a variant of SEQ ID NO: 112. In some embodiments, the variant comprises one or more amino acid substitutions in FR regions of SEQ ID NO: 112; in some embodiments, the amino acid substitutions are one or more amino acid substitutions selected from the group consisting of V5Q, A9P, K12V, A40S, P41H, 4M8I, V67A, and R83T.

In some embodiments, provided is the anti-DLL3 antibody according to any one of the foregoing, wherein: the DLL3-VH comprises the amino acid sequence of SEQ ID NO: 113, or a variant of SEQ ID NO: 113. In some embodiments, the variant comprises one or more amino acid substitutions in FR regions of SEQ ID NO: 113; in some embodiments, the amino acid substitutions are one or more amino acid substitutions selected from the group consisting of V5Q, S7F, A9P, E10V, K12V, A40S, P41H, A75S, and R83T.

In some embodiments, provided is the anti-DLL3 antibody according to any one of the foregoing, wherein: the DLL3-VH comprises the amino acid sequence of SEQ ID NO: 114, or a variant of SEQ ID NO: 114. In some embodiments, the variant comprises one or more amino acid substitutions in FR regions of SEQ ID NO: 114; in some embodiments, the amino acid substitutions are one or more amino acid substitutions selected from the group consisting of V5Q, S7F, A9P, E10V, K12V, M48I, V67A, A75S, and R83T.

In some embodiments, provided is the anti-DLL3 antibody according to any one of the foregoing, wherein: the DLL3-VH comprises the amino acid sequence of SEQ ID NO: 115, or a variant of SEQ ID NO: 115. In some embodiments, the variant comprises one or more amino acid substitutions in FR regions of SEQ ID NO: 115; in some embodiments, the amino acid substitutions are one or more amino acid substitutions selected from the group consisting of V5Q, A9P, K12V, A40S, P41H, M48I, and V67A.

In some embodiments, provided is the anti-DLL3 antibody according to any one of the foregoing, wherein: the DLL3-VL comprises the amino acid sequence of SEQ ID NO: 100 or a variant thereof. In some embodiments, the variant comprises one or more amino acid substitutions selected from the group consisting of D9K, P43S, G68A, and Y87H in FR regions of SEQ ID NO: 100.

In some embodiments, provided is the anti-DLL3 antibody according to any one of the foregoing, wherein: the DLL3-VL comprises the amino acid sequence of SEQ ID NO: 103 or a variant thereof. In some embodiments, the variant comprises one or more amino acid substitutions selected from the group consisting of A43S, S60D, and Q100S in FR regions of SEQ ID NO: 103.

In some embodiments, provided is the anti-DLL3 antibody according to any one of the foregoing, wherein:

i) the DLL3-VH comprises the amino acid sequence of SEQ ID NO: 43, 44, 45, 46, 47, 48, 49, 50, 51, 52, 53, 54, 55, 56, or 57, and the DLL3-VL comprises the amino acid sequence of SEQ ID NO: 63, 64, 65, 66, or 67; or the DLL3-VH comprises the amino acid sequence of SEQ ID NO: 56 or 57, and the DLL3-VL comprises the amino acid sequence of SEQ ID NO: 58, 59, 60, 61, 62, 63, 64, 65, 66, 67, or 68; or
ii) the DLL3-VH comprises the amino acid sequence of SEQ ID NO: 78, 79, 80, 81, or 82, and the DLL3-VL comprises the amino acid sequence of SEQ ID NO: 75, 76, or 77; or
iii) the DLL3-VH comprises the amino acid sequence of SEQ ID NO: 89, 90, 91, 92, 93, 94, or 95, and the DLL3-VL comprises the amino acid sequence of SEQ ID NO: 86, 87, or 88; or
iv) the DLL3-VH comprises the amino acid sequence of SEQ ID NO: 105, 106, 107, 108, 109, 110, 111, 112, 113, 114, 115, or 116, and the DLL3-VL comprises the amino acid sequence of SEQ ID NO: 100, 101, 102, 103, or 104.

In some embodiments, provided is the anti-DLL3 antibody according to any one of the foregoing, wherein:

the DLL3-VH comprises the amino acid sequence of SEQ ID NO: 43, 44, or 45, and the DLL3-VL comprises the amino acid sequence of SEQ ID NO: 58, 59, or 60; or
the DLL3-VH comprises the amino acid sequence of SEQ ID NO: 47, 48, 49, 53, 54, or 55, and the DLL3-VL comprises the amino acid sequence of SEQ ID NO: 59; or
the DLL3-VH comprises the amino acid sequence of SEQ ID NO: 43, 44, or 45, and the DLL3-VL comprises the amino acid sequence of SEQ ID NO: 60; or
the DLL3-VH comprises the amino acid sequence of SEQ ID NO: 46, 48, 53, 54, or 55, and the DLL3-VL comprises the amino acid sequence of SEQ ID NO: 61; or
the DLL3-VH comprises the amino acid sequence of SEQ ID NO: 54, and the DLL3-VL comprises the amino acid sequence of SEQ ID NO: 63 or 64; or
the DLL3-VH comprises the amino acid sequence of SEQ ID NO: 56 or 57, and the DLL3-VL comprises the amino acid sequence of SEQ ID NO: 65, 66, or 67.

In some embodiments, provided is the anti-DLL3 antibody according to any one of the foregoing, wherein:

the DLL3-VH comprises the amino acid sequence of SEQ ID NO: 78 or 79, and the DLL3-VL comprises the amino acid sequence of SEQ ID NO: 75, 76, or 77; or
the DLL3-VH comprises the amino acid sequence of SEQ ID NO: 78, 79, 80, 81, or 82, and the DLL3-VL comprises the amino acid sequence of SEQ ID NO: 77.

In some embodiments, provided is the anti-DLL3 antibody according to any one of the foregoing, wherein: the DLL3-VH comprises the amino acid sequence of SEQ ID NO: 89, 90, 91, 92, 93, or 94, and the DLL3-VL comprises the amino acid sequence of SEQ ID NO: 86 or 87; or the DLL3-VH comprises the amino acid sequence of SEQ ID NO: 95, and the DLL3-VL comprises the amino acid sequence of SEQ ID NO: 87.

In some embodiments, provided is the anti-DLL3 antibody according to any one of the foregoing, wherein:

the DLL3-VH comprises the amino acid sequence of SEQ ID NO: 105, 106, 107, 108, 109, 110, or 111, and the DLL3-VL comprises the amino acid sequence of SEQ ID NO: 100, 101, or 102; or
the DLL3-VH comprises the amino acid sequence of SEQ ID NO: 112, 113, 114, or 115, and the DLL3-VL comprises the amino acid sequence of SEQ ID NO: 100, 103, or 104; or
the DLL3-VH comprises the amino acid sequence of SEQ ID NO: 109, and the DLL3-VL comprises the amino acid sequence of SEQ ID NO: 103.

In some embodiments, provided is the anti-DLL3 antibody according to any one of the foregoing, wherein:

i) the DLL3-VH comprises the amino acid sequence of SEQ ID NO: 54, and the DLL3-VL comprises the amino acid sequence of SEQ ID NO: 63; or

the DLL3-VH comprises the amino acid sequence of SEQ ID NO: 54, and the DLL3-VL comprises the amino acid sequence of SEQ ID NO: 61; or
the DLL3-VH comprises the amino acid sequence of SEQ ID NO: 54, and the DLL3-VL comprises the amino acid sequence of SEQ ID NO: 64; or

the DLL3-VH comprises the amino acid sequence of SEQ ID NO: 56, and the DLL3-VL comprises the amino acid sequence of SEQ ID NO: 65; or
the DLL3-VH comprises the amino acid sequence of SEQ ID NO: 56, and the DLL3-VL comprises the amino acid sequence of SEQ ID NO: 66; or
the DLL3-VH comprises the amino acid sequence of SEQ ID NO: 56, and the DLL3-VL comprises the amino acid sequence of SEQ ID NO: 67; or
the DLL3-VH comprises the amino acid sequence of SEQ ID NO: 57, and the DLL3-VL comprises the amino acid sequence of SEQ ID NO: 65; or
the DLL3-VH comprises the amino acid sequence of SEQ ID NO: 57, and the DLL3-VL comprises the amino acid sequence of SEQ ID NO: 66; or
the DLL3-VH comprises the amino acid sequence of SEQ ID NO: 57, and the DLL3-VL comprises the amino acid sequence of SEQ ID NO: 67; or

ii) the DLL3-VH comprises the amino acid sequence of SEQ ID NO: 81, and the DLL3-VL comprises the amino acid sequence of SEQ ID NO: 77; or
iii) the DLL3-VH comprises the amino acid sequence of SEQ ID NO: 91, and the DLL3-VL comprises the amino acid sequence of SEQ ID NO: 87; or
iv) the DLL3-VH comprises the amino acid sequence of SEQ ID NO: 109, and the DLL3-VL comprises the amino acid sequence of SEQ ID NO: 100.

In some embodiments, provided is the anti-DLL3 antibody according to the foregoing, wherein:

i) the DLL3-VH comprises the amino acid sequence of SEQ ID NO: 12, and the DLL3-VL comprises the amino acid sequence of SEQ ID NO: 13; or
ii) the DLL3-VH comprises the amino acid sequence of SEQ ID NO: 14, and the DLL3-VL comprises the amino acid sequence of SEQ ID NO: 15; or
iii) the DLL3-VH comprises the amino acid sequence of SEQ ID NO: 16, and the DLL3-VL comprises the amino acid sequence of SEQ ID NO: 17; or
iv) the DLL3-VH comprises the amino acid sequence of SEQ ID NO: 18, and the DLL3-VL comprises the amino acid sequence of SEQ ID NO: 19.

In some embodiments, provided is the anti-DLL3 antibody according to any one of the foregoing, wherein:

the DLL3-VH comprises the amino acid sequence of SEQ ID NO: 54, and the DLL3-VL comprises the amino acid sequence of SEQ ID NO: 63; or
the DLL3-VH comprises the amino acid sequence of SEQ ID NO: 54, and the DLL3-VL comprises the amino acid sequence of SEQ ID NO: 61.

In some embodiments, provided is the anti-DLL3 antibody according to any one of the foregoing, wherein: the DLL3-VH comprises the amino acid sequence of SEQ ID NO: 81, and the DLL3-VL comprises the amino acid sequence of SEQ ID NO: 77.
In some embodiments, provided is the anti-DLL3 antibody according to any one of the foregoing, wherein: the DLL3-VH comprises the amino acid sequence of SEQ ID NO: 91, and the DLL3-VL comprises the amino acid sequence of SEQ ID NO: 87.
In some embodiments, provided is the anti-DLL3 antibody according to any one of the foregoing, wherein: the DLL3-VH comprises the amino acid sequence of SEQ ID NO: 109, and the DLL3-VL comprises the amino acid sequence of SEQ ID NO: 100.
In some embodiments, provided is the anti-DLL3 antibody according to any one of the foregoing, wherein the anti-DLL3 antibody is an antibody fragment.
In some embodiments, provided is the anti-DLL3 antibody according to any one of the foregoing, wherein the antibody fragment is a Fab, Fab', F(ab')2, Fd, Fv, scFv, dsFv, or dAb.
In some embodiments, provided is the anti-DLL3 antibody according to any one of the foregoing, wherein the anti-DLL3 antibody comprises a heavy chain constant region and a light chain constant region.
In some embodiments, provided is the anti-DLL3 antibody according to any one of the foregoing, wherein the heavy chain constant region is a heavy chain constant region of a human IgG.
In some embodiments, provided is the anti-DLL3 antibody according to any one of the foregoing, wherein the light chain constant region is a human κ or λ light chain constant region.
In some embodiments, provided is the anti-DLL3 antibody according to any one of the foregoing, wherein the heavy chain constant region is a heavy chain constant region of human IgG1.

In some embodiments, provided is the anti-DLL3 antibody according to any one of the foregoing, wherein the light chain constant region is a light chain constant region of human κ.

In some embodiments, provided is the anti-DLL3 antibody according to any one of the foregoing, wherein the heavy chain constant region comprises the amino acid sequence of SEQ ID NO: 41.

In some embodiments, provided is the anti-DLL3 antibody according to any one of the foregoing, wherein the light chain constant region comprises the amino acid sequence of SEQ ID NO: 42.

In some embodiments, provided is the anti-DLL3 antibody according to any one of the foregoing, wherein the anti-DLL3 antibody comprises a heavy chain and a light chain, wherein:

the heavy chain comprises an amino acid sequence having at least 85%, 86%, 87%, 88%, 89%, 90%, 91%, 92%, 93%, 94%, 95%, 96%, 97%, 98%, or 99% sequence identity to SEQ ID NO: 121, and the light chain comprises an amino acid sequence having at least 85%, 86%, 87%, 88%, 89%, 90%, 91%, 92%, 93%, 94%, 95%, 96%, 97%, 98%, or 99% sequence identity to SEQ ID NO: 122; or

the heavy chain comprises an amino acid sequence having at least 85%, 86%, 87%, 88%, 89%, 90%, 91%, 92%, 93%, 94%, 95%, 96%, 97%, 98%, or 99% sequence identity to SEQ ID NO: 123, and the light chain comprises an amino acid sequence having at least 85%, 86%, 87%, 88%, 89%, 90%, 91%, 92%, 93%, 94%, 95%, 96%, 97%, 98%, or 99% sequence identity to SEQ ID NO: 124; or

the heavy chain comprises an amino acid sequence having at least 85%, 86%, 87%, 88%, 89%, 90%, 91%, 92%, 93%, 94%, 95%, 96%, 97%, 98%, or 99% sequence identity to SEQ ID NO: 125, and the light chain comprises an amino acid sequence having at least 85%, 86%, 87%, 88%, 89%, 90%, 91%, 92%, 93%, 94%, 95%, 96%, 97%, 98%, or 99% sequence identity to SEQ ID NO: 126; or

the heavy chain comprises an amino acid sequence having at least 85%, 86%, 87%, 88%, 89%, 90%, 91%, 92%, 93%, 94%, 95%, 96%, 97%, 98%, or 99% sequence identity to SEQ ID NO: 127, and the light chain comprises an amino acid sequence having at least 85%, 86%, 87%, 88%, 89%, 90%, 91%, 92%, 93%, 94%, 95%, 96%, 97%, 98%, or 99% identity to SEQ ID NO: 128.

In some embodiments, provided is the anti-DLL3 antibody according to any one of the foregoing, wherein the anti-DLL3 antibody comprises a heavy chain and a light chain, wherein:

the heavy chain comprises the amino acid sequence of SEQ ID NO: 121, and the light chain comprises the amino acid sequence of SEQ ID NO: 122; or

the heavy chain comprises the amino acid sequence of SEQ ID NO: 123, and the light chain comprises the amino acid sequence of SEQ ID NO: 124;

the heavy chain comprises the amino acid sequence of SEQ ID NO: 125, and the light chain comprises the amino acid sequence of SEQ ID NO: 126; or

the heavy chain comprises the amino acid sequence of SEQ ID NO: 127, and the light chain comprises the amino acid sequence of SEQ ID NO: 128.

In some embodiments, provided is the anti-DLL3 antibody according to any one of the foregoing, wherein the anti-DLL3 antibody comprises a heavy chain and a light chain, wherein the heavy chain comprises the amino acid sequence of SEQ ID NO: 121, and the light chain comprises the amino acid sequence of SEQ ID NO: 122.

In some embodiments, provided is the anti-DLL3 antibody according to any one of the foregoing, wherein the anti-DLL3 antibody comprises a heavy chain and a light chain, wherein the heavy chain comprises the amino acid sequence of SEQ ID NO: 125, and the light chain comprises the amino acid sequence of SEQ ID NO: 126.

In some embodiments, provided is the anti-DLL3 antibody according to any one of the foregoing, wherein the anti-DLL3 antibody is a bispecific antibody.

In some embodiments, provided is the anti-DLL3 antibody according to any one of the foregoing, wherein the anti-DLL3 antibody is a bispecific antibody binding specifically to DLL3 and CD3.

In another aspect, the present disclosure provides a pharmaceutical composition comprising: a therapeutically effective amount of the antigen-binding molecule according to any one of the foregoing or the anti-DLL3 antibody according to any one of the foregoing, and one or more pharmaceutically acceptable carriers, diluents, buffers, or excipients. In some embodiments, the pharmaceutical composition also comprises at least one second therapeutic agent. In one example, the second therapeutic agent is any agent that is advantageously combined with a CD3/DLL3 bispecific antigen-binding molecule. In some embodiments, the second therapeutic agent is an antibody capable of binding specifically to CD28. In some embodiments, the second therapeutic agent is a bispecific antibody capable of binding specifically to CD28 and a tumor cell surface antigen (TAA). In some embodiments, the second therapeutic agent is a bispecific antibody capable of binding specifically to CD28 and DLL3.

In another aspect, the present disclosure also provides an isolated nucleic acid encoding the antigen-binding molecule according to any one of the foregoing or the anti-DLL3 antibody according to any one of the foregoing.

In another aspect, the present disclosure also provides a vector comprising the aforementioned isolated nucleic acid.

In another aspect, the present disclosure also provides a host cell comprising the aforementioned isolated nucleic acid or vector.

In another aspect, the present disclosure also provides a method for treating a tumor or cancer, the method comprising administering to a subject a therapeutically effective amount of the antigen-binding molecule according to any one of the foregoing or the anti-DLL3 antibody according to any one of the foregoing or the pharmaceutical composition according to any one of the foregoing.

In another aspect, the present disclosure also provides use of the antigen-binding molecule according to any one of the foregoing or the antibody according to any one of the foregoing or the pharmaceutical composition according to any one of the foregoing in the preparation of a medicament for treating a tumor or cancer.

In another aspect, the present disclosure also provides the antigen-binding molecule according to any one of the foregoing or the anti-DLL3 antibody according to any one of the foregoing or the pharmaceutical composition according to any one of the foregoing for use as a medicament. In some embodiments, the medicament is used for treating a tumor or cancer.

In some embodiments, the tumor or cancer according to any one of the foregoing is selected from the group consisting of: head and neck squamous cell carcinoma, head and neck cancer, brain cancer, neuroglioma, glioblastoma multiforme, neuroblastoma, central nervous system cancer, neuroendocrine tumor, throat cancer, pharyngeal squamous cell carcinoma, oral squamous cell carcinoma, nasopharyngeal cancer, esophageal cancer, thyroid cancer, malignant pleural mesothelioma, lung cancer, breast cancer, liver cancer, hepatobiliary cancer, pancreatic cancer, gastric cancer, gastrointestinal cancer, intestinal cancer, colon cancer, colorectal cancer, renal cancer, clear cell renal cell carcinoma, ovarian cancer, endometrial cancer, cervical cancer, bladder cancer, prostate cancer, testicular cancer, skin cancer, melanoma, large cell lung cancer, triple-negative breast cancer, lymphoma, and small cell lung cancer.

In some embodiments, the tumor or cancer according to the foregoing is a DLL3-expressing cancer.

In some embodiments, the tumor or cancer according to the foregoing is a solid tumor. In some embodiments, the tumor or cancer according to the foregoing is lung cancer.

In some embodiments, the tumor or cancer according to the foregoing is small cell lung cancer.

In some embodiments, the method for treating a tumor or cancer according to the foregoing further comprises the use of a second therapeutic agent. In some embodiments, the second therapeutic agent comprises an anti-tumor agent, radiotherapy, an antibody-drug conjugate, a bispecific antibody, a bispecific antibody conjugated to an anti-tumor agent, an immune checkpoint inhibitor, or a combination thereof.

In some embodiments, in the method for treating a tumor or cancer according to the foregoing, the second therapeutic agent is any agent that is advantageously combined with a CD3/DLL3 bispecific antigen-binding molecule. In some embodiments, the second therapeutic agent is an antibody capable of binding specifically to CD28. In some embodiments, the second therapeutic agent is a bispecific antibody capable of binding specifically to CD28 and a tumor cell surface antigen (TAA). In some embodiments, the second therapeutic agent is a bispecific antibody capable of binding specifically to CD28 and DLL3.

In some embodiments, in the method for treating a tumor or cancer according to the foregoing, the second therapeutic agent and the antigen-binding molecule according to any one of the present disclosure are administered simultaneously, sequentially, or separately.

The antigen-binding molecule provided by the present disclosure has the following characteristics: good therapeutic activity, safety, pharmacokinetic properties, and druggability (e.g., stability).

## BRIEF DESCRIPTION OF THE DRAWINGS

FIG. 1A shows a schematic diagram of the structure of Format1 of DLL3-CD3 bispecific antibodies, wherein Ob stands for Obscurin.
FIG. 1B shows a schematic diagram of the structure of Format2 of DLL3-CD3 bispecific antibodies.
FIG. 1C shows a schematic diagram of the structure of Format3 of DLL3-CD3 bispecific antibodies.
FIG. 2A shows experimental results for the binding of bispecific antibodies to DLL1.
The results show that the DLL3-CD3 bispecific antibodies of the present disclosure do not bind to DLL1.
FIG. 2B shows experimental results for the binding of bispecific antibodies to DLL4. The results show that the DLL3-CD3 bispecific antibodies of the present disclosure do not bind to DLL4.
FIG. 3 shows experimental results for the killing effects of bispecific antibodies on cells. The results show that the bispecific antibodies of the present disclosure have no killing effects on H460 cells not expressing DLL3.

FIG. 4A shows experimental results for the activation of T cells by bispecific antibodies, and the results show that the bispecific antibodies of the present disclosure have significant T cell activating effects in the presence of DLL3-expressing DLL3/H82 cells.

FIG. 4B shows that the bispecific antibodies of the present disclosure do not activate T cells in the presence of H460 negative cells not expressing DLL3.

FIG. 5A shows results for cytokine release experiments for bispecific antibodies, and the results show that PBMCs stimulated by the bispecific antibodies of the present disclosure secreted very low levels of IFNγ in the presence of H1184 cells.

FIG. 5B shows that PBMCs stimulated by the bispecific antibodies of the present disclosure secreted very low levels of IL-6 in the presence of H1184 cells.

## DETAILED DESCRIPTION

## Terminology

The terminology used herein is for the purpose of describing embodiments only and is not intended to be limiting. Unless defined otherwise, all technical and scientific terms used herein have the same meaning as commonly understood by one of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to". Unless otherwise specified, "comprise" includes "consist of". For example, for a DLL3-HCDR1 comprising the amino acid sequence of SEQ ID NO: 20, it specifically encompasses a DLL3-HCDR1 the amino acid sequence of which is set forth in SEQ ID NO: 20.

The three-letter and single-letter codes used in the present disclosure for amino acids are as described in J. Biol. Chem, 243, p3558 (1968).

The term "and/or", e.g., "X and/or Y" should be understood to mean "X and Y" or "X or Y" and should be used to provide explicit support for both meanings or either meaning.

As used herein, "CD3" and "CD3 fragment" refer to the well-known human CD3 protein or a fragment thereof unless specified as being from a non-human species (e.g., "mouse CD3", "mouse CD3 fragment", "monkey CD3", or "monkey CD3 fragment"). The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions, deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W. The term "antigen-binding molecule" is used in the broadest sense and encompasses a variety of molecules binding specifically to an antigen, including but not limited to antibodies, other polypeptides having antigen-binding activity, and antibody fusion proteins in which the two are fused, as long as they exhibit desired antigen-binding activity. The antigen-binding molecule herein comprises a variable region (VH) and a variable region (VL) which together comprise an antigen-binding domain. Illustratively, the antigen-binding molecules herein are bispecific antigen-binding molecules (e.g., bispecific antibodies).

The term "antibody" is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antibody fragments, or antigen-binding moieties), as long as they exhibit the desired antigen-binding activity. According to the context, those skilled can determine the specific meaning of "antibody".

"Natural antibody" refers to a naturally-occurring immunoglobulin molecule. For example, a native IgG antibody is a heterotetrameric glycoprotein of about 150,000 Daltons composed of two light chains and two heavy chains linked by a disulfide bond. From N-terminus to C-terminus, each heavy chain has one variable region (VH, also known as variable heavy domain or heavy chain variable region), followed by three constant domains (CH1, CH2, and CH3). Similarly, from N-terminus to C-terminus, each light chain has one variable region (VL, also known as variable light domain or light chain variable domain), followed by one constant light domain (light chain constant region, CL). The term "full-length antibody", "intact antibody", and "whole antibody" are used herein interchangeably and refer to an antibody having a substantially similar structure to a native antibody structure or having heavy chains comprising an Fc region as defined herein.

The term "bispecific antibody" refers to an antibody (including an antibody or an antigen-binding fragment thereof, such as a single-chain antibody) capable of binding specifically to two different antigens or at least two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies according to the number of the antigen-binding regions; the bispecific antibodies can be classified into symmetric bispecific antibodies and asymmetric bispecific antibodies according to the presence of horizontal symmetry in their structures. Among them, the bispecific antibodies based on antibody fragments, such as Fab fragments lacking Fc fragments, formed by binding 2 or more Fab fragments in one molecule, have low immunogenicity, small molecular weight and high tumor tissue permeability, and typical antibody structures of this type comprise bispecific antibodies, such as F(ab)2, scFv-Fab and (scFv)2-Fab; IgG-like bispecific antibodies (e.g., having Fc fragments) have relatively large molecular weight, and the Fc fragments facilitate later purification of the antibody and increase their solubility and stability, and the Fc fragments may further bind to the receptor FcRn and increase the serum half-life of the antibody, and typical structural models of bispecific antibodies comprise bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "variable region" or "variable domain" refers to a domain in an antigen-binding molecule that binds to an antigen. Herein, a heavy chain variable region in the antigen-binding moiety binding specifically to CD3 is denoted by CD3-VH, and a light chain variable region is denoted by CD3-VL; a heavy chain variable region in the antigen-binding moiety binding specifically to CD3 is denoted by CD3-VH, and a light chain variable region is denoted by CD3-VL. VH and VL each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Herein, the 3 CDRs in CD3-VH are denoted by CD3-HCDR1, CD3-HCDR2, and CD3-HCDR3, respectively; the 3 CDRs in CD3-VL are denoted by CD3-LCDR1, CD3-LCDR2, and CD3-LCDR3, respectively; the 3 CDRs in CD3-VH are denoted by CD3-HCDR1, CD3-HCDR2, and CD3-HCDR3, respectively; the 3 CDRs in CD3-VL are denoted by CD3-LCDR1, CD3-LCDR2, and CD3-LCDR3, respectively. Each VH and VL is, when viewed from N-terminus to C-terminus: FR1, CDR1, FR2, CDR2, FR3, CDR3, FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "Contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9: 2278), and the like. The corresponding relationships between the various numbering schemes are well known to those skilled in the art. The numbering schemes of the present disclosure are shown in Table 1 below.

Table 1. Relationships between CDR numbering schemes

| CDR | IMGT | Kabat | AbM | Chothia | Contact |
|------|--------|--------|--------|---------|---------|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs in the embodiments of the present disclosure.

The term "antibody fragment" or "antigen-binding fragment" refers to a molecule different from an intact antibody, which comprises a moiety of an intact antibody that retains the antigen-binding ability of the intact antibody. Examples of antibody fragments include, but are not limited to, Fv, Fab, Fab', Fab'-SH, F(ab')$_2$, single-domain antibodies, single-chain Fab (scFab), diabodies, linear antibodies, single-chain antibody molecules (e.g., scFv), and multispecific antibodies formed from antibody fragments. The term "Fc region" or "fragment crystallizable region" is used to define the C-terminal region of the heavy chain of an antibody, including native Fc regions and engineered Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1, IgG2 (IgG2A, IgG2B), IgG3, and IgG4. Unless otherwise indicated, the numbering scheme for the Fc region is the EU index.

The term "Titin chain" refers to a peptide fragment of 78-118 amino acids in length comprising a Titin Ig-like 152 domain in a Titin protein or a functional variant thereof, wherein the Titin chain is capable of binding to an Obscurin Ig-like 1 domain or an Obscurin-like Ig-like 1 domain to form a dimerized complex.

The term "Obscurin chain" refers to, in the Obscurin protein, a peptide fragment that is 87-117 amino acids in length and comprises an Obscurin Ig-like 1 domain, or a functional variant thereof, or to, in the Obscurin-like 1 protein, a peptide fragment that is 78-118 amino acids in length and comprises an Obscurin-like Ig-like 1 domain, or a functional variant thereof.

The Obscurin chain is capable of binding to the Titin chain to form a dimerized complex. The Titin chain and the Obscurin chain of the present disclosure can be used to replace the CH1 and CL in a Fab, respectively, to form a replaced Fab (Fab-S), and the replacement does not affect the binding of the antigen-binding molecule to an antigen.

The term "chimeric" antibody refers to an antibody in which a portion of the heavy and/or light chain is derived from a particular source or species and the remainder of the heavy and/or light chain is derived from a different source or species.

The term "humanized" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The term "affinity" refers to the overall strength of the non-covalent interaction between a single binding site of a molecule (e.g., an antibody) and its binding ligand (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. The term "kassoc" or "ka" refers to the association rate of a particular antibody-antigen interaction, while the term "kdis" or "kd" as used herein refers to the dissociation rate of a particular antibody-antigen interaction. As used herein, the term "KD" refers to the equilibrium dissociation constant, which is obtained from the ratio of kd to ka (i.e., kd/ka) and expressed as molar concentration (M). The KD value of an antibody can be measured using methods known in the art, such as surface plasmon resonance, ELISA, or solution equilibrium titration (SET).

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. In some embodiments, the antibody provided by the present disclosure is a monoclonal antibody.

The term "antigen" refers to a molecule or a portion of a molecule that is capable of being bound by a selective binding agent of an antigen-binding molecule (e.g., an antibody). An antigen may have one or more epitopes capable of interacting with different antigen-binding molecules (e.g., antibodies).

The term "epitope" refers to an area or region on an antigen that is capable of binding specifically to an antibody or an antigen-binding fragment thereof. Epitopes can be formed by contiguous amino acids (linear epitopes) or comprise non-contiguous amino acids (conformational epitopes), e.g., non-contiguous amino acids that are in spatial proximity to each other due to folding of an antigen (i.e., by tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

The term "capable of binding specifically", "binding specifically", or "binding" means that an antigen-binding molecule is capable of binding to a certain antigen or epitope with higher affinity than to other antigens or epitopes. Generally, an antibody binds to an antigen or epitope with an equilibrium dissociation constant (KD) of about $1\times10^{-7}$ M or less (e.g., about $1\times10^{-8}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by FACS or surface plasmon resonance. However, an antibody binding specifically to an antigen or an epitope thereof may have cross-reactivity to other related antigens, e.g. to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

The term "not bind" or "no binding" means that the antigen-binding molecule is not capable of binding to a certain antigen or an epitope thereof in the manner of the specific binding described above, for example, when the antibody binds to the antigen or the epitope thereof with an equilibrium dissociation constant (KD) of about $1\times10^{-6}$ M or greater.

The term "antigen-binding moiety" refers to a polypeptide molecule binding specifically to a target antigen. A specific antigen-binding moiety includes an antigen-binding domain of an antibody, e.g., comprises a heavy chain variable region and a light chain variable region. The term "antigen-binding moiety binding specifically to CD3" refers to a moiety that is capable of binding to CD3 or an epitope thereof with sufficient affinity, such that a molecule containing the moiety can be used as a diagnostic agent and/or therapeutic agent targeting CD3. For example, the antigen-binding moiety binding specifically to CD3 has the following equilibrium dissociation constant (KD): < about 10 nM, as measured by surface plasmon resonance. Antigen-binding moieties include antibody fragments as defined herein, e.g., a Fab, replaced Fab, or scFv.

The term "linker" refers to a linker unit that links two polypeptide fragments. Herein, the linkers present in the same structural formula may be identical or different. The linker may be a peptide linker comprising one or more amino acids, typically about 1-30, 2-24, or 3-15 amino acids. The linkers used herein may be identical or different. When "-" appears in a structural formula, it means that the units on both sides are directly linked by a covalent bond.

"Tm" is the temperature of dissolution and denaturation (endogenous fluorescence). When the protein is denatured (by heating or by a denaturant), the tertiary structure is opened and the aromatic amino acid microenvironment changes, resulting in a change in the emission fluorescence spectrum. In the present disclosure, Tm1 refers to the temperature at which the fluorescence value changes to half of the maximum value. "Tonset" is the onset temperature of denaturation. It refers to the temperature at which the protein begins to denature, i.e., the temperature at which the fluorescence value begins to change.

"Tagg" is the onset temperature of aggregation. The temperature at which the sample begins to aggregate is monitored by detecting aggregates at two wavelengths of 266 nm and 473 nm by static light scattering. Tagg 266 refers to the onset temperature of aggregation monitored at 266 nm.

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding the antigen-binding molecule refers to one or more nucleic acid molecules encoding antibody heavy and light chains (or fragments thereof), including such nucleic acid molecule(s) in a single vector or separate vectors, and such nucleic acid molecule(s) present at one or more locations in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with degenerate bases and/or deoxyinosine residues.

It should be understood that although specific coding nucleotide sequences are not provided in the present disclosure, those skilled can determine the corresponding coding nucleotide sequences based on amino acid sequences according to codon rules and host codon preferences.

The terms "polypeptide" and "protein" are used interchangeably herein and refer to a polymer of amino acid residues. The terms apply to amino acid polymers in which one or more amino acid residues are corresponding artificial chemical mimics of naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term "sequence identity" refers to the degree (percentage) to which the amino acids/nucleic acids of two sequences are identical at equivalent positions when the two sequences are optimally aligned. In the alignment process, gaps may be allowed to be introduced, if necessary, to achieve the maximum percent sequence identity, but any conservative substitution is not considered a part that constitutes sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "fusion" or "linkage" means that components (e.g., antigen-binding moieties and Fc domains) are covalently linked directly or via a linker.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that is applied to transform a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be completely identical to parent cells in terms of nucleic acid content and may contain mutations. As used herein, the term includes mutant progenies that have the same function or biological activity as the cells screened or selected from the primary transformed cells. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens*, *Pichia minuta (Ogataea minuta, Pichia lindneri) Pichiaopuntiae, Pichia thermotolerans, Pichia salictaria, Pichia guercuum, Pichia pijperi*, *Pichia stiptis, Pichia methanolica,* Pichia, *Saccharomycescerevisiae,* Saccharomyces, *Hansenula polymorpha,* Kluyveromyces, *Kluyveromyces lactis, Candida albicans, Aspergillus nidulans, Aspergillus niger, Aspergillus oryzae, Trichoderma reesei, Chrysosporium lucknowense, Fusarium* sp., *Fusarium gramineum, Fusarium venenatum, Physcomitrella patens,* and *Neurospora crassa.* Pichia, any Saccharomyces, *Hansenula polymorpha,* any Kluyveromyces, *Candida albicans,* any Aspergillus, *Trichoderma reesei, Chrysosporium lucknowense,* any Fusarium, *Yarrowia lipolytica,* and *Neurospora crassa.* The host cell of the present patent does not include objects not authorized by the Patent Laws.

"Optional" or "optionally" means that the event or circumstance subsequently described may, but does not necessarily, occur, and this description includes instances where the event or circumstance occurs or does not occur.

The term "pharmaceutical composition" refers to a mixture comprising one or more of the antigen-binding molecules or antibodies described herein and other chemical components; the other components are, for example, physiological/pharmaceutically acceptable carriers and excipients.

The term "pharmaceutically acceptable carrier, diluent, buffer, or excipient" refers to an ingredient in a pharmaceutical formulation that is different from the active ingredient and is not toxic to the subject. Pharmaceutically acceptable carriers, diluents, buffers, or excipients include, but are not limited to, buffers, excipients, stabilizers, or preservatives.

The term "subject" or "individual" includes humans and non-human animals. Non-human animals include all vertebrates (e.g., mammals and non-mammals) such as non-human primates (e.g., cynomolgus monkeys), sheep, dogs, cows, chickens, amphibians, and reptiles. Unless clearly indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells or tissues isolated from a subject, as well as fluids, cells or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

"Treatment" or "treat" (and grammatical variations thereof) refers to clinical intervention intended to be applied to the treated individual, which may be performed either for prophylactic purposes or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antigen-binding molecule of the present disclosure is used to delay the development of or slow the progression of disease.

The terms "recurrence", "relapse", or "relapsed" refer to the return of a cancer or disease after clinical assessment of the disappearance of disease. A diagnosis of distant cancer metastasis or local recurrence can be considered a relapse.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage caused by or associated with a disease state. In some examples, the effective amount is a therapeutically effective amount or a prophylactically effective amount.

"Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way.

"Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a subject.

**Antigen-Binding Molecules of the Present Disclosure**

The present disclosure provides an antigen-binding molecule having a number of advantageous properties, such as good *in vitro* killing activity, therapeutic activity, safety, pharmacokinetic properties, and druggability (e.g., yield, purity, and stability).

**Exemplary Antigen-Binding Molecules**

The antigen-binding molecule of the present disclosure includes bispecific antigen-binding molecules (e.g., bispecific antibodies) binding specifically to DLL3 and CD3 and anti-DLL3 antibodies. Particularly, the antigen-binding molecule of the present disclosure has any one of the following properties:

a. A high affinity for DLL3. In some embodiments, the anti-DLL3 antibody or antigen-binding molecule binds to human DLL3 with a KD of less than $4 \times 10^{-9}$ M, $3 \times 10^{-9}$ M, $2 \times 10^{-9}$ M, $1 \times 10^{-9}$ M, $9 \times 10^{-10}$ M, $8 \times 10^{-10}$ M, $7 \times 10^{-10}$ M, $6 \times 10^{-10}$ M, $5 \times 10^{-10}$ M, $4 \times 10^{-10}$ M, $3 \times 10^{-10}$ M, $2 \times 10^{-10}$ M, $1 \times 10^{-10}$ M, or $9 \times 10^{-11}$ M, as measured by surface plasmon resonance. In some embodiments, the antigen-binding molecule binds to DLL3 protein with an EC50 of less than 0.5 nM, 0.4 nM, 0.3 nM, 0.2 nM, 0.1 nM, 0.09 nM, 0.08 nM, or 0.07 nM, as determined by ELISA.

b. A high affinity for DLL3 expressed on cell surfaces. In some embodiments, the anti-DLL3 antibody binds to H1184 cells with an EC50 of less than 0.2 nM, 0.1 nM, 0.09 nM, 0.08 nM, 0.07 nM, 0.06 nM, 0.05 nM, or 0.04 nM.

c. A specific killing effect on DLL3-expressing cells. In some embodiments, the antigen-binding molecule may specifically kill DLL3-expressing SHP77 cells and H1184 cells, wherein the killing IC50 against H1184 cells is less than 30 pM, 25 pM, 20 pM, 15 pM, 14 pM, 13 pM, 12 pM, 11 pM, 10 pM, 9 pM, 8 pM, or 7 pM.

d. *In vitro* activating activity for T cells. In some embodiments, the antigen-binding molecule activates Jurkat Lucia™ NFAT cells with an EC50 of less than 20 pM, 19 pM, 18 pM, 17 pM, 16 pM, 15 pM, 14 pM, 13 pM, 12 pM, 11 pM, 10 pM, 9 pM, 8 pM, or 7 pM.

e. Induction of low-level release of cytokines (IL6 and IFNγ).

e. Stronger *in vivo* therapeutic activity. In some embodiments, the antigen-binding molecule may significantly inhibit the growth of subcutaneous xenograft tumors of SHP77 and H1184 cells in mice.

The present disclosure provides an antigen-binding molecule comprising at least one antigen-binding moiety binding specifically to DLL3 and at least one antigen-binding moiety binding specifically to CD3; the antigen-binding moiety binding specifically to DLL3 comprises a DLL3-VH and a DLL3-VL, and the antigen-binding moiety binding specifically to CD3 comprises a CD3-VH and a CD3-VL. The present disclosure also provides an anti-DLL3 antibody capable of binding specifically to DLL3, the antibody comprising a DLL3-VH and a DLL3-VL. Specifically, the examples herein disclose antibody series 6, 98, 110, and 30. Taking antibodies 6 and 110 as examples, the antibodies or antigen-binding molecules herein are described below.

Provided is an antigen-binding molecule binding specifically to DLL3 and CD3, wherein:

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22;

and the DLL3-VL comprises: a DLL3-LCDR1 comprising the amino acid sequence of SEQ ID NO: 23, a DLL3-LCDR2 comprising the amino acid sequence of SEQ ID NO: 24, 71, 72, or 73, and a DLL3-LCDR3 comprising the amino acid sequence of SEQ ID NO: 25 or 74.

Provided is an antigen-binding molecule binding specifically to DLL3 and CD3 or an anti-DLL3 antibody, wherein: the DLL3-VH comprises: a DLL3-HCDR1 comprising the amino acid sequence of SEQ ID NO: 20, a DLL3-HCDR2 comprising the amino acid sequence of SEQ ID NO: 32, 96, 97, 98, or 99, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36.

Provided is an antigen-binding molecule binding specifically to DLL3 and CD3, wherein: the DLL3-VH comprises a DLL3-HCDR1 set forth in SEQ ID NO: 20, a DLL3-HCDR2 set forth in SEQ ID NO: 21, 69, or 70, and a DLL3-HCDR3 set forth in SEQ ID NO: 22, and the DLL3-VL comprises a DLL3-LCDR1 set forth in SEQ ID NO: 23, a DLL3-LCDR2 set forth in SEQ ID NO: 24, 71, 72, or 73, and a DLL3-LCDR3 set forth in SEQ ID NO: 25 or 74.

Provided is an antigen-binding molecule binding specifically to DLL3 and CD3 or an anti-DLL3 antibody, wherein: the DLL3-VH comprises a DLL3-HCDR1 set forth in SEQ ID NO: 20, a DLL3-HCDR2 set forth in SEQ ID NO: 32, 96, 97, 98, or 99, and a DLL3-HCDR3 set forth in SEQ ID NO: 33, and the DLL3-VL comprises a DLL3-LCDR1 set forth in SEQ ID NO: 34, a DLL3-LCDR2 set forth in SEQ ID NO: 35, and a DLL3-LCDR3 set forth in SEQ ID NO: 36.

Provided is an antigen-binding molecule binding specifically to DLL3 and CD3, wherein: the DLL3-VH comprises a DLL3-HCDR1 set forth in SEQ ID NO: 20, a DLL3-HCDR2 set forth in SEQ ID NO: 21, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 71, and a DLL3-LCDR3 set forth in SEQ ID NO: 25.

Provided is an antigen-binding molecule binding specifically to DLL3 and CD3 or an anti-DLL3 antibody, wherein:

the DLL3-VH comprises a DLL3-HCDR1 set forth in SEQ ID NO: 20, a DLL3-HCDR2 set forth in SEQ ID NO: 21, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 24, and a DLL3-LCDR3 set forth in SEQ ID NO: 25; or

the DLL3-VH comprises a DLL3-HCDR1 set forth in SEQ ID NO: 20, a DLL3-HCDR2 set forth in SEQ ID NO: 21, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 72, and a DLL3-LCDR3 set forth in SEQ ID NO: 25; or the DLL3-VH comprises a DLL3-HCDR1 set forth in SEQ ID NO: 20, a DLL3-HCDR2 set forth in SEQ ID NO: 69, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 71, and a DLL3-LCDR3 set forth in SEQ ID NO: 74; or the DLL3-VH comprises a DLL3-HCDR1 set forth in SEQ ID NO: 20, a DLL3-HCDR2 set forth in SEQ ID NO: 69, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 24, and a DLL3-LCDR3 set forth in SEQ ID NO: 74; or the DLL3-VH comprises a DLL3-HCDR1 set forth in SEQ ID NO: 20, a DLL3-HCDR2 set forth in SEQ ID NO: 69, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 71, and a DLL3-LCDR3 set forth in SEQ ID NO: 25; or the DLL3-VH comprises a DLL3-HCDR1 set forth in SEQ ID NO: 20, a DLL3-HCDR2 set forth in SEQ ID NO: 70, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 71, and a DLL3-LCDR3 set forth in SEQ ID NO: 25; or the DLL3-VH comprises a DLL3-HCDR1 set forth in SEQ ID NO: 20, a DLL3-HCDR2 set forth in SEQ ID NO: 70, and a DLL3-HCDR3 set forth in SEQ ID NO: 22; and the DLL3-VL comprises a DLL3-LCDR1 set forth in SEQ ID NO: 23, a DLL3-LCDR2 set forth in SEQ ID NO: 24, and a DLL3-LCDR3 set forth in SEQ ID NO: 74.

Provided is an antigen-binding molecule binding specifically to DLL3 and CD3 or an anti-DLL3 antibody, wherein: the DLL3-VH comprises a DLL3-HCDR1 set forth in SEQ ID NO: 20, a DLL3-HCDR2 set forth in SEQ ID NO: 96, and a DLL3-HCDR3 set forth in SEQ ID NO: 33; and the DLL3-VL comprises a DLL3-LCDR1 set forth in SEQ ID NO: 34, a DLL3-LCDR2 set forth in SEQ ID NO: 35, and a DLL3-LCDR3 set forth in SEQ ID NO: 36.

In some embodiments, in the antigen-binding molecule or the anti-DLL3 antibody according to the foregoing, the DLL3-VH and/or the DLL3-VL are/is murine or humanized. In some embodiments, the DLL3-VH and/or the DLL3-VL are/is humanized.

In some embodiments, the FR1, FR2, FR3, and FR4 of the humanized DLL3-VH have at least 60%, 70%, 80%, or 90% sequence identity to the FR1, FR2, FR3, and FR4 of SEQ ID NO: 50, 51, or 52; the FR1, FR2, FR3, and FR4 of the humanized DLL3-VL have at least 60%, 70%, 80%, or 90% sequence identity to the FR1, FR2, FR3, and FR4 of SEQ ID NO: 62 or 68.

In some embodiments, the humanized DLL3-VH comprises a FR1, a FR2, and a FR3 derived from IGHV1-3*01, IGHV7-4-1*02, or IGHV3-73*01 and a FR4 derived from IGHJ6*01, and is unsubstituted or has one or more amino acid substitutions selected from the group consisting of 1E, 5Q, 27Y, 30T, 38K, 43K, 48I, 67A, 68A, 69L, 71V, 73K, 75S, 76N, and 93A; and/or the humanized DLL3-VL comprises a FR1, a FR2, and a FR3 derived from IGKV1-16*01 or IGKV3-20*02 and a FR4 derived from IGKJ4*01, and is unsubstituted or has one or more amino acid substitutions selected from the group consisting of 1N, 36L, 42K, 43S, 44F, 46G, 69A, 71Y, 79Q, and 85D. In some embodiments, the amino acid positions in the above variable regions are defined according to the Kabat numbering scheme.

In some embodiments, the humanized DLL3-VH comprises the amino acid sequence of SEQ ID NO: 50 or a variant thereof. In some embodiments, the variant comprises one or more amino acid substitutions selected from the group consisting of 1E, 43K, 48I, 67A, 69L, 71V, 73K, and 76N in FR regions of SEQ ID NO: 50.

In some embodiments, the humanized DLL3-VH comprises the amino acid sequence of SEQ ID NO: 51 or a variant thereof. In some embodiments, the variant comprises one or more amino acid substitutions selected from the group consisting of 1E, 5Q, 38K, 68A, 69L, 71V, 73K, 75S, and 76N in FR regions of SEQ ID NO: 51.

In some embodiments, the humanized DLL3-VH comprises the amino acid sequence of SEQ ID NO: 52 or a variant thereof. In some embodiments, the variant comprises one or more amino acid substitutions selected from the group consisting of 27Y, 30T, 69L, 71V, 73K, and 93A in FR regions of SEQ ID NO: 52.

In some embodiments, the humanized DLL3-VL comprises the amino acid sequence of SEQ ID NO: 62 or a variant thereof. In some embodiments, the variant comprises one or more amino acid substitutions selected from the group consisting of 36L, 43S, 44F, 46G, 69A, 71Y, and 85D in FR regions of SEQ ID NO: 62.

In some embodiments, the humanized DLL3-VL comprises the amino acid sequence of SEQ ID NO: 68 or a variant thereof. In some embodiments, the variant comprises one or more amino acid substitutions selected from the group consisting of 36L, 42K, 46G, 69A, 71Y, 79Q, and 85D in FR regions of SEQ ID NO: 68.

In some embodiments, the FR1, FR2, FR3, and FR4 of the humanized DLL3-VH have at least 60%, 70%, 80%, or 90% sequence identity to the FR1, FR2, FR3, and FR4 of SEQ ID NO: 95; the FR1, FR2, FR3, and FR4 of the humanized DLL3-VL have at least 60%, 70%, 80%, or 90% sequence identity to the FR1, FR2, FR3, and FR4 of SEQ ID NO: 88.

In some embodiments, the humanized DLL3-VH comprises a FR1, a FR2, and a FR3 derived from IGHV1-3*01 and a FR4 derived from IGHJ6*01, and is unsubstituted or has one or more amino acid substitutions selected from the group consisting of 1E, 24T, 44S, 71V, and 73K; and/or the humanized DLL3-VL comprises a FR1, a FR2, and a FR3 derived from IGKV4-1*01 and a FR4 derived from IGKJ2*01, and is unsubstituted or has one or more amino acid substitutions selected from the group consisting of 1N, 9K, 43S, 68A, and 85D. In some embodiments, the amino acid

**EP 4 545 564 A1**

positions in the above variable regions are defined according to the Kabat numbering scheme.

In some embodiments, the humanized DLL3-VH comprises the amino acid sequence of SEQ ID NO: 95 or a variant thereof. In some embodiments, the variant comprises one or more amino acid substitutions selected from the group consisting of 1E, 24T, 44S, 71V, and 73K in FR regions of SEQ ID NO: 95. In some embodiments, the humanized DLL3-VL comprises the amino acid sequence of SEQ ID NO: 88 or a variant thereof. In some embodiments, the variant comprises one or more amino acid substitutions selected from the group consisting of 1N, 9K, 43S, 68A, and 85D in FR regions of SEQ ID NO: 88.

In some embodiments, the humanized DLL3-VH comprises the amino acid sequence of SEQ ID NO: 51 or a variant thereof; in some embodiments, the variant comprises one or more amino acid substitutions selected from the group consisting of 1E, 38K, 68A, 69L, 71V, 73K, 75S, and 76N in FR regions of SEQ ID NO: 51; and the humanized DLL3-VL comprises the amino acid sequence of SEQ ID NO: 63 or a variant thereof; in some embodiments, the variant comprises one or more amino acid substitutions in FR regions of SEQ ID NO: 63; in some embodiments, the amino acid substitutions are one or more amino acid substitutions selected from the group consisting of 43 S and 44F.

In some embodiments, the humanized DLL3-VH comprises the amino acid sequence of SEQ ID NO: 91 or a variant of SEQ ID NO: 91; in some embodiments, the variant comprises one or more amino acid substitutions in FR regions of SEQ ID NO: 91; and the humanized DLL3-VL comprises the amino acid sequence of SEQ ID NO: 88 or a variant thereof; in some embodiments, the variant comprises one or more amino acid substitutions selected from the group consisting of 1N, 9K, 43S, 68A, and 85D in FR regions of SEQ ID NO: 88.

In some embodiments, the variable regions and CDRs are defined according to the Kabat numbering scheme.

In some embodiments, provided is the antigen-binding molecule according to any one of the foregoing, wherein the amino acid sequence of the DLL3-VH has at least 90%, 95%, 96%, 97%, 98%, or 99% sequence identity to SEQ ID NO: 12, 43, 44, 45, 46, 47, 48, 49, 50, 51, 52, 53, 54, 55, 56, or 57, and the amino acid sequence of the DLL3-VL has at least 90%, 95%, 96%, 97%, 98%, or 99% sequence identity to SEQ ID NO: 13, 58, 59, 60, 61, 62, 63, 64, 65, 66, 67, or 68. In some embodiments, the amino acid sequence of the DLL3-VH is set forth in SEQ ID NO: 12, 43, 44, 45, 46, 47, 48, 49, 50, 51, 52, 53, 54, 55, 56, or 57, and the amino acid sequence of the DLL3-VL is set forth in SEQ ID NO: 13, 58, 59, 60, 61, 62, 63, 64, 65, 66, 67, or 68.

In some embodiments, provided is the anti-DLL3 antibody according to any one of the foregoing, wherein the amino acid sequence of the DLL3-VH has at least 90%, 95%, 96%, 97%, 98%, or 99% sequence identity to SEQ ID NO: 43, 44, 45, 46, 47, 48, 49, 50, 51, 52, 53, 54, 55, 56, or 57, and the amino acid sequence of the DLL3-VL has at least 90%, 95%, 96%, 97%, 98%, or 99% sequence identity to SEQ ID NO: 63, 64, 65, 66, or 67. In some embodiments, the amino acid sequence of the DLL3-VH is set forth in SEQ ID NO: 43, 44, 45, 46, 47, 48, 49, 50, 51, 52, 53, 54, 55, 56, or 57, and the amino acid sequence of the DLL3-VL is set forth in SEQ ID NO: 63, 64, 65, 66, or 67. In some embodiments, provided is the anti-DLL3 antibody according to any one of the foregoing, wherein the amino acid sequence of the DLL3-VH has at least 90%, 95%, 96%, 97%, 98%, or 99% sequence identity to SEQ ID NO: 56 or 57, and the amino acid sequence of the DLL3-VL has at least 90%, 95%, 96%, 97%, 98%, or 99% sequence identity to SEQ ID NO: 58, 59, 60, 61, 62, 63, 64, 65, 66, 67, or 68. In some embodiments, the amino acid sequence of the DLL3-VH is set forth in SEQ ID NO: 56 or 57, and the amino acid sequence of the DLL3-VL is set forth in SEQ ID NO: 58, 59, 60, 61, 62, 63, 64, 65, 66, 67, or 68.

In some embodiments, in the antigen-binding molecule or the anti-DLL3 antibody according to any one of the foregoing, the amino acid sequence of the DLL3-VH is set forth in SEQ ID NO: 54, and the amino acid sequence of the DLL3-VL is set forth in SEQ ID NO: 63; or

the amino acid sequence of the DLL3-VH is set forth in SEQ ID NO: 54, and the amino acid sequence of the DLL3-VL is set forth in SEQ ID NO: 61; or
the amino acid sequence of the DLL3-VH is set forth in SEQ ID NO: 54, and the amino acid sequence of the DLL3-VL is set forth in SEQ ID NO: 64; or
the amino acid sequence of the DLL3-VH is set forth in SEQ ID NO: 56, and the amino acid sequence of the DLL3-VL is set forth in SEQ ID NO: 65; or
the amino acid sequence of the DLL3-VH is set forth in SEQ ID NO: 56, and the amino acid sequence of the DLL3-VL is set forth in SEQ ID NO: 67; or
the amino acid sequence of the DLL3-VH is set forth in SEQ ID NO: 56, and the amino acid sequence of the DLL3-VL is set forth in SEQ ID NO: 65; or
the amino acid sequence of the DLL3-VH is set forth in SEQ ID NO: 57, and the amino acid sequence of the DLL3-VL is set forth in SEQ ID NO: 65; or
the amino acid sequence of the DLL3-VH is set forth in SEQ ID NO: 57, and the amino acid sequence of the DLL3-VL is set forth in SEQ ID NO: 67; or
the amino acid sequence of the DLL3-VH is set forth in SEQ ID NO: 57, and the amino acid sequence of the DLL3-VL is set forth in SEQ ID NO: 65.

In some embodiments, provided is the antigen-binding molecule or the anti-DLL3 antibody according to any one of the foregoing, wherein the amino acid sequence of the DLL3-VH has at least 90%, 95%, 96%, 97%, 98%, or 99% sequence identity to SEQ ID NO: 16, 89, 90, 91, 92, 93, 94, or 95, and the amino acid sequence of the DLL3-VL has at least 90%, 95%, 96%, 97%, 98%, or 99% sequence identity to SEQ ID NO: 17, 86, 87, or 88. In some embodiments, the amino acid sequence of the DLL3-VH is set forth in SEQ ID NO: 16, 89, 90, 91, 92, 93, 94, or 95, and the amino acid sequence of the DLL3-VL is set forth in SEQ ID NO: 17, 86, 87, or 88.

In some embodiments, in the antigen-binding molecule or the anti-DLL3 antibody according to any one of the foregoing, the amino acid sequence of the DLL3-VH is set forth in SEQ ID NO: 91, and the amino acid sequence of the DLL3-VL is set forth in SEQ ID NO: 87.

In some embodiments, the anti-DLL3 antibody according to any one of the foregoing comprises a heavy chain and a light chain, wherein:

the amino acid sequence of the heavy chain is set forth in SEQ ID NO: 121, and the amino acid sequence of the light chain is set forth in SEQ ID NO: 122; or
the amino acid sequence of the heavy chain is set forth in SEQ ID NO: 125, and the amino acid sequence of the light chain is set forth in SEQ ID NO: 126.

In some embodiments, provided is the antigen-binding molecule according to the above, wherein:

i) the CD3-VH comprises a CD3-HCDR1 set forth in SEQ ID NO: 129, a CD3-HCDR2 set forth in SEQ ID NO: 130, and a CD3-HCDR3 set forth in SEQ ID NO: 131; and the CD3-VL comprises a CD3-LCDR1 set forth in SEQ ID NO: 132, a CD3-LCDR2 set forth in SEQ ID NO: 133, and a CD3-LCDR3 set forth in SEQ ID NO: 134; or
ii) the CD3-VH comprises a CD3-HCDR1 set forth in SEQ ID NO: 129, a CD3-HCDR2 set forth in SEQ ID NO: 130, and a CD3-HCDR3 set forth in SEQ ID NO: 135; and the CD3-VL comprises a CD3-LCDR1 set forth in SEQ ID NO: 132, a CD3-LCDR2 set forth in SEQ ID NO: 133, and a CD3-LCDR3 set forth in SEQ ID NO: 134.

In some embodiments, the variable regions and CDRs are defined according to the Kabat numbering scheme.

In some embodiments, provided is the antigen-binding molecule according to any one of the foregoing, wherein the CD3-VH and/or the CD3-VL are/is murine or humanized. In some embodiments, the CD3-VH and/or the CD3-VL are/is humanized.

In some embodiments, provided is the antigen-binding molecule according to any one of the foregoing, wherein the amino acid sequence of the CD3-VH is set forth in SEQ ID NO: 136 or 138, and the amino acid sequence of the CD3-VL is set forth in SEQ ID NO: 137.

In some embodiments, the antigen-binding molecule according to any one of the foregoing comprises:

a first chain set forth in SEQ ID NO: 152, a second chain set forth in SEQ ID NO: 153, a third chain set forth in SEQ ID NO: 154, and a fourth chain set forth in SEQ ID NO: 155; or
a first chain set forth in SEQ ID NO: 152, a second chain set forth in SEQ ID NO: 153, a third chain set forth in SEQ ID NO: 156, and a fourth chain set forth in SEQ ID NO: 155.

In some embodiments, the antigen-binding molecule according to any one of the foregoing comprises:

a first chain set forth in SEQ ID NO: 157, a second chain set forth in SEQ ID NO: 158, and a third chain set forth in SEQ ID NO: 159; or
a first chain set forth in SEQ ID NO: 157, a second chain set forth in SEQ ID NO: 158, and a third chain set forth in SEQ ID NO: 160.

In some embodiments, the antigen-binding molecule according to any one of the foregoing comprises:

a first chain set forth in SEQ ID NO: 161 and a second chain set forth in SEQ ID NO: 162; or
a first chain set forth in SEQ ID NO: 163 and a second chain set forth in SEQ ID NO: 162; or
a first chain set forth in SEQ ID NO: 185 and a second chain set forth in SEQ ID NO: 162; or
a first chain set forth in SEQ ID NO: 186 and a second chain set forth in SEQ ID NO: 162; or
a first chain set forth in SEQ ID NO: 259 and a second chain set forth in SEQ ID NO: 162; or
a first chain set forth in SEQ ID NO: 260 and a second chain set forth in SEQ ID NO: 162; or
a first chain set forth in SEQ ID NO: 187 and a second chain set forth in SEQ ID NO: 188; or
a first chain set forth in SEQ ID NO: 187 and a second chain set forth in SEQ ID NO: 189; or
a first chain set forth in SEQ ID NO: 190 and a second chain set forth in SEQ ID NO: 188; or

a first chain set forth in SEQ ID NO: 190 and a second chain set forth in SEQ ID NO: 189; or
a first chain set forth in SEQ ID NO: 191 and a second chain set forth in SEQ ID NO: 188; or
a first chain set forth in SEQ ID NO: 191 and a second chain set forth in SEQ ID NO: 189.

In some embodiments, the antigen-binding molecule according to any one of the foregoing comprises:

a first chain set forth in SEQ ID NO: 171, a second chain set forth in SEQ ID NO: 172, a third chain set forth in SEQ ID NO: 154, and a fourth chain set forth in SEQ ID NO: 155; or
a first chain set forth in SEQ ID NO: 171, a second chain set forth in SEQ ID NO: 172, a third chain set forth in SEQ ID NO: 156, and a fourth chain set forth in SEQ ID NO: 155.

In some embodiments, the antigen-binding molecule according to any one of the foregoing comprises:

a first chain set forth in SEQ ID NO: 173, a second chain set forth in SEQ ID NO: 174, and a third chain set forth in SEQ ID NO: 159; or
a first chain set forth in SEQ ID NO: 173, a second chain set forth in SEQ ID NO: 174, and a third chain set forth in SEQ ID NO: 160.

In some embodiments, the antigen-binding molecule according to any one of the foregoing comprises:

a first chain set forth in SEQ ID NO: 175 and a second chain set forth in SEQ ID NO: 176; or
a first chain set forth in SEQ ID NO: 177 and a second chain set forth in SEQ ID NO: 176.

The antigen-binding molecules comprising antibody series 98 and 30 disclosed according to the examples herein have a technical solution scope equivalent to that of the antibody series 6 and 110 described above.

## Structure of Antigen-Binding Molecule

The bispecific antigen-binding molecule of the present disclosure is not limited to a particular molecular structure, as long as it has the desired antigen-binding function. For example, the bispecific antigen-binding molecule herein may be bivalent (1 + 1), trivalent (2 + 1), or tetravalent (2 + 2). The antigen-binding moieties in the antigen-binding molecule may be any antibody fragments having antigen-binding activity that are fused via a peptide linker. The peptide linkers of the present disclosure (e.g., linkers 1 to 8) can be any suitable peptide chains, as long as the antigen-binding molecules are capable of exhibiting the desired antigen-binding activity. For example, the peptide linkers may be flexible peptides comprising 1-50 or 3-20 amino acid residues. In some embodiments, the peptide linkers each independently have a structure of (GGGGS)n, wherein n is 1, 2, or 3. In some embodiments, the sequences of the linker 1 and linker 2 are both GGGGS (SEQ ID NO: 147). In some embodiments, the sequence of the linker 3 is GGGGSGGGGSGGGGS (SEQ ID NO: 148). In some embodiments, the sequence of the linker 4 is GGGGS (SEQ ID NO: 147). In some embodiments, the sequence of the linker 5 is GGGSGGGG (SEQ ID NO: 149). In some embodiments, the sequences of linker 6 and linker 8 are G (SEQ ID NO: 150). In some embodiments, the sequence of linker 7 is GGGGSGGGG (SEQ ID NO: 151). Illustratively, the antigen-binding molecule of the present disclosure comprises one first chain having a structure represented by formula (a-1), one second chain having a structure represented by formula (b-1), one third chain having a structure represented by formula (c), and one fourth chain having a structure represented by formula (d):

(a-1) [DLL3-VH]-[GGGGS]-[Titin chain]-[Fc2],
(b-1) [DLL3-VL]-[GGGGS]-[Obscurin chain],
(c) [CD3-VH]-[CH1]-[Fc1], and
(d) [CD3-VL]-[CL],
the structures represented by formulas (a-1), (b-1), (c), and (d) are arranged from N-terminus to C-terminus.

An exemplary antigen-binding molecule comprises:

one first chain set forth in SEQ ID NO: 171, one second chain set forth in SEQ ID NO: 172, one third chain set forth in SEQ ID NO: 154, and one fourth chain set forth in SEQ ID NO: 155; or
one first chain set forth in SEQ ID NO: 171, one second chain set forth in SEQ ID NO: 172, one third chain set forth in SEQ ID NO: 156, and one fourth chain set forth in SEQ ID NO: 155; or
one first chain set forth in SEQ ID NO: 152, one second chain set forth in SEQ ID NO: 153, one third chain set forth in SEQ ID NO: 154, and one fourth chain set forth in SEQ ID NO: 155; or

EP 4 545 564 A1

one first chain set forth in SEQ ID NO: 152, one second chain set forth in SEQ ID NO: 153, one third chain set forth in SEQ ID NO: 156, and one fourth chain set forth in SEQ ID NO: 155; or
one first chain set forth in SEQ ID NO: 164, one second chain set forth in SEQ ID NO: 165, one third chain set forth in SEQ ID NO: 154, and one fourth chain set forth in SEQ ID NO: 155; or
one first chain set forth in SEQ ID NO: 164, one second chain set forth in SEQ ID NO: 165, one third chain set forth in SEQ ID NO: 156, and one fourth chain set forth in SEQ ID NO: 155; or
one first chain set forth in SEQ ID NO: 178, one second chain set forth in SEQ ID NO: 179, one third chain set forth in SEQ ID NO: 154, and one fourth chain set forth in SEQ ID NO: 155; or
one first chain set forth in SEQ ID NO: 178, one second chain set forth in SEQ ID NO: 179, one third chain set forth in SEQ ID NO: 156, and one fourth chain set forth in SEQ ID NO: 155.

Illustratively, the antigen-binding molecule comprises one first chain having a structure represented by formula (e), one second chain having a structure represented by formula (f), and one third chain having a structure represented by formula (g-1):

(e) [DLL3-VL]-[CL],
(f) [DLL3-VL]-[CH1]-[Fc2], and
(g-1) [CD3-VH]-[GGGGSGGGGSGGGGS]-[CD3-VL]-[GGGGS]-[Fc1];
the structures represented by formulas (e), (f), and (g-1) are arranged from N-terminus to C-terminus.

An exemplary antigen-binding molecule comprises:

one first chain set forth in SEQ ID NO: 173, one second chain set forth in SEQ ID NO: 174, and one third chain set forth in SEQ ID NO: 160; or
one first chain set forth in SEQ ID NO: 173, one second chain set forth in SEQ ID NO: 174, and one third chain set forth in SEQ ID NO: 159; or
one first chain set forth in SEQ ID NO: 157, one second chain set forth in SEQ ID NO: 158, and one third chain set forth in SEQ ID NO: 159; or
one first chain set forth in SEQ ID NO: 157, one second chain set forth in SEQ ID NO: 158, and one third chain set forth in SEQ ID NO: 160; or
one first chain set forth in SEQ ID NO: 166, one second chain set forth in SEQ ID NO: 167, and one third chain set forth in SEQ ID NO: 159; or
one first chain set forth in SEQ ID NO: 166, one second chain set forth in SEQ ID NO: 167, and one third chain set forth in SEQ ID NO: 160; or
one first chain set forth in SEQ ID NO: 180, one second chain set forth in SEQ ID NO: 181, and one third chain set forth in SEQ ID NO: 159; or
one first chain set forth in SEQ ID NO: 180, one second chain set forth in SEQ ID NO: 181, and one third chain set forth in SEQ ID NO: 160.

Illustratively, the antigen-binding molecule comprises one first chain having a structure represented by formula (i-1) and one second chain having a structure represented by formula (h-1):

(h-1) [DLL3-VL]-[GGGSGGGG]-[CD3-VH]-[G]-[Fc1], and
(i-1) [CD3-VL]-[GGGGSGGGG]-[DLL3-VH]-[G]-[Fc2];
the structures represented by formulas (h-1) and (i-1) are arranged from N-terminus to C-terminus.

An exemplary antigen-binding molecule comprises:

one first chain set forth in SEQ ID NO: 185 and one second chain set forth in SEQ ID NO: 162; or
one first chain set forth in SEQ ID NO: 161 and one second chain set forth in SEQ ID NO: 162; or
one first chain set forth in SEQ ID NO: 163 and one second chain set forth in SEQ ID NO: 162; or
one first chain set forth in SEQ ID NO: 259 and one second chain set forth in SEQ ID NO: 162; or
one first chain set forth in SEQ ID NO: 260 and one second chain set forth in SEQ ID NO: 162; or
one first chain set forth in SEQ ID NO: 168 and one second chain set forth in SEQ ID NO: 169; or
one first chain set forth in SEQ ID NO: 170 and one second chain set forth in SEQ ID NO: 169; or
one first chain set forth in SEQ ID NO: 175 and one second chain set forth in SEQ ID NO: 176; or
one first chain set forth in SEQ ID NO: 177 and one second chain set forth in SEQ ID NO: 176; or
one first chain set forth in SEQ ID NO: 182 and one second chain set forth in SEQ ID NO: 183; or

one first chain set forth in SEQ ID NO: 184 and one second chain set forth in SEQ ID NO: 183; or
one first chain set forth in SEQ ID NO: 186 and one second chain set forth in SEQ ID NO: 162; or
one first chain set forth in SEQ ID NO: 187 and one second chain set forth in SEQ ID NO: 188; or
one first chain set forth in SEQ ID NO: 187 and one second chain set forth in SEQ ID NO: 189; or
one first chain set forth in SEQ ID NO: 190 and one second chain set forth in SEQ ID NO: 188; or
one first chain set forth in SEQ ID NO: 190 and one second chain set forth in SEQ ID NO: 189; or
one first chain set forth in SEQ ID NO: 191 and one second chain set forth in SEQ ID NO: 188; or
one first chain set forth in SEQ ID NO: 191 and one second chain set forth in SEQ ID NO: 189.

**Variants of Antigen-Binding Molecules**

In certain embodiments, amino acid sequence variants of the antigen-binding molecules provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antigen-binding molecule. Any combination of deletion, insertion, and substitution can be made to obtain the final construct, provided that the final construct possesses the desired characteristics, e.g., antigen binding.

**Variants with Substitutions, Insertions, and Deletions**

In certain embodiments, antigen-binding molecule variants having one or more amino acid substitutions are provided. Substitutions may be made in the CDRs and FRs. Conservative substitutions are shown in Table 2 under the heading of "Preferred substitution". More substantial changes are provided in Table 2 under the heading of "exemplary substitution", and as further described below with reference to amino acid side chain classes. Amino acid substitutions can be introduced into an antibody of interest and the products are screened for a desired activity, e.g., retained/improved antigen binding, reduced immunogenicity, or improved ADCC or CDC.

Table 2. Amino acid substitutions

| Original residue | Exemplary substitution | Preferred substitution |
|---|---|---|
| Ala(A) | Val; Leu; Ile | Val |
| Arg(R) | Lys; Gln; Asn | Lys |
| Asn(N) | Gln; His; Asp,Lys; Arg | Gln |
| Asp(D) | Glu; Asn | Glu |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gln; Lys; Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |

(continued)

| Original residue | Exemplary substitution | Preferred substitution |
|---|---|---|
| Val(V) | Ile; Leu; Met; Phe; Ala; norleucine | Leu |

According to common side-chain properties, amino acids can be grouped as follows:

(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

A non-conservative substitution refers to using a member of one class to replace a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g. binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling.

In certain embodiments, substitutions, insertions or deletions may occur within one or more CDRs, as long as such changes do not substantially reduce the ability of the antibody to bind to the antigen. For example, conservative changes (e.g., conservative substitutions as provided herein) that do not substantially reduce binding affinity may be made in CDRs. In certain embodiments of the variant VH and VL sequences provided above, each CDR is unaltered or contains no more than 1, 2, or 3 amino acid substitutions.

A useful method for identifying residues or regions of an antibody that can be used as mutagenesis targets is called "alanine scanning mutagenesis". In this method, a residue or group of residues (e.g., charged residues such as Arg, Asp, His, Lys, and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include: amino- and/or carboxy-terminal fusions to 1 residue or polypeptides of 100 or more residues in length, and intrasequence insertions of single or multiple amino acid residues. Examples of terminal insertions include an antibody with an N-terminal methionyl residue. Other insertional variants of the antibody molecule include the fusion of the N- or C-terminus of the antibody fused to an enzyme (or a polypeptide that increases the serum half-life of the antibody).

**Engineering of Fab**

In one aspect, in the antigen-binding molecule of the present disclosure, one of the antigen-binding moiety binding specifically to CD3 and the antigen-binding moiety binding specifically to DLL3 is a replaced Fab, and the replaced Fab comprises a heavy chain variable region, a light chain variable region, a Titin chain, and an Obscurin chain. In the replaced Fab, the original CH1 and CL of the Fab are replaced with the Titin chain and the Obscurin chain. Illustratively, the sequences of the Titin chain and the Obscurin chain are shown in Tables 3-1 and 3-2. In the present disclosure, the combinations of the Titin chain and the Obscurin chain with the heavy chain variable region and the light chain variable region are interchangeable.

Table 3-1. The amino acid sequences of Titin chains

| No. | SEQ ID NO | Amino acid sequence |
|---|---|---|
| T.0 | 192 | GIPPKIEALPSDISIDEGKVLTVACAFTGEPTPEVTWSCGGRKIHSQEQGRFHIENTDDLTTLIIMDVQKQDGGLYTLSLGNEFGSDSATVNIHIRSI |
| T.1 | 193 | GIPPKIECLPSDISIDEGKVLTVASAFTGEPTPEVTWSTGGRKIHSQEQGRFHIENTDDLTTLIIMDVQKQDGGLYTLSLGNEFGSDSATVNIHIRSI |
| T.2 | 194 | GIPPKIEALPSDISIDEGKCLTVASAFTGEPTPEVTWSTGGRKIHSQEQGRFHIENTDDLTTLIIMDVQKQDGGLYTLSLGNEFGSDSATVNIHIRSI |
| T.3 | 195 | GIPPKIEALPSDISIDEGKVLTVASCFTGEPTPEVTWSTGGRKIHSQEQGRFHIENTDDLTTLIIMDVQKQDGGLYTLSLGNEFGSDSATVNIHIRSI |
| T.4 | 196 | GIPPKIEALPSDISIDEGKVLCVASAFTGEPTPEVTWSTGGRKIHSQEQGRFHIENTDDLTTLIIMDVQKQDGGLYTLSLGNEFGSDSATVNIHIRSI |
| T.5 | 197 | KAGIRGIPPKIECLPSDISIDEGKVLTVASAFTGEPTPEVTWSTGGRKIHSQEQGRFHIENTDDLTTLIIMDVQKQDGGLYTLSLGNEFGSDSATVNIHIRSI |
| T.6 | 198 | KAGIRGIPPKIEALPSDISIDEGKVLTVACAFTGEPTPEVTWSCGGRKIHSQEQGRFHIENTDDLTTLIIMDVQKQDGGLYTLSLGNEFGSDSATVNIHIRSI |
| T.7 | 199 | GIPPKIECLPSDISIDEGKVLTVASAFTGEPTPEVTWSTGGRKIHSQEQGRFHIENTDDLTTLIISDVQKQDGGLYSLSLGNEFGSDSATVNIHIRSI |
| T.8 | 200 | GIPPKIECLPSDISIDEGKVLTVASAFTGEPTPEVTWSTGGRKIHSQEQGRFHIENTDDLTTLIIKDVQRQDGGLYTLTLRNEFGSDSATVNIHIRSI |
| T.9 | 201 | GIWPKIECLPIDLSIDEGKVLMVASAFTGEPTPEVTWSTGGRKIHSQEQGRFHIENTDDLTTLIIMDVQKQDGGLYTLSLGMEFGSDSATVNIHIRSI |
| T.10 | 202 | GIPPKIECLPIDISIDEGKVLTVASAFTGEPTPEVTWSTGGRKIHSQEQGRFHIENTDDLTTLIIKDVQKQDGGLYTLTLRNEFGSDSATVNIHIRSI |

(continued)

| No. | SEQ ID NO | Amino acid sequence |
|---|---|---|
| T.11 | 203 | GIPPKIECLPSDISIDEGKVLTVASAFTGEPTPEVTWSTSG KKISSEEGGRFHIESTEDLTTLIIMDVQKQDGGVYTLSLG NDLGSDSATVNIHIRSI |
| T.12 | 204 | GIPPKIECLPSDISIDEGKVLTVASAFTGEPTPEVTWSTGG RKIHSEEGGRFHIESTEDLTTLIIMDVQKQDGGVYTLSLG NEFGSDSATVNIHIRSI |
| T.13 | 205 | GIPPKIECLPSDISIDEGKVLTVASAFTGEPTPEVTWSTGG RKIHSQEQGRFHIESTEDLTTLIIMDVQKQDGGVYTLSLG NEFGSDSATVNIHIRSI |
| T.14 | 206 | GIPPKIECLPSDISIDEGKVLTVASAFTGEPTPEVTWSTGG RKIHSQEQGRFHIESTEDLTTLIISDVQKQDGGLYSLSLG NEFGSDSATVNIHIRSI |
| T.15 | 207 | KAGIRGIPPKIECLPSDISIDEGKVLTVASAFTGEPTPEVT WSTGGRKIHSQEQGRFHIESTEDLTTLIISDVQKQDGGLY SLSLGNEFGSDSATVNIHIRSI |
| T.16 | 208 | GIPPKIECLPIDISIDEGKVLTVASAFTGEPTPEVTWSTGG RKIHSQEQGRFHIENTDDSTTLTIKDVQKQDGGLYTLTL RNEFGSDSATVNIHIRSI |
| T.17 | 209 | GIPPKIECLPIDISIDEGKCLTVASAFTGEPTPEVTWSTGG RKIHSQEQGRFHIENTDDSTTLTIKDVQKQDGGLYTLTL RNEFGSDSATVNIHIRSI |
| T.18 | 210 | GIPPKIECLPIDISIDEGKVLTVASCFTGEPTPEVTWSTGG RKIHSQEQGRFHIENTDDSTTLTIKDVQKQDGGLYTLTL RNEFGSDSATVNIHIRSI |

Table 3-2. The amino acid sequences of Obscurin chains

| No. | SEQ ID NO | Amino acid sequence |
|---|---|---|
| O.0 | 211 | SGAPRFLTRPKAFVVSVGKDATLSCQIVGNPTPQVSWEK DQQPVAAGARFRLAQDGDLYRLTILDLALGDSGQYVCR ARNAIGEAFAAVGLQVDAEA |
| OL.0 | 212 | QGSPPCFLRFPRPVRVVSGAEAELKCVVLGEPPPVVVWE KGGQQLAASERLSFPADGAEHGLLLTAALPTDAGVYVC RARNAAGEAYAAAAVTVLEPP |

(continued)

| No. | SEQ ID NO | Amino acid sequence |
|---|---|---|
| O.1 | 213 | SGAPRFLTRPKAFVVSVGKDATLSCQIVGNPTPQVSWEK DQQPVAAGARFRLAQDGDLYRLTILDLALGDSGQYVCR ARNAIGEAFACVGLQVDAEA |
| O.2 | 214 | SGCPRFLTRPKAFVVSVGKDATLSCQIVGNPTPQVSWEK DQQPVAAGARFRLAQDGDLYRLTILDLALGDSGQYVCR ARNAIGEAFAAVGLQVDAEA |
| O.3 | 215 | SGAPRFLTCPKAFVVSVGKDATLSCQIVGNPTPQVSWEK DQQPVAAGARFRLAQDGDLYRLTILDLALGDSGQYVCR ARNAIGEAFAAVGLQVDAEA |
| O.4 | 216 | SGAPRFLTRPKAFVVSVGKDATLSSQIVGNPTPQVSWEK DQQPVAAGARFRLAQDGDLYRLTILDLALGDSGQYVSR ARNAIGEAFACVGLQVDAEA |
| O.5 | 217 | SGCPRFLTRPKAFVVSVGKDATLSSQIVGNPTPQVSWEK DQQPVAAGARFRLAQDGDLYRLTILDLALGDSGQYVSR ARNAIGEAFAAVGLQVDAEA |
| O.6 | 218 | SGAPRFLTCPKAFVVSVGKDATLSSQIVGNPTPQVSWEK DQQPVAAGARFRLAQDGDLYRLTILDLALGDSGQYVSR ARNAIGEAFAAVGLQVDAEA |
| O.7 | 219 | SGAPRFKTRPKAFVVSVGKDATLSSQIVGNPTPQVSWEK DQQPVAAGARFRLAQDGDLYRLKILDLALGDSGQYVSR ARNAIGEAFACVGLQVDAEA |
| O.8 | 220 | SGAPRFKTRPKAFVVSVGKDATLSSQIVGNPTPQVSWEK DQQPVAAGARFRLAQDGDLYRLHILDLALGDSGQYVSR ARNAIGEAFACVGLQVDAEA |
| O.9 | 221 | SGAPRFLTRPLAFVVSVGKDATLSSQIVGNPTPQVSWEK DQQPVAAGARFRLAQDGDLYRLKILDLALGDSGQYVSR ARNAIGEAFACVGLQVDAEA |
| O.10 | 222 | SGAPRFLTRPLAFVVSVGKDATLSSQIVGNPTPQVSWEK DQQPVAAGARFRLAQDGDLYRLHILDLALGDSGQYVSR ARNAIGEAFACVGLQVDAEA |
| O.11 | 223 | DQPQFSGAPRFLTRPKAFVVSVGKDATLSSQIVGNPTPQ VSWEKDQQPVAAGARFRLAQDGDLYRLTILDLALGDS GQYVSRARNAIGEAFACVGLQVDAEA |

(continued)

| No. | SEQ ID NO | Amino acid sequence |
|---|---|---|
| O.12 | 224 | DQPQFSGAPRFKTRPKAFVVSVGKDATLSSQIVGNPTPQVSWEKDQQPVAAGARFRLAQDGDLYRLKILDLALGDSGQYVSRARNAIGEAFACVGLQVDAEA |
| O.13 | 225 | DQPQFSGAPRFKTRPKAFVVSVGKDATLSSQIVGNPTPQVSWEKDQQPVAAGARFRLAQDGDLYRLHILDLALGDSGQYVSRARNAIGEAFACVGLQVDAEA |
| O.14 | 226 | DQPQFSGAPRFRTRPKAFVVSVGKDATLSSQIVGNPTPQVSWEKDQQPVAAGARFRLAQDGDLYRLKILDLALGDSGQYVSRARNAIGEAFACVGLQVDAEA |
| O.15 | 227 | DQPQFSGAPRFRTRPKAFVVSVGKDATLSSQIVGNPTPQVSWEKDQQPVAAGARFRLAQDGDLYRLHILDLALGDSGQYVSRARNAIGEAFACVGLQVDAEA |
| O.16 | 228 | DQPQFSGAPRFLTRPKAFVVSVGKDATLSCQIVGNPTPQVSWEKDQQPVAAGARFRLAQDGDLYRLTILDLALGDSGQYVCRARNAIGEAFAAVGLQVDAEA |
| OL.1 | 229 | QGSPPEFLRFPRPVRVVSGAEAELKCVVLGEPPPVVVWEKGGQQLAASERLSFPADGAEHGLLLTAALPTDAGCYVCRARNAAGEAYAAAAVTVLEPP |
| OL.2 | 230 | QGSPPEFLRFPRPVRVVSGAEAELKCVVLGEPPPVVVWEKGGQQLAASERLSFPADGAEHGLLLTAALPTDAGVYVCRARNAACEAYAAAAVTVLEPP |
| OL.3 | 231 | QGSPPEFLRFPRPVRVVSGAEAELKCVVLGEPPPVVVWEKGGQQLAASERLSFPADGAEHGLLLTAALPTDAGVYVCRARNAAGECYAAAAVTVLEPP |
| OL.4 | 232 | QGSPPEFLRFPRPVRVVSGAEAELKSVVLGEPPPVVVWEKGGQQLAASERLSFPADGAEHGLLLTAALPTDAGCYVSRARNAAGEAYAAAAVTVLEPP |
| OL.5 | 233 | QGSPPEFLRFPRPVRVVSGAEAELKSVVLGEPPPVVVWEKGGQQLAASERLSFPADGAEHGLLLTAALPTDAGVYVSRARNAACEAYAAAAVTVLEPP |
| OL.6 | 234 | QGSPPEFLRFPRPVRVVSGAEAELKSVVLGEPPPVVVWEKGGQQLAASERLSFPADGAEHGLLLTAALPTDAGVYVSRARNAAGECYAAAAVTVLEPP |

(continued)

| No. | SEQ ID NO | Amino acid sequence |
|---|---|---|
| O.17 | 235 | SGAPRFLTRPKSYTVSVGKDASLSSQIVGNPTPQVSWEK DQQPVAAGARFRLAQDGDLYRLKILDLALGDSGQYVSR ARNAIGEAFACVGLQVDAEA |
| O.18 | 236 | SEAPRFLTRPLAFVVSEGKDATLSSQIVGDPPPEVTWEK DQQPITAGARFRLAQDGDVYRLEILDLQLSDSGQYVSR ARNAIGEAFACVGLQVDAEG |
| O.19 | 237 | SGAPRFLTRPLAFVVSVGKLAMLSSQIVGNPTPQVSWEK DQQPVAAGARFRLLQDGDLYRLKILDLALGDSGQYVSR ARNAIGEAFACVGLQVDAEA |
| O.20 | 238 | SGAPRFLTRPLAFVVSVGKDATLSSQIVGNPTPQVSWEK DKQPVTAGARFRLAQDGDLYRLKILDLQLSDSGQYVSR ARNAIGEAFACLGLQVDAEA |
| O.21 | 239 | SGAPRFLTRPLAFVVSVGKDATLSSQIVGNPTPQVSWEK DQLPVTAGARFRLAQDGDLYRLKILDLTLSDSGQYVSR ARNAIGEAFACVGLEVGAEA |
| O.22 | 240 | SGAPRFLTRPLSYVVSVGKDASLSSQIVGNPTPQVSWEK DQLPVTAGARFRLAQDGDLYRLKILDLQLSDSGQYVSR ARNAIGEAFACVGLEVGAEA |
| O.23 | 241 | DQPQFSGAPRFLTRPLSYVVSVGKDASLSSQIVGNPTPQ VSWEKDQLPVTAGARFRLAQDGDLYRLKILDLQLSDSG QYVSRARNAIGEAFACVGLEVGAEA |
| O.24 | 242 | SGAPRFLTRPKAFVVSVGKDATLSSQIVGNPFPQVSWEK DKQPVTAGVRFRLAQDGDLYRLKILDLQLSDSGQYVSR ARNAIGEAFACLGLQVDAEA |
| O.25 | 243 | SGAPRFLTRPKASVVSVGKDATLSSQIVGNPFPQVSWEK DKQPVTAGVRFRLAQDGDLYRLKILDLQLSDSGQYVSR ARNAHGEAFACLGLQVDAEA |
| O.26 | 244 | SGCPRFLTRPKASVVSVGKDATLSSQIVGNPFPQVSWEK DKQPVTAGVRFRLAQDGDLYRLKILDLQLSDSGQYVSR ARNAHGEAFACLGLQVDAEA |
| O.27 | 245 | SGAPRFLTCPKASVVSVGKDATLSSQIVGNPFPQVSWEK DKQPVTAGVRFRLAQDGDLYRLKILDLQLSDSGQYVSR ARNAHGEAFACLGLQVDAEA |

(continued)

| No. | SEQ ID NO | Amino acid sequence |
|---|---|---|
| O.28 | 246 | SGAPRFLTRPKASVVSVGKDATLSCQIVGNPFPQVSWEK DKQPVTAGVRFRLAQDGDLYRLKILDLQLSDSGQYVCR ARNAHGEAFACLGLQVDAEA |
| O.29 | 247 | SGCPRFLTRPKASVVSVGKDATLSCQIVGNPFPQVSWEK DKQPVTAGVRFRLAQDGDLYRLKILDLQLSDSGQYVCR ARNAHGEAFACLGLQVDAEA |
| O.30 | 248 | SGAPRFLTCPKASVVSVGKDATLSCQIVGNPFPQVSWEK DKQPVTAGVRFRLAQDGDLYRLKILDLQLSDSGQYVCR ARNAHGEAFACLGLQVDAEA |
| O.31 | 249 | SGAPRFLTRPKASVVSVGKDATLSSQIVGNPFPQVSWEK DKQPVTAGVRFRLAQDGDLYRLKILDCQLSDSGQYVSR ARNAHGEAFACLGLQCDAEA |
| O.32 | 250 | SGCPRFLTRPKASVVSVGKDATLSSQIVGNPFPQVSWEK DKQPVTAGVRFRLAQDGDLYRLKILDCQLSDSGQYVSR ARNAHGEAFACLGLQCDAEA |
| O.33 | 251 | SGAPRFLTCPKASVVSVGKDATLSSQIVGNPFPQVSWEK DKQPVTAGVRFRLAQDGDLYRLKILDCQLSDSGQYVSR ARNAHGEAFACLGLQCDAEA |

## Engineering of Fc region

In one aspect, the Fc region of the antigen-binding molecule of the present disclosure comprises one or more amino acid substitutions that reduce its binding to an Fc receptor, e.g., its binding to an Fcγ receptor, and reduce or eliminate effector functions. A natural IgG Fc region, specifically an $IgG_1$ Fc region or an $IgG_4$ Fc region, may cause the antigen-binding molecule of the present disclosure to target cells expressing an Fc receptor, rather than cells expressing an antigen. The engineered Fc region of the present disclosure exhibits reduced binding affinity for an Fc receptor and/or reduced effector functions. In some embodiments, the engineered Fc region shows no less than 50%, 80%, 90%, or 95% reduction in binding affinity for an Fc receptor as compared to the native Fc region. In some embodiments, the Fc receptor is an Fcγ receptor. In some embodiments, the Fc receptor is a human Fcγ receptor, e.g., FcγRI, FcγRIIa, FcyRIIB, or FcγRIIIa. In some embodiments, the engineered Fc region also has reduced binding affinity for a complement (e.g., C1q) as compared to the native Fc region. In some embodiments, the engineered Fc region has no reduced binding affinity for a neonatal Fc receptor (FcRn) as compared to the native Fc region. In some examples, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the IgG1 Fc region, amino acid residue substitutions at positions such as positions 238, 265, 269, 270, 297, 327, and 329 can reduce effector functions. In some embodiments, the Fc region is a human IgG1 Fc region, and the amino acid residues at positions 234 and 235 are A, as numbered according to the EU index. For the $IgG_4$ Fc region, amino acid residue substitutions at positions such as position 228 can reduce effector functions.

The antigen-binding molecule may also comprise disulfide bond engineering, such as 354C in the first subunit and 349C in the second subunit. To increase the serum half-life of the antigen-binding molecule, 252Y, 254T, and 256E mutations may be introduced. When the antigen-binding molecule comprises different binding moieties fused to the two subunits of the Fc region, it may lead to undesired homodimerization. To improve yield and purity, it is thus advantageous to introduce modifications that promote heterodimerization in the Fc region of the antigen-binding molecule of the present disclosure. In some embodiments, the Fc region of the present disclosure comprises engineering according to the knob-into-hole (KIH) technique; the method relates to introducing a knob structure at the interface of the first subunit and introducing a hole structure at the interface of the second subunit. As such, the knob structure can be positioned in the hole structure, promoting the formation of a heterodimer and inhibiting the production of a homodimer. The knob structure is constructed by substituting a small amino acid side chain at the interface of the first subunit with a larger side chain (e.g., tyrosine or tryptophan). The hole structure is created at the interface of the second subunit by substituting a large amino acid side chain with a smaller amino acid side chain (e.g., alanine or threonine). The knob structure and the hole structure are prepared by altering the nucleic acid encoding the polypeptide. Optional amino acid substitutions are shown in the table below:

Table 4. Combinations of KIH mutations

| First subunit | T366Y | T366W | T394W | F405W | T366W | T366Y F405A | T366W F405W | F405W Y407A |
|---|---|---|---|---|---|---|---|---|
| Second subunit | Y407T | Y407A | F405A | T394S | T366S L368A Y407V | T394W Y407T | T394W Y407A | T366W T394S |

In addition to the knob-into-hole technique, other techniques for modifying the CH3 domain of the heavy chain to achieve heterodimerization are also known in the art, e.g., WO1996027011A1, WO1998050431, EP1870459, WO2007110205, WO2009089004, WO2010129304, WO201190754, WO2011143545, WO2012058768, WO2013157954, and WO2013096291.

The C-terminus of the Fc region may be an intact C-terminus ending with amino acid residues PGK or a truncated C-terminus, e.g., a truncated C-terminus in which one or two C-terminal amino acid residues are removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without a K447 residue and/or G446 + K447 residues removed. In some embodiments, a composition of intact antibodies comprises antibody populations having a mixture of antibodies with and without a K447 residue and/or G446 + K447 residues.

## Recombination Method

The antigen-binding molecule may be produced using recombination methods. For these methods, one or more isolated nucleic acids encoding the antigen-binding molecule are provided.

In the case of a native antibody, a native antibody fragment, or a bispecific antibody with homodimeric heavy chains, two nucleic acids are required, one for the light chain or a fragment thereof and the other for the heavy chain or a fragment thereof. Such nucleic acids encode an amino acid sequence comprising the VL of the antibody and/or an amino acid sequence comprising the VH of the antibody (e.g., the light and/or heavy chain(s) of the antibody). These nucleic acids can be on the same expression vector or different expression vectors.

In the case of a bispecific antibody with heterodimeric heavy chains, four nucleic acids, for example, are required, one for a first light chain, one for a first heavy chain comprising a first heteromonomeric Fc-region polypeptide, one for a second light chain, and one for a second heavy chain comprising a second heteromonomeric Fc-region polypeptide. The four nucleic acids can be contained in one or more nucleic acid molecules or expression vectors. Generally, these nucleic acids are located on two or three expression vectors; that is, one vector can comprise more than one of these nucleic acids.

In one embodiment, the present disclosure provides an isolated nucleic acid encoding the aforementioned antibody. Such nucleic acids may independently encode any one of the aforementioned polypeptide chains. In another aspect, the present disclosure provides one or more vectors (e.g., expression vectors) comprising such nucleic acids. In another aspect, the present disclosure provides a host cell comprising such nucleic acids. In one embodiment, provided is a method for preparing an antigen-binding molecule, wherein the method comprises culturing a host cell comprising a nucleic acid encoding the antibody, as provided above, under conditions suitable for antibody expression, and optionally collecting the antibody from the host cell (or host cell culture medium).

For recombinant production of the antigen-binding molecule, the nucleic acid encoding the protein is isolated and inserted into one or more vectors for further cloning and/or expression in a host cell. Such nucleic acids can be readily isolated and sequenced using conventional procedures (e.g., by using oligonucleotide probes that are capable of binding specifically to genes encoding the heavy and light chains of an antibody), or produced by recombination methods or obtained by chemical synthesis.

Suitable host cells for cloning or expressing a vector encoding the antibody include prokaryotic or eukaryotic cells described herein. For example, the antibody can be produced in bacteria, particularly when the antibody does not require glycosylation and Fc effector functions. After expression, the antibody can be isolated from the bacterial cell paste in a soluble fraction and can be further purified.

In addition to prokaryotes, eukaryotic microorganisms such as filamentous fungi or yeast are suitable cloning or expression hosts for antibody-encoding vectors, including fungal and yeast strains, whose glycosylation pathways have been "humanized", such that an antibody with a partially or fully human glycosylation pattern is produced. Suitable host cells for expression of (glycosylated) antibodies may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978 and US6417429; vertebrate cells can also be used as hosts, e.g., mammalian cell lines adapted for growth in a suspension. Other examples of suitable mammalian host cell lines include an SV40-transformed monkey kidney CVI line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

## Diagnostic and Therapeutic Compositions

In certain embodiments, the antigen-binding molecule provided in the present disclosure can be used to detect the presence of DLL3 and/or DLL3 in a biological sample. As used herein, the term "detection" encompasses quantitative or qualitative detection. In certain embodiments, the biological sample includes a cell or tissue, such as tumor tissue.

In one embodiment, provided is an antigen-binding molecule for use in a diagnostic or detection method. In yet another aspect, provided is a method for detecting the presence of DLL3 and/or DLL3 in a biological sample. In certain embodiments, the method comprises contacting the biological sample with an antigen-binding molecule under suitable conditions and detecting whether a complex is formed between the detection agent and the antigen. Such methods may be *in vitro* or *in vivo* methods. In one embodiment, an antigen-binding molecule is used to select a subject suitable for treatment; for example, DLL3 and/or DLL3 are biomarkers for selecting a patient. Exemplary disorders that can be diagnosed using the antigen-binding molecule of the present disclosure are, for example, tumors or cancers.

In certain embodiments, provided is a labeled antigen-binding molecule. Labels include, but are not limited to, labels or moieties that are detected directly (such as fluorescent, chromophoric, electron-dense, chemiluminescent, and radioactive labels), and moieties that are detected indirectly (e.g., moieties that are detected indirectly through an enzymatic reaction or molecular interaction, such as enzymes or ligands).

In an additional aspect, provided is a pharmaceutical composition comprising the antigen-binding molecule, for example, for use in any of the following treatment methods. In one aspect, the pharmaceutical composition comprises any of the antigen-binding molecules provided herein and a pharmaceutically acceptable carrier. In another aspect, the pharmaceutical composition comprises any of the antigen-binding molecules provided herein and at least one additional therapeutic agent.

The pharmaceutical composition of the antigen-binding molecule described in the present disclosure is prepared by: mixing such an antigen-binding molecule having desired purity with one or more optional pharmaceutically acceptable carriers. The pharmaceutical composition is in the form of a lyophilized composition or an aqueous solution. Formulations for *in vivo* administration are generally sterile. Sterility can be readily achieved, for example, by filtration through a sterile filter membrane.

## Treatment Method and Route of Administration

Any of the antigen-binding molecules provided herein can be used in the treatment method.

In yet another aspect, the present disclosure provides use of the antigen-binding molecule in the manufacture or preparation of a medicament. In one embodiment, the medicament is used for treating a proliferative disease or tumor. And the medicament is present in an amount effective for the diseases described above. In some embodiments, the effective amount is a unit daily dose or a unit weekly dose. In one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the antigen-binding molecule, e.g., for any of the pharmaceutical uses or treatment methods described above. In another embodiment, the pharmaceutical composition also comprises at least one additional therapeutic agent.

The antigen-binding molecule of the present disclosure may be used alone or in combination with other agents for the treatment. For example, the antigen-binding molecule of the present disclosure may be co-administered with at least one additional therapeutic agent. In some embodiments, the additional therapeutic agent is a bispecific antibody binding specifically to DLL3 and CD28.

The antigen-binding molecule of the present disclosure (and any additional therapeutic agents) may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The antigen-binding molecule of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the method of administration, the timing of administration, and other factors known to medical practitioners. The antigen-binding molecule needs not be, but is optionally, formulated along with one or more agents currently used for preventing or treating the disorder. The effective amount of such additional agents depends on the amount of the antigen-binding molecule present in the pharmaceutical composition, the type of the disorder or treatment, and other factors discussed above. These are generally used in the same dosages and with routes of administration as described herein, or in about 1 to 99% of the dosages described herein, or in any dosage and by any route that is empirically/clinically determined to be appropriate.

For the prevention or treatment of a disease, the appropriate dosage of the antigen-binding molecule of the present disclosure (when used alone or in combination with one or more additional therapeutic agents) will depend on the type of disease to be treated, the type of therapeutic molecule, the severity and course of the disease, the purpose of the administration (prophylactic or therapeutic), previous treatment, clinical history and response to the therapeutic molecule of the patient, and the discretion of the attending physician. The therapeutic molecule is suitably administered to a patient in one or a series of treatments. Depending on the type and severity of the disease, about 1 $\mu$g/kg to 15 mg/kg of the antigen-binding molecule can be an initial candidate dosage for administration to the patient, whether, for example, by one or more separate administrations, or by continuous infusion. One typical daily dosage may range from about 1 $\mu$g/kg to 100 mg/kg or more, depending on the factors mentioned above. Accordingly, taking 50 kg body weight as an example, an exemplary unit daily dosage is 50 $\mu$g-5 g.

**Product (Kit)**

In another aspect of the present disclosure, provided is a product, which comprises materials useful for the treatment, prevention, and/or diagnosis of the disorders described above. The product comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the antigen-binding molecule of the present disclosure. The label or package insert indicates that the composition is used to treat the selected condition. Further, the product may comprise: (a) a first container containing a composition, wherein the composition comprises the antigen-binding molecule of the present disclosure; and (b) a second container containing a composition, wherein the composition comprises other cytotoxic agents or additional therapeutic agents. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further contain other materials as required, including other buffers, diluents, filters, needles, and syringes.

**Examples and Test Examples**

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

**Example 1. Antigen-Binding Molecules Comprising Titin Chain/Obscurin Chain**

The Titin /Obscurin chains of the present disclosure may be derived from any suitable polypeptide, including polypeptides from WO2021139758 (incorporated herein by reference in its entirety) and CN202110527339.7 and the patents which refer to it as a priority document (incorporated herein by reference in their entirety). Bispecific antibodies were constructed, where the CL was the kappa light chain constant region in WO2021139758, the amino acid sequences of the Titin and Obscurin chains are shown in Tables 3-1 and 3-2, and the linker sequences include GGGGS (SEQ ID NO: 147), ASTKG (SEQ ID NO: 255), or RTVAS (SEQ ID NO: 256). The amino acid sequences of the Fc1, Fc2, and CH1 in this example are shown below.

> Example 1-Fc1 (knob, SEQ ID NO: 143)

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPCREEMTKNQVSLWCLVKGFYPSDIAVE
WESNGQPENNYKTTPPVLDSDGSFFLYSKLTVDKSRWQQGNVFSCSVMHEAL
HNHYTQKSLSLSPGK

> Example 1-Fc2 (hole, SEQ ID NO: 144)

DKTHTCPPCPAPEAAGGPSVFLFPPKPKDTLMISRTPEVTCVVVDVSHEDPEVKF

NWYVDGVEVHNAKTKPREEQYNSTYRVVSVLTVLHQDWLNGKEYKCKVSNK
ALPAPIEKTISKAKGQPREPQVCTLPPSREEMTKNQVSLSCAVKGFYPSDIAVEW
ESNGQPENNYKTTPPVLDSDGSFFLVSKLTVDKSRWQQGNVFSCSVMHEALHN
HYTQKSLSLSPGK

> Example 1-CH1 (SEQ ID NO: 141).

**1.1. DI bispecific antibodies**

DI bispecific antibodies against hNGF and hRANKL, DI-2 to DI-20, which comprise a first heavy chain, a second heavy chain, a first light chain, and a second light chain as described below, were constructed with reference to Example 5 in WO2021139758:

the first heavy chain: when viewed from N-terminus to C-terminus, [VH1-I]-[linker 1]-[Obscurin chain]-[Fc2];
the first light chain: when viewed from N-terminus to C-terminus, [VL1-I]-[linker 2]-[ Titin chain];
the second heavy chain: when viewed from N-terminus to C-terminus, [VH2-D]-[CH1]-[Fc1]; and
the second light chain: when viewed from N-terminus to C-terminus, [VL2-D]-[CL]; where the VH1-I and VL1-I are the heavy chain variable region and the light chain variable region of I0 in WO2021139758, respectively, and the VH2-D and VL2-D are the heavy chain variable region and the light chain variable region of D0 in WO2021139758, respectively. The structures of the Obscurin chain, the Titin chain, the linker 1, and the linker 2 in the DI bispecific antibodies of this example are shown in Table 5 below.

Table 5. The Obscurin/Titin chains and linkers in the DI bispecific antibodies

| No. | First heavy chain | | First light chain | |
|---|---|---|---|---|
| | Obscurin chain | Linker 1 | Titin chain | Linker 2 |
| DI-2 | O.20 | GGGGS | T.10 | GGGGS |
| DI-3 | O.25 | GGGGS | T.16 | GGGGS |
| DI-4 | O.26 | GGGGS | T.17 | GGGGS |
| DI-5 | O.27 | GGGGS | T.18 | GGGGS |
| DI-6 | O.28 | GGGGS | T.16 | GGGGS |
| DI-7 | O.29 | GGGGS | T.17 | GGGGS |
| DI-8 | O.30 | GGGGS | T.18 | GGGGS |
| DI-9 | O.31 | GGGGS | T.16 | GGGGS |
| DI-10 | O.32 | GGGGS | T.17 | GGGGS |
| DI-11 | O.33 | GGGGS | T.18 | GGGGS |
| DI-12 | O.25 | ASTKG | T.16 | RTVAS |
| DI-13 | O.26 | ASTKG | T.17 | RTVAS |
| DI-14 | O.27 | ASTKG | T.18 | RTVAS |
| DI-15 | O.28 | ASTKG | T.16 | RTVAS |
| DI-16 | O.29 | ASTKG | T.17 | RTVAS |
| DI-17 | O.30 | ASTKG | T.18 | RTVAS |
| DI-18 | O.31 | ASTKG | T.16 | RTVAS |
| DI-19 | O.32 | ASTKG | T.17 | RTVAS |
| DI-20 | O.33 | ASTKG | T.18 | RTVAS |
| Note: The numbering of the Titin and Obscurin chains in the table is shown in Tables 3-1 and 3-2. | | | | |

The binding activity of the bispecific antibodies DI-2 to DI-20 to their antigens was measured using the method in Test Example 4 of WO2021139758. The thermal stability of the antibodies was studied. Study method: The antibodies were diluted with PBS to a concentration of 5 mg/mL, and their thermal stability was measured using a high throughput differential scanning fluorimeter (UNCHAINED; model: Unit). The experimental results show that there was no significant change in the antigen-binding activity of the engineered bispecific antibodies; additionally, there were significant increases in the Tm1 (°C) and Tonset (°C) of DI-4 to DI-8, DI-10 to DI-16, and DI-20 as compared to DI-2, suggesting that the thermal stability of the bispecific antibodies is better.

Table 6. The binding activity of the DI bispecific antibodies

| No. | RANKL | NGF | No. | RANKL | NGF |
|---|---|---|---|---|---|
| | EC50 (nM) | | | EC50 (nM) | |
| DI-2 | 0.3832 | 6.633 | DI-12 | 0.4125 | 6.5156 |
| DI-3 | 0.3613 | 5.7730 | DI-13 | 0.4440 | 5.5420 |
| DI-4 | 0.3959 | 6.2930 | DI-14 | 0.4182 | 3.2610 |
| DI-5 | 0.3290 | 6.1890 | DI-15 | 0.2206 | 5.2800 |
| DI-6 | 0.2509 | 5.6720 | DI-16 | 0.1474 | 5.5140 |
| DI-7 | 0.2557 | 6.6430 | DI-17 | 0.2329 | 6.7270 |
| DI-8 | 0.3643 | 7.6250 | DI-18 | 0.2662 | 5.9080 |
| DI-9 | 0.2944 | 8.4950 | DI-19 | 0.1843 | 5.9280 |
| DI-10 | 0.3460 | 7.1660 | DI-20 | 0.3184 | 6.4250 |

(continued)

| No. | RANKL | NGF | No. | RANKL | NGF |
|-----|-------|-----|-----|-------|-----|
| | EC50 (nM) | | | EC50 (nM) | |
| DI-11 | 0.3721 | 10.9600 | | | |

Table 7. The thermal stability of the DI bispecific antibodies

| No. | Tm1 (°C) | Tonset (°C) | No. | Tm1 (°C) | Tonset (°C) |
|-----|----------|-------------|-----|----------|-------------|
| DI-2 | 55.6 | 48.3 | DI-11 | 57.35 | - |
| DI-4 | 60.1 | 52.493 | DI-12 | 59.9 | 51.726 |
| DI-5 | 61 | 51.967 | DI-13 | 61 | 50.988 |
| DI-6 | 60.8 | 53.012 | DI-14 | 61.2 | 52.191 |
| DI-7 | 60.34 | 52.003 | DI-15 | 60.41 | 50.558 |
| DI-8 | 60.61 | 50.425 | DI-16 | 61.5 | 50.691 |
| DI-10 | 60.2 | 52.766 | DI-20 | 60.7 | 51.859 |

Solutions of the DI bispecific antibodies were prepared using a pH 5.5 buffer containing 10 mM acetic acid and 9% sucrose, and the solutions were incubated in a 40 °C incubator for four weeks. Following the incubation, the antibody solutions were concentrated to the concentration at the start of the incubation, and the solutions were observed for precipitate formation. The experimental results show that a precipitate formed in the solution of the DI-2 bispecific antibody group. DI-3 to DI-7 are more stable than DI-2.

Table 8. Precipitate formation in the solutions of the DI bispecific antibodies

| No. | Initial concentration | Concentration after solution concentration at week 4 | Precipitate formation in solution |
|-----|----------------------|------------------------------------------------------|-----------------------------------|
| DI-2 | 20 mg/mL | 20 mg/mL | Precipitate formed |
| DI-3 | 20 mg/mL | 20 mg/mL | No precipitation |
| DI-4 | 60 mg/mL | 60 mg/mL | No precipitation |
| DI-5 | 25 mg/mL | 25 mg/mL | No precipitation |
| DI-6 | 60 mg/mL | 60 mg/mL | No precipitation |
| DI-7 | 16 mg/mL | 16 mg/mL | No precipitation |

## 1.2. PL bispecific antibodies

PL bispecific antibodies against hPDL1 and hCTLA4, PL-1 to PL-19, which comprise a first heavy chain, a second heavy chain, a first light chain, and a second light chain as described below, were constructed:

the first heavy chain: when viewed from N-terminus to C-terminus, [VH1-P]-[linker 1]-[Obscurin chain]-[Fc1];
the first light chain: when viewed from N-terminus to C-terminus, [VL1-P]-[linker 2]-[ Titin chain];
the second heavy chain: when viewed from N-terminus to C-terminus, [VH2-L]-[CH1]-[Fc2]; and
the second light chain: when viewed from N-terminus to C-terminus, [VL2-L]-[CL]; where the VH1-P and VL1-P were the heavy chain variable region and the light chain variable region of the h1831K antibody in WO2020177733A1, respectively. The amino acid sequences of the VH2-L and VL2-L are shown below.
> VH2-L (SEQ ID NO: 257)

QVQLVESGGGVVQPGRSLRLSCAASGFTFSSYTMHWVRQAPGKGLEWVTFISY
DGNNKYYADSVKGRFTISRDNSKNTLYLQMNSLRAEDTAIYYCARTGWLGPF
DYWGQGTLVTVSS

> VL2-L (SEQ ID NO: 258)

EIVLTQSPGTLSLSPGERATLSCRASQSVGSSYLAWYQQKPGQAPRLLIYGAFSR
ATGIPDRFSGSGSGTDFTLTISRLEPEDFAVYYCQQYGSSPWTFGQGTKVEIK.

The structures of the Obscurin chain, the Titin chain, the linker 1, and the linker 2 in the PL bispecific antibodies of this example are shown in the table below.

Table 9. The Obscurin/Titin chains and linkers in the PL bispecific antibodies

| No. | First heavy chain | | First light chain | |
| | Obscurin chain | Linker 1 | Titin chain | Linker 2 |
|---|---|---|---|---|
| PL-1 | O.20 | GGGGS | T.10 | GGGGS |
| PL-2 | O.25 | GGGGS | T.16 | GGGGS |
| PL-3 | O.26 | GGGGS | T.17 | GGGGS |
| PL-4 | O.27 | GGGGS | T.18 | GGGGS |
| PL-5 | O.28 | GGGGS | T.16 | GGGGS |
| PL-6 | O.29 | GGGGS | T.17 | GGGGS |
| PL-7 | O.30 | GGGGS | T.18 | GGGGS |
| PL-8 | O.31 | GGGGS | T.16 | GGGGS |
| PL-9 | O.32 | GGGGS | T.17 | GGGGS |
| PL-10 | O.33 | GGGGS | T.18 | GGGGS |
| PL-11 | O.25 | ASTKG | T.16 | RTVAS |
| PL-12 | O.26 | ASTKG | T.17 | RTVAS |
| PL-13 | O.27 | ASTKG | T.18 | RTVAS |
| PL-14 | O.28 | ASTKG | T.16 | RTVAS |
| PL-15 | O.29 | ASTKG | T.17 | RTVAS |
| PL-16 | O.30 | ASTKG | T.18 | RTVAS |
| PL-17 | O.31 | ASTKG | T.16 | RTVAS |
| PL-18 | O.32 | ASTKG | T.17 | RTVAS |
| PL-19 | O.33 | ASTKG | T.18 | RTVAS |
| Note: The numbering of the Titin and Obscurin chains in the table is shown in Tables 3-1 and 3-2. | | | | |

The binding activity of the PL bispecific antibodies was measured with reference to the ELISA method in Test Example 4 of WO2021139758, where the hPDL1 and hCTLA4 antigens were purchased from Sino biology. The thermal stability of the antibodies was studied. Method: The antibodies were diluted with PBS to a concentration of 1.4-3 mg/mL, and their thermal stability was measured using a high throughput differential scanning fluorimeter (UNCHAINED; model: Unit). The experimental results show that the PL bispecific antibodies retained good antigen-binding activity; additionally, there were significant increases in the Tm1 (°C), Tagg 266 (°C), and Tonset (°C) of PL-2 to PL-19 as compared to PL-1, suggesting that the thermal stability of the bispecific antibodies is better.

Table 10. The binding activity of the PL bispecific antibodies

| No. | hPDL1 | hCTLA4 | No. | hPDL1 | hCTLA4 |
| | EC50 (nM) | | | EC50 (nM) | |
|---|---|---|---|---|---|
| PL-1 | 1.6 | 16.5 | PL-11 | 0.6 | 5.6 |
| PL-2 | 0.5 | 8.0 | PL-12 | 0.5 | 8.0 |

(continued)

| No. | hPDL1 | hCTLA4 | No. | hPDL1 | hCTLA4 |
|---|---|---|---|---|---|
| | EC50 (nM) | | | EC50 (nM) | |
| PL-3 | 0.6 | 7.9 | PL-13 | 0.5 | 4.4 |
| PL-4 | 0.7 | 6.5 | PL-14 | 1.3 | 6.0 |
| PL-5 | 0.7 | 6.7 | PL-15 | 1.4 | 3.2 |
| PL-6 | 0.8 | 5.3 | PL-16 | 1.7 | 34.9 |
| PL-7 | 0.5 | 7.3 | PL-17 | 1.4 | 6.2 |
| PL-8 | 1.4 | 14.5 | PL-18 | 1.6 | 6.7 |
| PL-9 | 0.6 | 5.5 | PL-19 | 1.6 | 6.5 |
| PL-10 | 0.6 | 6.9 | | | |

Table 11. The thermal stability of the PL bispecific antibodies

| No. | Tm1 (°C) | Tagg 266 (°C) | Tonset (°C) | No. | Tm1 (°C) | Tagg 266 (°C) | Tonset (°C) |
|---|---|---|---|---|---|---|---|
| PL-1 | 61.64 | 64.82 | 52.566 | PL-11 | 65.88 | - | 57.976 |
| PL-2 | 66.20 | 66.55 | 58.317 | PL-12 | 65.54 | 67.94 | - |
| PL-3 | 64.07 | 68.28 | 57.661 | PL-13 | 71.85 | 68.17 | 58.581 |
| PL-4 | 70.71 | 67.29 | 56.246 | PL-14 | 74.18 | 69.42 | 58.589 |
| PL-5 | 74.56 | 68.11 | 58.407 | PL-15 | 70.96 | 69.91 | 58.622 |
| PL-6 | 70.43 | 70.12 | 61.069 | PL-16 | 63.48 | 68.98 | 58.702 |
| PL-7 | 68.46 | 67.41 | 63.031 | PL-17 | 70.15 | 69.86 | 56.193 |
| PL-8 | 65.00 | - | - | PL-18 | - | 69.43 | - |
| PL-9 | 69.24 | 67.83 | 58.597 | PL-19 | - | 69.52 | 57.766 |
| PL-10 | 69.63 | 68.12 | 56.788 | | | | |

**1.3. HJ bispecific antibodies**

HJ bispecific antibodies against hIL5 and hTSLP, HJ-3 to HJ11, which comprise a first heavy chain, a second heavy chain, a first light chain, and a second light chain as described below, were constructed:

the first heavy chain: when viewed from N-terminus to C-terminus, [VH1-H]-[linker 1]-[Titin chain]-[Fc1];
the first light chain: when viewed from N-terminus to C-terminus, [VL1-H]-[linker 2]-[Obscurin chain];
the second heavy chain: when viewed from N-terminus to C-terminus, [VH2-J]-[CH1]-[Fc2]; and
the second light chain: when viewed from N-terminus to C-terminus, [VL2-J]-[CL]; where the VH1-H and VL1-I are the heavy chain variable region and the light chain variable region of H0 in WO2021139758, respectively, and the VH2-J and VL2-J are the heavy chain variable region and the light chain variable region of J1 in WO2021139758, respectively. The structures of the Obscurin chain, the Titin chain, the linker 1, and the linker 2 in the HJ bispecific antibodies of this example are shown in the table below.

Table 12. The Obscurin/Titin chains and linkers in the HJ bispecific antibodies

| No. | First heavy chain | | First light chain | |
|---|---|---|---|---|
| | Titin chain | Linker 1 | Obscurin chain | Linker 2 |
| HJ-3 | T.10 | GGGGS | O.20 | GGGGS |
| HJ-5 | T.16 | GGGGS | O.25 | GGGGS |
| HJ-6 | T.16 | GGGGS | O.27 | GGGGS |

(continued)

| No. | First heavy chain | | First light chain | |
|---|---|---|---|---|
| | Titin chain | Linker 1 | Obscurin chain | Linker 2 |
| HJ-7 | T.16 | GGGGS | O.28 | GGGGS |
| HJ-8 | T.16 | ASTKG | O.25 | RTVAS |
| HJ-9 | T.16 | ASTKG | O.27 | RTVAS |
| HJ-10 | T.16 | ASTKG | O.28 | RTVAS |
| HJ-11 | T.16 | ASTKG | O.29 | RTVAS |

The antigen-binding activity of the HJ bispecific antibodies was measured with reference to the method in Test Example 4 in WO2021139758. The thermal stability of the antibodies was studied. Method: Diluted solutions of the HJ bispecific antibodies were prepared with a pH 5.5 buffer containing 10 mM acetic acid and 9% sucrose and then concentrated by ultrafiltration to obtain HJ bispecific antibody solutions with different concentrations (the concentrations of the HJ bispecific antibodies are shown in Table 13-2). Subsequently, the concentrated solutions were incubated in a 40 °C incubator. At day 0 (i.e., before the 40 °C incubation, D0), day 7 (the 7th day of the 40 °C incubation, D7), day 14 (the 14th day of the 40 °C incubation, D14), day 21 (the 21st day of the 40 °C incubation, D21), and day 28 (the 28th day of the 40 °C incubation, D28), samples were taken and their SEC purity was tested. Immediately after 28 days of incubation at 40 °C, samples were taken and their CE-SDS purity was tested. The experimental results show that there was no significant change in the antigen-binding activity of the HJ bispecific antibodies constructed in the present disclosure; additionally, the thermal stability of the HJ-5 to HJ-11 bispecific antibodies is better than that of HJ-3.

Table 13-1. The binding activity of the HJ bispecific antibodies

| No. | hIL5 | hTSLP | No. | hIL5 | hTSLP |
|---|---|---|---|---|---|
| | EC50 (nM) | | | EC50 (nM) | |
| HJ-3 | 17.38 | 3.234 | HJ-8 | 18.74 | 3.332 |
| HJ-5 | 15.96 | 2.639 | HJ-9 | 10.86 | 3.184 |
| HJ-6 | 37.05 | 2.884 | HJ-10 | 20.81 | 3.173 |
| HJ-7 | 17.69 | 2.182 | HJ-11 | 9.464 | 3.005 |

Table 13-2. The results for the accelerated stability testing of the HJ bispecific antibodies

| No. | D0 concentration (mg/mL) | D0 SEC purity (%) | D28 SEC purity (%) | D28△ SEC purity (%) | D28 non-reduced CE-SDS purity (%) |
|---|---|---|---|---|---|
| HJ-3 | 50 | 98 | 84 | 14 | 67 |
| HJ-5 | 60.8 | 98.09 | 93.86 | 4.23 | 82.93 |
| HJ-6 | 54 | 98.14 | 89.68 | 8.46 | 80.76 |
| HJ-7 | 56.8 | 97.7 | 92.72 | 4.98 | 85.47 |
| HJ-8 | 62.6 | 93.39 | 84.28 | 9.11 | 73.03 |
| HJ-9 | 53.5 | 90.01 | 85.93 | 4.08 | 80.32 |
| HJ-10 | 69.8 | 90.9 | 88.31 | 2.59 | 80.4 |
| HJ-11 | 50.4 | 92.55 | 90.89 | 1.66 | 82.96 |

**Example 2. Preparation of DLL3 Antigens, Proteins for Detection, and Stably Transfected Cell Strains**

**2.1. Construction of cell strain highly expressing DLL3**

A pCDH lentiviral expression vector plasmid comprising SEQ ID NO: 1-2 (synthesized by GENEWIZ), along with a pVSVG or pCMV lentiviral packaging vector, was transfected into 293T cells (Cell Bank of the Chinese Academy of Sciences, GNHu17) using Lipofectamine 3000 (Invitrogen, L3000015). The virus-containing culture medium supernatants were collected, filtered, and subjected to ultracentrifugation. After the supernatants were discarded, resuspension was performed in 0.2 mL of sterile PBS. The concentrated viruses were then used to infect Chinese hamster ovary cells CHO-s (Invitrogen, R80007), DMS53 (ATCC, CRL-2062), and H82 (ATCC, HTB-175). After two to three weeks of screening with puromycin, FACS single-cell sorting was performed. The selected monoclonal cell strains were expanded and cryopreserved for subsequent experiments.

A pCDH-CMV-MCS-EF1-puro lentiviral expression vector plasmid comprising the LUC and GFP genes (synthesized by GENEWIZ), along with a pVSVG or pCMV lentiviral packaging vector, was transfected into 293T cells (Cell Bank of the Chinese Academy of Sciences, GNHu17) using Lipofectamine 3000 (Invitrogen, L3000015). The virus-containing culture medium supernatants were collected, filtered, and subjected to ultracentrifugation. After the supernatants were discarded, resuspension was performed in 0.2 mL of sterile PBS. SHP77 (ATCC, CRL-2195), H1184 (ATCC, CRL-5858), and H460 (Cell Bank of Chinese Academy of Sciences, TCHu205) were separately infected with the concentrated virus, screened using puromycin for two to three weeks, and subjected to FACS single-cell sorting. The selected monoclonal cell strains were expanded and cryopreserved for subsequent experiments.

The sequences of the related proteins used are as follows:

1. Human DLL3 full-length protein (SEQ ID NO: 1):

MVSPRMSGLLSQTVILALIFLPQTRPAGVFELQIHSFGPGPGPGAPRSPCSARLPC
RLFFRVCLKPGLSEEAAESPCALGAALSARGPVYTEQPGAPAPDLPLPDGLLQV
PFRDAWPGTFSFIIETWREELGDQIGGPAWSLLARVAGRRRLAAGGPWARDIQR
AGAWELRFSYRARCEPPAVGTACTRLCRPRSAPSRCGPGLRPCAPLEDECEAPL
VCRAGCSPEHGFCEQPGECRCLEGWTGPLCTVPVSTSSCLSPRGPSSATTGCLVP
GPGPCDGNPCANGGSCSETPRSFECTCPRGFYGLRCEVSGVTCADGPCFNGGLC

VGGADPDSAYICHCPPGFQGSNCEKRVDRCSLQPCRNGGLCLDLGHALRCRCR
AGFAGPRCEHDLDDCAGRACANGGTCVEGGGAHRCSCALGFGGRDCRERADP
CAARPCAHGGRCYAHFSGLVCACAPGYMGARCEFPVHPDGASALPAAPPGLR
PGDPQRYLLPPALGLLVAAGVAGAALLLVHVRRRGHSQDAGSRLLAGTPEPSV
HALPDALNNLRTQEGSGDGPSSSVDWNRPEDVDPQGIYVISAPSIYAREVATPL
FPPLHTGRAGQRQHLLFPYPSSILSVK

2. Cynomolgus monkey DLL3 full-length protein (SEQ ID NO: 2):

MVSPRMSRLLSQTVILALIFIPQARPAGVFELQIHSFGPGPGPGAPRSPCSARGPC
RLFFRVCLKPGLSEEAAESPCALGAALSARGPVYTEQPEAPAPDLPLPNGLLQV
PFRDAWPGTFSLIIETWREELGDQIGGPAWSLLARVTRRRRLAAGGPWARDIQR
AGAWELRFSYRARCELPAVGTACTRLCRPRSAPSRCGPGLRPCAPLEDECEAPP
VCRAGCSLEHGFCEQPGECRCLEGWTGPLCMVPVSTSSCLGLRGPSSTTTGCLV
PGPGPCDGNPCANGGSCSETPGSFECTCPRGFYGLRCEVSGVTCADGPCFNGGL
CVGGADPDSAYICHCPPGFQGSNCEKRVDRCSLQPCRNGGLCLDLGHALRCRC
RAGFAGPRCEHDLDDCAGRACANGGTCVEGGAHRCSCALGFGGRNCRERA
DPCAARPCAHGGRCYAHFSGLVCACAPGYMGARCEFPVHPDGVSALPAAPPG
LRPGDPQRYLLPPALGLLVAAGVAGAALLLVHVRRRGHAQDAGSRLLAGTPEP
SVHALPDALNNLRTQEGPGDVPSSSVDWNRPEDVDSRGIYVISAPSIYAREVAM
PLFPPLHTGRAGQRQNLLFPFPSSILSVK

3. Rat DLL3 full-length protein (SEQ ID NO: 3):

MVSLQVSSLPQTLILAFLLPQALPAGVFELQIHSFGPGPGPGTPRSPCNARGPCRL
FFRVCLKPGVSQEAAESLCALGAALSTSGPVYTEQPGVPAAALSLPDGLVRVPF
LDAWPGTFSLIIETWREQLGERAAGPAWNLLARVAGRRRLAAGAPWARDVQR
TGAWELHFSYRARCEPPAVGAACARLCRSRSAPSRCGPGLRPCTPFPDECEAPR
ESLTVCRAGCSPEHGYCEEPDECHCLEGWTGPLCTVPVSTSSCLNSRVSGPAGT
GCLLPGPGPCDGNPCANGGSCSETPGSFECACPRGFYGPRCEVSGVTCADGPCF
NGGLCVGGEDPDSAYVCHCPPAFQGSNCERRVDRCSLQPCQNGGLCLDLGHA
LRCRCRAGFAGPRCEHDLDDCAGRACANGGTCVEGGGARRCSCALGFGGRDC
RERADPCASRPCAHGGRCYAHFSGLVCACAPGYMGVRCEFAVRPGDADAVPA
APRGLRQADSQRFLLPPALGLLAAAALAGAALLLIHVRRRGPGRDTGTRLLSGT
REPSVHTLPDALNNLRLQDGAGDGPTSSADWNHPEDGDSRSIYVIPAPSIYARE
A

4. Mouse DLL3 full-length protein (SEQ ID NO: 4):

MVSLQVSPLSQTLILAFLLPQALPAGVFELQIHSFGPGPGLGTPRSPCNARGPCR
LFFRVCLKPGVSQEATESLCALGAALSTSVPVYTEHPGESAAALPLPDGLVRVP
FRDAWPGTFSLVIETWREQLGEHAGGPAWNLLARVVGRRRLAAGGPWARDV
QRTGTWELHFSYRARCEPPAVGAACARLCRSRSAPSRCGPGLRPCTPFPDECEA
PSVCRPGCSPEHGYCEEPDECRCLEGWTGPLCTVPVSTSSCLNSRVPGPASTGCL

LPGPGPCDGNPCANGGSCSETSGSFECACPRGFYGLRCEVSGVTCADGPCFNGG LCVGGEDPDSAYVCHCPPGFQGSNCEKRVDRCSLQPCQNGGLCLDLGHALRCR CRAGFAGPRCEHDLDDCAGRACANGGTCVEGGGSRRCSCALGFGGRDCRERA DPCASRPCAHGGRCYAHFSGLVCACAPGYMGVRCEFAVRPDGADAVPAAPRG LRQADPQRFLLPPALGLLVAAGLAGAALLVIHVRRRGPGQDTGTRLLSGTREPS VHTLPDALNNLRLQDGAGDGPSSSADWNHPEDGDSRSIYVIPAPSIYAREDWLI QVLF

5. Human DLL1 full-length protein (SEQ ID NO: 5):

MGSRCALALAVLSALLCQVWSSGVFELKLQEFVNKKGLLGNRNCCRGGAGPP PCACRTFFRVCLKHYQASVSPEPPCTYGSAVTPVLGVDSFSLPDGGGADSAFSN PIRFPFGFTWPGTFSLIIEALHTDSPDDLATENPERLISRLATQRHLTVGEEWSQD LHSSGRTDLKYSYRFVCDEHYYGEGCSVFCRPRDDAFGHFTCGERGEKVCNPG WKGPYCTEPICLPGCDEQHGFCDKPGECKCRVGWQGRYCDECIRYPGCLHGTC QQPWQCNCQEGWGGLFCNQDLNYCTHHKPCKNGATCTNTGQGSYTCSCRPG YTGATCELGIDECDPSPCKNGGSCTDLENSYSCTCPPGFYGKICELSAMTCADG PCFNGGRCSDSPDGGYSCRCPVGYSGFNCEKKIDYCSSSPCSNGAKCVDLGDA YLCRCQAGFSGRHCDDNVDDCASSPCANGGTCRDGVNDFSCTCPPGYTGRNC SAPVSRCEHAPCHNGATCHERGHRYVCECARGYGGPNCQFLLPELPPGPAVVD LTEKLEGQGGPFPWVAVCAGVILVLMLLLGCAAVVVCVRLRLQKHRPPADPC RGETETMNNLANCQREKDISVSIIGATQIKNTNKKADFHGDHSADKNGFKARY PAVDYNLVQDLKGDDTAVRDAHSKRDTKCQPQGSSGEEKGTPTTLRGGEASE RKRPDSGCSTSKDTKYQSVYVISEEKDECVIATEV

6. Human DLL4 full-length protein (SEQ ID NO: 6):

MAAASRSASGWALLLLVALWQQRAAGSGVFQLQLQEFINERGVLASGRPCEP
GCRTFFRVCLKHFQAVVSPGPCTFGTVSTPVLGTNSFAVRDDSSGGGRNPLQLP
FNFTWPGTFSLIIEAWHAPGDDLRPEALPPDALISKIAIQGSLAVGQNWLLDEQT
STLTRLRYSYRVICSDNYYGDNCSRLCKKRNDHFGHYVCQPDGNLSCLPGWTG
EYCQQPICLSGCHEQNGYCSKPAECLCRPGWQGRLCNECIPHNGCRHGTCSTP
WQCTCDEGWGGLFCDQDLNYCTHHSPCKNGATCSNSGQRSYTCTCRPGYTGV
DCELELSECDSNPCRNGGSCKDQEDGYHCLCPPGYYGLHCEHSTLSCADSPCFN
GGSCRERNQGANYACECPPNFTGSNCEKKVDRCTSNPCANGGQCLNRGPSRM
CRCRPGFTGTYCELHVSDCARNPCAHGGTCHDLENGLMCTCPAGFSGRRCEVR
TSIDACASSPCFNRATCYTDLSTDTFVCNCPYGFVGSRCEFPVGLPPSFPWVAVS
LGVGLAVLLVLLGMVAVAVRQLRLRRPDDGSREAMNNLSDFQKDNLIPAAQL
KNTNQKKELEVDCGLDKSNCGKQQNHTLDYNLAPGPLGRGTMPGKFPHSDKS
LGEKAPLRLHSEKPECRISAICSPRDSMYQSVCLISEERNECVIATEV.

**2.2. Preparation of antigens**

Human DLL3 (Uniprot, Q9NYJ7), cynomolgus monkey DLL3 (Uniprot, A0A2K5WSR4), and mouse DLL3 (Uniprot, 088516) sequences were used as templates to design human DLL3 ECD fusion proteins containing different tags, and the fusion proteins were cloned onto pTT5 vectors. After expression by 293E cells, antigens were obtained. The amino acid sequences of the related proteins are as follows:

1. His-hDLL3 (ECD) (SEQ ID NO: 7):

METDTLLLWVLLLWVPGSTGHHHHHHHHHHGGGGSGGGGSAGVFELQIHSF
GPGPGPGAPRSPCSARLPCRLFFRVCLKPGLSEEAAESPCALGAALSARGPVYTE
QPGAPAPDLPLPDGLLQVPFRDAWPGTFSFIIETWREELGDQIGGPAWSLLARV
AGRRRLAAGGPWARDIQRAGAWELRFSYRARCEPPAVGTACTRLCRPRSAPSR
CGPGLRPCAPLEDECEAPLVCRAGCSPEHGFCEQPGECRCLEGWTGPLCTVPVS
TSSCLSPRGPSSATTGCLVPGPGPCDGNPCANGGSCSETPRSFECTCPRGFYGLR
CEVSGVTCADGPCFNGGLCVGGADPDSAYICHCPPGFQGSNCEKRVDRCSLQP
CRNGGLCLDLGHALRCRCRAGFAGPRCEHDLDDCAGRACANGGTCVEGGGA
HRCSCALGFGGRDCRERADPCAARPCAHGGRCYAHFSGLVCACAPGYMGARC
EFPVHPDGASALPAAPPGLRPGDPQRYL

Note: The signal peptide sequence is highlighted with a dotted line; the his tag and the linker are single underlined; and the extracellular region of DLL3 is double underlined.

2. Fc-hDLL3 (ECD) (SEQ ID NO: 8):

METDTLLLWVLLLWVPGSTGEPKSSDKTHTCPPCPAPELLGGPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSAGVFE
LQIHSFGPGPGPGAPRSPCSARLPCRLFFRVCLKPGLSEEAAESPCALGAALSAR
GPVYTEQPGAPAPDLPLPDGLLQVPFRDAWPGTFSFIIETWREELGDQIGGPAW
SLLARVAGRRRLAAGGPWARDIQRAGAWELRFSYRARCEPPAVGTACTRLCRP
RSAPSRCGPGLRPCAPLEDECEAPLVCRAGCSPEHGFCEQPGECRCLEGWTGPL
CTVPVSTSSCLSPRGPSSATTGCLVPGPGPCDGNPCANGGSCSETPRSFECTCPR
GFYGLRCEVSGVTCADGPCFNGGLCVGGADPDSAYICHCPPGFQGSNCEKRVD
RCSLQPCRNGGLCLDLGHALRCRCRAGFAGPRCEHDLDDCAGRACANGGTCV
EGGGAHRCSCALGFGGRDCRERADPCAARPCAHGGRCYAHFSGLVCACAPGY
MGARCEFPVHPDGASALPAAPPGLRPGDPQRYL

Note: The signal peptide sequence is highlighted with a dotted line; the Fc tag and the linker are single underlined; and the extracellular region of DLL3 is double underlined.

3. hDLL3 (ECD)-strep twin (SEQ ID NO: 9):

METDTLLLWVLLLWVPGSTGAGVFELQIHSFGPGPGPGAPRSPCSARLPCRLFF
RVCLKPGLSEEAAESPCALGAALSARGPVYTEQPGAPAPDLPLPDGLLQVPFRD
AWPGTFSFIIETWREELGDQIGGPAWSLLARVAGRRRLAAGGPWARDIQRAGA
WELRFSYRARCEPPAVGTACTRLCRPRSAPSRCGPGLRPCAPLEDECEAPLVCR
AGCSPEHGFCEQPGECRCLEGWTGPLCTVPVSTSSCLSPRGPSSATTGCLVPGPG
PCDGNPCANGGSCSETPRSFECTCPRGFYGLRCEVSGVTCADGPCFNGGLCVG
GADPDSAYICHCPPGFQGSNCEKRVDRCSLQPCRNGGLCLDLGHALRCRCRAG
FAGPRCEHDLDDCAGRACANGGTCVEGGGAHRCSCALGFGGRDCRERADPCA
ARPCAHGGRCYAHFSGLVCACAPGYMGARCEFPVHPDGASALPAAPPGLRPG
DPQRYLWSHPQFEKGGGSGGGSGGSAWSHPQFEK

Note: The signal peptide sequence is highlighted with a dotted line; the extracellular region of DLL3 is double-underlined; and the strep twin tag is single-underlined.

4. cynoDLL3 (ECD)-strep twin (SEQ ID NO: 10):

METDTLLLWVLLLWVPGSTGAGVFELQIHSFGPGPGPGAPRSPCSARGPCRLFF
RVCLKPGLSEEAAESPCALGAALSARGPVYTEQPEAPAPDLPLPNGLLQVPFRD
AWPGTFSLIIETWREELGDQIGGPAWSLLARVTRRRRLAAGGPWARDIQRAGA
WELRFSYRARCELPAVGTACTRLCRPRSAPSRCGPGLRPCAPLEDECEAPPVCR
AGCSLEHGFCEQPGECRCLEGWTGPLCMVPVSTSSCLGLRGPSSTTTGCLVPGP
GPCDGNPCANGGSCSETPGSFECTCPRGFYGLRCEVSGVTCADGPCFNGGLCV
GGADPDSAYICHCPPGFQGSNCEKRVDRCSLQPCRNGGLCLDLGHALRCRCRA
GFAGPRCEHDLDDCAGRACANGGTCVEGGGAHRCSCALGFGGRNCRERADPC
AARPCAHGGRCYAHFSGLVCACAPGYMGARCEFPVHPDGVSALPAAPPGLRP
GDPQRYLWSHPQFEKGGGSGGGSGGSAWSHPQFEK

Note: The signal peptide sequence is highlighted with a dotted line; the extracellular region of DLL3 is double-underlined; and the strep twin tag is single-underlined.

5. mouDLL3 (ECD)-strep twin (SEQ ID NO: 11):

METDTLLLWVLLLWVPGSTGAGVFELQIHSFGPGPGLGTPRSPCNARGPCRLFF
RVCLKPGVSQEATESLCALGAALSTSVPVYTEHPGESAAALPLPDGLVRVPFRD
AWPGTFSLVIETWREQLGEHAGGPAWNLLARVVGRRRLAAGGPWARDVQRT
GTWELHFSYRARCEPPAVGAACARLCRSRSAPSRCGPGLRPCTPFPDECEAPSV
CRPGCSPEHGYCEEPDECRCLEGWTGPLCTVPVSTSSCLNSRVPGPASTGCLLP
GPGPCDGNPCANGGSCSETSGSFECACPRGFYGLRCEVSGVTCADGPCFNGGL
CVGGEDPDSAYVCHCPPGFQGSNCEKRVDRCSLQPCQNGGLCLDLGHALRCR
CRAGFAGPRCEHDLDDCAGRACANGGTCVEGGGSRRCSCALGFGGRDCRERA
DPCASRPCAHGGRCYAHFSGLVCACAPGYMGVRCEFAVRPDGADAVPAAPRG
LRQADPQRFLWSHPQFEKGGGSGGGSGGSAWSHPQFEK

Note: The signal peptide sequence is highlighted with a dotted line; the extracellular region of DLL3 is double-underlined; and the strep twin tag is single-underlined.

## Example 3. Preparation of Anti-Human DLL3 Antibodies

### 3.1. Screening and identification of anti-human DLL3 hybridoma antibodies

Monoclonal antibodies against human DLL3 were prepared using the hybridoma technique. The immunization method was as follows:

For the first group, cross-immunization was performed using the adjuvants TiterMax® Gold Adjuvant (Sigma Cat No. T2684) and Thermo Imject® Alum (Thermo Cat No. 77161), with His-hDLL3 (ECD) (SEQ ID NO: 7) as the immunogen. The ratio of the antigen to the adjuvant (TiterMax® Gold Adjuvant) was 1:1, and the ratio of the antigen to the adjuvant (Thermo Imject® Alum) was 3:1. The dose for the first immunization was 50 μg/mouse/immunization, and the dose for boost immunization was 25 μg/mouse/immunization. The antibody titer in the mouse serum was determined by ELISA.

The immunizing antigens for the second group of mice were DLL3 CHO-s cells and Fc-hDLL3 (ECD) (SEQ ID NO: 8), and immunizations were alternately performed with cell and protein antigens. Cell immunization was performed by injecting intraperitoneally into each mouse 0.1 mL of a cell suspension diluted with normal saline to a concentration of $10^8$/mL. For the Fc-hDLL3 (ECD) antigen, cross-immunizations were performed using the adjuvants TiterMax® Gold Adjuvant (Sigma Cat No. T2684) and Thermo Imject® Alum (Thermo Cat No. 77161). The ratio of the antigen to the adjuvant (TiterMax® Gold Adjuvant) was 1:1, and the ratio of the antigen to the adjuvant (Thermo Imject® Alum) was 3:1.

The dose for the first immunization was 50 µg/mouse/immunization, and the dose for boost immunization was 25 µg/mouse/immunization. The antibody titer in the mouse serum was determined by ELISA.

Spleen lymphocytes and the myeloma cells, Sp2/0 cells (ATCC® CRL-8287™), were fused by following an optimized electrofusion procedure to obtain hybridoma cells. Based on the growth density of hybridoma cells, the hybridoma culture supernatant was assayed using an ELISA method with binding to DLL3 protein and a FACS method with binding to DLL3 CHO-s cells. Clones that bind to human DLL3 protein, monkey DLL3 protein, and DLL3 CHO-s cells but do not bind to wild-type CHO-s cells were selected, cryopreserved, expanded, conserved, and subcloned one to two times in time until single cell clones were obtained. Through the screening experiment above, hybridoma clones mAb6, mAb98, and mAb110 were obtained.

The hybridoma clones were expanded, and RNA was extracted. Reverse transcription amplification (RT-PCR) was performed using a degenerate primer of mouse-Ig, and the variable region sequences of the antibodies were finally obtained.

The heavy chain variable region of mAb6 (SEQ ID NO: 12):

EVQLQQSGPVLVKPGASVKMSCKASGYTFTDYYMNWVKQSHGKSLEWIGIINP
YSGVTIYNHKFKGKATLTVDKSSNTAYMELNSLTSEDSAVYYCARQDSLLDYA
MDYWGQGTSVTVSS

The light chain variable region of mAb6 SEQ ID NO: 13):

DILMTQSPSSMSVSLGDTVSITCHASQGISGNMGWLQQKPGKSFKGLIYHGANL
EDGVPSRFSGSGSGADYSLTISSLESEDFADYYCVQYAQFPYTFGGGTKLEIK
The heavy chain variable region of mAb98 (SEQ ID NO: 14):
EVQLVESGGGLVKPGGSLKLSCAASGFTFSDYGIHWVRQAPEKGLEWVAYISS
GGYTIYYADTVKGRFTISRDNAKNTLFLQMTSLRSEDTAMYYCARGDYDDIFY
TMDYWGQGTSVTVSS

The light chain variable region of mAb98 (SEQ ID NO: 15):

DIQMTQTTSSLSASLGDRVTISCRASRDITKFLNWYQQKPDGTVKLLIYYTSRLH
SGVPSRFSGSGSGTDYSLTISNLDQEDIATYFCQQGNTLPWTFGGGTKLDIK

The heavy chain variable region of mAb110 (SEQ ID NO: 16):

EVQLQQSGPVLVKPGASVKMSCKTSGYTFTDYYMNWVKQSHGKSLEWIGIINP
YNGGTSYTQKFKGKATLTVDKSSSTAYMELNSLTSEDSTVYFCARHFYDSSYL
TYSTMDYWGQGTSVTVSS

The light chain variable region of mAb110 (SEQ ID NO: 17):

NIVMTQSPKSMSMSVGERVTLSCKASENVGIFVSWYQQKPKQSPKLLIYGASN
RYTGVPDRFTGSGSATDFTLTISSVQAEDLADYYCGQSHSYPLTFGTGTKLELK.

3.2. Protocol for screening immune repertoire for antibodies, and antibody sequences

Total RNA was extracted from spleen cells of immunized mice using the Trizol reagent and reverse-transcribed into cDNA. Degenerate primers were used to amplify the heavy and light chain variable region sequences of the antibodies, with the cDNA as a template, and the amplified fragments were constructed into an optimized phage display vector. The constructed vector was then introduced into an E. coli TG1 strain through electroporation to obtain a DLL3 immune library. The obtained DLL3 immune library was packaged into phages, and two runs of library screening were conducted using biotinylated human DLL3 protein. The single clones obtained from the screening were inoculated into 96-well plates for phage packaging. Phages that could bind to human DLL3 protein and monkey DLL3 protein were identified by ELISA, and the corresponding single clones were sequenced to finally obtain the variable region sequences of the anti-DLL3 antibodies.

The heavy chain variable region of mAb30 (SEQ ID NO: 18):

EVRLQQFGPVLVKPGASVKMSCKASGYTFTDYYMNWVKQSHGKSLEWIGIINP YNGDTSYNQKFRGKATLTVDKSSSTAYMELNSLTSEDSAVYYCARYHYGPHL EGYFDVWGTGTTLTVSS

The light chain variable region of mAb30 (SEQ ID NO: 19):

DIVMTQSPKSMSMSVGERVTLSCKASENVGYVSWYQQKPEQSPKLLIYGASNR YTGVPDRFTGSGSATDFTLTISSVQAEDLADYHCGQSYSYPLTFGSGTKLEIK.

Table 14. The CDRs of antibodies

| Antibody | Name | Sequence | Sequence No. |
|---|---|---|---|
| mAb6 | HCDR1 | DYYMN | SEQ ID NO: 20 |
| | HCDR2 | IINPYSGVTIYNHKFKG | SEQ ID NO: 21 |
| | HCDR3 | QDSLLDYAMDY | SEQ ID NO: 22 |
| | LCDR1 | HASQGISGNMG | SEQ ID NO: 23 |
| | LCDR2 | HGANLED | SEQ ID NO: 24 |
| | LCDR3 | VQYAQFPYT | SEQ ID NO: 25 |
| mAb98 | HCDR1 | DYGIH | SEQ ID NO: 26 |
| | HCDR2 | YISSGGYTIYY ADTVKG | SEQ ID NO: 27 |
| | HCDR3 | GDYDDIFYTMDY | SEQ ID NO: 28 |
| | LCDR1 | RASRDITKFLN | SEQ ID NO: 29 |
| | LCDR2 | YTSRLHS | SEQ ID NO: 30 |
| | LCDR3 | QQGNTLPWT | SEQ ID NO: 31 |
| mAb110 | HCDR1 | DYYMN | SEQ ID NO: 20 |
| | HCDR2 | IINPYNGGTSYTQKFKG | SEQ ID NO: 32 |
| | HCDR3 | HFYDSSYLTYSTMDY | SEQ ID NO: 33 |
| | LCDR1 | KASENVGIFVS | SEQ ID NO: 34 |
| | LCDR2 | GASNRYT | SEQ ID NO: 35 |
| | LCDR3 | GQSHSYPLT | SEQ ID NO: 36 |

(continued)

| Antibody | Name | Sequence | Sequence No. |
|---|---|---|---|
| mAb30 | HCDR1 | DYYMN | SEQ ID NO: 20 |
| | HCDR2 | IINPYNGDTSYNQKFRG | SEQ ID NO: 37 |
| | HCDR3 | YHYGPHLEGYFDV | SEQ ID NO: 38 |
| | LCDR1 | KASENVGYVS | SEQ ID NO: 39 |
| | LCDR2 | GASNRYT | SEQ ID NO: 35 |
| | LCDR3 | GQSYSYPLT | SEQ ID NO: 40 |
| Note: The amino acid residues of the CDRs of the VH/VL were determined and annotated using the Kabat numbering scheme. | | | |

The heavy chain variable regions and the light chain variable regions of the murine antibodies were cloned into pTT5 vector plasmids containing a human IgG1 heavy chain constant region set forth in SEQ ID NO: 41 and a κ light chain constant region set forth in SEQ ID NO: 42, and HEK293 cells were transfected with the resulting plasmids to obtain anti-DLL3 chimeric antibodies M6CHI, M98CHI, M110CHI, and P30CHI.

The heavy chain constant region of human IgG1 (SEQ ID NO: 41):

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQ
KSLSLSPGK

The light chain constant region of human κ (hκ) (SEQ ID NO: 42):

RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC.

**Example 4. Humanization of Anti-DLL3 Monoclonal Antibodies**

Through comparison with a Kabat human antibody heavy and light chain variable region germline gene database, heavy and light chain variable region germline genes with high homology were selected as templates, and the CDRs of the murine antibodies were grafted onto the corresponding human templates to form variable region sequences structured as FR1-CDR1-FR2-CDR2-FR3-CDR3-FR4. Certain amino acids in the variable regions were subjected to amino acid substitution, and the variable regions were recombined with constant regions (for example, the human IgG1 heavy chain constant region set forth in SEQ ID NO: 41 and the human κ light chain constant region set forth in SEQ ID NO: 42) to obtain full-length humanized antibodies.

**4-1. Humanization of antibody mAb6**

For the antibody mAb6, the human germline light chain templates were IGKV1-16*01 or IGKV3-20*02 and IGKJ4*01, and the human germline heavy chain templates were IGHV1-3*01, IGHV7-4-1*02, or IGHV3-73*01 and IGHJ6*01. Optionally, the amino acid residue(s) at position(s) 1, 36, 42, 43, 44, 46, 52, 56, 69, 71, 79, 85, and/or 94 in the light chain variable region of the humanized antibody were/was substituted; and/or the amino acid residue(s) at position(s) 1, 27, 30, 38, 43, 48, 67, 68, 69, 71, 73, 75, 76, 93, 56, 58, and/or 5 in the heavy chain variable region of the humanized antibody were/was substituted.

Table 15. Humanization templates and corresponding point mutations for the mAb6 murine antibody

| hu VL | | huVH | |
|---|---|---|---|
| VL1 | Graft (IGKV1-16*01) + F36L, S46G | VH1 | GraftIGHV1-3*01) + Q1E, R71V, T73K |
| VL2 | Graft(IGKV1-16*01) + F36L, S46G, T69A, F71Y | VH2 | Graft(IGHV1-3*01) + Q1E, I69L, R71V, T73K, S76N |
| VL3 | Graft(IGKV1-16*01) + F36L, A43S, P44F, S46G, T69A, F71Y | VH3 | Graft(IGHV1-3*01) + Q1E, M48I, V67A, I69L, R71V, T73K, S76N |
| VL4 | Graft(IGKV1-16*01) + F36L, S46G, T69A, F71Y, T85D | VH4 | Graft(IGHV1-3*01) + Q1E, Q43K, I69L, R71V, T73K, S76N |
| VL5 | Graft (IGKV1-16*01) | VH5 | Graft(IGHV7-4-1*02) + Q1E, L71V, T73K, F69L |
| VL6 | Graft(IGKV1-16*01) + F36L, S46G, T69A, F71Y, T85D +D56E | VH6 | Graft(IGHV7-4-1*02) + Q1E, F69L, L71V, T73K, V75S, S76N |
| VL7 | Graft(IGKV1-16*01) + F36L, S46G, T69A, F71Y, T85D +D56S | VH7 | Graft(IGHV3-73*01) + F27Y, S30T, I69L, R71V, D73K, T93A |
| VL8 | Graft(IGKV1-16*01) + F36L, S46G, T69A, F71Y, T85D +F94Y, D56E | VH8 | Graft(IGHV1-3 *01) |
| VL9 | Graft(IGKV1-16*01) + F36L, S46G, T69A, F71Y, T85D +F94Y, D1N, A52R, D56E | VH9 | Graft(IGHV7-4-1*02) |
| VL10 | Graft(IGKV3-20*02)+ Y36L, Q42K,L46G, T69A, F71Y, E79Q, V85D + D56E | VH10 | Graft(IGHV3-73*01) |
| VL11 | Graft(IGKV3-20*02) + D56E | VH11 | Graft(IGHV7-4-1*02) + Q1E, R38K, F69L, L71V, T73K, V75S, S76N |
| | | VH12 | Graft(IGHV7-4-1*02) + Q1E, V68A, F69L, L71V, T73K, V75S, S76N, |
| | | VH13 | Graft(IGHV7-4-1*02) + Q1E, F69L, L71V, T73K, V75S, S76N, R38K, V68A |
| | | VH14 | Graft(IGHV7-4-1*02) + Q1E, F69L, L71V, T73K, V75S, S76N, -V68A +I58T |
| | | VH15 | Graft(IGHV7-4-1*02) + Q1E, F69L, L71V, T73K, V75S, S76N, -V68A +V56S, V5Q |
| FR4 | IGKJ4*01 | FR4 | IGHJ6*01 |

Note: The amino acid positions in the table are numbered according to the Kabat numbering scheme. F36L means that the F at position 36, according to the Kabat numbering scheme, is mutated to L. The same applies hereinafter.

The sequences of the variable regions of the hu6 humanized antibodies obtained are as follows:

hu6 VH1 (SEQ ID NO: 43)

**E**VQLVQSGAEVKKPGASVKVSCKASGYTFT<u>DYYMN</u>WVRQAPGQRLEWMG<u>II NPYSGVTIYNHKFKG</u>RVTIT**V**D**K**SASTAYMELSSLRSEDTAVYYCAR<u>QDSLLD YAMDY</u>WGQGTTVTVSS

hu6 VH2 (SEQ ID NO: 44)

**E**VQLVQSGAEVKKPGASVKVSCKASGYTFT<u>DYYMN</u>WVRQAPGQRLEWMG<u>II NPYSGVTIYNHKFKGR</u>VT**L**T**V**D**K**SA**N**TAYMELSSLRSEDTAVYYCAR<u>QDSLLD YAMDY</u>WGQGTTVTVSS

hu6 VH3 (SEQ ID NO: 45)

**E**VQLVQSGAEVKKPGASVKVSCKASGYTFT<u>DYYMN</u>WVRQAPGQRLEW**I**G<u>IIN PYSGVTIYNHKFKGR</u>**A**T**L**T**V**D**K**SA**N**TAYMELSSLRSEDTAVYYCAR<u>QDSLLDY AMDY</u>WGQGTTVTVSS

hu6 VH4 (SEQ ID NO: 46)

**E**VQLVQSGAEVKKPGASVKVSCKASGYTFT<u>DYYMN</u>WVRQAPG**K**RLEWMG<u>II NPYSGVTIYNHKFKGR</u>VT**L**T**V**D**K**SA**N**TAYMELSSLRSEDTAVYYCAR<u>QDSLLD YAMDY</u>WGQGTTVTVSS

hu6 VH5 (SEQ ID NO: 47)

**E**VQLVQSGSELKKPGASVKVSCKASGYTFT<u>DYYMN</u>WVRQAPGQGLEWMG<u>IIN PYSGVTIYNHKFKGR</u>FV**L**S**V**D**K**SVSTAYLQISSLKAEDTAVYYCAR<u>QDSLLDY</u>

<u>AMDY</u>WGQGTTVTVSS

hu6 VH6 (SEQ ID NO: 48)

**E**VQLVQSGSELKKPGASVKVSCKASGYTFT<u>DYYMN</u>WVRQAPGQGLEWMG<u>IIN PYSGVTIYNHKFKGR</u>FV**L**S**V**D**K**S**SN**TAYLQISSLKAEDTAVYYCAR<u>QDSLLDY AMDY</u>WGQGTTVTVSS

hu6 VH7 (SEQ ID NO: 49)

**E**VQLVESGGGLVQPGGSLKLSCAASG**Y**TF**T**<u>DYYMN</u>WVRQASGKGLEWVG<u>IIN PYSGVTIYNHKFKGR</u>FT**L**S**V**D**K**SKNTAYLQMNSLKTEDTAVYYC**A**R<u>QDSLLD YAMDY</u>WGQGTTVTVSS

hu6VH8 (SEQ ID NO: 50) (Graft IGHV1-3*01)

**Q**VQLVQSGAEVKKPGASVKVSCKASGYTFT<u>DYYMN</u>WVRQAPGQRLEWMG<u>II NPYSGVTIYNHKFKGR</u>VTITRDTSASTAYMELSSLRSEDTAVYYCAR<u>QDSLLDY AMDY</u>WGQGTTVTVSS

hu6VH9 (SEQ ID NO: 51) (Graft IGHV7-4-1*02)

QVQLVQSGSELKKPGASVKVSCKASGYTFT<u>DYYMN</u>WVRQAPGQGLEWMG<u>IIN</u><u>PYSGVTIYNHKFKG</u>RFVFSLDTSVSTAYLQISSLKAEDTAVYYCAR<u>QDSLLDYA</u><u>MDY</u>WGQGTTVTVSS

hu6VH10 (SEQ ID NO: 52) (Graft IGHV3-73*01)

**E**VQLVESGGGLVQPGGSLKLSCAASGFTFS<u>DYYMN</u>WVRQASGKGLEWVG<u>IINP</u><u>YSGVTIYNHKFKG</u>RFTISRDDSKNTAYLQMNSLKTEDTAVYYCTR<u>QDSLLDYA</u><u>MDY</u>WGQGTTVTVSS

hu6VH11 (SEQ ID NO: 53)

**E**VQLVQSGSELKKPGASVKVSCKASGYTFT<u>DYYMN</u>WV**K**QAPGQGLEWMG<u>IIN</u><u>PYSGVTIYNHKFKG</u>RFV**L**S**V**D**K**S**SN**TAYLQISSLKAEDTAVYYCAR<u>QDSLLDY</u><u>AMDY</u>WGQGTTVTVSS

hu6VH12 (SEQ ID NO: 54)

**E**VQLVQSGSELKKPGASVKVSCKASGYTFT<u>DYYMN</u>WVRQAPGQGLEWMG<u>IIN</u><u>PYSGVTIYNHKFKG</u>RF**AL**S**V**D**K**S**SN**TAYLQISSLKAEDTAVYYCAR<u>QDSLLDY</u><u>AMDY</u>WGQGTTVTVSS

hu6VH13 (SEQ ID NO: 55)

**E**VQLVQSGSELKKPGASVKVSCKASGYTFT<u>DYYMN</u>WV**K**QAPGQGLEWMG<u>IIN</u><u>PYSGVTIYNHKFKG</u>RF**AL**S**V**D**K**S**SN**TAYLQISSLKAEDTAVYYCAR<u>QDSLLDY</u><u>AMDY</u>WGQGTTVTVSS

hu6VH14 (SEQ ID NO: 56)

**E**VQLVQSGSELKKPGASVKVSCKASGYTFT<u>DYYMN</u>WVRQAPGQGLEWMG<u>IIN</u><u>PYSGVT</u>**T**<u>YNHKFKG</u>RF**AL**S**V**D**K**S**SN**TAYLQISSLKAEDTAVYYCAR<u>QDSLLDY</u>

<u>AMDY</u>WGQGTTVTVSS

hu6VH15 (SEQ ID NO: 57)

**E**VQL**Q**QSGSELKKPGASVKVSCKASGYTFT<u>DYYMN</u>WVRQAPGQGLEWMG<u>IIN</u><u>PYSG</u>**S**<u>TIYNHKFKG</u>RF**AL**S**V**D**K**S**SN**TAYLQISSLKAEDTAVYYCAR<u>QDSLLDYA</u><u>MDY</u>WGQGTTVTVSS

hu6 VL1 (SEQ ID NO: 58)

DIQMTQSPSSLSASVGDRVTITC<u>HASQGISGNMGW</u>**L**QQKPGKAPK**G**LIY<u>HGAN</u><u>LED</u>GVPSRFSGSGSGTDFTLTISSLQPEDFATYYC<u>VQYAQFPYTF</u>GGGTKVEIK

hu6 VL2 (SEQ ID NO: 59)

DIQMTQSPSSLSASVGDRVTITC**HASQGISGNMGW<u>L</u>**QQKPGKAPK<u>**G**</u>LIYH**GAN
LEDG**VPSRFSGSGSG<u>**AD**</u><u>**Y**</u>TLTISSLQPEDFATYYC<u>**VQYAQFPYT**</u>FGGGTKVEIK

hu6 VL3 (SEQ ID NO: 60)

DIQMTQSPSSLSASVGDRVTITC**HASQGISGNMGW<u>L</u>**QQKPGK<u>**SF**</u>K<u>**G**</u>LIYH**GAN
LEDG**VPSRFSGSGSG<u>**AD**</u><u>**Y**</u>TLTISSLQPEDFATYYC<u>**VQYAQFPYT**</u>FGGGTKVEIK

hu6 VL4 (SEQ ID NO: 61)

DIQMTQSPSSLSASVGDRVTITC**HASQGISGNMGW<u>L</u>**QQKPGKAPK<u>**G**</u>LIYH**GAN
LEDG**VPSRFSGSGSG<u>**AD**</u><u>**Y**</u>TLTISSLQPEDFA<u>**D**</u>YYC<u>**VQYAQFPYT**</u>FGGGTKVEIK

hu6VL5 (SEQ ID NO: 62) (Graft IGKV1-16*01)

DIQMTQSPSSLSASVGDRVTITC**HASQGISGNMGW**FQQKPGKAPKSLIYH**GANL
E**<u>**D**</u>GVPSRFSGSGSGTDFTLTISSLQPEDFATYYC<u>**VQYAQFPYT**</u>FGGGTKVEIK

hu6VL6 (SEQ ID NO: 63)

DIQMTQSPSSLSASVGDRVTITC**HASQGISGNMGW<u>L</u>**QQKPGKAPK<u>**G**</u>LIYH**GAN
LEE**GVPSRFSGSGSG<u>**AD**</u><u>**Y**</u>TLTISSLQPEDFA<u>**D**</u>YYC<u>**VQYAQFPYT**</u>FGGGTKVEIK

hu6VL7 (SEQ ID NO: 64)

DIQMTQSPSSLSASVGDRVTITC**HASQGISGNMGW<u>L</u>**QQKPGKAPK<u>**G**</u>LIYH**GAN
LES**GVPSRFSGSGSG<u>**AD**</u><u>**Y**</u>TLTISSLQPEDFA<u>**D**</u>YYC<u>**VQYAQFPYT**</u>FGGGTKVEIK

hu6VL8 (SEQ ID NO: 65)

DIQMTQSPSSLSASVGDRVTITC**HASQGISGNMGW<u>L</u>**QQKPGKAPK<u>**G**</u>LIYH**GAN
LEE**GVPSRFSGSGSG<u>**AD**</u><u>**Y**</u>TLTISSLQPEDFA<u>**D**</u>YYC<u>**VQYAQ<u>Y</u>PYT**</u>FGGGTKVEIK

hu6VL9 (SEQ ID NO: 66)

<u>**N**</u>IQMTQSPSSLSASVGDRVTITC**HASQGISGNMGW<u>L</u>**QQKPGKAPK<u>**G**</u>LIYH**G<u>R</u>N
LEE**GVPSRFSGSGSG<u>**AD**</u><u>**Y**</u>TLTISSLQPEDFA<u>**D**</u>YYC<u>**VQYAQ<u>Y</u>PYT**</u>FGGGTKVEIK

hu6VL10 (SEQ ID NO: 67)
Graft (IGK V3-20*02) + Y36L, Q42K, L46G, T69A, F71Y, E79Q, V85D + D56E

EIVLTQSPATLSLSPGERATLSC**HASQGISGNMGW<u>L</u>**QQKPG<u>**K**</u>APR<u>**G**</u>LIYH**GANL
EE**GIPARFSGSGSG<u>**AD**</u><u>**Y**</u>TLTISRL<u>**Q**</u>PEDFA<u>**D**</u>YYC<u>**VQYAQFPYT**</u>FGGGTKVEIK

hu6VL11 (SEQ ID NO: 68) Graft (IGKV3-20*02) + D56E

EIVLTQSPATLSLSPGERATLSC<u>HASQGISGNMG</u>WYQQKPGQAPRLLIY<u>HGANL</u>
<u>E<b>E</b></u>GIPARFSGSGSGTDFTLTISRLEPEDFAVYYC<u>VQYAQFPYT</u>FGGGTKVEIK

Note: CDRs are single underlined, amino acid substitution sites are double underlined, and the rest are FR regions. The same applies hereinafter.

The CDRs of the humanized antibodies of mAb6 are as follows:

Table 16. The CDRs of the humanized antibodies of mAb6

| Variable region | CDR | Sequence | SEQ ID NO |
|---|---|---|---|
| hu6VH14 | HCDR2 | INPYSGVT**T**YNHKFKG | 69 |
| hu6VH15 | HCDR2 | INPYSG**S**TIYNHKFKG | 70 |
| hu6VL6/VL8/VL10/VL11 | LCDR2 | HGANLE**E** | 71 |
| hu6VL7 | LCDR2 | HGANLE**S** | 72 |
| hu6VL9 | LCDR2 | HG**R**NLE**E** | 73 |
| hu6VL8/VL9 | LCDR3 | VQYAQ**Y**PYT | 74 |

**3-2. Humanization of mAb98**

For the antibody mAb98, the human germline light chain templates were IGKV1-39*01 and IGKJ4*01, and the human germline heavy chain templates were IGHV3-7*01 and IGHJ6*01. Optionally, the amino acid residue(s) at position(s) 42, 44, and/or 71 in the light chain variable region of the humanized antibody were/was substituted; and/or the amino acid residue(s) at position(s) 34, 57, and/or 98 in the heavy chain variable region of the humanized antibody were/was substituted.

Table 17. Humanization templates and corresponding point mutations for the mAb98 murine antibody

| hu98**VL** | | hu98**VH** | |
|---|---|---|---|
| VL1 | Graft(IGKV1-39*01) | VH1 | Graft(IGHV3-7*01) |
| VL2 | Graft(IGKV1-39*01) + P44V, F71Y | VH2 | Graft(IGHV3-7*01) + D98E |
| VL3 | Graft(IGKV1-39*01) + K42G, P44V, F71Y | VH3 | Graft(IGHV3-7*01) + I34M, D98E |
| | | VH4 | Graft(IGHV3-7*01) + I57S, D98E |
| | | VH5 | Graft(IGHV3-7*01) + I34M, I57S, D98E |
| FR4 | IGKJ4*01 | FR4 | IGHJ6*01 |

The sequences of the variable regions of the hu98 humanized antibodies obtained are as follows:

hu98VL1 Graft (IGKV1-39*01) (SEQ ID NO: 75)

DIQMTQSPSSLSASVGDRVTITC<u>RASRDITKFLN</u>WYQQKPGKAPKLLIY<u>YTSRLH</u>
<u>S</u>GVPSRFSGSGSGTDFTLTISSLQPEDFATYYC<u>QQGNTLPWT</u>FGGGTKVEIK

hu98VL2 Graft (IGKV1-39*01) + P44V, F71Y (SEQ ID NO: 76)

DIQMTQSPSSLSASVGDRVTITC<u>RASRDITKFLN</u>WYQQKPGKA<u><b>V</b></u>KLLIY<u>YTSRL</u>
<u>H</u>SGVPSRFSGSGSGTD<u><b>Y</b></u>TLTISSLQPEDFATYYC<u>QQGNTLPWT</u>FGGGTKVEIK

hu98VL3 Graft (IGKV1-39*01) + K42G, P44V, F71Y (SEQ ID NO: 77)

DIQMTQSPSSLSASVGDRVTITC<u>RASRDITKFL</u>NWYQQKPG<u>G</u>A<u>V</u>KLLIY<u>YTSRL</u><u>HS</u>GVPSRFSGSGSGTD<u>Y</u>TLTISSLQPEDFATYYC<u>QQGNTLPWT</u>FGGGTKVEIK

hu98VH1 Graft (IGHV3-7*01) (SEQ ID NO: 78)

EVQLVESGGGLVQPGGSLRLSCAASGFTFS<u>DYGIH</u>WVRQAPGKGLEWVA<u>YISS</u><u>GGYTIYYADTVKG</u>RFTISRDNAKNSLYLQMNSLRAEDTAVYYCAR<u>GDYDDIFY</u><u>TMDY</u>WGQGTTVTVSS

hu98VH2 Graft (IGHV3-7*01) + D98E (SEQ ID NO: 79)

EVQLVESGGGLVQPGGSLRLSCAASGFTFS<u>DYGIH</u>WVRQAPGKGLEWVA<u>YISS</u><u>GGYTIYYADTVKG</u>RFTISRDNAKNSLYLQMNSLRAEDTAVYYCAR<u>GDY**E**DIFY</u><u>TMDY</u>WGQGTTVTVSS

hu98VH3 (SEQ ID NO: 80)

EVQLVESGGGLVQPGGSLRLSCAASGFTFS<u>DYG**M**H</u>WVRQAPGKGLEWVA<u>YIS</u><u>SGGYTIYYADTVKG</u>RFTISRDNAKNSLYLQMNSLRAEDTAVYYCAR<u>GDY**E**DIF</u><u>YTMDY</u>WGQGTTVTVSS

hu98VH4 (SEQ ID NO: 81)

EVQLVESGGGLVQPGGSLRLSCAASGFTFS<u>DYGIH</u>WVRQAPGKGLEWVA<u>YISS</u><u>GGYT**S**YYADTVKG</u>RFTISRDNAKNSLYLQMNSLRAEDTAVYYCAR<u>GDY**E**DIF</u><u>YTMDY</u>WGQGTTVTVSS

hu98VH5 (SEQ ID NO: 82)

EVQLVESGGGLVQPGGSLRLSCAASGFTFS<u>DYG**M**H</u>WVRQAPGKGLEWVA<u>YIS</u><u>SGGYT**S**YYADTVKG</u>RFTISRDNAKNSLYLQMNSLRAEDTAVYYCAR<u>GDY**E**DIF</u><u>YTMDY</u>WGQGTTVTVSS.

Table 18. The CDRs of hu98

| Variable region | CDR | Sequence | SEQ ID NO |
|---|---|---|---|
| hu98VH2/VH3/VH4/VH5 | HCDR3 | GDY**E**DIFYTMDY | 83 |
| hu98VH3/VH5 | HCDR1 | DYG**M**H | 84 |
| hu98VH4/VH5 | HCDR2 | YISSGGYT**S**YYADTVKG | 85 |

**3-3. Humanization of mAb110**

For the antibody mAb110, the human germline light chain templates were IGKV4-1*01 and IGKJ2*01, and the human germline heavy chain templates were IGHV1-3*01 and IGHJ6*01. Optionally, the amino acid residue(s) at position(s) 1, 9, 43, 68, and/or 85 in the light chain variable region of the humanized antibody were/was substituted; and/or the amino acid residue(s) at position(s) 1, 24, 44, 71, 73, 54, and/or 55 in the heavy chain variable region of the humanized antibody were/was substituted.

Table 19. Humanization templates and corresponding point mutations for the mAb110 murine antibody

| hu110VL | | hu110VH | |
|---------|---|---------|---|
| VL1 | Graft(IGKV4-1*01) + P43S, G68A | VH1 | Graft(IGHV1-3*01) + R71V, T73K + Q1E |
| VL2 | Graft(IGKV4-1*01) + D1N, D9K, P43S, G68A, V85D | VH2 | Graft(IGHV1-3*01) + A24T, R44S, R71V, T73K + Q1E |
| VL3 | Graft(IGKV4-1*01) | VH3 | Graft(IGHV1-3*01) + A24T, R44S, R71V, T73K + Q1E, N54Q |
| | | VH4 | Graft(IGHV1-3*01) + A24T, R44S, R71V, T73K + Q1E, N54S |
| | | VH5 | Graft(IGHV1-3*01) + A24T, R44S, R71V, T73K + Q1E, N54T |
| | | VH6 | Graft(IGHV1-3*01) + A24T, R44S, R71V, T73K + Q1E, G55V |
| | | VH7 | Graft(IGHV1-3*01) |
| FR4 | IGKJ2*01 | FR4 | IGHJ6*01 |

The sequences of the variable regions of the hu110 humanized antibodies obtained are as follows:

hu110VL1 Graft (IGKV4-1*01) + P43S, G68A (SEQ ID NO: 86)

DIVMTQSPDSLAVSLGERATINC**KASENVGIFVS**WYQQKPGQ**S**PKLLIY**GASNR
YT**GVPDRFSGSGS**A**TDFTLTISSLQAEDVAVYYC**GQSHSYPL**TFGQGTKLEIK

hu110VL2 Graft (IGKV4-1*01) + D1N, D9K, P43S, G68A, V85D (SEQ ID NO: 87)

**N**IVMTQSP**K**SLAVSLGERATINC**KASENVGIFVS**WYQQKPGQ**S**PKLLIY**GASNR
YT**GVPDRFSGSGS**A**TDFTLTISSLQAEDVA**D**YYC**GQSHSYPL**TFGQGTKLEIK

hu110VL3 Graft (IGKV4-1*01): (SEQ ID NO: 88)

DIVMTQSPDSLAVSLGERATINC**KASENVGIFVS**WYQQKPGQPPKLLIY**GASNR
YT**GVPDRFSGSGSGTDFTLTISSLQAEDVAVYYC**GQSHSYPL**TFGQGTKLEIK

hu110VH1 Graft (IGHV1-3*01) + R71V, T73K + Q1E (SEQ ID NO: 89)

**E**VQLVQSGAEVKKPGASVKVSCKASGYTFT**DYYMN**WVRQAPGQRLEWMG**II
NPYNGGT**SYTQKFKGRVTIT**V**D**K**SASTAYMELSSLRSEDTAVYYCAR**HFYDSS

**YLTYSTMDY**WGQGTTVTVSS

hu110VH2 Graft (IGHV1-3*01) + A24T, R44S, R71V, T73K + Q1E (SEQ ID NO: 90)

**E**VQLVQSGAEVKKPGASVKVSCK**T**SGYTFT**DYYMN**WVRQAPGQ**S**LEWMG**II
N
PYNGGT**SYTQKFKGRVTIT**V**D**K**SASTAYMELSSLRSEDTAVYYCAR**HFYDSSY
LTYSTMDY**WGQGTTVTVSS

hu110VH3 Graft (IGHV1-3*01) + A24T, R44S, R71V, T73K + Q1E, N54Q (SEQ ID NO: 91)

EVQLVQSGAEVKKPGASVKVSCKTSGYTFTDYYMNWVRQAPGQSLEWMGIINPYQGGTSYTQKFKGRVTITVDKSASTAYMELSSLRSEDTAVYYCARHFYDSSYLTYSTMDYWGQGTTVTVSS

hu110VH4 Graft (IGHV1-3*01) + A24T, R44S, R71V, T73K + Q1E, N54S (SEQ ID NO: 92)

EVQLVQSGAEVKKPGASVKVSCKTSGYTFTDYYMNWVRQAPGQSLEWMGIINPYSGGTSYTQKFKGRVTITVDKSASTAYMELSSLRSEDTAVYYCARHFYDSSYLTYSTMDYWGQGTTVTVSS

hu110VH5 Graft (IGHV1-3*01) + A24T, R44S, R71V, T73K + Q1E, N54T (SEQ ID NO: 93)

EVQLVQSGAEVKKPGASVKVSCKTSGYTFTDYYMNWVRQAPGQSLEWMGIINPYTGGTSYTQKFKGRVTITVDKSASTAYMELSSLRSEDTAVYYCARHFYDSSYLTYSTMDYWGQGTTVTVSS

hu110VH6 Graft (IGHV1-3*01) + A24T, R44S, R71V, T73K + Q1E, G55V (SEQ ID NO: 94)

EVQLVQSGAEVKKPGASVKVSCKTSGYTFTDYYMNWVRQAPGQSLEWMGIINPYNVGTSYTQKFKGRVTITVDKSASTAYMELSSLRSEDTAVYYCARHFYDSSYLTYSTMDYWGQGTTVTVSS

hu110VH7 Graft (IGHV1-3*01) (SEQ ID NO: 95)

QVQLVQSGAEVKKPGASVKVSCKASGYTFTDYYMNWVRQAPGQRLEWMGIINPYNGGTSYTQKFKGRVTITRDTSASTAYMELSSLRSEDTAVYYCARHFYDSSYLTYSTMDYWGQGTTVTVSS.

Table 20. The CDRs of hu110

| Variable region | CDR | Sequence | Sequence No. |
|---|---|---|---|
| hu110VH3 | HCDR2 | IINPY**Q**GGTSYTQKFKG | 96 |
| hu110VH4 | HCDR2 | IINPY**S**GGTSYTQKFKG | 97 |
| hu110VH5 | HCDR2 | IINPY**T**GGTSYTQKFKG | 98 |
| hu110VH6 | HCDR2 | IINPYN**V**GTSYTQKFKG | 99 |

**3-4. Humanization of mAb30**

For the antibody mAb30, the human germline light chain templates were IGKV4-1*01 or IGKV1-39*01 and IGKJ2*01, and the human germline heavy chain templates were IGHV1-3*01 and IGHJ6*01. Optionally, the amino acid residue(s) at position(s) 9, 43, 60, 68, 87, and/or 100 in the light chain variable region of the humanized antibody were/was substituted; and/or the amino acid residue(s) at position(s) 1, 3, 5, 7, 9, 10, 12, 40, 44, 41, 48, 67, 69, 71, 73, 75, 83, 54, and/or 55 in the heavy chain variable region of the humanized antibody were/was substituted.

Table 21. Humanization templates and corresponding point mutations for the mAb30 murine antibody

| hu30**VL** | | hu30**VH** | |
|---|---|---|---|
| VL1 | Graft(IGKV4-1*01) | VH1 | Graft(IGHV1-3*01) + R44S, R71V, T73K + Q1E |

(continued)

| hu30**VL** | | hu30**VH** | |
|---|---|---|---|
| VL2 | Graft(IGKV4-1*01) + G68A, Y87H | VH2 | Graft(IGHV1-3*01) + Q3R, R44S, I69L, R71V, T73K+ Q1E |
| VL3 | Graft(IGKV4-1*01) + D9K, P43S, G68A, Y87H | VH3 | Graft(IGHV1-3*01) + Q3R, R44S, I69L, R71V, T73K + Q1E, N54Q |
| VL4 | Graft(IGKV1-39*01) | VH4 | Graft(IGHV1-3*01) + Q3R, R44S, I69L, R71V, T73K + Q1E, N54S |
| VL5 | Graft(IGKV1-39*01) + A43S, S60D, Q100S | VH5 | Graft(IGHV1-3*01) + Q3R, R44S, I69L, R71V, T73K + Q1E, N54T |
| | | VH6 | Graft(IGHV1-3*01) + Q3R, R44S, I69L, R71V, T73K + Q1E, G55V |
| | | VH7 | Graft(IGHV1-3*01) + Q3R, V5Q, A9P, E10V, K12V, R44S, I69L, R71V, T73K + Q1E |
| | | VH8 | Graft(IGHV1-3*01) + Q3R, S7F, E10V, R44S, I69L, R71V, T73K, A75S + Q1E, N54T |
| | | VH9 | Graft(IGHV1-3*01) + Q3R, R44S, M48I, V67A, I69L, R71V, T73K + Q1E, N54T |
| | | VH10 | Graft(IGHV1-3*01) + Q3R, A40S, P41H, R44S, I69L, R71V, T73K + Q1E, N54T |
| | | VH11 | Graft(IGHV1-3*01) + Q3R, S7F, E10V, R44S, I69L, R71V, T73K, A75S, R83T + Q1E, N54T |
| | | VH12 | Graft(IGHV1-3 *01) |
| FR4 | IGKJ2*01 | FR4 | IGHJ6*01 |

The sequences of the variable regions of the hu30 humanized antibodies obtained are as follows:

hu30VL1 Graft (IGKV4-1*01) (SEQ ID NO: 100)

DIVMTQSPDSLAVSLGERATINCKASENVGYVSWYQQKPGQPPKLLIYGASNR
YTGVPDRFSGSGSGTDFTLTISSLQAEDVAVYYCGQSYSYPLTFGQGTKLEIK

hu30VL2 Graft (IGKV4-1*01) + G68A, Y87H (SEQ ID NO: 101)

DIVMTQSPDSLAVSLGERATINCKASENVGYVSWYQQKPGQPPKLLIYGASNR
YTGVPDRFSGSGS**A**TDFTLTISSLQAEDVAVY**H**CGQSYSYPLTFGQGTKLEIK

hu30VL3 Graft (IGKV4-1*01) + D9K, P43S, G68A, Y87H (SEQ ID NO: 102)

DIVMTQSP**K**SLAVSLGERATINCKASENVGYVSWYQQKPGQ**S**PKLLIYGASNR
YTGVPDRFSGSGS**A**TDFTLTISSLQAEDVAVY**H**CGQSYSYPLTFGQGTKLEIK

hu30VL4 Graft (IGKV1-39*01) (SEQ ID NO: 103)

DIQMTQSPSSLSASVGDRVTITCKASENVGYVSWYQQKPGKAPKLLIYGASNR
YTGVPSRFSGSGSGTDFTLTISSLQPEDFATYYCGQSYSYPLTFGQGTKLEIK

hu30VL5 Graft (IGKV1-39*01) + A43S, S60D, Q100S (SEQ ID NO: 104)

DIQMTQSPSSLSASVGDRVTITC<u>KASENVGYVS</u>WYQQKPGK<u>S</u>PKLLIY<u>GASNRY</u><br>
<u>T</u>GVP<u>D</u>RFSGSGSGTDFTLTISSLQPEDFATYYC<u>GQSYSYPLT</u>FG<u>S</u>GTKLEIK

hu30VH1 Graft (IGHV1-3*01) + R44S, R71V, T73K + Q1E (SEQ ID NO: 105)

<u>E</u>VQLVQSGAEVKKPGASVKVSCKASGYTFT<u>DYYMN</u>WVRQAPGQ<u>S</u>LEWMG<u>IIN</u><br>
<u>PYNGDTSYNQKFRG</u>RVTIT<u>V</u>D<u>K</u>SASTAYMELSSLRSEDTAVYYCAR<u>YHYGPHL</u><br>
<u>EGYFD</u>VWGQGTTVTVSS

hu30VH2 Graft (IGHV1-3*01) + Q3R, R44S, I69L, R71V, T73K + Q1E (SEQ ID NO: 106)

<u>E</u>V<u>R</u>LVQSGAEVKKPGASVKVSCKASGYTFT<u>DYYMN</u>WVRQAPGQ<u>S</u>LEWMG<u>IIN</u><br>
<u>PYNGDTSYNQKFRG</u>RVT<u>L</u>T<u>V</u>D<u>K</u>SASTAYMELSSLRSEDTAVYYCAR<u>YHYGPH</u><br>
<u>LEGYFD</u>VWGQGTTVTVSS

hu30VH3 Graft (IGHV1-3*01) + Q3R, R44S, I69L, R71V, T73K + Q1E, N54Q (SEQ ID NO: 107)

<u>E</u>V<u>R</u>LVQSGAEVKKPGASVKVSCKASGYTFT<u>DYYMN</u>WVRQAPGQ<u>S</u>LEWMG<u>IIN</u><br>
<u>PY<u>Q</u>GDTSYNQKFRG</u>RVT<u>L</u>T<u>V</u>D<u>K</u>SASTAYMELSSLRSEDTAVYYCAR<u>YHYGPH</u>

<u>LEGYFD</u>VWGQGTTVTVSS

hu30VH4 Graft (IGHV1-3*01) + Q3R, R44S, I69L, R71V, T73K + Q1E, N54S (SEQ ID NO: 108)

<u>E</u>V<u>R</u>LVQSGAEVKKPGASVKVSCKASGYTFT<u>DYYMN</u>WVRQAPGQ<u>S</u>LEWMG<u>IIN</u><br>
<u>PY<u>S</u>GDTSYNQKFRG</u>RVT<u>L</u>T<u>V</u>D<u>K</u>SASTAYMELSSLRSEDTAVYYCAR<u>YHYGPHL</u><br>
<u>EGYFD</u>VWGQGTTVTVSS

hu30VH5 Graft (IGHV1-3*01) + Q3R, R44S, I69L, R71V, T73K + Q1E, N54T (SEQ ID NO: 109)

<u>E</u>V<u>R</u>LVQSGAEVKKPGASVKVSCKASGYTFT<u>DYYMN</u>WVRQAPGQ<u>S</u>LEWMG<u>IIN</u><br>
<u>PY<u>T</u>GDTSYNQKFRG</u>RVT<u>L</u>T<u>V</u>D<u>K</u>SASTAYMELSSLRSEDTAVYYCAR<u>YHYGPH</u><br>
<u>LEGYFD</u>VWGQGTTVTVSS

hu30VH6 Graft (IGHV1-3*01) + Q3R, R44S, I69L, R71V, T73K + Q1E, G55V (SEQ ID NO: 110)

<u>E</u>V<u>R</u>LVQSGAEVKKPGASVKVSCKASGYTFT<u>DYYMN</u>WVRQAPGQ<u>S</u>LEWMG<u>IIN</u><br>
<u>PYN<u>V</u>DTSYNQKFRG</u>RVT<u>L</u>T<u>V</u>D<u>K</u>SASTAYMELSSLRSEDTAVYYCAR<u>YHYGPH</u><br>
<u>LEGYFD</u>VWGQGTTVTVSS

hu30VH7 Graft (IGHV1-3*01) + Q3R, V5Q, A9P, E10V, K12V, R44S, I69L, R71V, T73K + Q1E (SEQ ID NO: 111)

<u>E</u>V<u>R</u>L<u>Q</u>QSG<u>PV</u>V<u>V</u>KPGASVKVSCKASGYTFT<u>DYYMN</u>WVRQAPGQ<u>S</u>LEWMG<u>I</u><br>
<u>I</u>NPYNGDTSYNQKFRG</u>RVT<u>L</u>T<u>V</u>D<u>K</u>SASTAYMELSSLRSEDTAVYYCAR<u>YHYGP</u><br>
<u>HLEGYFD</u>VWGQGTTVTVSS

hu30VH8 Graft (IGHV1-3*01) + Q3R, S7F, E10V, R44S, I69L, R71V, T73K, A75S + Q1E, N54T (SEQ ID NO: 112)

**E**V**R**LVQ**F**GA**V**VKKPGASVKVSCKASGYTFT<u>DYYMN</u>WVRQAPGQ**S**LEWMG<u>II</u> <u>NPY</u>**T**<u>GDTSYNQKFRG</u>RVT**L**T**V**D**K**S**S**STAYMELSSLRSEDTAVYYCAR<u>YHYGP</u> <u>HLEGYFD</u>VWGQGTTVTVSS

hu30VH9 Graft (IGHV1-3*01) + Q3R, R44S, M48I, V67A, I69L, R71V, T73K + Q1E, N54T (SEQ ID NO: 113)

**E**V**R**LVQSGAEVKKPGASVKVSCKASGYTFT<u>DYYMN</u>WVRQAPGQ**S**LEW**I**G<u>II</u> <u>N</u> <u>PY</u>**T**<u>GDTSYNQKFRG</u>R**A**T**L**T**V**D**K**SASTAYMELSSLRSEDTAVYYCAR<u>YHYGPH</u> <u>LEGYFD</u>VWGQGTTVTVSS

hu30VH10 Graft (IGHV1-3*01) + Q3R, A40S, P41H, R44S, I69L, R71V, T73K + Q1E, N54T (SEQ ID NO: 114)

**E**V**R**LVQSGAEVKKPGASVKVSCKASGYTFT<u>DYYMN</u>WVRQ**SH**GQ**S**LEWMG<u>II</u> <u>NPY</u>**T**<u>GDTSYNQKFRG</u>RVT**L**T**V**D**K**SASTAYMELSSLRSEDTAVYYCAR<u>YHYGP</u> <u>HLEGYFD</u>VWGQGTTVTVSS

hu30VH11 Graft (IGHV1-3*01) + Q3R, S7F, E10V, R44S, I69L, R71V, T73K, A75S, R83T + Q1E, N54T (SEQ ID NO: 115)

**E**V**R**LVQ**F**GA**V**VKKPGASVKVSCKASGYTFT<u>DYYMN</u>WVRQAPGQ**S**LEWMG<u>II</u> <u>NPY</u>**T**<u>GDTSYNQKFRG</u>RVT**L**T**V**D**K**S**S**STAYMELSSL**T**SEDTAVYYCAR<u>YHYGP</u> <u>HLEGYFD</u>VWGQGTTVTVSS

hu30VH12 Graft (IGHV1-3*01) (SEQ ID NO: 116)

QVQLVQSGAEVKKPGASVKVSCKASGYTFT<u>DYYMN</u>WVRQAPGQRLEWMG<u>II</u> <u>NPYN</u><u>GDTSYNQKFRG</u>RVTITRDTSASTAYMELSSLRSEDTAVYYCAR<u>YHYGPH</u> <u>LEGYFD</u>VWGQGTTVTVSS.

Table 22. The CDRs of hu30

| Variable region | CDR | Sequence | Sequence No. |
|---|---|---|---|
| hu30VH3 | HCDR2 | IINPY**Q**GDTSYNQKFRG | SEQ ID NO: 117 |
| hu30VH4 | HCDR2 | IINPY**S**GDTSYNQKFRG | SEQ ID NO: 118 |
| hu30VH5/VH8/V H9/VH10/VH11 | HCDR2 | IINPY**T**GDTSYNQKFRG | SEQ ID NO: 119 |
| hu30VH6 | HCDR2 | IINPYN**V**DTSYNQKFRG | SEQ ID NO: 120 |

The heavy chain variable region and the light chain variable region of the humanized antibodies were recombined with a heavy chain constant region and a light chain constant region, respectively. The sequence of an exemplary heavy chain constant region is SEQ ID NO: 41, and the sequence of an exemplary light chain constant region is SEQ ID NO: 42. Full-length humanized antibodies were obtained. Variable region combinations for specific humanized antibodies are shown in the table below.

Table 23-1. Humanized antibodies of mAb6

| Variable region | VL1 | VL2 | VL3 | VL4 | VL5 |
|---|---|---|---|---|---|
| VH1 | hu6L1H1 | hu6L2H1 | hu6L3H1 | hu6L4H1 | hu6L5H1 |
| VH2 | hu6L1H2 | hu6L2H2 | hu6L3H2 | hu6L4H2 | hu6L5H2 |

(continued)

| Variable region | VL1 | VL2 | VL3 | VL4 | VL5 |
|---|---|---|---|---|---|
| VH3 | hu6L1H3 | hu6L2H3 | hu6L3H3 | hu6L4H3 | hu6L5H3 |
| VH4 | hu6L1H4 | hu6L2H4 | hu6L3H4 | hu6L4H4 | hu6L5H4 |
| VH5 | hu6L1H5 | hu6L2H5 | hu6L3H5 | hu6L4H5 | hu6L5H5 |
| VH6 | hu6L1H6 | hu6L2H6 | hu6L3H6 | hu6L4H6 | hu6L5H6 |
| VH7 | hu6L1H7 | hu6L2H7 | hu6L3H7 | hu6L4H7 | hu6L5H7 |
| VH8 | hu6L1H8 | hu6L2H8 | hu6L3H8 | hu6L4H8 | hu6L5H8 |
| VH9 | hu6L1H9 | hu6L2H9 | hu6L3H9 | hu6L4H9 | hu6L5H9 |
| VH10 | hu6L1H10 | hu6L2H10 | hu6L3H10 | hu6L4H10 | hu6L5H10 |
| VH11 | hu6L1H11 | hu6L2H11 | hu6L3H11 | hu6L4H11 | hu6L5H11 |
| VH12 | hu6L1H12 | hu6L2H12 | hu6L3H12 | hu6L4H12 | hu6L5H12 |
| VH13 | hu6L1H13 | hu6L2H13 | hu6L3H13 | hu6L4VH13 | hu6L5H13 |
| VH14 | hu6L1H14 | hu6L2H14 | hu6L3H14 | hu6L4VH14 | hu6L5H14 |
| VH15 | hu6L1H15 | hu6L2H15 | hu6L3H15 | hu6L4VH15 | hu6L5H15 |
| Note: hu6L1H1 indicates that the antibody comprises the heavy chain variable region hu6VH1 and the light chain variable region hu6VL1 and that the sequence of the heavy chain constant region is SEQ ID NO: 41 and the sequence of the light chain constant region is SEQ ID NO: 42. The same applies to the others. | | | | | |

Table 23-2. Humanized antibodies of mAb6

| Variable region | VL6 | VL7 | VL8 | VL9 | VL10 | VL11 |
|---|---|---|---|---|---|---|
| VH1 | hu6L6H1 | hu6L7H1 | hu6L8H1 | hu6L9H1 | hu6L10H1 | hu6L11H1 |
| VH2 | hu6L6H2 | hu6L7H2 | hu6L8H2 | hu6L9H2 | hu6L10H2 | hu6L11H2 |
| VH3 | hu6L6H3 | hu6L7H3 | hu6L8H3 | hu6L9H3 | hu6L10H3 | hu6L11H3 |
| VH4 | hu6L6H4 | hu6L7H4 | hu6L8H4 | hu6L9H4 | hu6L10H4 | hu6L11H4 |
| VH5 | hu6L6H5 | hu6L7H5 | hu6L8H5 | hu6L9H5 | hu6L10H5 | hu6L11H5 |
| VH6 | hu6L6H6 | hu6L7H6 | hu6L8H6 | hu6L9H6 | hu6L10H6 | hu6L11H6 |
| VH7 | hu6L6H7 | hu6L7H7 | hu6L8H7 | hu6L9H7 | hu6L10H7 | hu6L11H7 |
| VH8 | hu6L6H8 | hu6L7H8 | hu6L8H8 | hu6L9H8 | hu6L10H8 | hu6L11H8 |
| VH9 | hu6L6H9 | hu6L7H9 | hu6L8H9 | hu6L9H9 | hu6L10H9 | hu6L11H9 |
| VH10 | hu6L6H10 | hu6L7H10 | hu6L8H10 | hu6L9H10 | hu6L10H10 | hu6L11H10 |
| VH11 | hu6L6H11 | hu6L7H11 | hu6L8H11 | hu6L9H11 | hu6L10H11 | hu6L11H11 |
| VH12 | hu6L6H12 | hu6L7H12 | hu6L8H12 | hu6L9H12 | hu6L10H12 | hu6L11H12 |
| VH13 | hu6L6H13 | hu6L7H13 | hu6L8H13 | hu6L9VH13 | hu6L10H13 | hu6L11H13 |
| VH14 | hu6L6H14 | hu6L7H14 | hu6L8H14 | hu6L9VH14 | hu6L10H14 | hu6L11H14 |
| VH15 | hu6L6H15 | hu6LH15 | hu6L8H15 | hu6L9VH15 | hu6L10H15 | hu6L11H15 |

Table 23-3. Humanized antibodies of mAb98

| Variable region | VL1 | VL2 | VL3 |
|---|---|---|---|
| VH1 | hu98L1H1 | hu98L2H1 | hu98L3H1 |
| VH2 | hu98L1H2 | hu98L2H2 | hu98L3H2 |
| VH3 | hu98L1H3 | hu98L2H3 | hu98L3H3 |

(continued)

| Variable region | VL1 | VL2 | VL3 |
|---|---|---|---|
| VH4 | hu98L1H4 | hu98L2H4 | hu98L3H4 |
| VH5 | hu98L1H5 | hu98L2H5 | hu98L3H5 |

Note: hu98L1H1 indicates that the antibody comprises the heavy chain variable region hu98VH1 and the light chain variable region hu98VL1 and that the sequence of the heavy chain constant region is SEQ ID NO: 41 and the sequence of the light chain constant region is SEQ ID NO: 42. The same applies to the others.

Table 23-4. Humanized antibodies of mAb110

| Variable region | VL1 | VL2 | VL3 |
|---|---|---|---|
| VH1 | hu110L1H1 | hu110L2H1 | hu110L3H1 |
| VH2 | hu110L1H2 | hu110L2H2 | hu110L3H2 |
| VH3 | hu110L1H3 | hu110L2H3 | hu110L3H3 |
| VH4 | hu110L1H4 | hu110L2H4 | hu110L3H4 |
| VH5 | hu110L1H5 | hu110L2H5 | hu110L3H5 |
| VH6 | hu110L1H6 | hu110L2H6 | hu110L3H6 |
| VH7 | hu110L1H7 | hu110L2H7 | hu110L3H7 |

Note: hu110L1H1 indicates that the antibody comprises the heavy chain variable region hu110VH1 and the light chain variable region hu110VL1 and that the sequence of the heavy chain constant region is SEQ ID NO: 41 and the sequence of the light chain constant region is SEQ ID NO: 42. The same applies to the others.

Table 23-5. Humanized antibodies of mAb30

| Variable region | VL1 | VL2 | VL3 | VL4 | VL5 |
|---|---|---|---|---|---|
| VH1 | hu30L1H1 | hu30L2H1 | hu30L3H1 | hu30L4H1 | hu30L5H1 |
| VH2 | hu30L1H2 | hu30L2H2 | hu30L3H2 | hu30L4H2 | hu30L5H2 |
| VH3 | hu30L1H3 | hu30L2H3 | hu30L3H3 | hu30L4H3 | hu30L5H3 |
| VH4 | hu30L1H4 | hu30L2H4 | hu30L3H4 | hu30L4H4 | hu30L5H4 |
| VH5 | hu30L1H5 | hu30L2H5 | hu30L3H5 | hu30L4H5 | hu30L5H5 |
| VH6 | hu30L1H6 | hu30L2H6 | hu30L3H6 | hu30L4H6 | hu30L5H6 |
| VH7 | hu30L1H7 | hu30L2H7 | hu30L3H7 | hu30L4H7 | hu30L5H7 |
| VH8 | hu30L1H8 | hu30L2H8 | hu30L3H8 | hu30L4H8 | hu30L5H8 |
| VH9 | hu30L1H9 | hu30L2H9 | hu30L3H9 | hu30L4H9 | hu30L5H9 |
| VH10 | hu30L1H10 | hu30L2H10 | hu30L3H10 | hu30L4H10 | hu30L5H10 |
| VH11 | hu30L1H11 | hu30L2H11 | hu30L3H11 | hu30L4H11 | hu30L5H11 |
| VH12 | hu30L1H12 | hu30L2H12 | hu30L3H12 | hu30L4H12 | hu30L5H12 |

Note: hu30L1H1 indicates that the antibody comprises the heavy chain variable region hu30VH1 and the light chain variable region hu30VL1 and that the sequence of the heavy chain constant region is SEQ ID NO: 41 and the sequence of the light chain constant region is SEQ ID NO: 42. The same applies to the others.

The above antibodies were cloned, expressed, and purified, and through a protein binding assay (Test Example 3), a cell binding assay (Test Example 2), and Biacore (Test Example 1), humanized antibodies with good activity were finally selected. The heavy and light chain amino acid sequences of exemplary humanized antibodies are as follows:

The heavy chain of Hu6 (also referred to as hu6L4H12) (SEQ ID NO: 121):

EVQLVQSGSELKKPGASVKVSCKASGYTFTDYYMNWVRQAPGQGLEWMGIIN
PYSGVTIYNHKFKGRFALSVDKSSNTAYLQISSLKAEDTAVYYCARQDSLLDYA
MDYWGQGTTVTVSS*ASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
K*

The light chain of Hu6 (also referred to as hu6L4H12) (SEQ ID NO: 122):

DIQMTQSPSSLSASVGDRVTITCHASQGISGNMGWLQQKPGKAPKGLIYHGAN
LEDGVPSRFSGSGSGADYTLTISSLQPEDFADYYCVQYAQFPYTFGGGTKVEIK
*RTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC*

The heavy chain of Hu98 (also referred to as hu98L3H4) (SEQ ID NO: 123):

EVQLVESGGGLVQPGGSLRLSCAASGFTFSDYGIHWVRQAPGKGLEWVAYISS
GGYTSYYADTVKGRFTISRDNAKNSLYLQMNSLRAEDTAVYYCARGDYEDIF
YTMDYWGQGTTVTVSS*ASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQKSLSL
SPGK*

The light chain of Hu98 (also referred to as hu98L3H4) (SEQ ID NO: 124):

DIQMTQSPSSLSASVGDRVTITCRASRDITKFLNWYQQKPGGAVKLLIYYTSRL
HSGVPSRFSGSGSGTDYTLTISSLQPEDFATYYCQQGNTLPWTFGGGTKVEIK*RT
VAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC*

The heavy chain of Hu110 (also referred to as hu110L2H3) (SEQ ID NO: 125):

EVQLVQSGAEVKKPGASVKVSCKTSGYTFTDYYMNWVRQAPGQSLEWMGIIN
PYQGGTSYTQKFKGRVTITVDKSASTAYMELSSLRSEDTAVYYCARHFYDSSY

LTYSTMDYWGQGTTVTVSS*ASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEWESN GQPENNYKTTPPVLDSDGSFFLYSKLTVDKSRWQQGNVFSCSVMHEALHNHYTQKS LSLSPGK*

The light chain of Hu110 (also referred to as hu110L2H3) (SEQ ID NO: 126):

NIVMTQSPKSLAVSLGERATINCKASENVGIFVSWYQQKPGQSPKLLIYGASNR YTGVPDRFSGSGSATDFTLTISSLQAEDVADYYCGQSHSYPLTFGQGTKLEIK*RT VAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC*

The heavy chain of Hu30 (also referred to as hu30L1H5) (SEQ ID NO: 127):

EVRLVQSGAEVKKPGASVKVSCKASGYTFTDYYMNWVRQAPGQSLEWMGIIN PYTGDTSYNQKFRGRVTLTVDKSASTAYMELSSLRSEDTAVYYCARYHYGPHL EGYFDVWGQGTTVTVSS*ASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKA LPAPIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNHYTQKSL SLSPGK*

The light chain of Hu30 (also referred to as hu30L1H5) (SEQ ID NO: 128):

DIVMTQSPDSLAVSLGERATINCKASENVGYVSWYQQKPGQPPKLLIYGASNR YTGVPDRFSGSGSGTDFTLTISSLQAEDVAVYYCGQSYSYPLTFGQGTKLEIK*RT VAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC*

Note: the underlined parts in the sequences indicate the variable regions, and the italicized parts indicate the constant regions.

**Example 5. Preparation of Anti-DLL3-CD3 Bispecific Antibodies**

**5.1. CD3 antibodies used in present disclosure**

The CD3-binding moiety of the present disclosure may be derived from any suitable antibody. Specifically, S107E and I2C were used in the examples of the present disclosure, and their variable regions and CDR sequences are as follows:

Table 24. The CDRs of S107E

| Antibody | Name | Sequence | No. |
|---|---|---|---|
| S107E | HCDR1 | KYAMN | SEQ ID NO: 129 |
| | HCDR2 | RIRSKYNNYATYYADSVKD | SEQ ID NO: 130 |
| | HCDR3 | HGNFGN**E**YISYWAY | SEQ ID NO: 131 |
| | LCDR1 | GSSTGAVTSGNYPN | SEQ ID NO: 132 |
| | LCDR2 | GTKFLAP | SEQ ID NO: 133 |
| | LCDR3 | VLWYSNRWV | SEQ ID NO: 134 |
| I2C | HCDR1 | KYAMN | SEQ ID NO: 129 |
| | HCDR2 | RIRSKYNNYATYYADSVKD | SEQ ID NO: 130 |
| | HCDR3 | HGNFGNSYISYWAY | SEQ ID NO: 135 |
| | LCDR1 | GSSTGAVTSGNYPN | SEQ ID NO: 132 |
| | LCDR2 | GTKFLAP | SEQ ID NO: 133 |
| | LCDR3 | VLWYSNRWV | SEQ ID NO: 134 |

The variable region sequences of S107E are as follows:

> S107E-VH (SEQ ID NO: 136)

EVQLVESGGGLVQPGGSLKLSCAASGFTFN<u>KYAMN</u>WVRQAPGKGLEWVA<u>RIRSKYNNYATYYADSVKD</u>RFTISRDDSKNTAYLQMNNLKTEDTAVYYCVR<u>HGNFGNEYISYWAY</u>WGQGTLVTVSS

> S107E-VL (SEQ ID NO: 137)

QTVVTQEPSLTVSPGGTVTLTC<u>GSSTGAVTSGNYPN</u>WVQQKPGQAPRGLIG<u>GTKFLAP</u>GTPARFSGSLLGGKAALTLSGVQPEDEAEYYC<u>VLWYSNRWV</u>FGGGTKLTVL

The variable region sequences of I2C are as follows:

> I2C-VH (SEQ ID NO: 138)

EVQLVESGGGLVQPGGSLKLSCAASGFTFN<u>KYAMN</u>WVRQAPGKGLEWVA<u>RIRSKYNNYATYYADSVKD</u>RFTISRDDSKNTAYLQMNNLKTEDTAVYYCVR<u>HGNFGNSYISYWAY</u>WGQGTLVTVSS

> I2C-VL (SEQ ID NO: 137)

QTVVTQEPSLTVSPGGTVTLTC<u>GSSTGAVTSGNYPN</u>WVQQKPGQAPRGLIG<u>GTKFLAP</u>GTPARFSGSLLGGKAALTLSGVQPEDEAEYYC<u>VLWYSNRWV</u>FGGGTKLTVL

Note: CDRs are underlined.
> S107E-scFv (SEQ ID NO: 139):

EVQLVESGGGLVQPGGSLKLSCAASGFTFNKYAMNWVRQAPGKGLEWVARIR SKYNNYATYYADSVKDRFTISRDDSKNTAYLQMNNLKTEDTAVYYCVRHGNF

GNEYISYWAYWGQGTLVTVSS*GGGGSGGGGSGGGGS*QTVVTQEPSLTVSPGGT VTLTCGSSTGAVTSGNYPNWVQQKPGQAPRGLIGGTKFLAPGTPARFSGSLLG GKAALTLSGVQPEDEAEYYCVLWYSNRWVFGGGTKLTVL

> I2C-scFv (SEQ ID NO: 140):

EVQLVESGGGLVQPGGSLKLSCAASGFTFNKYAMNWVRQAPGKGLEWVARIR SKYNNYATYYADSVKDRFTISRDDSKNTAYLQMNNLKTEDTAVYYCVRHGNF GNSYISYWAYWGQGTLVTVSS*GGGGSGGGGSGGGGS*QTVVTQEPSLTVSPGGT VTLTCGSSTGAVTSGNYPNWVQQKPGQAPRGLIGGTKFLAPGTPARFSGSLLG GKAALTLSGVQPEDEAEYYCVLWYSNRWVFGGGTKLTVL

Note: Linkers are double underlined.

**5.2. Structures of DLL3-CD3 bispecific antibodies of present disclosure**

The structures of the DLL3-CD3 bispecific antibody molecules of the present disclosure are Format1, Format2, and Format3, and the specific structures are as follows:

**1. Format1** is an asymmetric structure Hot-Ig. An intact molecule has four chains in total, and the four chains are all different and are specifically as follows:

chain 1: VL (anti-DLL3)-linker 1-Obscurin;
chain 2: VH (anti-DLL3)-linker 1-Titin-IgG1Fc (Hole, L234A, L235A, Y349C, T366S, L368A, Y407V);
chain 3: VH (anti-CD3)-IgG1 (CH1)-IgG1Fc (Knob, L234A/L235A/S354C/T366W); and
chain 4: VL (anti-CD3)-hλ.

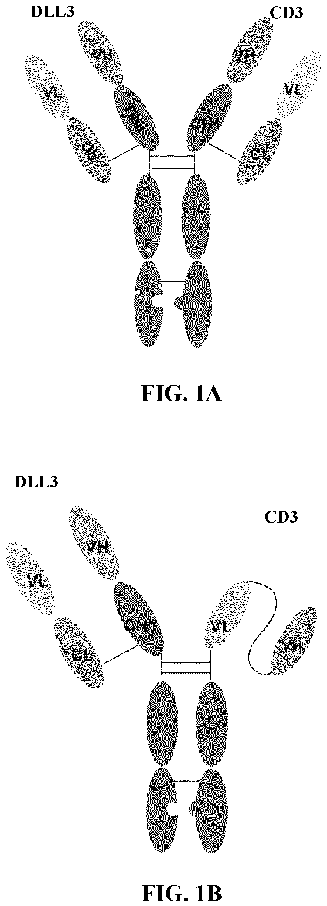

A schematic diagram of Format1 is shown in FIG. 1A, where Ob stands for Obscurin.
**2. Format2** is an asymmetric structure molecule Y body. An intact molecule contains 3 chains, and the 3 chains are specifically as follows:

chain 1: VL (anti-DLL3)-hx;
chain 2: VH (anti-DLL3-IgG1 (CH1)-IgG1Fc (Hole, L234A/L235A/Y349C/T366S/L368A/Y407V); and
chain 3: VH (anti-CD3)-linker 2-VL (anti-CD3)-linker 1-IgG1Fc (Knob, L234A/L235A/S354C/T366W).

A schematic diagram of Format2 is shown in FIG. 1B.
**3. Format3** is a diabody. An intact molecule contains two chains, and the two chains are specifically as follows:

chain 1: VL (anti-DLL3)-linker 3-VH (anti-CD3)-linker 4-Fc (Knob, L234A, L235A, G237A, Y349C, T366W); and
chain 2: VL (anti-CD3)-linker 5-VH (anti-DLL3)-linker 4-Fc (hole, L234A, L235A, G237A, S354C, T366S, L368A, Y407A).

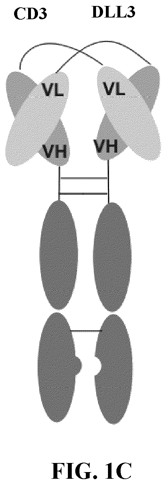

A schematic diagram of Format3 is shown in FIG. 1C.

The related sequences are as follows:

> IgG$_1$ (CH1) (SEQ ID NO: 141)

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSC

> hλ (SEQ ID NO: 142)

GQPKANPTVTLFPPSSEELQANKATLVCLISDFYPGAVTVAWKADGSPVKAGV
ETTKPSKQSNNKYAASSYLSLTPEQWKSHRSYSCQVTHEGSTVEKTVAPTEC

> IgG1Fc (Knob, L234A/L235A/S354C/T366W) (SEQ ID NO: 143)

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPCREEMTKNQVSLWCLVKGFYPSDIAVE
WESNGQPENNYKTTPPVLDSDGSFFLYSKLTVDKSRWQQGNVFSCSVMHEAL
HNHYTQKSLSLSPGK

> IgG1Fc (Hole, L234A, L235A, Y349C, T366S, L368A, Y407V) (SEQ ID NO: 144)

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSREEMTKNQVSLSCAVKGFYPSDIAVEW
ESNGQPENNYKTTPPVLDSDGSFFLVSKLTVDKSRWQQGNVFSCSVMHEALHN
HYTQKSLSLSPGK

> Obscurin (SEQ ID NO: 246)

SGAPRFLTRPKASVVSVGKDATLSCQIVGNPFPQVSWEKDKQPVTAGVRFRLA
QDGDLYRLKILDLQLSDSGQYVCRARNAHGEAFACLGLQVDAEA

> Titin (SEQ ID NO: 208)

GIPPKIECLPIDISIDEGKVLTVASAFTGEPTPEVTWSTGGRKIHSQEQGRFHIENT
DDSTTLTIKDVQKDGGLYTLTLRNEFGSDSATVNIHIRSI

> Fc (Knob, L234A, L235A, G237A, Y349C, T366W) (SEQ ID NO: 145)

CPPCPAPEAAGAPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAP
IEKTISKAKGQPREPQVCTLPPSREEMTKNQVSLWCLVKGFYPSDIAVEWESNG
QPENNYKTTPPVLDSDGSFFLYSKLTVDKSRWQQGNVFSCSVMHEALHNHYT
QKSLSLSPGK

> Fc (hole, L234A, L235A, G237A, S354C, T366S, L368A, Y407V) (SEQ ID NO: 146)

CPPCPAPE**AA**G**A**PSVFLFPPKPKDTLMISRTPEVTCVVVDVSHEDPEVKFNWYV

DGVEVHNAKTKPREEQYNSTYRVVSVLTVLHQDWLNGKEYKCKVSNKALPAP
IEKTISKAKGQPREPQVYTLPP**C**REEMTKNQVSL**SC**AVKGFYPSDIAVEWESNG
QPENNYKTTPPVLDSDGSFFL**V**SKLTVDKSRWQQGNVFSCSVMHEALHNHYT
QKSLSLSPGK

> Linker 1 (SEQ ID NO: 147)
GGGGS
> Linker 2 (SEQ ID NO: 148)
GGGGSGGGGSGGGGS
> Linker 3 (SEQ ID NO: 149)
GGGSGGGG
> Linker 4 (SEQ ID NO: 150)
G
> Linker 5 (SEQ ID NO: 151)
GGGGSGGGG.

Based on the amino acid sequences of humanized antibodies of mAb6, mAb98, mAb110, and mAb30, the following bispecific antibodies were constructed.

Table 25. The bispecific antibodies of the present disclosure

| No. | Chain 1 | Chain 2 | Chain 3 | Chain 4 |
|---|---|---|---|---|
| hu6-S107E-hot Ig | hu6VL-G4S-Obscurin | hu6VH-G4S-Titin-hole | S107EVH-CH1 -knob | CD3VL -hλ |
| Format 1 | SEQ ID NO: 152 | SEQ ID NO: 153 | SEQ ID NO: 154 | SEQ ID NO: 155 |
| hu6-I2C-hot Ig | hu6VL-G4S-Obscurin | hu6VH-G4S-Titin-hole | I2CVH-CH1-k nob | CD3VL -hλ |
| Format 1 | SEQ ID NO: 152 | SEQ ID NO: 153 | SEQ ID NO: 156 | SEQ ID NO: 155 |
| hu6-S107E-Y body | hu6VL-hκ | hu6VH-CH1-hole | S107E_scFv-k nob | |
| Format 2 | SEQ ID NO: 157 | SEQ ID NO: 158 | SEQ ID NO: 159 | |
| hu6-I2C-Y body | hu6VL-hκ | hu6VH-CH1-hole | I2C_scFv-knob | |
| Format 2 | SEQ ID NO: 157 | SEQ ID NO: 158 | SEQ ID NO: 160 | |
| hu6-S107E-diabody | hu6VL-S 107EVH-knob | CD3VL-hu6VH-hole | | |
| Format 3 | SEQ ID NO: 161 | SEQ ID NO: 162 | | |
| hu6-I2C-diabody | hu6VL-I2CVH-kn ob | CD3VL-hu6VH-hole | | |
| Format 3 | SEQ ID NO: 163 | SEQ ID NO: 162 | | |
| hu98-S107E-hot Ig | hu98VL-G4S-Obscurin | hu98VH-G4S-Titi n-hole | S107EVH-CH1 -knob | CD3VL -hλ |
| Format 1 | SEQ ID NO: 164 | SEQ ID NO: 165 | SEQ ID NO: 154 | SEQ ID NO: 155 |
| hu98-I2C-hot Ig | hu98VL-G4S-Obscurin | hu98VH-G4S-Titi n-hole | I2CVH-CH1-k nob | CD3VL -hλ |
| Format 1 | SEQ ID NO: 164 | SEQ ID NO: 165 | SEQ ID NO: 156 | SEQ ID NO: 155 |
| hu98-S107E-Y body | hu98VL-hκ | hu98VH-CH1-hol e | S107E_scFv-k nob | |
| Format 2 | SEQ ID NO: 166 | SEQ ID NO: 167 | SEQ ID NO: 159 | |

(continued)

| No. | Chain 1 | Chain 2 | Chain 3 | Chain 4 |
|---|---|---|---|---|
| hu98-I2C-Y body | hu98VL-hκ | hu98VH-CH1-hol e | I2C_scFv-knob | |
| Format 2 | SEQ ID NO: 166 | SEQ ID NO: 167 | SEQ ID NO: 160 | |
| hu98-S107E-diabody | hu98VL-S107EV H-knob | CD3VL-hu98VH-hole | | |
| Format 3 | SEQ ID NO: 168 | SEQ ID NO: 169 | | |
| hu98-I2C-diabody | hu98VL-12CVH-k nob | CD3VL-hu98VH-hole | | |
| Format 3 | SEQ ID NO: 170 | SEQ ID NO: 169 | | |
| hu110-S107E-hot Ig | hu110VL-G4S-Ob scurin | hu110VH-G4S-Tit in-hole | S107EVH-CH1 -knob | CD3VL -hλ |
| Format 1 | SEQ ID NO: 171 | SEQ ID NO: 172 | SEQ ID NO: 154 | SEQ ID NO: 155 |
| hu110-I2C-hot Ig | hu110VL-G4S-Ob scurin | hu110VH-G4S-Tit in-hole | I2CVH-CH1-k nob | CD3VL -hλ |
| Format 1 | SEQ ID NO: 171 | SEQ ID NO: 172 | SEQ ID NO: 156 | SEQ ID NO: 155 |
| hu110-S107E-Y body | hu110VL-hκ | hu110VH-CH1-ho le | S107E_scFv-k nob | |
| Format 2 | SEQ ID NO: 173 | SEQ ID NO: 174 | SEQ ID NO: 159 | |
| hu110-I2C-Y body | hu110VL-hκ | hu110VH-CH1-ho le | I2C_scFv-knob | |
| Format 2 | SEQ ID NO: 173 | SEQ ID NO: 174 | SEQ ID NO: 160 | |
| hu110-S107E-diabod y | hu110VL-S107EV H-knob | CD3VL-hu110VH -hole | | |
| Format 3 | SEQ ID NO: 175 | SEQ ID NO: 176 | | |
| hu110-I2C-diabody | hu110VL-I2CVH-knob | CD3VL-hu110VH -hole | | |
| Format 3 | SEQ ID NO: 177 | SEQ ID NO: 176 | | |
| hu30-S107E-hot Ig | hu30VL-G4S-Obs curin | hu30VH-G4S-Titi n-hole | S107EVH-CH1 -knob | CD3VL -hλ |
| Format 1 | SEQ ID NO: 178 | SEQ ID NO: 179 | SEQ ID NO: 154 | SEQ ID NO: 155 |
| hu30-I2C-hot Ig | hu30VL-G4S-Obs curin | hu30VH-G4S-Titi n-hole | I2CVH-CH1-k nob | CD3VL -hλ |
| Format 1 | SEQ ID NO: 178 | SEQ ID NO: 179 | SEQ ID NO: 156 | SEQ ID NO: 155 |
| hu30-S107E-Y body | hu30VL-hκ | hu30VH-CH1-hol e | S107E_scFv-k nob | |
| Format 2 | SEQ ID NO: 180 | SEQ ID NO: 181 | SEQ ID NO: 159 | |
| hu30-I2C-Y body | hu30VL-hκ | hu30VH-CH1-hol e | I2C_scFv-knob | |
| Format 2 | SEQ ID NO: 180 | SEQ ID NO: 181 | SEQ ID NO: 160 | |
| hu30-S107E-diabody | hu30VL-S107EV H-knob | CD3VL-hu30VH-hole | | |
| Format 3 | SEQ ID NO: 182 | SEQ ID NO: 183 | | |
| hu30-I2C-diabody | hu30VL-I2CVH-k nob | CD3VL-hu30VH-hole | | |
| Format 3 | SEQ ID NO: 184 | SEQ ID NO: 183 | | |
| hu6(D56E)-S107E-di abody | hu6VL (D56E) -S107EVH-knob | CD3VL-hu6VH-h ole | | |

(continued)

| No. | Chain 1 | Chain 2 | Chain 3 | Chain 4 |
|---|---|---|---|---|
| Format 3 | SEQ ID NO: 185 | SEQ ID NO: 162 | | |
| hu6(D56S)-S107E-diabody | hu6VL (D56S)-S107EVH-knob | CD3VL-hu6VH-hole | | |
| Format 3 | SEQ ID NO: 186 | SEQ ID NO: 162 | | |
| hu6(L8H14)-S107E-diabody | hu6VL8 -S107EVH-knob | CD3VL-hu6VH14-hole | | |
| Format 3 | SEQ ID NO: 187 | SEQ ID NO: 188 | | |
| hu6(L8H15)-S107E-diabody | hu6VL8 -S107EVH-knob | CD3VL-hu6VH15-hole | | |
| Format 3 | SEQ ID NO: 187 | SEQ ID NO: 189 | | |
| hu6(L9H14)-S107E-diabody | hu6VL9-S107EV ' H-knob | CD3VL-hu6VH14-hole | | |
| Format 3 | SEQ ID NO: 190 | SEQ ID NO: 188 | | |
| hu6(L9H15)-S107E-diabody | hu6VL9-S107EV H-knob | CD3VL-hu6VH15-hole | | |
| Format 3 | SEQ ID NO: 190 | SEQ ID NO: 189 | | |
| hu6(L10H14)-S107E-diabody | hu6VL10-S107EV H-knob | CD3VL-hu6VH14-hole | | |
| Format 3 | SEQ ID NO: 191 | SEQ ID NO: 188 | | |
| hu6(L10H15)-S107E-diabody | hu6VL10-S107EV H-knob | CD3VL-hu6VH15-hole | | |
| Format 3 | SEQ ID NO: 191 | SEQ ID NO: 189 | | |
| hu6(D56E)-I2C-diabody | hu6VL (D56E)-I2CVH-knob | CD3VL-hu6VH-hole | | |
| Format 3 | SEQ ID NO: 259 | SEQ ID NO: 162 | | |
| hu6(D56S)-I2C-diabody | hu6VL (D56S)-I2CVH-knob | CD3VL-hu6VH-hole | | |
| Format 3 | SEQ ID NO: 260 | SEQ ID NO: 162 | | |
| Note: hu6-S107E-hot Ig indicates that the molecule uses variable regions of mAb6 and its humanized antibodies as the DLL3-binding domain, uses a variable region of S107E as the CD3-binding domain, and uses hot Ig as the molecular structure. The same applies to the others. | | | | |

The amino acid sequences of the DLL3-CD3 bispecific antibodies of the present **disclosure** are as follows:

> hu6VL-G4S-Obscurin (SEQ ID NO: 152)

DIQMTQSPSSLSASVGDRVTITCHASQGISGNMGWLQQKPGKAPKGLIYHGAN
LEDGVPSRFSGSGSGADYTLTISSLQPEDFADYYCVQYAQFPYTFGGGTKVEIK
*GGGGS*SGAPRFLTRPKASVVSVGKDATLSCQIVGNPFPQVSWEKDKQPVTAGV
RFRLAQDGDLYRLKILDLQLSDSGQYVCRARNAHGEAFACLGLQVDAEA

> hu6VH-G4S-Titin-hole (SEQ ID NO: 153)

EVQLVQSGSELKKPGASVKVSCKASGYTFTDYYMNWVRQAPGQGLEWMGIIN PYSGVTIYNHKFKGRFALSVDKSSNTAYLQISSLKAEDTAVYYCARQDSLLDYA MDYWGQGTTVTVSS*GGGGS*GIPPKIECLPIDISIDEGKVLTVASAFTGEPTPEVT WSTGGRKIHSQEQGRFHIENTDDSTTLTIKDVQKQDGGLYTLTLRNEFGSDSAT VNIHIRSIDKTHTCPPCPAPEAAGGPSVFLFPPKPKDTLMISRTPEVTCVVVDVSH EDPEVKFNWYVDGVEVHNAKTKPREEQYNSTYRVVSVLTVLHQDWLNGKEY KCKVSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFSCS VMHEALHNHYTQKSLSLSPGK

> S107EVH-CH1-knob (SEQ ID NO: 154)

<u>EVQLVESGGGLVQPGGSLKLSCAASGFTFNKYAMNWVRQAPGKGLEWVARIR</u> <u>SKYNNYATYYADSVKDRFTISRDDSKNTAYLQMNNLKTEDTAVYYCVRHGNF</u> <u>GNEYISYWAYWGQGTLVTVSSASTKGPSVFPLAPSSKSTSGGTAALGCLVKDY</u> <u>FPEPVTVSWNSGALTSGVHTFPAVLQSSGLYSLSSVVTVPSSSLGTQTYICNVNH</u> <u>KPSNTKVDKKVEPKSC</u>DKTHTCPPCPAPE**AA**GGPSVFLFPPKPKDTLMISRTPEV TCVVVDVSHEDPEVKFNWYVDGVEVHNAKTKPREEQYNSTYRVVSVLTVLHQ DWLNGKEYKCKVSNKALPAPIEKTISKAKGQPREPQVYTLPP**C**REEMTKNQVS LW**C**LVKGFYPSDIAVEWESNGQPENNYKTTPPVLDSDGSFFLYSKLTVDKSRW QQGNVFSCSVMHEALHNHYTQKSLSLSPGK

> **CD3VL-h**λ (SEQ ID NO: 155)

<u>QTVVTQEPSLTVSPGGTVTLTCGSSTGAVTSGNYPNWVQQKPGQAPRGLIGGT</u> <u>KFLAPGTPARFSGSLLGGKAALTLSGVQPEDEAEYYCVLWYSNRWVFGGGTK</u> <u>LTVL</u>GQPKANPTVTLFPPSSEELQANKATLVCLISDFYPGAVTVAWKADGSPVK AGVETTKPSKQSNNKYAASSYLSLTPEQWKSHRSYSCQVTHEGSTVEKTVAPT EC

> I2CVH-CH1-knob (SEQ ID NO: 156)

<u>EVQLVESGGGLVQPGGSLKLSCAASGFTFNKYAMNWVRQAPGKGLEWVARIR</u> <u>SKYNNYATYYADSVKDRFTISRDDSKNTAYLQMNNLKTEDTAVYYCVRHGNF</u> <u>GNSYISYWAYWGQGTLVTVSS</u>ASTKGPSVFPLAPSSKSTSGGTAALGCLVKDY

FPEPVTVSWNSGALTSGVHTFPAVLQSSGLYSLSSVVTVPSSSLGTQTYICNVNH
KPSNTKVDKKVEPKSCDKTHTCPPCPAPEAAGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPCREEMTKNQVS
LWCLVKGFYPSDIAVEWESNGQPENNYKTTPPVLDSDGSFFLYSKLTVDKSRW
QQGNVFSCSVMHEALHNHYTQKSLSLSPGK

> hu6VL-hκ (SEQ ID NO: 157)

<u>DIQMTQSPSSLSASVGDRVTITCHASQGISGNMGWLQQKPGKAPKGLIYHGAN
LEDGVPSRFSGSGSGADYTLTISSLQPEDFADYYCVQYAQFPYTFGGGTKVEIK</u>
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

> hu6VH-CH1-hole (SEQ ID NO: 158)

<u>EVQLVQSGSELKKPGASVKVSCKASGYTFTDYYMNWVRQAPGQGLEWMGIIN
PYSGVTIYNHKFKGRFALSVDKSSNTAYLQISSLKAEDTAVYYCARQDSLLDYA
MDYWGQGTTVTVSS</u>ASTKGPSVFPLAPSSKSTSGGTAALGCLVKDYFPEPVTV
SWNSGALTSGVHTFPAVLQSSGLYSLSSVVTVPSSSLGTQTYICNVNHKPSNTK
VDKKVEPKSCDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVCTLPPSREEMTKNQVSLSCAV
KGFYPSDIAVEWESNGQPENNYKTTPPVLDSDGSFFLVSKLTVDKSRWQQGNV
FSCSVMHEALHNHYTQKSLSLSPGK

> S107E_scFv-knob (SEQ ID NO: 159)

<u>EVQLVESGGGLVQPGGSLKLSCAASGFTFNKYAMNWVRQAPGKGLEWVARIR
SKYNNYATYYADSVKDRFTISRDDSKNTAYLQMNNLKTEDTAVYYCVRHGNF
GNEYISYWAYWGQGTLVTVSS*GGGGSGGGGSGGGGS*QTVVTQEPSLTVSPGGT
VTLTCGSSTGAVTSGNYPNWVQQKPGQAPRGLIGGTKFLAPGTPARFSGSLLG
GKAALTLSGVQPEDEAEYYCVLWYSNRWVFGGGTKLTVL*GGGGS*</u>DKTHTCPP
CPAPEAAGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIE
KTISKAKGQPREPQVYTLPPCREEMTKNQVSLWCLVKGFYPSDIAVEWESNGQ
PENNYKTTPPVLDSDGSFFLYSKLTVDKSRWQQGNVFSCSVMHEALHNHYTQ
KSLSLSPGK

> I2C_scFv-knob (SEQ ID NO: 160)

EVQLVESGGGLVQPGGSLKLSCAASGFTFNKYAMNWVRQAPGKGLEWVARIR
SKYNNYATYYADSVKDRFTISRDDSKNTAYLQMNNLKTEDTAVYYCVRHGNF
GNSYISYWAYWGQGTLVTVSS*GGGGSGGGGSGGGGS*QTVVTQEPSLTVSPGGT

VTLTCGSSTGAVTSGNYPNWVQQKPGQAPRGLIGGTKFLAPGTPARFSGSLLG
GKAALTLSGVQPEDEAEYYCVLWYSNRWVFGGGTKLTVL*GGGGS*DKTHTCPP
CPAPEAAGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIE
KTISKAKGQPREPQVYTLPPCREEMTKNQVSLWCLVKGFYPSDIAVEWESNGQ
PENNYKTTPPVLDSDGSFFLYSKLTVDKSRWQQGNVFSCSVMHEALHNHYTQ
KSLSLSPGK

> hu6VL-S107EVH-knob (SEQ ID NO: 161)

DIQMTQSPSSLSASVGDRVTITCHASQGISGNMGWLQQKPGKAPKGLIYHGAN
LEDGVPSRFSGSGSGADYTLTISSLQPEDFADYYCVQYAQFPYTFGGGTKVEIK
*GGGSGGGG*EVQLVESGGGLVQPGGSLKLSCAASGFTFNKYAMNWVRQAPGK
GLEWVARIRSKYNNYATYYADSVKDRFTISRDDSKNTAYLQMNNLKTEDTAV
YYCVRHGNFGNEYISYWAYWGQGTLVTVSS*G*CPPCPAPEAAGAPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTLP
PSREEMTKNQVSLWCLVKGFYPSDIAVEWESNGQPENNYKTTPPVLDSDGSFF
LYSKLTVDKSRWQQGNVFSCSVMHEALHNHYTQKSLSLSPGK

> CD3VL-hu6VH-hole (SEQ ID NO: 162)

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVL*GGGGSGGGGG*EVQLVQSGSELKKPGASVKVSCKASGYTFTDYYMNWVR
QAPGQGLEWMGIINPYSGVTIYNHKFKGRFALSVDKSSNTAYLQISSLKAEDTA
VYYCARQDSLLDYAMDYWGQGTTVTVSS*G*CPPCPAPEAAGAPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPC
REEMTKNQVSLSCAVKGFYPSDIAVEWESNGQPENNYKTTPPVLDSDGSFFLVS
KLTVDKSRWQQGNVFSCSVMHEALHNHYTQKSLSLSPGK

> hu6VL-I2CVH-knob (SEQ ID NO: 163)

DIQMTQSPSSLSASVGDRVTITCHASQGISGNMGWLQQKPGKAPKGLIYHGAN
LEDGVPSRFSGSGSGADYTLTISSLQPEDFADYYCVQYAQFPYTFGGGTKVEIK
*GGGSGGGG*EVQLVESGGGLVQPGGSLKLSCAASGFTFNKYAMNWVRQAPGK
GLEWVARIRSKYNNYATYYADSVKDRFTISRDDSKNTAYLQMNNLKTEDTAV
YYCVRHGNFGNSYISYWAYWGQGTLVTVSS*G*CPPCPAPEAAGAPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTLP
PSREEMTKNQVSLWCLVKGFYPSDIAVEWESNGQPENNYKTTPPVLDSDGSFF

LYSKLTVDKSRWQQGNVFSCSVMHEALHNHYTQKSLSLSPGK

> hu98VL-G4S-Obscurin (SEQ ID NO: 164)

DIQMTQSPSSLSASVGDRVTITCRASRDITKFLNWYQQKPGGAVKLLIYYTSRL
HSGVPSRFSGSGSGTDYTLTISSLQPEDFATYYCQQGNTLPWTFGGGTKVEIK*G*
*GGGS*SGAPRFLTRPKASVVSVGKDATLSCQIVGNPFPQVSWEKDKQPVTAGVR
FRLAQDGDLYRLKILDLQLSDSGQYVCRARNAHGEAFACLGLQVDAEA

> hu98VH-G4S-Titin-hole (SEQ ID NO: 165)

EVQLVESGGGLVQPGGSLRLSCAASGFTFSDYGIHWVRQAPGKGLEWVAYISS
GGYTSYYADTVKGRFTISRDNAKNSLYLQMNSLRAEDTAVYYCARGDYEDIFY
TMDYWGQGTTVTVSS*GGGGS*GIPPKIECLPIDISIDEGKVLTVASAFTGEPTPEVT
WSTGGRKIHSQEQGRFHIENTDDSTTLTIKDVQKDGGLYTLTLRNEFGSDSAT
VNIHIRSIDKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVCTLPPSREEMTKNQVSLSCAVKGFY
PSDIAVEWESNGQPENNYKTTPPVLDSDGSFFLVSKLTVDKSRWQQGNVFSCS
VMHEALHNHYTQKSLSLSPGK

> hu98VL-hκ (SEQ ID NO: 166)

DIQMTQSPSSLSASVGDRVTITCRASRDITKFLNWYQQKPGGAVKLLIYYTSRL
HSGVPSRFSGSGSGTDYTLTISSLQPEDFATYYCQQGNTLPWTFGGGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

> hu98VH-CH1-hole (SEQ ID NO: 167)

EVQLVESGGGLVQPGGSLRLSCAASGFTFSDYGIHWVRQAPGKGLEWVAYISS
GGYTSYYADTVKGRFTISRDNAKNSLYLQMNSLRAEDTAVYYCARGDYEDIFY
TMDYWGQGTTVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKKVEPKSCDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVCTLPPSREEMTKNQVSLSCA
VKGFYPSDIAVEWESNGQPENNYKTTPPVLDSDGSFFLVSKLTVDKSRWQQGN
VFSCSVMHEALHNHYTQKSLSLSPGK

> hu98VL-S107EVH-knob (SEQ ID NO: 168)

DIQMTQSPSSLSASVGDRVTITCRASRDITKFLNWYQQKPGGAVKLLIYYTSRL
HSGVPSRFSGSGSGTDYTLTISSLQPEDFATYYCQQGNTLPWTFGGGTKVEIK*G
GGSGGGG*EVQLVESGGGLVQPGGSLKLSCAASGFTFNKYAMNWVRQAPGKGL
EWVARIRSKYNNYATYYADSVKDRFTISRDDSKNTAYLQMNNLKTEDTAVYY

CVRHGNFGNEYISYWAYWGQGTLVTVSS*G*CPPCPAPEAAGAPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVCTLPPSR
EEMTKNQVSLWCLVKGFYPSDIAVEWESNGQPENNYKTTPPVLDSDGSFFLYS
KLTVDKSRWQQGNVFSCSVMHEALHNHYTQKSLSLSPGK

> CD3VL-hu98VH-hole (SEQ ID NO: 169)

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVL*GGGGSGGGG*EVQLVESGGGLVQPGGSLRLSCAASGFTFSDYGIHWVRQA
PGKGLEWVAYISSGGYTSYYADTVKGRFTISRDNAKNSLYLQMNSLRAEDTAV
YYCARGDYEDIFYTMDYWGQGTTVTVSS*G*CPPCPAPEAAGAPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPC
REEMTKNQVSLSCAVKGFYPSDIAVEWESNGQPENNYKTTPPVLDSDGSFFLVS
KLTVDKSRWQQGNVFSCSVMHEALHNHYTQKSLSLSPGK

> hu98VL-I2CVH-knob (SEQ ID NO: 170)

DIQMTQSPSSLSASVGDRVTITCRASRDITKFLNWYQQKPGGAVKLLIYYTSRL
HSGVPSRFSGSGSGTDYTLTISSLQPEDFATYYCQQGNTLPWTFGGGTKVEIK*G*
*GGSGGGG*EVQLVESGGGLVQPGGSLKLSCAASGFTFNKYAMNWVRQAPGKGL
EWVARIRSKYNNYATYYADSVKDRFTISRDDSKNTAYLQMNNLKTEDTAVYY
CVRHGNFGNSYISYWAYWGQGTLVTVSS*G*CPPCPAPEAAGAPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVCTLPPSR
EEMTKNQVSLWCLVKGFYPSDIAVEWESNGQPENNYKTTPPVLDSDGSFFLYS
KLTVDKSRWQQGNVFSCSVMHEALHNHYTQKSLSLSPGK

> hu110VL-G4S-Obscurin (SEQ ID NO: 171)

NIVMTQSPKSLAVSLGERATINCKASENVGIFVSWYQQKPGQSPKLLIYGASNR
YTGVPDRFSGSGSATDFTLTISSLQAEDVADYYCGQSHSYPLTFGQGTKLEIK*G*
*GGGS*SGAPRFLTRPKASVVSVGKDATLSCQIVGNPFPQVSWEKDKQPVTAGVR
FRLAQDGDLYRLKILDLQLSDSGQYVCRARNAHGEAFACLGLQVDAEA

> hu110VH-G4S-Titin-hole (SEQ ID NO: 172)

EVQLVQSGAEVKKPGASVKVSCKTSGYTFTDYYMNWVRQAPGQSLEWMGIIN
PYQGGTSYTQKFKGRVTITVDKSASTAYMELSSLRSEDTAVYYCARHFYDSSY
LTYSTMDYWGQGTTVTVSS*GGGGS*GIPPKIECLPIDISIDEGKVLTVASAFTGEPT
PEVTWSTGGRKIHSQEQGRFHIENTDDSTTLTIKDVQKDGGLYTLTLRNEFGS
DSATVNIHIRSIDKTHTCPPCPAPEAAGGPSVFLFPPKPKDTLMISRTPEVTCVVV

DVSHEDPEVKFNWYVDGVEVHNAKTKPREEQYNSTYRVVSVLTVLHQDWLN
GKEYKCKVSNKALPAPIEKTISKAKGQPREPQVCTLPPSREEMTKNQVSLSCAV
KGFYPSDIAVEWESNGQPENNYKTTPPVLDSDGSFFLVSKLTVDKSRWQQGNV
FSCSVMHEALHNHYTQKSLSLSPGK

> hu110VL-hκ (SEQ ID NO: 173)

NIVMTQSPKSLAVSLGERATINCKASENVGIFVSWYQQKPGQSPKLLIYGASNR
YTGVPDRFSGSGSATDFTLTISSLQAEDVADYYCGQSHSYPLTFGQGTKLEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

> hu110VH-CH1-hole (SEQ ID NO: 174)

EVQLVQSGAEVKKPGASVKVSCKTSGYTFTDYYMNWVRQAPGQSLEWMGIINPYQGGTSYTQKFKGRVTITVDKSASTAYMELSSLRSEDTAVYYCARHFYDSSYLTYSTMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK

> hu110VL-S107EVH-knob (SEQ ID NO: 175)

NIVMTQSPKSLAVSLGERATINCKASENVGIFVSWYQQKPGQSPKLLIYGASNRYTGVPDRFSGSGSATDFTLTISSLQAEDVADYYCGQSHSYPLTFGQGTKLEIK*GGGSGGGG*EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNEYISYWAYWGQGTLVTVSS*G*CPPCPAPEAAGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLWCLVKGFYPSDIAVEWESNGQPENNYKTTPPVLDSDGSFFLYSKLTVDKSRWQQGNVFSCSVMHEALHNHYTQKSLSLSPGK

> CD3VL-hu110VH-hole (SEQ ID NO: 176)

QTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL*GGGGSGGGG*EVQLVQSGAEVKKPGASVKVSCKTSGYTFTDYYMNWVRQAPGQSLEWMGIINPYQGGTSYTQKFKGRVTITVDKSASTAYMELSSLRSEDTAVYYCARHFYDSSYLTYSTMDYWGQGTTVTVSS*G*CPPCPAPEAAGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLSCAVKGFYPSDIAVEWESNGQPENNYKTTPPVLDSDGSFFLVSKLTVDKSRWQQGNVFSCSVMHEALHNHYTQKSLSLSPGK

> hu110VL-I2CVH-knob (SEQ ID NO: 177)

NIVMTQSPKSLAVSLGERATINCKASENVGIFVSWYQQKPGQSPKLLIYGASNR YTGVPDRFSGSGSATDFTLTISSLQAEDVADYYCGQSHSYPLTFGQGTKLEIK*G GGSGGGG*EVQLVESGGGLVQPGGSLKLSCAASGFTFNKYAMNWVRQAPGKGL EWVARIRSKYNNYATYYADSVKDRFTISRDDSKNTAYLQMNNLKTEDTAVYY CVRHGNFGNSYISYWAYWGQGTLVTVSS*G*CPPCPAPEAAGAPSVFLFPPKPKD TLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVCTLPPSR EEMTKNQVSLWCLVKGFYPSDIAVEWESNGQPENNYKTTPPVLDSDGSFFLYS KLTVDKSRWQQGNVFSCSVMHEALHNHYTQKSLSLSPGK

> hu30VL-G4S-Obscurin (SEQ ID NO: 178)

DIVMTQSPDSLAVSLGERATINCKASENVGYVSWYQQKPGQPPKLLIYGASNR YTGVPDRFSGSGSGTDFTLTISSLQAEDVAVYYCGQSYSYPLTFGQGTKLEIK*G GGGS*SGAPRFLTRPKASVVSVGKDATLSCQIVGNPFPQVSWEKDKQPVTAGVR FRLAQDGDLYRLKILDLQLSDSGQYVCRARNAHGEAFACLGLQVDAEA

> hu30VH-G4S-Titin-hole (SEQ ID NO: 179)

EVRLVQSGAEVKKPGASVKVSCKASGYTFTDYYMNWVRQAPGQSLEWMGIIN PYTGDTSYNQKFRGRVTLTVDKSASTAYMELSSLRSEDTAVYYCARYHYGPHL EGYFDVWGQGTTVTVSS*GGGGS*GIPPKIECLPIDISIDEGKVLTVASAFTGEPTPE VTWSTGGRKIHSQEQGRFHIENTDDSTTLTIKDVQKQDGGLYTLTLRNEFGSDS ATVNIHIRSIDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDWLNGK EYKCKVSNKALPAPIEKTISKAKGQPREPQVCTLPPSREEMTKNQVSLSCAVKG FYPSDIAVEWESNGQPENNYKTTPPVLDSDGSFFLVSKLTVDKSRWQQGNVFS CSVMHEALHNHYTQKSLSLSPGK

> hu30VL-hκ (SEQ ID NO: 180)

DIVMTQSPDSLAVSLGERATINCKASENVGYVSWYQQKPGQPPKLLIYGASNR YTGVPDRFSGSGSGTDFTLTISSLQAEDVAVYYCGQSYSYPLTFGQGTKLEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC

> hu30VH-CH1-hole (SEQ ID NO: 181)

EVRLVQSGAEVKKPGASVKVSCKASGYTFTDYYMNWVRQAPGQSLEWMGIIN PYTGDTSYNQKFRGRVTLTVDKSASTAYMELSSLRSEDTAVYYCARYHYGPHL

EGYFDVWGQGTTVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKKVEPKSCDKTHTCPPCPAPEAAGGPSVFLFPPKPKDTLMISRTPEVTCV VVDVSHEDPEVKFNWYVDGVEVHNAKTKPREEQYNSTYRVVSVLTVLHQDW LNGKEYKCKVSNKALPAPIEKTISKAKGQPREPQVCTLPPSREEMTKNQVSLSC AVKGFYPSDIAVEWESNGQPENNYKTTPPVLDSDGSFFLVSKLTVDKSRWQQG NVFSCSVMHEALHNHYTQKSLSLSPGK

> hu30VL-S107EVH-knob (SEQ ID NO: 182)

DIVMTQSPDSLAVSLGERATINCKASENVGYVSWYQQKPGQPPKLLIYGASNR YTGVPDRFSGSGSGTDFTLTISSLQAEDVAVYYCGQSYSYPLTFGQGTKLEIK*G GGSGGGG*EVQLVESGGGLVQPGGSLKLSCAASGFTFNKYAMNWVRQAPGKGL EWVARIRSKYNNYATYYADSVKDRFTISRDDSKNTAYLQMNNLKTEDTAVYY CVRHGNFGNEYISYWAYWGQGTLVTVSS*G*CPPCPAPEAAGAPSVFLFPPKPKD TLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVCTLPPSR EEMTKNQVSLWCLVKGFYPSDIAVEWESNGQPENNYKTTPPVLDSDGSFFLYS KLTVDKSRWQQGNVFSCSVMHEALHNHYTQKSLSLSPGK

> CD3VL-hu30VH-hole (SEQ ID NO: 183)

QTVVTQEPSLTVSPGGTVTLTCGSSTGAVTSGNYPNWVQQKPGQAPRGLIGGT KFLAPGTPARFSGSLLGGKAALTLSGVQPEDEAEYYCVLWYSNRWVFGGGTK LTVL*GGGGSGGGG*EVRLVQSGAEVKKPGASVKVSCKASGYTFTDYYMNWVR QAPGQSLEWMGIINPYTGDTSYNQKFRGRVTLTVDKSASTAYMELSSLRSEDT AVYYCARYHYGPHLEGYFDVWGQGTTVTVSS*G*CPPCPAPEAAGAPSVFLFPPK PKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTL PPCREEMTKNQVSLSCAVKGFYPSDIAVEWESNGQPENNYKTTPPVLDSDGSFF LVSKLTVDKSRWQQGNVFSCSVMHEALHNHYTQKSLSLSPGK

> hu30VL-I2CVH-knob (SEQ ID NO: 184)

DIVMTQSPDSLAVSLGERATINCKASENVGYVSWYQQKPGQPPKLLIYGASNR
YTGVPDRFSGSGSGTDFTLTISSLQAEDVAVYYCGQSYSYPLTFGQGTKLEIK*G*
*GGSGGGG*EVQLVESGGGLVQPGGSLKLSCAASGFTFNKYAMNWVRQAPGKGL
EWVARIRSKYNNYATYYADSVKDRFTISRDDSKNTAYLQMNNLKTEDTAVYY
CVRHGNFGNSYISYWAYWGQGTLVTVSS*G*CPPCPAPEAAGAPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVCTLPPSR
EEMTKNQVSLWCLVKGFYPSDIAVEWESNGQPENNYKTTPPVLDSDGSFFLYS

KLTVDKSRWQQGNVFSCSVMHEALHNHYTQKSLSLSPGK

> hu6VL (D56E)-S107EVH-knob (hu6 (D56E)-S107E-diabody-C1 (SEQ ID NO: 185)

DIQMTQSPSSLSASVGDRVTITCHASQGISGNMGWLQQKPGKAPKGLIYHGAN
LEEGVPSRFSGSGSGADYTLTISSLQPEDFADYYCVQYAQFPYTFGGGTKVEIK
*GGGSGGGG*EVQLVESGGGLVQPGGSLKLSCAASGFTFNKYAMNWVRQAPGK
GLEWVARIRSKYNNYATYYADSVKDRFTISRDDSKNTAYLQMNNLKTEDTAV
YYCVRHGNFGNEYISYWAYWGQGTLVTVSS*G*CPPCPAPEAAGAPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTLP
PSREEMTKNQVSLWCLVKGFYPSDIAVEWESNGQPENNYKTTPPVLDSDGSFF
LYSKLTVDKSRWQQGNVFSCSVMHEALHNHYTQKSLSLSPGK

> hu6VL (D56E)-I2CVH-knob (SEQ ID NO: 259)

DIQMTQSPSSLSASVGDRVTITCHASQGISGNMGWLQQKPGKAPKGLIYHGAN
LEEGVPSRFSGSGSGADYTLTISSLQPEDFADYYCVQYAQFPYTFGGGTKVEIK
*GGGSGGGG*EVQLVESGGGLVQPGGSLKLSCAASGFTFNKYAMNWVRQAPGK
GLEWVARIRSKYNNYATYYADSVKDRFTISRDDSKNTAYLQMNNLKTEDTAV
YYCVRHGNFGNSYISYWAYWGQGTLVTVSS*G*CPPCPAPEAAGAPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTLP
PSREEMTKNQVSLWCLVKGFYPSDIAVEWESNGQPENNYKTTPPVLDSDGSFF
LYSKLTVDKSRWQQGNVFSCSVMHEALHNHYTQKSLSLSPGK

> hu6VL (D56S)-I2CVH-knob (SEQ ID NO: 260)

DIQMTQSPSSLSASVGDRVTITC<u>HASQGISGNMG</u>WLQQKPGKAPKGLIY<u>HGAN</u>
<u>LES</u>GVPSRFSGSGSGADYTLTISSLQPEDFADYYC<u>VQYAQFPYT</u>FGGGTKVEIK*G*
*GGSGGGG*EVQLVESGGGLVQPGGSLKLSCAASGFTFNKYAMNWVRQAPGKGL
EWVARIRSKYNNYATYYADSVKDRFTISRDDSKNTAYLQMNNLKTEDTAVYY
CVRHGNFGNSYISYWAYWGQTLVTVSS*G*CPPCPAPEAAGAPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVCTLPPSR
EEMTKNQVSLWCLVKGFYPSDIAVEWESNGQPENNYKTTPPVLDSDGSFFLYS
KLTVDKSRWQQGNVFSCSVMHEALHNHYTQKSLSLSPGK

> hu6VL (D56S)-S107EVH-knob (SEQ ID NO: 186)

DIQMTQSPSSLSASVGDRVTITC<u>HASQGISGNMG</u>WLQQKPGKAPKGLIY<u>HGAN</u>
<u>LES</u>GVPSRFSGSGSGADYTLTISSLQPEDFADYYC<u>VQYAQFPYT</u>FGGGTKVEIK*G*
*GGSGGGG*EVQLVESGGGLVQPGGSLKLSCAASGFTFNKYAMNWVRQAPGKGL
EWVARIRSKYNNYATYYADSVKDRFTISRDDSKNTAYLQMNNLKTEDTAVYY

CVRHGNFGNEYISYWAYWGQGTLVTVSS*G*CPPCPAPEAAGAPSVFLFPPKPKD
TLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVCTLPPSR
EEMTKNQVSLWCLVKGFYPSDIAVEWESNGQPENNYKTTPPVLDSDGSFFLYS
KLTVDKSRWQQGNVFSCSVMHEALHNHYTQKSLSLSPGK

> hu6VL8-S107EVH-knob (SEQ ID NO: 187)

DIQMTQSPSSLSASVGDRVTITC<u>HASQGISGNMG</u>WLQQKPGKAPKGLIY<u>HGAN</u>
<u>LEE</u>GVPSRFSGSGSGADYTLTISSLQPEDFADYYC<u>VQYAQ**Y**PYT</u>FGGGTKVEIK
*GGGSGGGGG*EVQLVESGGGLVQPGGSLKLSCAASGFTFNKYAMNWVRQAPGK
GLEWVARIRSKYNNYATYYADSVKDRFTISRDDSKNTAYLQMNNLKTEDTAV
YYCVRHGNFGNEYISYWAYWGQGTLVTVSS*G*CPPCPAPEAAGAPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTLP
PSREEMTKNQVSLWCLVKGFYPSDIAVEWESNGQPENNYKTTPPVLDSDGSFF
LYSKLTVDKSRWQQGNVFSCSVMHEALHNHYTQKSLSLSPGK

> CD3VL-hu6VH14-hole (SEQ ID NO: 188)

QTVVTQEPSLTVSPGGTVTLTCGSSTGAVTSGNYPNWVQQKPGQAPRGLIGGT KFLAPGTPARFSGSLLGGKAALTLSGVQPEDEAEYYCVLWYSNRWVFGGGTK LTVL*GGGGSGGGG*EVQLVQSGSELKKPGASVKVSCKASGYTFT<u>DYYMN</u>WVR QAPGQGLEWMGI<u>INPYSGVTT</u>YNHKFKGRFALSVDKSSNTAYLQISSLKAEDT AVYYCAR<u>QDSLLDYAMDY</u>WGQGTTVTVSS*G*CPPCPAPEAAGAPSVFLFPPKPK DTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPP CREEMTKNQVSLSCAVKGFYPSDIAVEWESNGQPENNYKTTPPVLDSDGSFFL VSKLTVDKSRWQQGNVFSCSVMHEALHNHYTQKSLSLSPGK

> CD3VL-hu6VH15-hole (SEQ ID NO: 189)

QTVVTQEPSLTVSPGGTVTLTCGSSTGAVTSGNYPNWVQQKPGQAPRGLIGGT KFLAPGTPARFSGSLLGGKAALTLSGVQPEDEAEYYCVLWYSNRWVFGGGTK LTVL*GGGGSGGGG*EVQL**QQ**SGSELKKPGASVKVSCKASGYTFT<u>DYYMN</u>WVR QAPGQGLEWMGI<u>INPYSGSTI</u>YNHKFKGRFALSVDKSSNTAYLQISSLKAEDTA VYYCAR<u>QDSLLDYAMDY</u>WGQGTTVTVSS*G*CPPCPAPEAAGAPSVFLFPPKPKD TLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPC REEMTKNQVSLSCAVKGFYPSDIAVEWESNGQPENNYKTTPPVLDSDGSFFLVS KLTVDKSRWQQGNVFSCSVMHEALHNHYTQKSLSLSPGK

> hu6VL9-S107EVH-knob (SEQ ID NO: 190)

<u>N</u>IQMTQSPSSLSASVGDRVTITC<u>HASQGISGNMG</u>WLQQKPGKAPKGLIY<u>HGRN LEE</u>GVPSRFSGSGSGADYTLTISSLQPEDFADYYC<u>VQYAQYPYT</u>FGGGTKVEIK *GGGSGGGG*EVQLVESGGGLVQPGGSLKLSCAASGFTFNKYAMNWVRQAPGK GLEWVARIRSKYNNYATYYADSVKDRFTISRDDSKNTAYLQMNNLKTEDTAV YYCVRHGNFGNEYISYWAYWGQGTLVTVSS*G*CPPCPAPEAAGAPSVFLFPPKP KDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVCTLP PSREEMTKNQVSLWCLVKGFYPSDIAVEWESNGQPENNYKTTPPVLDSDGSFF LYSKLTVDKSRWQQGNVFSCSVMHEALHNHYTQKSLSLSPGK

> hu6VL10-S107EVH-knob (SEQ ID NO: 191)

EIVLTQSPATLSLSPGERATLSC*HASQGISGNMGW***L*QQKPG***K***APR***G***LIYHGANL***EE***GIPARFSGSGSG***AD***Y***TLTISRL***Q***PEDFA***D***YYC*VQYAQFPYT*FGGGTKVEIK*GGGSGGGG*EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNEYISYWAYWGQTLVTVSS*G*CPPCPAPEAAGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLWCLVKGFYPSDIAVEWESNGQPENNYKTTPPVLDSDGSFFLYSKLTVDKSRWQQGNVFSCSVMHEALHNHYTQKSLSLSPGK.

The control molecules used in the present disclosure were AMG-75, which was prepared with reference to WO2017021349, and BI-764532, which was prepared with reference to WO2019234220. The sequences of the control molecules are as follows:

AMG-757 (SEQ ID NO: 252):

QVQLQESGPGLVKPSETLSLTCTVSGGSISSYYWSWIRQPPGKCLEWIGYVYYSGTTNYNPSLKSRVTISVDTSKNQFSLKLSSVTAADTAVYYCASIAVTGFYFDYWGQGTLVTVSSGGGGSGGGGSGGGGSEIVLTQSPGTLSLSPGERVTLSCRASQRVNNNYLAWYQQRPGQAPRLLIYGASSRATGIPDRFSGSGSGTDFTLTISRLEPEDFAVYYCQQYDRSPLTFGCGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGDKTHTCPPCPAPELLGGPSVFLFPPKPKDTLMISRTPEVTCVVVDVSHEDPEVKFNWYVDGVEVHNAKTKPCEEQYGSTYRCVSVLTVLHQDWLNGKEYKCKVSNKALPAPIEKTISKAKGQPREPQVYTLPPSREEMT

KNQVSLTCLVKGFYPSDIAVEWESNGQPENNYKTTPPVLDSDGSFFLYSKLTVDKSRWQQGNVFSCSVMHEALHNHYTQKSLSLSPGGGGSGGGGSGGGGSGGGGSGGGGSGGGGSDKTHTCPPCPAPELLGGPSVFLFPPKPKDTLMISRTPEVTCVVVDVSHEDPEVKFNWYVDGVEVHNAKTKPCEEQYGSTYRCVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

BI-764532:

> BI-764532 chain 1 (SEQ ID NO: 253):

DIQMTQSPSAMSASVGDRVTITCRASQGISNYLVWFQQKPGKAPKRLIYAVSSL
YSGVPSRFSGSGSGTEFTLTISSLQPEDFATYYCLQHDSYPYTFGQGTKLEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGECG
GGGSEGKSSGSGSESKSTEGKSSGSGSESKSTGGGGSQVQLVQSGAEVKKPGAS
VKVSCKASGYTFTSYYVHWVRQAPGQGLEWMVIINPGGGTTSYAQKFLGRVT
MTRDTSTNTVYMELKSLRSEDTAVYYCARGEAVTGNYFYYGMDVWGQGTTV
TVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKRVEPKSCD
KTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKA
LPAPIEKTISKAKGQPREPQVYTLPPSREEMTKNQVSLWCLVKGFYPSDIAVEW
ESNGQPENNYKTTPPVLDSDGSFFLYSKLTVDKSRWQQGNVFSCSVMHEALHN
HYTQKSLSLSPG

> BI-764532 chain 2 (SEQ ID NO: 254):

EAVVTQEPSLTVSPGGTVTLTCRSSTGAVTTSNYANWVQEKPGQLPRGLIGGTN
KRAPWVPARFSGSLLGGKAALTLSGAQPEDEAEYFCALWYSNLWVFGGGTKL
TVLGQPKAAPSVTLFPPSSEELQANKATLVCLISDFYPGAVKVAWKADGSPVNT
GVETTTPSKQSNNKYAASSYLSLTPEQWKSHRSYSCQVTHEGSTVEKTVAPAE
CSGGGGSEGKSSGSGSESKSTEGKSSGSGSESKSTGGGGSEVQLVESGGGLVQP
GGSLKLSCAASGFTFNTYAMNWVRQAPGKGLEWVARIRSKYNNYATYYADS
VKDRFTISRDDSKNTAYLQMNNLKTEDTAVYYCVRHGNFGNSYVSWFAYWG
QGTLVTVSAASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEP
KSCDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKV
SNKALPAPIEKTISKAKGQPREPQVYTLPPSREEMTKNQVSLSCAVKGFYPSDIA
VEWESNGQPENNYKTTPPVLDSDGSFFLVSKLTVDKSRWQQGNVFSCSVMHE
ALHNRFTQKSLSLSPG.

**Test Examples**

**Test Example 1: Biacore Antibody Affinity Assay**

Test antibodies were affinity-captured with a Protein A biosensor chip (Cat. # 29127556, Cytiva) for 18 seconds, and then the antigens human DLL3 (ACRO, DLL3-H52H4), cynomolgus monkey DLL3 (KACTUS, DLL-RM103), mouse DLL3 (KACTUS, DLL-MM103), human CD3D&3E (ACRO, CDD-H52W1), and monkey CD3D&3E (ACRO, CDD-C52W4) were allowed to flow through the surface of the chip for 180 seconds, followed by 600 seconds of dissociation. Reaction signals were detected in real time using a Biacore 8K (Cytiva) instrument to obtain association and dissociation curves. After the dissociation in each experimental cycle, the chip was washed and regenerated with a 10 mM glycine-hydrochloric acid solution (pH 1.5, Cat. # BR-1003-54, Cytiva). Data fitting was performed using a 1:1 Model. The results are shown in Table 26-1 to Table 26-9.

Table 26-1. The affinities of antibodies for human DLL3 (based on clone 6)

| Antibody | ka (1/Ms) | kd (1/s) | KD (M) |
|---|---|---|---|
| M6CHI | 9.65E+05 | 6.57E-04 | 6.81E-10 |
| hu6L1H1 | 7.98E+05 | 2.25E-03 | 2.81E-09 |
| hu6L1H2 | 7.87E+05 | 1.73E-03 | 2.19E-09 |
| hu6L1H3 | 8.12E+05 | 2.24E-03 | 2.76E-09 |
| hu6L2H1 | 7.09E+05 | 1.55E-03 | 2.19E-09 |
| hu6L2H2 | 7.04E+05 | 1.16E-03 | 1.64E-09 |
| hu6L2H3 | 6.95E+05 | 1.39E-03 | 2.01E-09 |
| hu6L3H1 | 7.15E+05 | 1.44E-03 | 2.02E-09 |
| hu6L3H2 | 7.16E+05 | 1.14E-03 | 1.59E-09 |
| hu6L3H3 | 7.18E+05 | 1.35E-03 | 1.88E-09 |
| hu6L4H4 | 7.43E+05 | 1.08E-03 | 1.45E-09 |
| hu6L2H5 | 8.10E+05 | 7.84E-04 | 9.69E-10 |
| hu6L2H6 | 8.63E+05 | 7.56E-04 | 8.77E-10 |
| hu6L2H7 | 6.70E+05 | 2.05E-03 | 3.06E-09 |
| hu6L2H11 | 2.06E+06 | 7.49E-04 | 3.63E-10 |
| hu6L2H12 | 1.98E+06 | 9.13E-04 | 4.61E-10 |
| hu6L2H13 | 2.10E+06 | 8.03E-04 | 3.82E-10 |
| hu6L4H6 | 1.91E+06 | 8.11E-04 | 4.24E-10 |
| hu6L4H 11 | 2.14E+06 | 6.72E-04 | 3.14E-10 |
| hu6L4H12 | 2.06E+06 | 8.05E-04 | 3.91E-10 |
| hu6L4H13 | 2.16E+06 | 6.95E-04 | 3.22E-10 |

Table 26-2. The affinities of antibodies for human DLL3 (based on clone 98)

| Antibody | ka (1/Ms) | kd (1/s) | KD (M) |
|---|---|---|---|
| M98CHI | 1.59E+06 | 6.82E-04 | 4.29E-10 |
| hu98L1H1 | 1.55E+06 | 1.71E-03 | 1.10E-09 |
| hu98L1H2 | 1.56E+06 | 3.07E-03 | 1.97E-09 |
| hu98L2H1 | 1.45E+06 | 3.89E-04 | 2.69E-10 |
| hu98L2H2 | 1.22E+06 | 6.68E-04 | 5.50E-10 |
| hu98L3H1 | 1.60E+06 | 3.72E-04 | 2.32E-10 |
| hu98L3H2 | 1.58E+06 | 7.32E-04 | 4.64E-10 |
| hu98L3H4 | 1.36E+06 | 1.51E-03 | 1.11E-09 |
| hu98L3H5 | 1.42E+06 | 1.73E-03 | 1.22E-09 |

Table 26-3. The affinities of antibodies for human DLL3 (based on clone 110)

| Antibody | ka (1/Ms) | kd (1/s) | KD (M) |
|---|---|---|---|
| hu110L1H1 | 6.14E+05 | 4.17E-05 | 6.80E-11 |
| hu110L1H2 | 5.20E+05 | 4.26E-05 | 8.19E-11 |
| hu110L1H3 | 5.64E+05 | 4.75E-05 | 8.43E-11 |
| hu110L1H4 | 5.27E+05 | 4.80E-05 | 9.11E-11 |
| hu110L1H5 | 4.54E+05 | 6.41E-05 | 1.41E-10 |
| hu110L1H6 | 5.20E+05 | 5.32E-05 | 1.02E-10 |
| hu110L2H1 | 5.09E+05 | 5.84E-05 | 1.15E-10 |
| hu110L2H2 | 5.21E+05 | 6.25E-05 | 1.20E-10 |
| hu110L2H3 | 5.50E+05 | 6.45E-05 | 1.17E-10 |
| hu110L2H4 | 4.96E+05 | 5.11E-05 | 1.03E-10 |
| hu110L2H5 | 4.34E+05 | 8.96E-05 | 2.06E-10 |
| hu110L2H6 | 4.11E+05 | 6.86E-05 | 1.67E-10 |

Table 26-4. The affinities of antibodies for human DLL3 (based on clone 30)

| Antibody | ka (1/Ms) | kd (1/s) | KD (M) |
|---|---|---|---|
| hu30L1H1 | 8.57E+05 | 7.87E-04 | 9.19E-10 |
| hu30L1H2 | 9.89E+05 | 1.31E-03 | 1.32E-09 |
| hu30L1H3 | 9.29E+05 | 1.85E-03 | 1.99E-09 |
| hu30L1H4 | 9.05E+05 | 1.16E-03 | 1.28E-09 |
| hu30L1H5 | 1.23E+06 | 1.17E-03 | 9.52E-10 |
| hu30L1H6 | 1.04E+06 | 1.43E-03 | 1.38E-09 |
| hu30L1H7 | 9.85E+05 | 1.15E-03 | 1.16E-09 |
| hu30L1H8 | 4.25E+05 | 1.09E-03 | 2.57E-09 |
| hu30L1H9 | 4.19E+05 | 9.81E-04 | 2.34E-09 |
| hu30L1H10 | 4.40E+05 | 9.64E-04 | 2.19E-09 |
| hu30L1H11 | 4.14E+05 | 1.17E-03 | 2.83E-09 |
| hu30L2H1 | 8.67E+05 | 5.71E-04 | 6.58E-10 |
| hu30L2H2 | 9.14E+05 | 9.21E-04 | 1.01E-09 |
| hu30L2H3 | 9.81E+05 | 1.38E-03 | 1.41E-09 |
| hu30L2H4 | 9.66E+05 | 1.04E-03 | 1.07E-09 |
| hu30L2H5 | 9.35E+05 | 9.07E-04 | 9.69E-10 |
| hu30L2H6 | 9.15E+05 | 9.88E-04 | 1.08E-09 |
| hu30L2H7 | 9.00E+05 | 9.83E-04 | 1.09E-09 |
| hu30L3H1 | 1.63E+06 | 5.90E-04 | 3.62E-10 |
| hu30L3H2 | 8.55E+05 | 7.62E-04 | 8.91E-10 |
| hu30L3H3 | 1.05E+06 | 1.31E-03 | 1.25E-09 |
| hu30L3H4 | 1.08E+06 | 9.56E-04 | 8.82E-10 |
| hu30L3H5 | 1.12E+06 | 8.20E-04 | 7.35E-10 |
| hu30L3H6 | 9.52E+05 | 8.94E-04 | 9.39E-10 |

(continued)

| Antibody | ka (1/Ms) | kd (1/s) | KD (M) |
|---|---|---|---|
| hu30L3H7 | 1.05E+06 | 8.53E-04 | 8.15E-10 |
| hu30L4H5 | 4.40E+05 | 9.20E-04 | 2.09E-09 |
| hu30L4H8 | 3.43E+05 | 9.42E-04 | 2.74E-09 |
| hu30L4H9 | 3.92E+05 | 1.01E-03 | 2.57E-09 |
| hu30L4H10 | 4.27E+05 | 9.19E-04 | 2.16E-09 |
| hu30L4H11 | 3.98E+05 | 1.13E-03 | 2.84E-09 |
| hu30L5H5 | 4.46E+05 | 8.82E-04 | 1.98E-09 |
| hu30L5H8 | 4.00E+05 | 1.02E-03 | 2.54E-09 |
| hu30L5H9 | 3.99E+05 | 9.89E-04 | 2.48E-09 |
| hu30L5H10 | 4.64E+05 | 9.49E-04 | 2.05E-09 |
| hu30L5H11 | 3.91E+05 | 1.20E-03 | 3.06E-09 |

Table 26-5. The affinities of bispecific antibodies for human DLL3

| Antibody | ka (1/Ms) | kd (1/s) | KD (M) |
|---|---|---|---|
| hu6(D56E)-S 107E-diabody | 4.22E+05 | 4.62E-04 | 1.09E-09 |
| hu6(D56E)-I2C-diabody | 4.22E+05 | 4.83E-04 | 1.14E-09 |
| hu110-S107E-hot Ig | 2.49E+05 | 2.82E-05 | 1.13E-10 |
| hu110-I2C-hot Ig | 2.28E+05 | 2.72E-05 | 1.19E-10 |
| hu110-I2C-Y body | 2.13E+05 | 2.66E-05 | 1.25E-10 |

Table 26-6. The affinities of bispecific antibodies for cynomolgus monkey DLL3

| Antibody | ka (1/Ms) | kd (1/s) | KD (M) |
|---|---|---|---|
| hu6(D56E)-S107E-diabody | 2.64E+05 | 4.88E-04 | 1.85E-09 |
| hu6(D56E)-I2C-diabody | 2.51E+05 | 4.93E-04 | 1.96E-09 |
| hu110-S107E-hot Ig | 1.23E+05 | 6.43E-05 | 5.23E-10 |
| hu110-I2C-hot Ig | 1.02E+05 | 6.26E-05 | 6.16E-10 |
| hu110-I2C-Y body | 8.81E+04 | 6.71E-05 | 7.62E-10 |

Table 26-7. The affinities of bispecific antibodies for mouse DLL3

| Antibody | ka (1/Ms) | kd (1/s) | KD (M) |
|---|---|---|---|
| hu6(D56E)-S107E-diabody | 3.78E+05 | 4.51E-03 | 1.19E-08 |
| hu6(D56E)-I2C-diabody | 3.47E+05 | 4.85E-03 | 1.40E-08 |
| hu110-S107E-hot Ig | No binding | | |
| hu110-I2C-hot Ig | No binding | | |
| hu110-I2C-Y body | No binding | | |

Table 26-8. The affinities of bispecific antibodies for human CD3

| Antibody | ka (1/Ms) | kd (1/s) | KD (M) |
|---|---|---|---|
| hu6(D56E)-S 107E-diabody | 1.93E+05 | 1.12E-02 | 5.82E-08 |
| hu6(D56E)-I2C-diabody | 1.12E+06 | 2.13E-03 | 1.90E-09 |

(continued)

| Antibody | ka (1/Ms) | kd (1/s) | KD (M) |
|---|---|---|---|
| hu110-S107E-hot Ig | 1.60E+05 | 7.49E-03 | 4.67E-08 |
| hu110-I2C-hot Ig | 8.81E+05 | 1.68E-03 | 1.91E-09 |
| hu110-I2C-Y body | 9.44E+05 | 2.87E-03 | 3.04E-09 |

Table 26-9. The affinities of bispecific antibodies for cynomolgus monkey CD3

| Antibody | ka (1/Ms) | kd (1/s) | KD (M) |
|---|---|---|---|
| hu6(D56E)-S107E-diabody | 2.66E+05 | 1.23E-02 | 4.60E-08 |
| hu6(D56E)-I2C-diabody | 1.32E+06 | 2.04E-03 | 1.54E-09 |
| hu110-S107E-hot Ig | 1.77E+05 | 7.00E-03 | 3.96E-08 |
| hu110-I2C-hot Ig | 9.99E+05 | 1.49E-03 | 1.50E-09 |
| hu110-I2C-Y body | 1.12E+06 | 2.61E-03 | 2.32E-09 |

The results show that all the humanized antibodies and chimeric antibodies binding specifically to DLL3 of the present disclosure have good affinities for human DLL3; the bispecific antibodies have good affinities for both human and cynomolgus monkey DLL3 and CD3.

**Test Example 2: DLL3 Cell-Level FACS Binding Assay**

Cells of the DLL3-expressing small cell lung cancer cell lines H1184 (ATCC, Cat. No. CRL-5858), DLL3/H82, DLL3/SHP77, hDLL3/CHO-s, and cynoDLL3/CHO-s and CD3-expressing Jurkat cells (ATCC, TIB-152) were suspended in a FACS buffer (1% BSA + pH 7.4 PBS) to form $1 \times 10^6$/mL cell suspensions, and the suspensions were added to 96-well round-bottom plates at 100 μL/well. The plates were centrifuged at 300 g for 5 min, and the supernatant was removed. Different concentrations of test antibodies were added at 100 μL/well. The plates were incubated in the dark in a refrigerator at 4 °C for 1 h. After 3 centrifugal washes at 300 g, working concentrations of APC anti-human IgG Fc (BioLegend, 410712) or PE F(ab')2-goat anti-human IgG (invitrogen, H10104) were added. The plates were incubated in the dark in a refrigerator at 4 °C for 40 min. After 3 centrifugal washes at 300 g, the geometric mean fluorescence intensity was measured on an Invitrogen flow cytometer, and the EC50 values for the binding of the antibodies to DLL3-expressing cells were calculated. The results are shown in Table 27-1 to Table 27-6.

Table 27-1. The binding activity of antibodies to H1184 cells expressing human DLL3

| Antibody | Emax | EC50 (nM) |
|---|---|---|
| hu6L2H2 | 2193 | 0.06 |
| hu6L4H4 | 2312 | 0.06 |
| hu6L2H5 | 2486 | 0.06 |
| hu6L2H6 | 2422 | 0.05 |
| hu6L2H7 | 2196 | 0.07 |
| hu6L2H 11 | 1959 | 0.04 |
| hu6L2H12 | 2145 | 0.10 |
| hu6L2H13 | 2105 | 0.10 |
| hu6L4H11 | 2188 | 0.07 |
| hu6L4H12 | 2131 | 0.05 |
| hu6L4H13 | 2118 | 0.09 |

Table 27-2. The binding activity of antibodies to H1184 cells expressing human DLL3

| Antibody | Emax | EC50 |
|---|---|---|
| hu98L1H1 | 2818 | 0.09 |
| hu98L2H1 | 2466 | 0.06 |
| hu98L2H2 | 2307 | 0.04 |
| hu98L3H1 | 2439 | 0.05 |
| hu98L3H2 | 2346 | 0.04 |
| hu98L3H4 | 1599 | 0.05 |
| hu98L3H5 | 1543 | 0.05 |

Table 27-3. The binding activity of antibodies to H82 cells overexpressing human DLL3

| Antibody | Emax | EC50 (nM) |
|---|---|---|
| hu110L1H1 | 4467.96 | 1.15 |
| hu110L1H2 | 4697.79 | 1.31 |
| hu110L1H3 | 4562.33 | 1.15 |
| hu110L1H4 | 4581.31 | 1.51 |
| hu110L1H5 | 4610.74 | 1.61 |
| hu110L1H6 | 4616.13 | 1.66 |
| hu110L2H1 | 4719.05 | 1.25 |
| hu110L2H2 | 4754.73 | 1.25 |
| hu110L2H3 | 4574.56 | 1.32 |
| hu110L2H4 | 4597.66 | 1.41 |
| hu110L2H5 | 4673.18 | 1.71 |
| hu110L2H6 | 5193.43 | 1.40 |

Table 27-4. The binding activity of antibodies to H82 cells overexpressing human DLL3

| Antibody | Emax | EC50 (nM) |
|---|---|---|
| hu30L1H1 | 5866 | 0.74 |
| hu30L1H2 | 6049 | 0.86 |
| hu30L1H3 | 5558 | 0.75 |
| hu30L1H4 | 5358 | 0.57 |
| hu30L1H5 | 6799 | 0.88 |
| hu30L1H6 | 5751 | 0.56 |
| hu30L2H1 | 5741 | 0.89 |
| hu30L2H2 | 4914 | 0.61 |
| hu30L2H3 | 4724 | 0.59 |
| hu30L2H4 | 5441 | 0.54 |
| hu30L2H5 | 5573 | 0.65 |
| hu30L2H6 | 5422 | 0.69 |
| hu30L2H7 | 5583 | 0.83 |
| hu30L3H1 | 5530 | 0.51 |
| hu30L3H2 | 5386 | 0.44 |

(continued)

| Antibody | Emax | EC50 (nM) |
|---|---|---|
| hu30L3H3 | 5303 | 0.43 |
| hu30L3H4 | 5338 | 0.43 |
| hu30L3H5 | 5251 | 0.43 |
| hu30L3H6 | 5340 | 0.46 |
| hu30L3H7 | 5448 | 0.43 |

Table 27-5. The binding activity of antibodies to SHP77 cells expressing human DLL3

| Antibody | Emax | EC50 (nM) |
|---|---|---|
| hu30L1H5 | 1543 | 6.45 |
| hu30L1H8 | 1375 | 5.67 |
| hu30L1H10 | 1630 | 7.99 |
| hu30L4H5 | 1460 | 5.44 |
| hu30L4H8 | 1422 | 5.3 |
| hu30L4H9 | 1370 | 9.94 |
| hu30L4H11 | 1519 | 7.82 |
| hu30L5H5 | 1610 | 7.48 |
| hu30L5H9 | 1561 | 9.6 |

Table 27-6. The binding activity of bispecific antibodies to cells

| Antibody | hDLL3/CHO-S EC50 (nM) | CynoDLL3/CHO-S EC50 (nM) | Jurkat EC50 (nM) |
|---|---|---|---|
| hu6(D56E)-S 107E-diabody | 1.00 | 1.17 | 13.62 |
| hu6(D56E)-I2C-diabody | 3.48 | 2.97 | 4.61 |
| hu110-S107E-hot Ig | 5.07 | 5.01 | 7.74 |
| hu110-I2C-Y body | 4.52 | 4.92 | 3.83 |
| hu110-I2C-hot Ig | 4.91 | 4.92 | 3.27 |

The results show that the anti-DLL3 humanized antibodies of the present disclosure have good capacities to bind to cells expressing human DLL3; the bispecific antibodies have good capacities to bind to cells expressing human and cynomolgus monkey DLL3 and CD3.

**Test Example 3: DLL3 Protein-Level ELISA Binding Assay**

Streptavidin (abcam, ab136200, 1 $\mu$g/mL) was plated at 100 $\mu$L/well, and the plates were incubated overnight at 4 °C. The plates were washed 3 times with 250 $\mu$L of PBST solution (PBS containing 0.1% Tween 20) per well. The plates were blocked with 250 $\mu$L of 5% milk per well at 37 °C for 2 h. The plates were washed 3 times with 250 $\mu$L of PBST solution per well. Biotinylated DLL3 antigen (1 $\mu$g/mL) (SEQ ID NO: 9) was added, and the plates were incubated at 37 °C for 1 h. The plates were washed 3 times with 250 $\mu$L of PBST solution per well. Antibodies (maximum concentration 400 nM, serially diluted 4-fold) were formulated, and incubation was performed at 37 °C for 1 h. The plates were washed 6 times with 250 $\mu$L of PBST solution per well. Working concentrations of human IgG (H+L)-HRP (Jackson, 109-035-003, 1:4000 dilution) were added at 100 $\mu$L/well, and the plates were incubated at 37 °C for 1 h. The plates were washed 6 times with 250 $\mu$L of PBST solution per well. TMB (KPL, 5120-0077) substrate solution was added at 100 $\mu$L/well, and color development was performed at room temperature for 5-10 min. 1 M $H_2SO_4$ was added at 100 $\mu$L/well to stop the color development, and microplate reader (Molecular Devices, VERSA max) readings at 450 nm were taken.

Table 28. The binding activity of bispecific antibodies to human DLL3 protein

| Antibody | EC50 (nM) | Emax |
|---|---|---|
| hu6(D56E)-S 107E-diabody | 0.49 | 0.33 |
| hu6(D56E)-I2C-diabody | 0.49 | 0.36 |
| hu110-S107E-hot Ig | 0.11 | 0.45 |
| hu110-I2C-Y body | 0.07 | 0.48 |
| hu110-I2C-hot Ig | 0.08 | 0.48 |

The results show that the bispecific antibodies of the present disclosure have good affinities for human DLL3 protein.

**Test Example 4: ELISA Binding Assay with DLL1 and DLL4 Proteins**

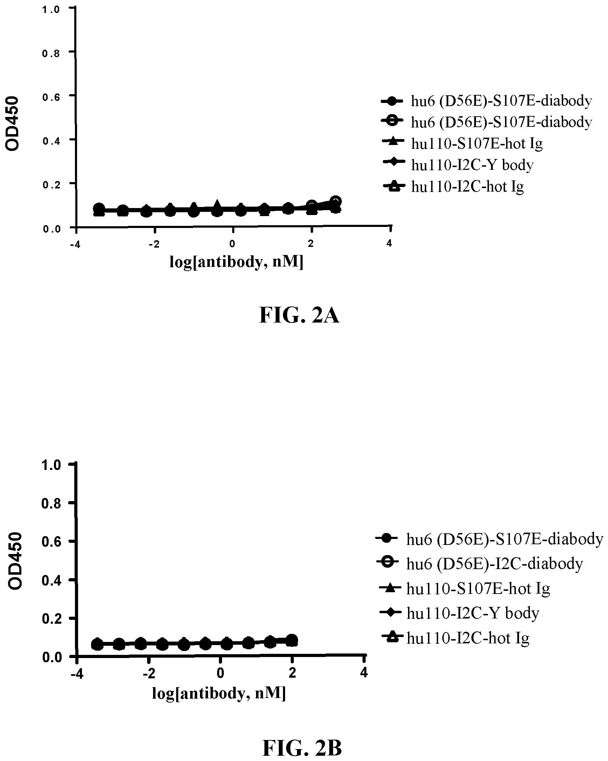

DLL1 (ACRO, DL1-H52H8, 1 μg/mL) and DLL4 (ACRO, DL4-H5227, 1 μg/mL) were plated at 100 μL/well, and the plates were incubated overnight at 4 °C. The plates were washed 3 times with 250 μL of PBST solution (PBS containing 0.1% Tween 20) per well. The plates were blocked with 250 μL of 5% milk per well at 37 °C for 2 h. The plates were washed 3 times with 250 μL of PBST solution per well. Test antibodies (maximum concentration 100 nM, serially diluted 4-fold) were added, and the plates were incubated at 37 °C for 1 h. The plates were washed 6 times with 250 μL of PBST solution per well. Working concentrations of human IgG (H+L)-HRP (Jackson, 109-035-003, 1:4000 dilution) were added at 100 μL/well, and the plates were incubated at 37 °C for 1 h. The plates were washed 6 times with 250 μL of PBST solution per well. TMB (KPL, 5120-0077) substrate solution was added at 100 μL/well, and color development was performed at room temperature for 5-10 min. 1 M $H_2SO_4$ was added at 100 μL/well to stop the color development, and microplate reader (Molecular Devices, VERSA max) readings at 450 nm were taken. The results are shown in FIGs. 2A to 2B.
The results show that the binding of the bispecific antibodies of the present disclosure to human DLL1 and DLL4 proteins was undetectable.

**Test Example 5. Killing Activity of Bispecific Antibodies of Present Disclosure Against Cells**

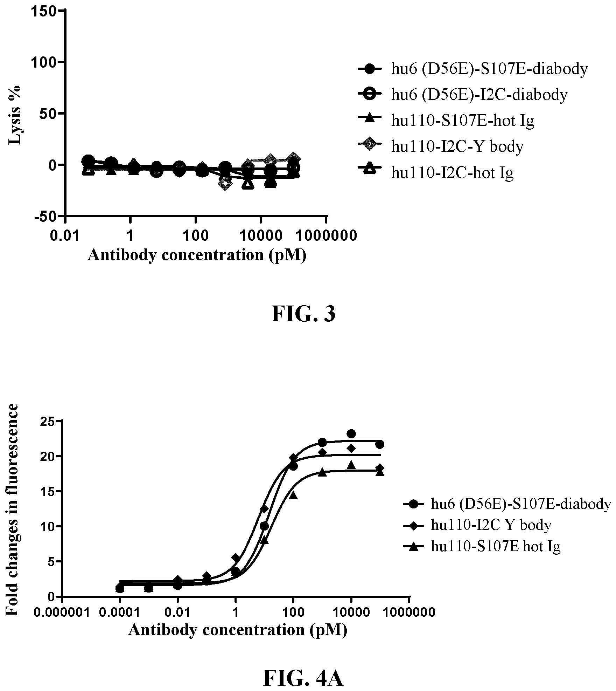

In this test example, the killing activity of bispecific antibodies as T cell engager molecules against tumor cells was studied. The target-specific cytotoxic activity of the bispecific antibodies of the present disclosure was evaluated, with LUC-GFP-expressing SHP77, H1184, and H460 stably transfected cell lines as target cells.
SHP77/lucG cells were cultured in 1640 + 10% FBS + 10 μg/mL puromycin complete culture medium and passaged 2-3 times a week. H1184/lucG cells were cultured in 1640 + 5% FBS + 10 μg/mL puromycin complete culture medium and passaged once a week. H460/lucG cells were cultured in 1640 + 10% FBS complete culture medium and passaged 2-3 times a week. Cryopreserved PBMCs (Milestone) were thawed and resuspended in 1640 + 10% FBS complete culture medium. The suspension was placed in a T75 culture flask and incubated for 4 h (at a density of 2E6 cells/mL). PBMCs were harvested, centrifuged, resuspended in 1640 + 10% FBS, and counted, and the cell count was adjusted to 1.5E6 cells/mL. The above PBMC suspension was mixed with a target cell suspension in equal volumes, and the mixture was added at 100 μL/well to ensure that the E:T Ratio was 10: 1. In addition, a PBMC ONLY group was set up, and the above PBMC suspension was mixed with 1640 + 10% FBS complete culture medium in equal volumes. Antibodies were 5-fold diluted with 1640 + 10% FBS culture medium with an initial concentration of 600 nM (6× the final concentration), and 9 dose points were obtained and added at 20 μL/well. The treated cells were cultured in a 37 °C, 5% $CO_2$ incubator for 48 h. The plates were taken out and centrifuged at 1000 rpm for 3 min, and 50 μL of the supernatant was pipetted into a new 3788 plate for cytokine detection and stored at -20 °C. 50 μL of ONE-GloTMLuciferase (Promega, E6120) was added to the culture plates. After 5 min of incubation at room temperature, luminescence was measured using Vendor, and the killing effects of the antibodies at the different concentrations were calculated. Dose-response curve plots were created based on the logarithmic concentrations and signal values of the antibodies using Graphpad Prism8.0 software, and the IC50 for antibody-mediated killing was obtained. PBMC wells containing the target cells and containing no added antibodies were set to 0% inhibition, and the maximum inhibition rate Imax was calculated for the antibodies. The results are shown in Tables 29-1 to 29-4, Table 30, and FIG. 3.

Table 29-1. The cytotoxic activity of antibodies against SHP77

| Experiment 1 | Imax (%) | IC50 (pM) |
|---|---|---|
| hu6-S 107E-diabody | 99 | 69.85 |

(continued)

| Experiment 1 | Imax (%) | IC50 (pM) |
|---|---|---|
| hu6-I2C-diabody | 100 | 7.50 |
| hu98-I2C-diabody | 100 | 13.47 |

Table 29-2. The cytotoxic activity of antibodies against SHP77

| Experiment 2 | Imax (%) | IC50 (pM) |
|---|---|---|
| AMG-757 | 74 | 74.66 |
| hu6-I2C-diabody | 75 | 33.14 |
| hu6(D56E)-I2C-diabody | 78 | 20.25 |
| hu6(D56S)-I2C-diabody | 78 | 24.49 |

Table 29-3. The cytotoxic activity of antibodies against SHP77

| Experiment 3 | Imax (%) | IC50 (pM) |
|---|---|---|
| hu6(D56E)-S 107E-diabody | 96 | 83.04 |
| hu6(L8H14)-S107E-diabody | 93 | 3596.22 |
| hu6(L8H15)-S107E-diabody | 97 | 294.78 |
| hu6(L9H14)-S107E-diabody | 98 | 1576.15 |
| hu6(L9H15)-S107E-diabody | 96 | 168.09 |
| hu6(L10H14)-S107E-diabody | 95 | 307.53 |
| hu6(L10H15)-S107E-diabody | 95 | 85.57 |

Table 29-4. The cytotoxic activity of antibodies against SHP77

| Experiment 4 | Imax (%) | IC50 (pM) |
|---|---|---|
| hu6(D56E)-I2C-diabody | 99 | 4.17 |
| hu110-I2C-Y body | 100 | 33.82 |
| hu110-I2C-hot Ig | 96 | 22.18 |
| BI-764532 | 97 | 44.63 |

Table 30. The killing of H1184 cells by bispecific antibodies

| Antibody | Imax (%) | IC50 (pM) |
|---|---|---|
| hu6(D56E)-S107E-diabody | 97 | 19.68 |
| hu110-I2C-Y body | 98 | 6.42 |
| hu110-S107E-hot Ig | 92 | 29.54 |

The results show that the bispecific antibodies of the present disclosure have relatively strong killing effects on the DLL3-expressing cells SHP77 and H1184 and have no killing effects on H460 cells not expressing DLL3 (FIG. 3).

**Test Example 6. *In Vitro* T cell Activation Activity of Bispecific Antibodies of Present Disclosure**

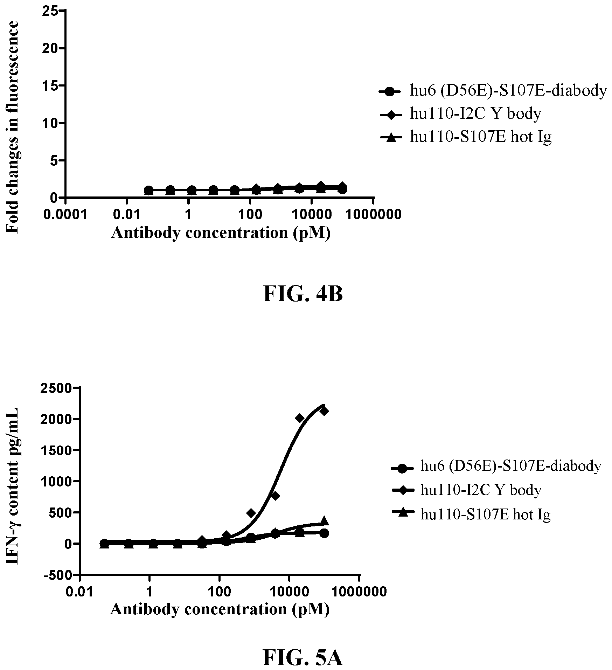

H82/DLL3 cells were cultured in 1640 + 10% FBS complete culture medium and passaged 2-3 times a week with a passage ratio of about 1:3. H460 cells were cultured in 1640 + 10% FBS complete culture medium and passaged 2-3 times a week with a passage ratio of about 1:10. Jurkat Lucia™ NFAT cells (InvivoGen) were cultured in 1640 + 10% FBS + 100 μg/mL zeocin (InvivoGen, ant-zn-1) complete culture medium and passaged 2-3 times a week with a passage ratio of about 1:10.Jurkat Lucia™ NFAT cells were harvested, centrifuged at 1000 rpm for 3 min, resuspended, and counted, and

128

the cell count was adjusted to 1E6 cells/mL. Target cells H82/DLL3 cells were harvested, centrifuged at 1000 rpm for 3 min, resuspended, and counted, and the cell count was adjusted to 2E5 cells/mL. H82/DLL3 and H460 cells were harvested, centrifuged at 1000 rpm for 3 min, resuspended, and counted, and the cell count was adjusted to 4E5 cells/mL. The Jurkat Lucia™ NFAT and target cells described above were mixed in equal volumes, and the mixture was added at 100 μL/well to ensure that the E:T Ratio for H82/DLL3 cells was 5:1 and the E:T Ratio for H82/DLL3 and H460 cells was 2.5:1. Antibodies were 5-fold diluted with 1640 + 10% FBS culture medium with an initial concentration of 600 nM ($6\times$ the final concentration), and 9 dose points were obtained and added at 20 μL/well. The treated cells were cultured in a 37 °C, 5% $CO_2$ incubator for 5 h. QUANTI-Luc™ Gold reagent (Invivogen, rep-qlcg5) was dissolved in 25 mL of $ddH_2O$. 50 μL of QUANTI-Luc™ Gold was added. After 5 min of incubation at room temperature, luminescence was measured using Vendor, and the activating effects of the antibodies at the different concentrations on Jurkat Lucia™ NFAT cells were calculated. Plots were created using Graphpad Prism8.0 software, and the results are shown in FIGs. 4A to 4B and Table 31.

Formula for fold changes in activation N = fluorescence reading of test antibody/fluorescence reading of negative well

Table 31. Results for the activation of T cells by bispecific antibodies

| Antibody | Fold changes in activation | EC50 (pM) |
|---|---|---|
| hu6(D56E)-S107E-diabody | 22.20 | 15.21 |
| hu110-I2C Y body | 20.18 | 6.36 |
| hu110-S107E hot Ig | 17.98 | 17.69 |

The results show that the bispecific antibodies of the present disclosure activated T cells only in the presence of DLL3-expressing DLL3/H82 cells and did not activate T cells in the presence of H460 negative cells not expressing DLL3.

**Test Example 7. Cytokine Release Levels for Bispecific Antibodies of Present Disclosure**

**1. Release levels of cytokine IFNγ for bispecific antibodies of present disclosure**

The secretion of cytokine IFNγ in the stimulation of PBMCs with DLL3/CD3 bispecific antibodies in the presence of H1184 cells was evaluated by HTRF.

The supernatants collected in the cell killing experiments (Test Example 5), cryopreserved in a freezer at -20 °C, were taken out, thawed at room temperature, and shaken for good uniformity. Powdered IFNγ standard was dissolved in sterile water, and the solution of the standard was diluted with 1640 + 10% FBS culture medium to the desired concentration. A kit for detection of IFNγ (CISBIO, 62HIFNGPEG) was taken out and equilibrated to room temperature, and two detection antibodies in the kit were 20-fold diluted with a detection buffer. 16 μL of a cell supernatant (10-fold diluted for IFNγ detection) and the IFNγ standard were added to a 96-well plate, and 4 μL of the dilution of a corresponding detection antibody was added. The plate was shaken for good uniformity, centrifuged at 1000 rpm for 1 min, and incubated overnight at room temperature. The samples incubated overnight were taken out and centrifuged at 1000 rpm for 1 min, and the absorbance at 665 nm and 620 nm was measured using a PHERAstar multi-mode microplate reader. Data were analyzed using Graphpad Prism 8, and the results are shown in FIG. 5A.

The results show that the bispecific antibodies of the present disclosure caused very low levels of IFNγ release.

**2. Release levels of cytokine IL-6 for bispecific antibodies of present disclosure**

The secretion of cytokine IL-6 in the stimulation of PBMCs with DLL3/CD3 bispecific antibodies in the presence of H1184 cells was evaluated by ELISA.

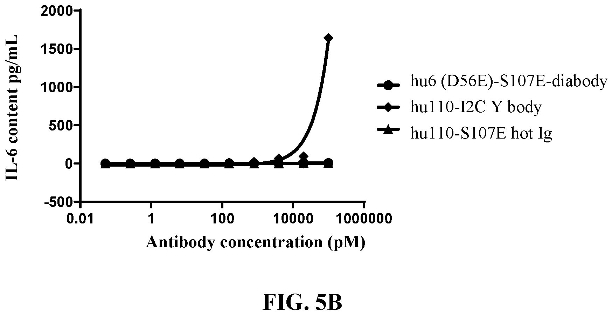

The supernatants collected in the cell killing experiments (Test Example 5), cryopreserved in a freezer at -20 °C, were taken out, thawed at room temperature, and shaken for good uniformity. The samples were 5-fold diluted with a sample diluent for later use. Before cytokine detection, an IL-6 ELISA kit (NeoBioscience Technology Co., Ltd., EHC007.96) was taken out and equilibrated to normal temperature. IL-6 standard was diluted and detection was performed according to the instructions. OD450 values were measured, and data were analyzed using Graphpad Prism 8. The results are shown in FIG. 5B.

The results show that the bispecific antibodies of the present disclosure caused very low levels of IL6 release, indicating that the bispecific antibodies of the present disclosure have better safety.

## Evaluation of *In Vivo* Activity

**Test Example 8. Efficacy of Bispecific Antibodies of Present Disclosure in SHP77 Subcutaneous Xenograft Tumor Model**

In the present disclosure, severe combined immunodeficiency NOG mice were inoculated with SHP-77 cells, then intraperitoneally injected with human PBMCs, and, after tumorigenesis, dosed in groups.

Female NOG mice weighing about 15-17 g were purchased from Vital River. SHP-77 cells were from ATCC. Human PBMCs were purchased from Milestone (Shanghai) Biotechnologies Co. Ltd., Cat. No: ID#A10S115034.

100 $\mu$L of SHP-77 cells ($4 \times 10^6$ cells/mouse) were inoculated subcutaneously into the right flank of NOG mice. On the day of inoculation, cryopreserved hPBMCs were thawed. On day 2 post-inoculation, each mouse was intraperitoneally injected with 100 $\mu$L of a cell suspension of hPBMCs ($5 \times 10^6$ cells). Eight days after the inoculation, when the tumor volume was ~120 mm$^3$, mice that were overweight or had tumors that were either too big or too small were excluded. Mice were randomized into 5 groups of 8 according to tumor volume, and administration of an equimolar amount of antibody was started on the day of grouping. The doses are shown in Table 32. The corresponding equimolar amount of antibody was administered by intraperitoneal injection 3 times in total, once every 5 days, until day 15. The tumor volume and body weight were measured twice a week, and data were recorded.

Tumor volume (V) was calculated using the formula:

$$V = 1/2 \times L_{long} \times L_{short}^2$$

Relative tumor proliferation rate T/C (%) = (T - T$_0$)/(C - C$_0$) $\times$ 100%, where T and C represent the tumor volumes of the treatment group and the control group at the end of the experiment; T$_0$ and C$_0$ represent the tumor volumes at the beginning of the experiment.

$$\text{Tumor growth inhibition rate (TGI) (\%)} = 100 - \text{T/C (\%)}.$$

Table 32. The efficacy of antibodies in the SHP77 subcutaneous xenograft tumor model (day 15 post-dose)

| Group | Dose (mg/kg) | Tumor growth inhibition rate TGI (%) |
|---|---|---|
| Vehicle | / | / |
| AMG-757 | 1 | 87 |
| hu6(D56E)-S107E-diabody | 0.96 | 107 |
| hu110-S107E-hot Ig | 1.38 | 94 |
| hu110-I2C-Y body | 1.19 | 106 |

The results show that the bispecific antibodies of the present disclosure can significantly inhibit the growth of SHP-77 xenograft tumors.

**Test Example 9. Efficacy of Bispecific Antibodies of Present Disclosure in H1184 Subcutaneous Xenograft Tumor Model**

In the present disclosure, severe combined immunodeficiency NOG mice were inoculated with H1184 cells, then intraperitoneally injected with human PBMCs, and, after tumorigenesis, dosed in groups.

Female NOG mice weighing about 15-17 g were purchased from Vital River. H1184 cells were from ATCC. Human PBMCs were purchased from Milestone (Shanghai) Biotechnologies Co. Ltd., Cat. No: ID#A10S115034.

200 $\mu$L of H1184 cells ($3.5 \times 10^6$ cells, containing 50% MatriGel) were inoculated subcutaneously into the right flank of each NOG mouse. On day 11 post-inoculation, 8 vials of cryopreserved hPBMCs (ID#A10S115034) were thawed. On day 12 post-inoculation, each mouse was intraperitoneally injected with 100 $\mu$L of a cell suspension of human PBMCs ($5 \times 10^6$ cells). Eighteen days after the inoculation, when the tumor volume was ~140 mm$^3$, mice that were overweight or had tumors that were either too big or too small were excluded. Mice were randomized into groups of 8 according to tumor volume, and administration of an equimolar amount of antibody was started on the day of grouping. The doses are shown in Table 33. The antibody was administered by intraperitoneal injection 4 times in total, twice a week. The tumor volume and body weight were measured twice a week, and data were recorded.

Data were recorded using Excel statistical software: the average values were calculated as avg; the SD values were calculated as STDEV; the SEM values were calculated as STDEV/SQRT (number of animals per group); GraphPad Prism software was used for plotting, and statistical analysis of the data was performed using Two-way ANOVA, One-way ANOVA, or t-test.

Tumor volume (V) was calculated using the formula:

$$V = 1/2 \times L_{long} \times L_{short}^2$$

Relative tumor proliferation rate T/C (%) = (T - $T_0$)/(C - $C_0$) × 100%, where T and C represent the tumor volumes of the treatment group and the control group at the end of the experiment; $T_0$ and $C_0$ represent the tumor volumes at the beginning of the experiment.

$$\text{Tumor growth inhibition rate (TGI) (\%)} = 100 - T/C\ (\%).$$

Table 33. The efficacy of antibodies in the H1184 subcutaneous xenograft tumor model (day 15 post-dose)

| Group | Dose (mg/kg) | Tumor growth inhibition rate TGI (%) |
|---|---|---|
| Vehicle | / | / |
| AMG-757 | 1 | 13 |
| hu6(D56E)-S 107E-diabody | 0.96 | 55 |
| hu110-I2C-Y body | 1.19 | 52 |

The results show that the bispecific antibodies of the present disclosure can significantly inhibit the growth of H1184 xenograft tumors.

Although the foregoing invention has been described in detail by way of drawings and examples for purposes of clarity of understanding, the description and examples should not be construed as limiting the scope of the present disclosure. The disclosures of all the patents and scientific literature cited herein are clearly incorporated by reference in their entirety.

**Claims**

1. An antigen-binding molecule binding specifically to DLL3 and CD3, comprising at least one antigen-binding moiety binding specifically to DLL3 and at least one antigen-binding moiety binding specifically to CD3, wherein:

   the antigen-binding moiety binding specifically to DLL3 comprises a heavy chain variable region DLL3-VH and a light chain variable region DLL3-VL, and
   the antigen-binding moiety binding specifically to CD3 comprises a heavy chain variable region CD3-VH and a light chain variable region CD3-VL, wherein:

   i) the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, 24, 72, or 73, and a DLL3-LCDR3 comprising the amino acid sequence of SEQ ID NO: 25 or 74; or
   ii) the DLL3-VH comprises: a DLL3-HCDR1 comprising the amino acid sequence of SEQ ID NO: 26 or 84, a DLL3-HCDR2 comprising the amino acid sequence of SEQ ID NO: 85 or 27, and a DLL3-HCDR3 comprising the amino acid sequence of SEQ ID NO: 83 or 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or
   iii) the DLL3-VH comprises: a DLL3-HCDR1 comprising the amino acid sequence of SEQ ID NO: 20, a DLL3-HCDR2 comprising the amino acid sequence of SEQ ID NO: 96, 32, 97, 98, or 99, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

iv) the DLL3-VH comprises: a DLL3-HCDR1 comprising the amino acid sequence of SEQ ID NO: 20, a DLL3-HCDR2 comprising the amino acid sequence of SEQ ID NO: 119, 37, 117, 118, or 120, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40;

preferably,

i) the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

ii) the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or
the DLL3-VH comprises: a DLL3-HCDR1 comprising the amino acid sequence of SEQ ID NO: 84, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or
the DLL3-VH comprises: a DLL3-HCDR1 comprising the amino acid sequence of SEQ ID NO: 84, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or
the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

iii) the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or
iv) the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40;

more preferably,
the antigen-binding molecule binds to human DLL3 at 25 °C with a KD of less than $4 \times 10^{-9}$ M, as measured by surface plasmon resonance.

2. The antigen-binding molecule binding specifically to DLL3 and CD3 according to claim 1, wherein:

i) the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 131; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134; or
ii) the CD3-VH comprises: a CD3-HCDR1 comprising the amino acid sequence of SEQ ID NO: 129, a CD3-HCDR2 comprising the amino acid sequence of SEQ ID NO: 130, and a CD3-HCDR3 comprising the amino acid sequence of SEQ ID NO: 135; and the CD3-VL comprises: a CD3-LCDR1 comprising the amino acid sequence of SEQ ID NO: 132, a CD3-LCDR2 comprising the amino acid sequence of SEQ ID NO: 133, and a CD3-LCDR3 comprising the amino acid sequence of SEQ ID NO: 134.

3. The antigen-binding molecule binding specifically to DLL3 and CD3 according to claim 1 or 2, wherein:

i) the DLL3-VH comprises the amino acid sequence of SEQ ID NO: 54, 12, 43, 44, 45, 46, 47, 48, 49, 50, 51, 52, 53, 55, 56, or 57, and the DLL3-VL comprises the amino acid sequence of SEQ ID NO: 63, 13, 58, 59, 60, 61, 62, 64, 65, 66, 67, or 68; or

ii) the DLL3-VH comprises the amino acid sequence of SEQ ID NO: 81, 14, 78, 79, 80, or 82, and the DLL3-VL comprises the amino acid sequence of SEQ ID NO: 77, 15, 75, or 76; or

iii) the DLL3-VH comprises the amino acid sequence of SEQ ID NO: 91, 16, 89, 90, 92, 93, 94, or 95, and the DLL3-VL comprises the amino acid sequence of SEQ ID NO: 87, 17, 86, or 88; or

iv) the DLL3-VH comprises the amino acid sequence of SEQ ID NO: 109, 18, 105, 106, 107, 108, 110, 111, 112, 113, 114, 115, or 116, and the DLL3-VL comprises the amino acid sequence of SEQ ID NO: 100, 19, 101, 102, 103, or 104;

preferably,

i) the DLL3-VH comprises the amino acid sequence of SEQ ID NO: 54, and the DLL3-VL comprises the amino acid sequence of SEQ ID NO: 63; or

the DLL3-VH comprises the amino acid sequence of SEQ ID NO: 54, and the DLL3-VL comprises the amino acid sequence of SEQ ID NO: 61; or
the DLL3-VH comprises the amino acid sequence of SEQ ID NO: 54, and the DLL3-VL comprises the amino acid sequence of SEQ ID NO: 64; or
the DLL3-VH comprises the amino acid sequence of SEQ ID NO: 56, and the DLL3-VL comprises the amino acid sequence of SEQ ID NO: 65; or
the DLL3-VH comprises the amino acid sequence of SEQ ID NO: 56, and the DLL3-VL comprises the amino acid sequence of SEQ ID NO: 66; or
the DLL3-VH comprises the amino acid sequence of SEQ ID NO: 56, and the DLL3-VL comprises the amino acid sequence of SEQ ID NO: 67; or
the DLL3-VH comprises the amino acid sequence of SEQ ID NO: 57, and the DLL3-VL comprises the amino acid sequence of SEQ ID NO: 65; or
the DLL3-VH comprises the amino acid sequence of SEQ ID NO: 57, and the DLL3-VL comprises the amino acid sequence of SEQ ID NO: 66; or
the DLL3-VH comprises the amino acid sequence of SEQ ID NO: 57, and the DLL3-VL comprises the amino acid sequence of SEQ ID NO: 67; or

ii) the DLL3-VH comprises the amino acid sequence of SEQ ID NO: 81, and the DLL3-VL comprises the amino acid sequence of SEQ ID NO: 77; or

iii) the DLL3-VH comprises the amino acid sequence of SEQ ID NO: 91, and the DLL3-VL comprises the amino acid sequence of SEQ ID NO: 87; or

iv) the DLL3-VH comprises the amino acid sequence of SEQ ID NO: 109, and the DLL3-VL comprises the amino acid sequence of SEQ ID NO: 100.

4. The antigen-binding molecule binding specifically to DLL3 and CD3 according to any one of claims 1 to 3, wherein:

i) the CD3-VH comprises the amino acid sequence of SEQ ID NO: 136, and the CD3-VL comprises the amino acid sequence of SEQ ID NO: 137; or

ii) the CD3-VH comprises the amino acid sequence of SEQ ID NO: 138, and the CD3-VL comprises the amino acid sequence of SEQ ID NO: 137.

5. The antigen-binding molecule binding specifically to DLL3 and CD3 according to any one of claims 1 to 4, wherein:

the antigen-binding moiety binding specifically to DLL3 comprises a Titin chain and an Obscurin chain capable of forming a dimer; or

the antigen-binding moiety binding specifically to CD3 comprises a Titin chain and an Obscurin chain capable of forming a dimer;

preferably,

the Titin chain comprises an amino acid sequence selected from the group consisting of SEQ ID NO: 192 to SEQ ID NO: 210, and

the Obscurin chain comprises an amino acid sequence selected from the group consisting of SEQ ID NO: 211 to SEQ ID NO: 251;

more preferably,

the Titin chain comprises the amino acid sequence of SEQ ID NO: 208, and the Obscurin chain comprises the amino acid sequence of SEQ ID NO: 246.

6.  The antigen-binding molecule binding specifically to DLL3 and CD3 according to any one of claims 1 to 5, comprising an Fc region, wherein preferably, the Fc region is a human IgG Fc region; further preferably, the Fc region is a human IgG1 Fc region;

    more preferably, the Fc region comprises one or more amino acid substitutions capable of reducing binding of the Fc region to an Fcγ receptor;
    most preferably, the Fc region is a human IgG1 Fc region and has amino acids A at positions 234 and 235, with numbering in accordance with the EU index.

7.  The antigen-binding molecule binding specifically to DLL3 and CD3 according to any one of claims 1 to 6, comprising an Fc region comprising a first subunit Fc1 and a second subunit Fc2 capable of associating with each other, wherein the Fc1 and Fc2 each independently have one or more amino acid substitutions reducing homodimerization of the Fc region;

    preferably, the Fc1 has a knob structure according to the knob-into-hole technique, and the Fc2 has a hole structure according to the knob-into-hole technique;
    more preferably, the Fc1 has an amino acid W at position 366, and the Fc2 has an amino acid S at position 366, an amino acid A at position 368, and an amino acid V at position 407, with numbering in accordance with the EU index;
    most preferably, the Fc1 comprises the amino acid sequence of SEQ ID NO: 143, and the Fc2 comprises the amino acid sequence of SEQ ID NO: 144; or the Fc1 comprises the amino acid sequence of SEQ ID NO: 145, and the Fc2 comprises the amino acid sequence of SEQ ID NO: 146.

8.  The antigen-binding molecule binding specifically to DLL3 and CD3 according to claim 7, wherein the antigen-binding molecule comprises one antigen-binding moiety binding specifically to DLL3 and one antigen-binding moiety binding specifically to CD3; the antigen-binding moiety binding specifically to DLL3 is a replaced Fab comprising a Titin chain and an Obscurin chain capable of forming a dimer, and the antigen-binding moiety binding specifically to CD3 is a Fab; or

    the antigen-binding moiety binding specifically to DLL3 is a Fab, and the antigen-binding moiety binding specifically to CD3 is a replaced Fab comprising a Titin chain and an Obscurin chain capable of forming a dimer; preferably,
    the antigen-binding molecule binding specifically to DLL3 and CD3 comprises one first chain having a structure represented by formula (a), one second chain having a structure represented by formula (b), one third chain having a structure represented by formula (c), and one fourth chain having a structure represented by formula (d):

    formula (a)       [DLL3-VH]-[linker 1]-[Titin chain]-[Fc2],

    formula (b)       [DLL3-VL]-[linker 2]-[Obscurin chain],

    formula (c)       [CD3-VH]-[CH1]-[Fc1],

    and

    formula (d)       [CD3-VL]-[CL],

    wherein the linker 1 and linker 2 are identical or different peptide linkers; or the linker 1 and/or linker 2 are/is absent;
    the structures represented by formulas (a), (b), (c), and (d) are arranged from N-terminus to C-terminus;
    the Titin chain and the Obscurin chain are interchangeable;
    the Fc1 and the Fc2 are interchangeable;
    more preferably, the antigen-binding molecule binding specifically to DLL3 and CD3 comprises:

    one first chain comprising the amino acid sequence of SEQ ID NO: 171, one second chain comprising the amino acid sequence of SEQ ID NO: 172, one third chain comprising the amino acid sequence of SEQ ID NO: 154, and one fourth chain comprising the amino acid sequence of SEQ ID NO: 155; or
    one first chain comprising the amino acid sequence of SEQ ID NO: 171, one second chain comprising the amino acid sequence of SEQ ID NO: 172, one third chain comprising the amino acid sequence of SEQ ID NO: 156, and one fourth chain comprising the amino acid sequence of SEQ ID NO: 155; or

one first chain comprising the amino acid sequence of SEQ ID NO: 152, one second chain comprising the amino acid sequence of SEQ ID NO: 153, one third chain comprising the amino acid sequence of SEQ ID NO: 154, and one fourth chain comprising the amino acid sequence of SEQ ID NO: 155; or

one first chain comprising the amino acid sequence of SEQ ID NO: 152, one second chain comprising the amino acid sequence of SEQ ID NO: 153, one third chain comprising the amino acid sequence of SEQ ID NO: 156, and one fourth chain comprising the amino acid sequence of SEQ ID NO: 155; or

one first chain comprising the amino acid sequence of SEQ ID NO: 164, one second chain comprising the amino acid sequence of SEQ ID NO: 165, one third chain comprising the amino acid sequence of SEQ ID NO: 154, and one fourth chain comprising the amino acid sequence of SEQ ID NO: 155; or

one first chain comprising the amino acid sequence of SEQ ID NO: 164, one second chain comprising the amino acid sequence of SEQ ID NO: 165, one third chain comprising the amino acid sequence of SEQ ID NO: 156, and one fourth chain comprising the amino acid sequence of SEQ ID NO: 155; or

one first chain comprising the amino acid sequence of SEQ ID NO: 178, one second chain comprising the amino acid sequence of SEQ ID NO: 179, one third chain comprising the amino acid sequence of SEQ ID NO: 154, and one fourth chain comprising the amino acid sequence of SEQ ID NO: 155; or

one first chain comprising the amino acid sequence of SEQ ID NO: 178, one second chain comprising the amino acid sequence of SEQ ID NO: 179, one third chain comprising the amino acid sequence of SEQ ID NO: 156, and one fourth chain comprising the amino acid sequence of SEQ ID NO: 155.

9. The antigen-binding molecule binding specifically to DLL3 and CD3 according to claim 7, wherein the antigen-binding molecule comprises one antigen-binding moiety binding specifically to DLL3 and one antigen-binding moiety binding specifically to CD3; the antigen-binding moiety binding specifically to DLL3 is a Fab, and the antigen-binding moiety binding specifically to CD3 is an scFv;

preferably,
the antigen-binding molecule binding specifically to DLL3 and CD3 comprises one first chain having a structure represented by formula (e), one second chain having a structure represented by formula (f), and one third chain having a structure represented by formula (g):

formula (e)  [DLL3-VL]-[CL],

formula (f)  [DLL3-VH]-[CH1]-[Fc2],

and

formula (g)  [CD3-VH]-[linker 3]-[CD3-VL]-[linker 4]-[Fc1],

wherein the linker 3 and linker 4 are identical or different peptide linkers;
the structures represented by formulas (e), (f), and (g) are arranged from N-terminus to C-terminus;
the Fc1 and the Fc2 are interchangeable;
more preferably, the antigen-binding molecule binding specifically to DLL3 and CD3 comprises:

one first chain comprising the amino acid sequence of SEQ ID NO: 173, one second chain comprising the amino acid sequence of SEQ ID NO: 174, and one third chain comprising the amino acid sequence of SEQ ID NO: 160; or

one first chain comprising the amino acid sequence of SEQ ID NO: 173, one second chain comprising the amino acid sequence of SEQ ID NO: 174, and one third chain comprising the amino acid sequence of SEQ ID NO: 159; or

one first chain comprising the amino acid sequence of SEQ ID NO: 157, one second chain comprising the amino acid sequence of SEQ ID NO: 158, and one third chain comprising the amino acid sequence of SEQ ID NO: 159; or

one first chain comprising the amino acid sequence of SEQ ID NO: 157, one second chain comprising the amino acid sequence of SEQ ID NO: 158, and one third chain comprising the amino acid sequence of SEQ ID NO: 160; or

one first chain comprising the amino acid sequence of SEQ ID NO: 166, one second chain comprising the amino acid sequence of SEQ ID NO: 167, and one third chain comprising the amino acid sequence of SEQ ID NO: 159; or

one first chain comprising the amino acid sequence of SEQ ID NO: 166, one second chain comprising the amino acid sequence of SEQ ID NO: 167, and one third chain comprising the amino acid sequence of SEQ ID NO: 160; or

one first chain comprising the amino acid sequence of SEQ ID NO: 180, one second chain comprising the amino acid sequence of SEQ ID NO: 181, and one third chain comprising the amino acid sequence of SEQ ID NO: 159; or

one first chain comprising the amino acid sequence of SEQ ID NO: 180, one second chain comprising the amino acid sequence of SEQ ID NO: 181, and one third chain comprising the amino acid sequence of SEQ ID NO: 160.

10. The antigen-binding molecule binding specifically to DLL3 and CD3 according to claim 7, wherein the antigen-binding molecule comprises one antigen-binding moiety binding specifically to DLL3 and one antigen-binding moiety binding specifically to CD3;

the antigen-binding molecule binding specifically to DLL3 and CD3 comprises one first chain having a structure represented by formula (h) and one second chain having a structure represented by formula (i):

formula (h)          [DLL3-VL]-[linker 5]-[CD3-VH]-[linker 6]-[Fc1],

and

formula (i)          [CD3-VL]-[linker 7]-[DLL3-VH]-[linker 8]-[Fc2],

wherein the linker 5, linker 6, linker 7, and linker 8 are identical or different peptide linkers;
the structures represented by formulas (h) and (i) are arranged from N-terminus to C-terminus;
the Fc1 and the Fc2 are interchangeable;
preferably, wherein:

i) the DLL3-VH comprises the amino acid sequence of SEQ ID NO: 54, and the DLL3-VL comprises the amino acid sequence of SEQ ID NO: 63; and the CD3-VH comprises the amino acid sequence of SEQ ID NO: 136 or 138, and the CD3-VL comprises the amino acid sequence of SEQ ID NO: 137; or

the DLL3-VH comprises the amino acid sequence of SEQ ID NO: 54, and the DLL3-VL comprises the amino acid sequence of SEQ ID NO: 61; and the CD3-VH comprises the amino acid sequence of SEQ ID NO: 136 or 138, and the CD3-VL comprises the amino acid sequence of SEQ ID NO: 137; or
the DLL3-VH comprises the amino acid sequence of SEQ ID NO: 54, and the DLL3-VL comprises the amino acid sequence of SEQ ID NO: 64; and the CD3-VH comprises the amino acid sequence of SEQ ID NO: 136 or 138, and the CD3-VL comprises the amino acid sequence of SEQ ID NO: 137; or
the DLL3-VH comprises the amino acid sequence of SEQ ID NO: 56, and the DLL3-VL comprises the amino acid sequence of SEQ ID NO: 65; and the CD3-VH comprises the amino acid sequence of SEQ ID NO: 136 or 138, and the CD3-VL comprises the amino acid sequence of SEQ ID NO: 137; or
the DLL3-VH comprises the amino acid sequence of SEQ ID NO: 56, and the DLL3-VL comprises the amino acid sequence of SEQ ID NO: 66; and the CD3-VH comprises the amino acid sequence of SEQ ID NO: 136 or 138, and the CD3-VL comprises the amino acid sequence of SEQ ID NO: 137; or
the DLL3-VH comprises the amino acid sequence of SEQ ID NO: 56, and the DLL3-VL comprises the amino acid sequence of SEQ ID NO: 67; and the CD3-VH comprises the amino acid sequence of SEQ ID NO: 136 or 138, and the CD3-VL comprises the amino acid sequence of SEQ ID NO: 137; or
the DLL3-VH comprises the amino acid sequence of SEQ ID NO: 57, and the DLL3-VL comprises the amino acid sequence of SEQ ID NO: 65; and the CD3-VH comprises the amino acid sequence of SEQ ID NO: 136 or 138, and the CD3-VL comprises the amino acid sequence of SEQ ID NO: 137; or
the DLL3-VH comprises the amino acid sequence of SEQ ID NO: 57, and the DLL3-VL comprises the amino acid sequence of SEQ ID NO: 66; and the CD3-VH comprises the amino acid sequence of SEQ ID NO: 136 or 138, and the CD3-VL comprises the amino acid sequence of SEQ ID NO: 137; or
the DLL3-VH comprises the amino acid sequence of SEQ ID NO: 57, and the DLL3-VL comprises the amino acid sequence of SEQ ID NO: 67; and the CD3-VH comprises the amino acid sequence of SEQ ID NO: 136 or 138, and the CD3-VL comprises the amino acid sequence of SEQ ID NO: 137; or

ii) the DLL3-VH comprises the amino acid sequence of SEQ ID NO: 91, and the DLL3-VL comprises the amino acid sequence of SEQ ID NO: 87; and the CD3-VH comprises the amino acid sequence of SEQ ID NO: 136 or 138, and the CD3-VL comprises the amino acid sequence of SEQ ID NO: 137; or
iii) the DLL3-VH comprises the amino acid sequence of SEQ ID NO: 81, and the DLL3-VL comprises the amino acid sequence of SEQ ID NO: 77; and the CD3-VH comprises the amino acid sequence of SEQ ID NO: 136 or 138, and the CD3-VL comprises the amino acid sequence of SEQ ID NO: 137; or
iv) the DLL3-VH comprises the amino acid sequence of SEQ ID NO: 109, and the DLL3-VL comprises the amino acid sequence of SEQ ID NO: 100; and the CD3-VH comprises the amino acid sequence of SEQ ID NO: 136 or 138, and the CD3-VL comprises the amino acid sequence of SEQ ID NO: 137;

more preferably, the antigen-binding molecule binding specifically to DLL3 and CD3 comprises:

one first chain comprising the amino acid sequence of SEQ ID NO: 185 and one second chain comprising the amino acid sequence of SEQ ID NO: 162; or
one first chain comprising the amino acid sequence of SEQ ID NO: 161 and one second chain comprising the amino acid sequence of SEQ ID NO: 162; or
one first chain comprising the amino acid sequence of SEQ ID NO: 163 and one second chain comprising the amino acid sequence of SEQ ID NO: 162; or
one first chain comprising the amino acid sequence of SEQ ID NO: 259 and one second chain comprising the amino acid sequence of SEQ ID NO: 162; or
one first chain comprising the amino acid sequence of SEQ ID NO: 260 and one second chain comprising the amino acid sequence of SEQ ID NO: 162; or
one first chain comprising the amino acid sequence of SEQ ID NO: 168 and one second chain comprising the amino acid sequence of SEQ ID NO: 169; or
one first chain comprising the amino acid sequence of SEQ ID NO: 170 and one second chain comprising the amino acid sequence of SEQ ID NO: 169; or
one first chain comprising the amino acid sequence of SEQ ID NO: 175 and one second chain comprising the amino acid sequence of SEQ ID NO: 176; or
one first chain comprising the amino acid sequence of SEQ ID NO: 177 and one second chain comprising the amino acid sequence of SEQ ID NO: 176; or
one first chain comprising the amino acid sequence of SEQ ID NO: 182 and one second chain comprising the amino acid sequence of SEQ ID NO: 183; or
one first chain comprising the amino acid sequence of SEQ ID NO: 184 and one second chain comprising the amino acid sequence of SEQ ID NO: 183; or
one first chain comprising the amino acid sequence of SEQ ID NO: 186 and one second chain comprising the amino acid sequence of SEQ ID NO: 162; or
one first chain comprising the amino acid sequence of SEQ ID NO: 187 and one second chain comprising the amino acid sequence of SEQ ID NO: 188; or
one first chain comprising the amino acid sequence of SEQ ID NO: 187 and one second chain comprising the amino acid sequence of SEQ ID NO: 189; or
one first chain comprising the amino acid sequence of SEQ ID NO: 190 and one second chain comprising the amino acid sequence of SEQ ID NO: 188; or
one first chain comprising the amino acid sequence of SEQ ID NO: 190 and one second chain comprising the amino acid sequence of SEQ ID NO: 189; or
one first chain comprising the amino acid sequence of SEQ ID NO: 191 and one second chain comprising the amino acid sequence of SEQ ID NO: 188; or
one first chain comprising the amino acid sequence of SEQ ID NO: 191 and one second chain comprising the amino acid sequence of SEQ ID NO: 189.

11. An anti-DLL3 antibody capable of binding specifically to DLL3, comprising a heavy chain variable region DLL3-VH and a light chain variable region DLL3-VL, wherein:

i) the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 69 or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 24, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 21, 69, or 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

ii) the DLL3-VH comprises: a DLL3-HCDR1 comprising the amino acid sequence of SEQ ID NO: 26 or 84, a DLL3-HCDR2 comprising the amino acid sequence of SEQ ID NO: 85 or 27, and a DLL3-HCDR3 comprising the amino acid sequence of SEQ ID NO: 83 or 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

iii) the DLL3-VH comprises: a DLL3-HCDR1 comprising the amino acid sequence of SEQ ID NO: 20, a DLL3-HCDR2 comprising the amino acid sequence of SEQ ID NO: 96, 32, 97, 98, or 99, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

iv) the DLL3-VH comprises: a DLL3-HCDR1 comprising the amino acid sequence of SEQ ID NO: 20, a DLL3-HCDR2 comprising the amino acid sequence of SEQ ID NO: 119, 37, 117, 118, or 120, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40;

preferably,

i) the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 21, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 72, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 73, and a DLL3-LCDR3 comprising the amino acid sequence of SEQ ID NO: 74; or

the DLL3-VH comprises: a DLL3-HCDR1 comprising the amino acid sequence of SEQ ID NO: 20, a DLL3-HCDR2 comprising the amino acid sequence of SEQ ID NO: 69, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

the DLL3-VH comprises: a DLL3-HCDR1 comprising the amino acid sequence of SEQ ID NO: 20, a DLL3-HCDR2 comprising the amino acid sequence of SEQ ID NO: 70, and a DLL3-HCDR3 comprising the amino acid sequence of SEQ ID NO: 22; and the DLL3-VL comprises: a DLL3-LCDR1 comprising the amino acid sequence of SEQ ID NO: 23, a DLL3-LCDR2 comprising the amino acid sequence of SEQ ID NO: 71, and a DLL3-LCDR3 comprising the amino acid sequence of SEQ ID NO: 25; or

ii) the DLL3-VH comprises: a DLL3-HCDR1 comprising the amino acid sequence of SEQ ID NO: 26, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 84, a DLL3-HCDR2 comprising the amino acid sequence of SEQ ID NO: 27, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 84, a DLL3-HCDR2 comprising the amino acid sequence of SEQ ID NO: 85, and a DLL3-HCDR3 comprising the amino acid sequence of SEQ ID NO: 83; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

the DLL3-VH comprises: a DLL3-HCDR1 comprising the amino acid sequence of SEQ ID NO: 26, a DLL3-HCDR2 comprising the amino acid sequence of SEQ ID NO: 27, and a DLL3-HCDR3 comprising the amino acid sequence of SEQ ID NO: 28; and the DLL3-VL comprises: a DLL3-LCDR1 comprising the amino acid sequence of SEQ ID NO: 29, a DLL3-LCDR2 comprising the amino acid sequence of SEQ ID NO: 30, and a DLL3-LCDR3 comprising the amino acid sequence of SEQ ID NO: 31; or

iii) the DLL3-VH comprises: a DLL3-HCDR1 comprising the amino acid sequence of SEQ ID NO: 20, a DLL3-HCDR2 comprising the amino acid sequence of SEQ ID NO: 96, and a DLL3-HCDR3 comprising the amino acid sequence of SEQ ID NO: 33; and the DLL3-VL comprises: a DLL3-LCDR1 comprising the amino acid sequence of SEQ ID NO: 34, a DLL3-LCDR2 comprising the amino acid sequence of SEQ ID NO: 35, and a DLL3-LCDR3 comprising the amino acid sequence of SEQ ID NO: 36; or

iv) the DLL3-VH comprises: a DLL3-HCDR1 comprising the amino acid sequence of SEQ ID NO: 20, a DLL3-HCDR2 comprising the amino acid sequence of SEQ ID NO: 119, and a DLL3-HCDR3 comprising the amino acid sequence of SEQ ID NO: 38; and the DLL3-VL comprises: a DLL3-LCDR1 comprising the amino acid sequence of SEQ ID NO: 39, a DLL3-LCDR2 comprising the amino acid sequence of SEQ ID NO: 35, and a DLL3-LCDR3 comprising the amino acid sequence of SEQ ID NO: 40;

more preferably,

the anti-DLL3 antibody binds to human DLL3 at 25 °C with a KD of less than $4 \times 10^{-9}$ M, as measured by surface plasmon resonance.

12. The anti-DLL3 antibody according to claim 11, comprising a heavy chain variable region DLL3-VH and a light chain variable region DLL3-VL, wherein:

i) the DLL3-VH comprises the amino acid sequence of SEQ ID NO: 54, 43, 44, 45, 46, 47, 48, 49, 50, 51, 52, 53, 55, 56, or 57, and the DLL3-VL comprises the amino acid sequence of SEQ ID NO: 63, 64, 65, 66, or 67; or the DLL3-VH comprises the amino acid sequence of SEQ ID NO: 56 or 57, and the DLL3-VL comprises the amino acid sequence of SEQ ID NO: 58, 59, 60, 61, 62, 63, 64, 65, 66, 67, or 68; or

ii) the DLL3-VH comprises the amino acid sequence of SEQ ID NO: 81, 78, 79, 80, or 82, and the DLL3-VL comprises the amino acid sequence of SEQ ID NO: 77, 75, or 76; or

iii) the DLL3-VH comprises the amino acid sequence of SEQ ID NO: 91, 89, 90, 92, 93, 94, or 95, and the DLL3-VL comprises the amino acid sequence of SEQ ID NO: 87, 86, or 88; or

iv) the DLL3-VH comprises the amino acid sequence of SEQ ID NO: 109, 105, 106, 107, 108, 110, 111, 112, 113, 114, 115, or 116, and the DLL3-VL comprises the amino acid sequence of SEQ ID NO: 100, 101, 102, 103, or 104;

preferably,

i) the DLL3-VH comprises the amino acid sequence of SEQ ID NO: 54, and the DLL3-VL comprises the amino acid sequence of SEQ ID NO: 63; or

the DLL3-VH comprises the amino acid sequence of SEQ ID NO: 54, and the DLL3-VL comprises the amino acid sequence of SEQ ID NO: 61; or
the DLL3-VH comprises the amino acid sequence of SEQ ID NO: 54, and the DLL3-VL comprises the amino acid sequence of SEQ ID NO: 64; or
the DLL3-VH comprises the amino acid sequence of SEQ ID NO: 56, and the DLL3-VL comprises the amino acid sequence of SEQ ID NO: 65; or
the DLL3-VH comprises the amino acid sequence of SEQ ID NO: 56, and the DLL3-VL comprises the amino acid sequence of SEQ ID NO: 66; or
the DLL3-VH comprises the amino acid sequence of SEQ ID NO: 56, and the DLL3-VL comprises the amino acid sequence of SEQ ID NO: 67; or
the DLL3-VH comprises the amino acid sequence of SEQ ID NO: 57, and the DLL3-VL comprises the amino acid sequence of SEQ ID NO: 65; or
the DLL3-VH comprises the amino acid sequence of SEQ ID NO: 57, and the DLL3-VL comprises the amino acid sequence of SEQ ID NO: 66; or
the DLL3-VH comprises the amino acid sequence of SEQ ID NO: 57, and the DLL3-VL comprises the amino acid sequence of SEQ ID NO: 67; or

ii) the DLL3-VH comprises the amino acid sequence of SEQ ID NO: 81, and the DLL3-VL comprises the amino acid sequence of SEQ ID NO: 77; or
iii) the DLL3-VH comprises the amino acid sequence of SEQ ID NO: 91, and the DLL3-VL comprises the amino acid sequence of SEQ ID NO: 87; or
iv) the DLL3-VH comprises the amino acid sequence of SEQ ID NO: 109, and the DLL3-VL comprises the amino acid sequence of SEQ ID NO: 100.

13. The anti-DLL3 antibody according to claim 11 or 12, wherein the anti-DLL3 antibody is an antibody fragment; preferably, the antibody fragment is a Fab, Fab', F(ab')2, Fd, Fv, scFv, dsFv, or dAb.

14. The anti-DLL3 antibody according to any one of claims 11 to 13, comprising a heavy chain constant region and a light chain constant region, wherein preferably, the heavy chain constant region comprises the amino acid sequence of SEQ ID NO: 41, and/or the light chain constant region comprises the amino acid sequence of SEQ ID NO: 42.

15. The anti-DLL3 antibody according to claim 14, comprising a heavy chain and a light chain, wherein:

the heavy chain comprises an amino acid sequence having at least 85% sequence identity to SEQ ID NO: 121, and the light chain comprises an amino acid sequence having at least 85% sequence identity to SEQ ID NO: 122; or

the heavy chain comprises an amino acid sequence having at least 85% sequence identity to SEQ ID NO: 123, and the light chain comprises an amino acid sequence having at least 85% sequence identity to SEQ ID NO: 124; or

the heavy chain comprises an amino acid sequence having at least 85% sequence identity to SEQ ID NO: 125, and the light chain comprises an amino acid sequence having at least 85% sequence identity to SEQ ID NO: 126; or

the heavy chain comprises an amino acid sequence having at least 85% sequence identity to SEQ ID NO: 127, and the light chain comprises an amino acid sequence having at least 85% sequence identity to SEQ ID NO: 128; preferably,

the heavy chain comprises the amino acid sequence of SEQ ID NO: 121, and the light chain comprises the amino acid sequence of SEQ ID NO: 122; or

the heavy chain comprises the amino acid sequence of SEQ ID NO: 123, and the light chain comprises the amino acid sequence of SEQ ID NO: 124; or

the heavy chain comprises the amino acid sequence of SEQ ID NO: 125, and the light chain comprises the amino acid sequence of SEQ ID NO: 126; or

the heavy chain comprises the amino acid sequence of SEQ ID NO: 127, and the light chain comprises the amino acid sequence of SEQ ID NO: 128.

16. A pharmaceutical composition, comprising:
a therapeutically effective amount of the antigen-binding molecule binding specifically to DLL3 and CD3 according to any one of claims 1 to 10, or the anti-DLL3 antibody according to any one of claims 11 to 15, and one or more pharmaceutically acceptable carriers, diluents, buffers, or excipients.

17. An isolated nucleic acid, wherein the isolated nucleic acid encodes the antigen-binding molecule binding specifically to DLL3 and CD3 according to any one of claims 1 to 10, or the anti-DLL3 antibody according to any one of claims 11 to 15.

18. A host cell, comprising the isolated nucleic acid according to claim 17.

19. Use of the antigen-binding molecule binding specifically to DLL3 and CD3 according to any one of claims 1 to 10, or the anti-DLL3 antibody according to any one of claims 11 to 15, or the pharmaceutical composition according to claim 16 in the preparation of a medicament for treating a tumor or cancer, wherein preferably, the tumor or cancer is selected from the group consisting of:

head and neck squamous cell carcinoma, head and neck cancer, brain cancer, neuroglioma, glioblastoma multiforme, neuroblastoma, central nervous system cancer, neuroendocrine tumor, throat cancer, pharyngeal squamous cell carcinoma, oral squamous cell carcinoma, nasopharyngeal cancer, esophageal cancer, thyroid cancer, malignant pleural mesothelioma, lung cancer, breast cancer, liver cancer, hepatobiliary cancer, pancreatic cancer, gastric cancer, gastrointestinal cancer, intestinal cancer, colon cancer, colorectal cancer, renal cancer, clear cell renal cell carcinoma, ovarian cancer, endometrial cancer, cervical cancer, bladder cancer, prostate cancer, testicular cancer, skin cancer, melanoma, large cell lung cancer, triple-negative breast cancer, lymphoma, and small cell lung cancer;
more preferably, the tumor or cancer is a DLL3-expressing tumor or cancer.

FIG. 1A

FIG. 1B

FIG. 1C

FIG. 2A

FIG. 2B

FIG. 3

FIG. 4A

FIG. 4B

FIG. 5A

**FIG. 5B**

## INTERNATIONAL SEARCH REPORT

International application No.

**PCT/CN2023/101778**

### A. CLASSIFICATION OF SUBJECT MATTER

C07K 16/46(2006.01)i; C07K 16/30(2006.01)i; C07K 16/28(2006.01)i; C07K 19/00(2006.01)i; A61K 39/395(2006.01)i; A61P 35/00(2006.01)i; C12N 15/13(2006.01)i; C12N 5/10(2006.01)i

According to International Patent Classification (IPC) or to both national classification and IPC

### B. FIELDS SEARCHED

Minimum documentation searched (classification system followed by classification symbols)

IPC: C07K A61K A61P C12N

Documentation searched other than minimum documentation to the extent that such documents are included in the fields searched

Electronic data base consulted during the international search (name of data base and, where practicable, search terms used)

CNTXT; CNABS; ENTXTC; WPABSC; VEN; ENTXT; CJFD; CNKI; 万方, WANFANG; 读秀, DUXIU; ISI_Web of Science; NCBI; EMBL; STNext; 中国专利生物序列检索系统, China Patents Biological Sequence Database: 恒瑞, DLL3, CD3, 双特异性, 抗体, 序列检索, Hengrui, bispecific, antibody

### C. DOCUMENTS CONSIDERED TO BE RELEVANT

| Category* | Citation of document, with indication, where appropriate, of the relevant passages | Relevant to claim No. |
|---|---|---|
| A | CN 112513092 A (BOEHRINGER INGELHEIM INTERNATIONAL GMBH) 16 March 2021 (2021-03-16)<br>claims 1-41 | 1-19 |
| A | CN 108271376 A (AMGEN RESEARCH MUNICH GMBH) 10 July 2018 (2018-07-10)<br>claims 1-28 | 1-19 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **28 September 2023** | **07 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/101778**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

<div align="center">

**INTERNATIONAL SEARCH REPORT**
Information on patent family members

</div>

| International application No. |
| --- |
| **PCT/CN2023/101778** |

| Patent document cited in search report | | | Publication date (day/month/year) | Patent family member(s) | | | Publication date (day/month/year) |
| --- | --- | --- | --- | --- | --- | --- | --- |
| CN | 112513092 | A | 16 March 2021 | IL | 279235 | A | 31 January 2021 |
| | | | | KR | 20210018311 | A | 17 February 2021 |
| | | | | MX | 2020013348 | A | 09 March 2021 |
| | | | | CA | 3099956 | A1 | 12 December 2019 |
| | | | | JOP | 13 March 2020 | A1 | 06 December 2020 |
| | | | | ECSP | 20084864 | A | 31 May 2021 |
| | | | | PH | 12020552080 | A1 | 31 May 2021 |
| | | | | CR | 20200601 | A | 26 January 2021 |
| | | | | CL | 2020003105 | A1 | 11 June 2021 |
| | | | | MA | 52742 | A | 14 April 2021 |
| | | | | BR | 112020022898 | A2 | 23 February 2021 |
| | | | | JP | 2021526816 | A | 11 October 2021 |
| | | | | JP | 7133043 | B2 | 07 September 2022 |
| | | | | US | 2022251236 | A1 | 11 August 2022 |
| | | | | AU | 2019280900 | A1 | 19 November 2020 |
| | | | | US | 2019375849 | A1 | 12 December 2019 |
| | | | | US | 11332541 | B2 | 17 May 2022 |
| | | | | CO | 2020015172 | A2 | 21 December 2020 |
| | | | | PE | 20210321 | A1 | 16 February 2021 |
| | | | | EP | 3802598 | A1 | 14 April 2021 |
| | | | | JP | 2022180379 | A | 06 December 2022 |
| | | | | SG | 11202011134 | XA | 30 December 2020 |
| | | | | TW | 202016151 | A | 01 May 2020 |
| | | | | WO | 2019234220 | A1 | 12 December 2019 |
| CN | 108271376 | A | 10 July 2018 | IL | 296409 | A | 01 November 2022 |
| | | | | CL | 2020003046 | A1 | 09 April 2021 |
| | | | | EP | 3328889 | A1 | 06 June 2018 |
| | | | | EP | 3328889 | B1 | 23 June 2021 |
| | | | | ES | 2884211 | T3 | 10 December 2021 |
| | | | | JP | 2021000088 | A | 07 January 2021 |
| | | | | JP | 6827583 | B2 | 10 February 2021 |
| | | | | US | 2017037130 | A1 | 09 February 2017 |
| | | | | US | 10294300 | B2 | 21 May 2019 |
| | | | | CL | 2018000267 | A1 | 05 October 2018 |
| | | | | ZA | 202006983 | B | 29 March 2023 |
| | | | | AU | 2016302569 | A1 | 07 December 2017 |
| | | | | AU | 2016302569 | B2 | 25 August 2022 |
| | | | | HUE | 055112 | T2 | 28 October 2021 |
| | | | | US | 2020332002 | A1 | 22 October 2020 |
| | | | | US | 11591396 | B2 | 28 February 2023 |
| | | | | BR | 112018000475 | A2 | 18 September 2018 |
| | | | | JP | 2021061859 | A | 22 April 2021 |
| | | | | JP | 7166368 | B2 | 07 November 2022 |
| | | | | MA | 42530 | A | 24 March 2021 |
| | | | | MA | 42530 | B1 | 30 September 2021 |
| | | | | MX | 2021009793 | A | 08 September 2021 |
| | | | | LT | 3328889 | T | 10 August 2021 |
| | | | | TW | 201708261 | A | 01 March 2017 |
| | | | | TWI | 793062 | B | 21 February 2023 |
| | | | | KR | 20180033501 | A | 03 April 2018 |

Form PCT/ISA/210 (patent family annex) (July 2022)

International application No.

**PCT/CN2023/101778**

| Patent document cited in search report | Publication date (day/month/year) | Patent family member(s) | | Publication date (day/month/year) |
|---|---|---|---|---|
| | | KR | 102404077 B1 | 02 June 2022 |
| | | TN | 2017000549 A1 | 12 April 2019 |
| | | US | 2021309743 A1 | 07 October 2021 |
| | | NZ | 737339 A | 30 September 2022 |
| | | SG | 10201911548 QA | 30 January 2020 |
| | | EP | 3865514 A1 | 18 August 2021 |
| | | SI | 3328889 T1 | 30 September 2021 |
| | | MY | 184895 A | 30 April 2021 |
| | | HK | 1249525 A1 | 02 November 2018 |
| | | RS | 62248 B1 | 30 September 2021 |
| | | CR | 20180066 A | 29 May 2018 |
| | | WO | 2017021349 A1 | 09 February 2017 |
| | | KR | 20220075455 A | 08 June 2022 |
| | | AU | 2022268314 A1 | 15 December 2022 |
| | | JP | 2018527908 A | 27 September 2018 |
| | | JP | 6824245 B2 | 03 February 2021 |
| | | PT | 3328889 T | 25 August 2021 |
| | | CO | 2018000877 A2 | 19 April 2018 |
| | | MX | 2018001154 A | 23 May 2018 |
| | | HRP | 20211073 T1 | 01 October 2021 |
| | | US | 2019263907 A1 | 29 August 2019 |
| | | US | 10683351 B2 | 16 June 2020 |
| | | MD | 3328889 T2 | 31 October 2021 |
| | | JP | 2022191477 A | 27 December 2022 |
| | | IL | 256871 A | 29 March 2018 |
| | | IL | 256871 B | 01 October 2022 |
| | | IL | 256871 B2 | 01 February 2023 |
| | | PH | 12018500241 A1 | 13 August 2018 |
| | | CA | 2986848 A1 | 09 February 2017 |
| | | DK | 3328889 T3 | 23 August 2021 |
| | | PL | 3328889 T3 | 06 December 2021 |
| | | PE | 20181152 A1 | 17 July 2018 |
| | | JO | 3671 B1 | 27 August 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1996027011 A1
- WO 1998050431 A
- EP 1870459 A
- WO 2007110205 A
- WO 2009089004 A
- WO 2010129304 A
- WO 201190754 A
- WO 2011143545 A
- WO 2012058768 A
- WO 2013157954 A
- WO 2013096291 A
- US 5959177 A
- US 6040498 A
- US 6420548 B
- US 7125978 B
- US 6417429 B
- WO 2021139758 A
- CN 202110527339
- WO 2020177733 A1
- WO 2017021349 A
- WO 2019234220 A

### Non-patent literature cited in the description

- *J. Biol. Chem*, 1968, vol. 243, 3558
- **ARAN F. LABRIJN et al.** *Nature Reviews Drug Discovery*, 2019, vol. 18, 585-608
- **CHEN S1 et al.** *J Immunol Res.*, 11 February 2019, vol. 2019, 4516041
- **KABAT et al.** Sequences of Proteins of Immunological Interest. Public Health Service, National Institutes of Health, 1991
- **MARTIN**. ACR. Protein Sequence and Structure Analysis of Antibody Variable Domains[J. 2001
- **LEFRANC, M.P. et al.** *Dev. Comp. Immunol.*, 2003, vol. 27, 55-77
- *Front Immunol.*, 16 October 2018, vol. 9, 2278
- *Meth. Mol. Biol.*, 2004, vol. 248, 443-463
- *Prot. Sci.*, 2000, vol. 9, 487-496
- **HARLOW** ; **LANE**. Antibodies. Cold Spring Harbor Press
- **YAZAKI, P.** ; **WU, A.M.** Methods in Molecular Biology. Humana Press, 2004, vol. 248, 255-268
- Antibodies: A Laboratory Manual and Molecular Cloning: A Laboratory Manual. Cold Spring Harbor Laboratory